(19) Europäisches Patentamt
European Patent Office
Office européen des brevets

(11) EP 1 581 629 B1

(12) **EUROPEAN PATENT SPECIFICATION**

(45) Date of publication and mention of the grant of the patent:
**01.04.2015 Bulletin 2015/14**

(21) Application number: **03796633.0**

(22) Date of filing: **04.12.2003**

(51) Int Cl.:
***C12Q 1/68*** *(2006.01)*

(86) International application number:
**PCT/US2003/038539**

(87) International publication number:
**WO 2004/053066 (24.06.2004 Gazette 2004/26)**

(54) **METHODS FOR THE IDENTIFICATION, ASSESSMENT, AND TREATMENT OF PATIENTS WITH PROTEASOME INHIBITION THERAPY**

VERFAHREN ZUR IDENTIFIZIERUNG,BEURTEILUNG UND BEHANDLUNG VON PATIENTEN MIT EINER PROTEASOMEN INHIBITIONS THERAPIE

PROCEDES POUR IDENTIFIER, EVALUER ET TRAITER DES PATIENTS SUIVANT UNE THERAPIE D'INHIBITION DE PROTEASOME

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **06.12.2002 US 431514 P**

(43) Date of publication of application:
**05.10.2005 Bulletin 2005/40**

(73) Proprietor: **MILLENNIUM PHARMACEUTICALS, INC.**
**Cambridge, MA 02139 (US)**

(72) Inventors:
- **MULLIGAN, George**
  **Lexington, MA 02421 (US)**
- **BRYANT, Barbara, M.**
  **Cambridge, MA 02139 (US)**
- **MORRISSEY, Michael P.**
  **Brighton, MA 02135 (US)**
- **BOLT, Andrew**
  **Cambridge, MA 02138 (US)**
- **DAMOKOSH, Andrew, I.**
  **West Hartford, CT 06107 (US)**

(74) Representative: **Lock, Graham James et al**
**Fry Heath & Spence LLP**
**The Gables**
**Massetts Road**
**Horley**
**Surrey RH6 7DQ (GB)**

(56) References cited:

- **"Affymetrix GeneChip Human Genome U95 Set HG-U95A" GENBANK GEO, 11 March 2002 (2002-03-11), XP002293987**
- **AN W G ET AL: "PROTEASE INHIBITOR-INDUCED APOPTOSIS: ACCUMULATION OF WT P53, P21WAF1/CIP1, AND INDUCTION OF APOPTOSIS ARE INDEPENDENT MARKERS OF PROTEASOME INHIBITION" LEUKEMIA, MACMILLAN PRESS LTD, US, vol. 14, no. 7, July 2000 (2000-07), pages 1276-1283, XP009081378 ISSN: 0887-6924**
- **AGHAJANIAN CAROL ET AL: "A phase I trial of the novel proteasome inhibitor PS341 in advanced solid tumor malignancies." CLINICAL CANCER RESEARCH : AN OFFICIAL JOURNAL OF THE AMERICAN ASSOCIATION FOR CANCER RESEARCH AUG 2002, vol. 8, no. 8, August 2002 (2002-08), pages 2505-2511, XP002427558 ISSN: 1078-0432**
- **ZIMMERMANN J ET AL: "Proteasome inhibitor induced gene expression profiles reveal overexpression of transcriptional regulators ATF3, GADD153 and MAD1." ONCOGENE 8 JUN 2000, vol. 19, no. 25, 8 June 2000 (2000-06-08), pages 2913-2920, XP002427559 ISSN: 0950-9232**
- **ADAMS J. ET AL.: 'Protease Inhibitors: A Novel Class of Potent and Effective Antitumor Agents' CANCER RESEARCH vol. 59, 01 June 1999, pages 2615 - 2622, XP002168152**
- **ADAMS J. ET AL.: 'J. Development of The Proteasome Inhibitor PS-341' THE ONCOLOGIST vol. 7, February 2002, pages 9 - 16, XP003000262**


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- LIGHTCAP E.S. ET AL.: 'Proteasome Inhibition Measurements' CLINICAL APPLICATION. CLINICAL CHEMISTRY vol. 46, no. 5, 2000, pages 673 - 683, XP003000263
- HOCHWALD ET AL: 'Antineoplastic Therapy in Colorectal Cancer Through Proteasome Inhibition' THE AMERICAN SURGEON vol. 69, no. 1, January 2003, pages 15 - 23, XP008068421
- SUDIMACK J ET AL: "TARGETED DRUG DELIVERY VIA THE FOLATE RECEPTOR", ADVANCED DRUG DELIVERY REVIEWS, ELSEVIER, AMSTERDAM, NL, vol. 41, no. 2, 1 January 2000 (2000-01-01), pages 147-162, XP000990851, ISSN: 0169-409X, DOI: 10.1016/S0169-409X(99)00062-9

## Description

CROSS-REFERENCES TO RELATED APPLICATIONS

This application claims the benefit of U.S. Provisional Application Number 60/431,514, filed December 6, 2002.

BACKGROUND OF THE INVENTION

Proteasome inhibition represents an important recently developed strategy in cancer treatment. The proteasome is a multi-enzyme complex present in all cells which plays a role in degradation of proteins involved in regulation of the cell cycle. For example, King *et al.*, demonstrated that the ubiquitin-proteasome pathway plays an essential role in regulating cell cycle, neoplastic growth and metastasis. A number of key regulatory proteins, including p53, cyclins, and the cyclin-dependent kinases p21 and $p27^{KIP1}$, are temporally degraded during the cell cycle by the ubiquitin-proteasome pathway. The ordered degradation of these proteins is required for the cell to progress through the cell cycle and to undergo mitosis. See, *e.g.,* Science 274:1652-1659 (1996). Furthermore, the ubiquitin-proteasome pathway is required for transcriptional regulation. Palombella *et al*., teach that the activation of the transcription factor NF-kB is regulated by proteasome-mediated degradation of the inhibitor protein IkB. See International Patent Application Publication No. WO 95/25533. In turn, NF-kB plays a central role in the regulation of genes involved in the immune and inflammatory responses. For example, Read *et al.* demonstrated that the ubiquitin-proteasome pathway is required for expression of cell adhesion molecules, such as E-selectin, ICAM-1, and VCAM-1. See Immunity 2:493-506 (1995). Additional findings further support the role for proteasome inhibition in cancer therapy, as Zetter found that cell adhesion molecules are involved in tumor metastasis and angiogenesis *in vivo*, by directing the adhesion and extravastation of tumor cells to and from the vasculature to distant tissue sites within the body. See, *e.g*., Seminars in Cancer Biology 4:219-229 (1993). Moreover, Beg and Baltimore, found that NF-kB is an anti-apoptotic factor, and inhibition of NF-kB activation makes cells more sensitive to environmental stress and cytotoxic agents. See Science 274:782 (1996).

Adams *et al.* have described peptide boronic ester and acid compounds useful as proteasome inhibitors. See, *e.g.,* U.S. Patent No. 5,780,454 (1998), U.S. Patent No. 6,066,730 (2000), and U.S. Patent No. 6,083,903 (2000). They describe the use of the disclosed boronic ester and boronic acid compounds to reduce the rate of muscle protein degradation, to reduce the activity of NF-kB in a cell, to reduce the rate of degradation of p53 protein in a cell, to inhibit cyclin degradation in a cell, to inhibit the growth of a cancer cell, and to inhibit NF-kB dependent cell adhesion. Adams *et al.* have described one of the compounds, N-pyrazinecarbonyl-L-phenylalanine-L-leucineboronic acid (PS-341, now know as bortezomib) as having demonstrated antitumor activity in human tumor xenograft models. This particular compound has recently received approval for treatment of patients having relapsed refractory multiple myeloma, and is presently undergoing clinical trials in additional indications, including additional hematological cancers as well as solid tumors.

Because the proteasome plays a pervasive role in normal physiology as well as pathology, it is important to optimize (e.g., avoid excessive) proteasome inhibition when using proteasome inhibitors as therapeutic agents. Moreover, one of the continued problems with therapy in cancer patients is individual differences in response to therapies. With the narrow therapeutic index and the toxic potential of many available cancer therapies, this potentially contributes to many patients undergoing unnecessary ineffective and even harmful therapy regimens. If a designed therapy could be optimized to treat individual patients, such situations could be reduced or even eliminated. Accordingly, there is a need to identify particular cancer patients against which proteasome inhibitors are particularly effective, either alone or in combination with other chemotherapies. Also, there is a need to identify particular patients who respond well to treatment with a proteasome inhibitor (responders) versus those patient who do not respond to proteasome treatment (non-responders). It would therefore be beneficial to provide for the diagnosis, staging, prognosis, and monitoring of cancer patients, including, e.g., hematological cancer patients (e.g., multiple myeloma, leukemias, lymphoma, etc) as well as solid tumor cancer patients, who would benefit from proteasome inhibition therapies; or to indicate a predisposition of such patients to such preventative measures. The present invention is directed towards these needs.

DESCRIPTION OF THE INVENTION

The present invention is directed to the methods and kit defined in the appended claims. These methods typically include the determining the level of expression of two or more predictive markers in a patient's tumor (e.g., a patient's cancer cells), and identifying whether expression in the sample includes a pattern or profile of expression of a selected predictive marker or marker set which correlates with response or non-response to proteasome inhibition therapy.

The provided methods of the invention can eliminate ineffective or inappropriate use of proteasome inhibition therapy regimens.

The present methods and compositions are designed for use in diagnostics and therapeutics for a patient suffering from cancer. The cancer can be of the liquid or solid tumor type. Liquid tumors include tumors of hematological origin, including, e.g., myelomas (e.g., multiple myeloma), leukemias (e.g., Waldenstrom's syndrome, chronic lymphocytic leukemia, other leukemias), and lymphomas (e.g., B-cell lymphomas, non-Hodgkins lymphoma). Solid tumors can originate in organs, and include cancers such as lung, breast, prostate, ovary, colon, kidney, and liver.

Therapeutic agents for use in the methods of the invention include a new class of therapeutic agents known as proteosome inhibitors. One example of a proteosome inhibitor that was recently approved for treatment of relapsed refractory multiple myeloma patients and is presently being tested in clinical trials for additional indications is bortezomib. Other examples of proteosome inhibitors are known in the art and are described in further detail herein. Proteasome inhibition therapy regimens can also include additional therapeutic agents such as chemotherapeutic agents. Some examples of traditional chemotherapeutic agents are set forth in Table A. Alternatively or in combination with these chemotherapeutic agents, newer classes of chemotherapeutic agents can also be used in proteasome inhibition therapy.

One embodiment of the invention provides methods for determining a proteasome inhibition-based regimen for treating a tumor in a patient. Such methods are defined in the claims. A significant expression level of predictive marker or markers in the patient sample can be an indication that the patient is a responsive patient and would benefit from proteasome inhibition therapy when the predictive marker or marker set provided herein indicate such responsiveness. Additionally, a significant expression level of a predictive marker or markers in a patient can be an indication that the patient is a non-responsive patient and would not benefit from proteasome inhibition therapy when the marker or markers provided herein indicate such non-responsiveness.

Selected predictive markers for use in the methods comprise responsive predictive markers as indicated in Table 1, Table 2, and Table 3.

In certain aspects, the level of expression of predictive marker in the patient's tumor can be measured by isolating a sample of the tumor and performing analysis on the isolated sample, or a portion thereof. In another aspect, the level of expression of predictive marker in the patient's tumor can be measured using in vivo imaging techniques.

In certain aspects, determining the level of expression comprises detection of mRNA. Such detection can be carried out by any relevant method, including e.g., PCR, northern, nucleotide array detection, in vivo imaging using nucleic acid probes. In other aspects, determining the level of expression of the predictive marker comprises detection of protein. Such detection can be carried out using any relevant method for protein detection, including w.g., ELISA, western blot, immunoassay, protein array detection, in vivo imaging using peptide probes.

Determining the level of expression of a predictive marker can be compared to a predetermined standard control level of expression in order to evaluate if expression of a marker or marker set is significant and make an assessment for determining whether the patient is responsive or non-responsive. Additionally, determining the level of expression of a predictive marker can be compared to an internal control marker level of expression which is measured at the same time as the predictive marker in order to make an assessment for determining whether the patient is responsive or non-responsive. The level of expression may be determined as significantly over-expressed in certain aspects. The level of expression may be under-expressed in other aspects. In still other aspects, the level of expression is determined against a pre-determined standard as determined by the methods provided herein.

Methods of the invention can use at least two of the predictive markers set forth in any one of Table 1, Table 2, Table 3, Table 4, Table 5, Table 6, or Table 7. Additionally, the methods provided can use two, three, four, five, six, or more markers to form a predictive marker set. For example, marker sets selected from the markers in Table 1, Table 2 and/or Table 3 can be generated using the methods provided herein and can comprise between two, and all of the markers set forth in Table 1, Table 2 or Table 3 and each and every combination in between (e.g., four selected markers, 16 selected markers, 74 selected markers, etc.). In one embodiment, the markers comprise those set forth in Table 4, Table 5 or Table 6.

Methods of the invention further provide the ability to construct marker sets from the individual predictive markers set forth in Table 1 Table 2 and Table 3 using the methods described in further detail herein. In a further aspect, more than one marker set can be used in combination for the diagnostic, prognostic and treatment methods provided.

The methods of the invention can be performed such that determination of the level of expression of a predictive marker is measured prior to tumor therapy in order to identify whether the patient will be responsive to a proteasome inhibition therapy.

In addition, the methods of the invention can be performed concurrently with ongoing tumor therapy to determine if the patient is either responding to present proteasome inhibition therapy or will respond to additional therapy comprising proteasome inhibition therapy.

Still further, the methods of the invention can be performed after tumor therapy has been carried out in order to assess whether the patient will be responsive to future course of proteasome inhibition therapy.

Whether the methods are performed during ongoing tumor therapy or after a course of tumor therapy, the tumor therapy can comprise proteasome inhibition therapy or alternative forms of cancer therapy. The methods provided are designed to determine if the patient will benefit from additional or future proteasome inhibition therapy, and can include

such proteasome inhibition therapy alone or in combination with additional therapeutic agents.

The invention also relates to a kit as defined in the claims.

Provided are marker sets and methods for identification of marker sets comprising at least two isolated predictive markers set forth in Table 1, Table 2 and Table 3. The marker sets comprise reagents for detection of the relevant predictive markers set forth in Table 1, Table 2 and Table 3. Such reagents include nucleic acid probes, primers, antibodies, antibody derivatives, antibody fragments, and peptide probes.

Further provided are kits for use in determining a proteasome inhibition based regimen for treating a tumor in a patient. The kits of the invention include reagents for assessing predictive markers (e.g., at least two predictive markers) and predictive marker sets (e.g., at least two, three, four or more markers selected from Table 1, Table 2 and Table 3), as well as instructions for use in accordance with the methods provided herein. The kits provided contain nucleic acid probes for assessment of predictive markers. The kits provided contain antibody, antibody derivative antibody fragment, or peptide reagents for assessment of predictive markers.

According to the invention, the markers and marker sets are selected such that the positive predictive value of the methods of the invention is at least about 10%, preferably about 25%, more preferably about 50% and most preferably about 75%, 80%, 85%, or 90% or greater. Also preferred for use in the methods of the invention are markers that are differentially expressed in tumors, as compared to normal cells, by at least one-and-a-half-fold and preferably at least two-fold in at least about 20%, more preferably about 50%, and most preferably about 75% or more of any of the following conditions: partial responders, complete responders, minimal responders, and non-responders to proteasome inhibition therapy.

The present invention further provides previously unknown or unrecognized targets for the development of anti-cancer agents, e.g., chemotherapeutic compounds. The predictive markers and marker sets described herein also provide new targets either alone or in combination, which can be used for the development of novel therapeutics for cancers. Thus, nucleic acids and proteins represented by each of the markers described herein can be used as targets in developing treatments (either single agent or multiple agent) for cancers, including e.g, hematological malignancies or solid tumor malignancies.

Thus, additionally described herein are methods for use of the identified predictive markers, as well as the corresponding nucleic acid and polypeptides for screening methods for identification of novel compounds for use as anti-cancer therapeutics. Such newly identified compounds can be useful alone, or in combination with proteasome inhibition therapy as a complementary therapeutic.

The present invention is based, in part, on the identification of individual markers and marker sets that can be used to determine whether a tumor may be effectively treated by treatment with a proteasome inhibition therapy. For example, the compositions and methods provided herein can be used to determine whether a patient will be responsive or non-responsive to a proteasome inhibition therapeutic agent.

## Definitions

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the preferred methods and materials are described herein. In the case of conflict, the present specification, including definitions, will control.

The articles "a" and "an" are used herein to refer to one or to more than one (*i.e.* to at least one) of the grammatical object of the article. By way of example, "an element" means at least one element and can include more than one element.

A "marker" is a naturally-occurring polymer corresponding to at least one of the nucleic acids or proteins associated with Affymetrix probe set identifiers listed in any one of Table 1, Table 2 or Table 3 For example, markers include, without limitation, sense and anti-sense strands of genomic DNA (*i.e.* including any introns occurring therein), RNA generated by transcription of genomic DNA (*i.e.* prior to splicing), RNA generated by splicing of RNA transcribed from genomic DNA, and proteins generated by translation of spliced RNA (*i.e.* including proteins both before and after cleavage of normally cleaved regions such as transmembrane signal sequences). As used herein, "marker" may also include a cDNA made by reverse transcription of an RNA generated by transcription of genomic DNA (including spliced RNA). "marker set" is a group of markers. Markers of the present invention include the predictive markers identified in Table 1, Table 2, and Table 3.

A "Predictive Marker" or "predictive marker" as used herein, includes a marker which has been identified as having differential expression in tumor cells of a patient and is representative of a characteristic of a patient which is responsive in either a positive or negative manner to treatment with a proteasome inhibitor regimen. For example, a predictive marker includes a marker which is upregulated in a non-responsive patient; alternatively a predictive marker includes a marker which is upregulated in a responsive patient. Similarly, a predictive marker is intended to include those markers which are down-regulated in a non-responsive patient as well as those markers which are down-regulated in a responsive patient. Thus, as used herein, predictive marker is intended to include each and every one of these

possibilities, and further can include each one individually as a predictive marker; or alternatively can include one or more, or all of the characteristics collectively when reference is made to "predictive markers" or "predictive marker sets."

As used herein, a "naturally-occurring" nucleic acid molecule refers to an RNA or DNA molecule having a nucleotide sequence that occurs in nature (*e.g.* encodes a natural protein).

The term "probe" refers to any molecule which is capable of selectively binding to a specifically intended target molecule, for example a marker of the invention. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, and organic monomers.

The "normal" level of expression of a marker is the level of expression of the marker in cells in a similar environment or response situation, in a patient not afflicted with cancer. A normal level of expression of a marker may also refer to the level of expression of a "control sample", (*e.g.*, sample from a healthy subjects not having the marker associated disease). A control sample may be comprised of a control database. Alternatively, a "normal" level of expression of a marker is the level of expression of the marker in non-tumor cells in a similar environment or response situation from the same patient that the tumor is derived from.

"Over-expression" and "under-expression" of a marker refer to expression of the marker of a patient at a greater or lesser level, respectively, than normal level of expression of the marker (*e.g.* more than one and a half-fold, at least two-fold, at least threefold, greater or lesser level etc.).

"Complementary" refers to the broad concept of sequence complementarity between regions of two nucleic acid strands or between two regions of the same nucleic acid strand. It is known that an adenine residue of a first nucleic acid region is capable of forming specific hydrogen bonds ("base pairing") with a residue of a second nucleic acid region which is antiparallel to the first region if the residue is thymine or uracil. Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. Preferably, the first region comprises a first portion and the second region comprises a second portion, whereby, when the first and second portions are arranged in an antiparallel fashion, at least about 50%, and preferably at least about 75%, at least about 90%, or at least about 95% of the nucleotide residues of the first portion are capable of base pairing with nucleotide residues in the second portion. More preferably, all nucleotide residues of the first portion are capable of base pairing with nucleotide residues in the second portion.

"Homologous" as used herein, refers to nucleotide sequence similarity between two regions of the same nucleic acid strand or between regions of two different nucleic acid strands. When a nucleotide residue position in both regions is occupied by the same nucleotide residue, then the regions are homologous at that position. A first region is homologous to a second region if at least one nucleotide residue position of each region is occupied by the same residue. Homology between two regions is expressed in terms of the proportion of nucleotide residue positions of the two regions that are occupied by the same nucleotide residue. By way of example, a region having the nucleotide sequence 5'-ATTGCC-3' and a region having the nucleotide sequence 5'-TATGGC-3' share 50% homology. Preferably, the first region comprises a first portion and the second region comprises a second portion, whereby, at least about 50%, and preferably at least about 75%, at least about 90%, or at least about 95% of the nucleotide residue positions of each of the portions are occupied by the same nucleotide residue. More preferably, all nucleotide residue positions of each of the portions are occupied by the same nucleotide residue.

A marker is "fixed" to a substrate if it is covalently or non-covalently associated with the substrate such the substrate can be rinsed with a fluid (*e.g.* standard saline citrate, pH 7.4) without a substantial fraction of the marker dissociating from the substrate.

As used herein, "significant" expression, or a marker "significantly" expressed is intended to refer to differential expression of a predictive marker which is indicative of responsiveness or non-responsiveness. A marker or marker set in a patient is "significantly" expressed at a higher (or lower) level than the normal level of expression of a marker or marker set if the level of expression of the marker or marker set is greater or less, respectively, than the normal level by an amount greater than the standard error of the assay employed to assess expression,. Preferably a significant expression level is at least twice, and more preferably three, four, five or ten times that amount. Alternately, expression of the marker or marker set in the patient can be considered "significantly" higher or lower than the normal level of expression if the level of expression is at least about two, and preferably at least about three, four, or five times, higher or lower, respectively, than the normal level of expression of the marker or marker set. Still further, a "significant" expression level may refer to level which either meets or is above or below a pre-determined score for a predictive marker set as determined by methods provided herein.

A cancer or tumor is treated or diagnosed according to the methods described herein. "Cancer" or "tumor" is intended to include any neoplastic growth in a patient, including an inititial tumor and any metastases. The cancer can

be of the liquid or solid tumor type. Liquid tumors include tumors of hematological origin, including, e.g., myelomas (e.g., multiple myeloma), leukemias (e.g., Waldenstrom's syndrome, chronic lymphocytic leukemia, other leukemias), and lymphomas (e.g., B-cell lymphomas, non-Hodgkins lymphoma,). Solid tumors can originate in organs, and include cancers such as lung, breast, prostate, ovary, colon, kidney, and liver. As used herein, cancer cells, including tumor cells, refer to cells that divide at an abnormal (increased) rate. Cancer cells include, but are not limited to, carcinomas, such as squamous cell carcinoma, basal cell carcinoma, sweat gland carcinoma, sebaceous gland carcinoma, adenocarcinoma, papillary carcinoma, papillary adenocarcinoma, cystadenocarcinoma, medullary carcinoma, undifferentiated carcinoma, bronchogenic carcinoma, melanoma, renal cell carcinoma, hepatoma-liver cell carcinoma, bile duct carcinoma, cholangiocarcinoma, papillary carcinoma, transitional cell carcinoma, choriocarcinoma, semonoma, embryonal carcinoma, mammary carcinomas, gastrointestinal carcinoma, colonic carcinomas, bladder carcinoma, prostate carcinoma, and squamous cell carcinoma of the neck and head region; sarcomas, such as fibrosarcoma, myxosarcoma, liposarcoma, chondrosarcoma, osteogenic sarcoma, chordosarcoma, angiosarcoma, endotheliosarcoma, lymphangiosarcoma, synoviosarcoma and mesotheliosarcoma; hematologic cancers, such as myelomas, leukemias (e.g., acute myelogenous leukemia, chronic lymphocytic leukemia, granulocytic leukemia, monocytic leukemia, lymphocytic leukemia), and lymphomas (e.g., follicular lymphoma, mantle cell lymphoma, diffuse large Bcell lymphoma, malignant lymphoma, plasmocytoma, reticulum cell sarcoma, or Hodgkins disease); and tumors of the nervous system including glioma, meningoma, medulloblastoma, schwannoma or epidymoma.

A cancer is "responsive" to a therapeutic agent if its rate of growth is inhibited as a result of contact with the therapeutic agent, compared to its growth in the absence of contact with the therapeutic agent. Growth of a cancer can be measured in a variety of ways, for instance, the size of a tumor or the expression of tumor markers appropriate for that tumor type may be measured. For example, the response definitions used to identify markers associated with myeloma and its response to proteasome inhibition therapy, the Southwestern Oncology Group (SWOG) criteria as described in Blade et al., Br J Haematol. 1998 Sep;102(5):1115-23 were used (also see e.g., Table C). The quality of being responsive to a proteasome inhibition therapy is a variable one, with different cancers exhibiting different levels of "responsiveness" to a given therapeutic agent, under different conditions. Still further, measures of responsiveness can be assessed using additional criteria beyond growth size of a tumor, including patient quality of life, degree of metastases, etc. In addition, clinical prognostic markers and variables can be assessed (e.g., M protein in myeloma, PSA levels in prostate cancer) in applicable situations.

A cancer is "non-responsive" to a therapeutic agent if its rate of growth is not inhibited, or inhibited to a very low degree, as a result of contact with the therapeutic agent when compared to its growth in the absence of contact with the therapeutic agent. As stated above, growth of a cancer can be measured in a variety of ways, for instance, the size of a tumor or the expression of tumor markers appropriate for that tumor type may be measured. For example, the response definitions used to identify markers associated with non- response of multiple myeloma to therapeutic agents, the Southwestern Oncology Group (SWOG) criteria as described in Blade *et. al.* were used in the experiments described herein. The quality of being non-responsive to a therapeutic agent is a highly variable one, with different cancers exhibiting different levels of "non-responsiveness" to a given therapeutic agent, under different conditions. Still further, measures of non-responsiveness can be assessed using additional criteria beyond growth size of a tumor, including patient quality of life, degree of metastases, etc. In addition, clinical prognostic markers and variables can be assessed (e.g., M protein in myeloma, PSA levels in prostate cancer) in applicable situations.

"Treatment" shall mean preventing or inhibiting further tumor growth, as well as causing shrinkage of a tumor. Treatment is also intended to include prevention of metastasis of tumor. A tumor is "inhibited" or "treated" if at least one symptom (as determined by responsiveness/non-responsiveness indicators known in the art and described herein) of the cancer or tumor is alleviated, terminated, slowed, minimized, or prevented. Any amelioration of any symptom, physical or otherwise, of a tumor pursuant to treatment using any proteasome inhibitor, is within the scope of the invention.

As used herein, the term "agent" is defined broadly as anything that cancer cells, including tumor cells, may be exposed to in a therapeutic protocol. In the context of the present invention, such agents include, but are not limited to, proteasome inhibition agents, as well as chemotherapeutic agents as described in further detail herein.

"Proteasome inhibitor" shall mean any substance which directly or indirectly inhibits the 20S or 26S proteasome or the activity thereof. Preferably, such inhibition is specific, i.e., the proteasome inhibitor inhibits proteasome activity at a concentration that is lower than the concentration of the inhibitor required to produce another, unrelated biological effect. Preferably, the concentration of the proteasome inhibitor required for proteasome inhibition is at least 2-fold lower, more preferably at least 5-fold lower, even more preferably at least 10-fold lower, and most preferably at least 20-fold lower than the concentration required to produce an unrelated biological effect. Proteasome inhibitors include peptide aldehydes, peptide boronic acids, lactacystin and lactacystin analogues, vinyl sulfones, and alpha.'.beta.'-epoxyketones. Proteasome inhibitors are described in further detail herein.

A kit is any article of manufacture (*e.g.* a package or container) comprising at least one reagent, e.g. a probe, for specifically detecting a marker or marker set of the invention. The article of manufacture may be promoted, distributed, or sold as a unit for performing the methods of the present invention. The reagents included in such a kit comprise

probes/primers and/or antibodies for use in detecting responsive and non-predictive marker expression. In addition, the kits of the present invention may preferably contain instructions which describe a suitable detection assay. Such kits can be conveniently used, e.g., in clinical settings, to diagnose and evaluate patients exhibiting symptoms of cancer, in particular patients exhibiting the possible presence of an a cancer capable of treatment with proteasome inhibition therapy, including, e.g., hematological cancers e.g., myelomas (e.g., multiple myeloma), lymphomas (e.g., non-hodgkins lymphoma), leukemias, and solid tumors (e.g., lung, breast, ovarian, etc.).

The markers used in the methods or kits of the present invention, whose expression correlates with the response to an agent, are identified in Table 1, Table 2, Table 3, Table 4, Table 5, Table 6, and Table 7. By examining the expression of one or more of the identified markers or marker sets in a tumor, it is possible to determine which therapeutic agent or combination of agents will be most likely to reduce the growth rate of the cancer cells. By examining the expression of one or more of the identified markers or marker sets in a cancer, it is also possible to determine which therapeutic agent or combination of agents will be the least likely to reduce the growth rate of cancer cells. By examining the expression of one or more of the identified markers or marker sets, it is therefore possible to eliminate ineffective or inappropriate therapeutic agents It is also possible to identify new targets for anti-cancer agents by examining the expression of one or more markers or marker sets. Thus, in one embodiment, the tumor cells used in the methods of the present invention are from a bone marrow sample. Importantly, these determinations can be made on a patient by patient basis or on an agent by agent basis. Thus, one can determine whether or not a particular therapeutic treatment is likely to benefit a particular patient or group/class of patients, or whether a particular treatment should be continued.

Table 1 lists markers identified using statistical analysis applied to genes from 44 myeloma patient samples. The markers in Table 1 are significantly expressed in samples from patients that are either responsive or non-responsive to treatment with the proteasome inhibitor bortezomib. Thus, one would appreciate that the markers identified can function in a predictive model to prospectively identify patients' response to proteasome inhibition therapy, including response to bortezomib or other proteasome inhibition therapies known in the art as well as those described in further detail herein. In particular, the markers in Table 1 are correlated with a positive response to therapy (referred to herein as "non-predictive markers, (NR)"). A patient with a positive response (either complete, partial or minimal; see Table C) to therapy is hereinafter referred to as a "responder". Additionally, the predictive markers in Table 1 are correlated with a negative or poor response to an agent (referred to herein as "non-predictive markers, (NR)"). A patient with a poor response (called a progressive or refractory disease; see Table C) to treatment is hereinafter referred to as a "non-responder". A patient with no response to treatment is hereinafter referred to as "stable" (see Table C).

Table 2 lists markers identified using statistical analysis applied using a Cox proportional hazard analysis to determine predictors of time until disease progression (TTP) in patients with relapsed and refractory multiple myeloma. These markers are useful as additional predictive markers which are significantly expressed in patients who are likely to progress in disease at a faster rate, and less likely to be responsive to therapy than other patients. These predictive markers will serve as an additional factor in identification of patients likely to be responsive to proteasome inhibition therapy.

Table 3 lists markers identified using statistical analysis applied to genes from 44 myeloma samples. The predictive markers in Table 2 are significantly expressed in samples from myeloma patients whose disease is refractory to treatment with the proteasome inhibitor bortezomib. These predictive markers will further serve to distinguish refractory patients from those who will be either stable or responsive to treatment.

The invention also relates to various reagents and kits for diagnosing, staging, prognosing, monitoring and treating a cancer patient, (e.g., a patient with a liquid tumor or a solid tumor as described in further detail herein), with proteasome inhibition therapy.

According to the invention, the markers are selected such that the positive predictive value of the methods of the invention is at least about 10%, preferably about 25%, more preferably about 50% and most preferably about 90%. Also preferred for use in the methods of the invention are markers that are differentially expressed, as compared to normal cells, by at least two-fold in at least about 20%, more preferably about 50%, and most preferably about 75% of any of the following conditions: responsive patients (e.g., complete response, partial response, minimal response); and non-responsive patients (e.g., no change, relapse from response).

<u>Identification Of Responsive And Non-Predictive markers</u>

Described herein are markers that are expressed in a tumor that is responsive to proteasome inhibition therapy and whose expression correlates with responsiveness to that therapeutic agent. Also described herein are markers that are expressed in a tumor that is non-responsive to proteasome inhibition therapy and whose expression correlates with non-responsiveness to such therapy. The invention also features combinations of markers, referred to herein as "marker sets," that can predict patients that are likely to respond or not to respond to a proteasome inhibition therapy regimen.

Table 1 identifies markers whose expression correlates with responsiveness to a proteasome inhibitor. It is preferable to determine the expression of at least two or more of the identified predictive markers; or three or more of

the identified predictive markers comprising a set of the identified predictive markers.. Thus, it is preferable to assess the expression of a set or panel of predictive markers, *i.e.,* the expression profile of a predictive marker set.

Determining Responsiveness or Non-Responsiveness To An Agent

The expression level (including protein level) of the identified responsive and non-predictive markers may be used to: 1) determine if a patient can be treated by an agent or combination of agents; 2) determine if a patient is responding to treatment with an agent or combination of agents; 3) select an appropriate agent or combination of agents for treating a patient; 4) monitor the effectiveness of an ongoing treatment; 5) identify new proteasome inhibition therapy treatments (either single agent proteasome inhibitor agents or complementary agents which can be used alternatively or in combination with proteasome inhibition agents); 6) differentiate early versus late recurrence of a cancer; and 7) select an appropriate agent or combination of agents in treating early and late recurrence of a cancer. In particular, the identified responsive and non-predictive markers may be utilized to determine appropriate therapy, to monitor clinical therapy and human trials of a drug being tested for efficacy, and to develop new agents and therapeutic combinations.

In one embodiment of the invention, a cancer may be predisposed to respond to an agent if two or more of the corresponding predictive markers identified in Table 1,

Table 2 and Table 3 are significantly expressed. In another embodiment of the invention, the predisposition of a cancer to be responsive to an agent is determined by the methods of the present invention, wherein significant expression of the individual predictive markers of the marker sets identified in Table 4, Table 5, or Table 6 is evaluated. Likewise, the predisposition of a patient to be responsive to an agent is determined by the methods of the present invention, wherein a marker set generated using to the methods described herein wherein the markers comprising the marker set include predictive markers set forth in Table 1, Table 2, and/or Table 3, and the expression of the marker set is evaluated.

In another embodiment of the invention, a cancer may be predisposed to non-responsiveness to an agent if two or more of the corresponding non-predictive markers are significantly expressed. In another embodiment of the invention, a cancer may be predisposed to non-responsiveness to an agent if two or more of the corresponding predictive markers identified in Table 1, Table 2 and Table 3 are significantly expressed. In another embodiment of the invention, the predisposition of a cancer to be non-responsive to an agent is determined by the methods of the present invention, wherein significant expression of the individual predictive markers of the marker sets identified in Table 4, Table 5, or Table 6 is evaluated. Likewise, the predisposition of a patient to be non-responsive to an agent is determined by the methods of the present invention, wherein a marker set is generated using the methods described herein wherein the markers comprising the marker set include predictive markers set forth in Table 1, Table 2, and/or Table 3, and the expression of the marker set is evaluated.

In one aspect, the predictive marker or markers evaluated are selected from those set forth in Table 1. In yet another aspect the predictive marker or markers evaluated are selected from those set forth in Table 2. In still another aspect the predictive marker or markers evaluated are selected from those set forth in Table 3. Still a further aspect contemplates markers set forth in either Table 1 alone or in combination with markers set for the in Table 2 and/or Table 3, or alternatively, those markers set forth in Table 2 alone or in combination with Table 1 and/or Table 3.

The source of the cancer cells used in the present method will be based on how the method of the present invention is being used. For example, if the method is being used to determine whether a patient's cancer can be treated with an agent, or a combination of agents, then the preferred source of cancer cells will be cancer cells obtained from a tumor from the patient, e.g., a tumor biopsy (including a solid or a liquid tumor), a blood sample. Alternatively, a cancer cell line similar to the type of cancer being treated can be assayed. For example if multiple myeloma is being treated, then a myeloma cell line can be used. If the method is being used to predict or monitor the effectiveness of a therapeutic protocol, then a tissue or blood sample from the patient being treated is the preferred source. If the method is being used to identify new therapeutic agents or combinations, any cancer cells, *e.g.,* cells of a cancer cell line, can be used.

A skilled artisan can readily select and obtain the appropriate cancer cells that are used in the present method. For cancer cell lines, sources such as The National Cancer Institute, for the NCI-60 cells, are preferred. For cancer cells obtained from a patient, standard biopsy methods, such as a needle biopsy, can be employed.

Myeloma samples were used to identify the markers of the present invention. Further, the expression level of markers can be evaluated in other tissue types including disorders of related hematological cell types, including, e.g., Waldenstroms macrogobulinemia, Myelodysplastic syndrome and other hematological cancers including lymphomas, leukemias, as well as tumors of various solid tissues. It will thus be appreciated that cells from other hematologic malignancies including, e.g., B-cell Lymphomas, Non-Hodgkins Lymphoma, Waldenstrom's syndrome, or other leukemias will be useful in the methods of the present invention. Still further, the predictive markers predicting disease aggressiveness as well as responsiveness and non-responsiveness to proteasome inhibition therapeutic agents in solid tumors (e.g., lung, breast, prostate, ovary, colon, kidney, and liver), can also be useful in the methods of the present invention.

In the methods of the present invention, the level of expression of two or more predictive markers selected from the group consisting of the markers identified in Table 1 Table 2 and Table 3, is determined. As used herein, the level or amount of expression refers to the absolute level of expression of an mRNA encoded by the marker or the absolute level of expression of the protein encoded by the marker (*i.e*., whether or not expression is or is not occurring in the cancer cells).

Generally, it is preferable to determine the expression of two or more of the identified responsive or non-predictive markers, or three or more of the identified responsive or non-predictive markers, or still further a larger a set of the identified responsive and/or non-predictive markers, selected from the predictive markers identified in Table 1, Table 2 and Table 3. For example, Table 4, Table 5 and Table 6 set forth marker sets identified using the methods described herein and can be used in the methods of the present invention. Still further, additional and/or alternative marker sets comprising the predictive markers identified herein can be generated using the methods and predictive markers provided. Thus, it is possible to assess the expression of a panel of responsive and non-predictive markers using the methods and compositions provided herein.

As an alternative to making determinations based on the absolute expression level of selected markers, determinations may be based on normalized expression levels. Expression levels are normalized by correcting the absolute expression level of a responsive or non-predictive marker by comparing its expression to the expression of a control marker that is not a responsive or non-predictive marker, e.g., a housekeeping gene that is constitutively expressed. Suitable markers for normalization include housekeeping genes, such as the actin gene. Constitutively expressed genes are known in the art and can be identified and selected according to the relevant tissue and/or situation of the patient and the analysis methods. Such normalization allows one to compare the expression level in one sample, *e.g.,* a tumor sample, to another sample, *e.g.,* a non-tumor sample, or between samples from different sources.

Further, the expression level can be provided as a relative expression level. To determine a relative expression level of a marker or marker set, the level of expression of the predictive marker or marker set is determined for 10 or more individual samples, preferably 50 or more individual samples in order to establish a baseline, prior to the determination of the expression level for the sample in question. To establish a baseline measurement, mean expression level of each of the predictive markers or marker sets assayed in the larger number of samples is determined and this is used as a baseline expression level for the predictive markers or marker sets in question. The expression level of the marker or marker set determined for the test sample (absolute level of expression) is then divided by the mean expression value obtained for that marker or marker set. This provides a relative expression level and aids in identifying extreme cases of responsive or non-responsive-ness.

Preferably, the samples used will be from similar tumors or from non-cancerous cells of the same tissue origin as the tumor in question. The choice of the cell source is dependent on the use of the relative expression level data. For example, using tumors of similar types for obtaining a mean expression score allows for the identification of extreme cases of responsive or non-responsive-ness. Using expression found in normal tissues as a mean expression score aids in validating whether the responsive/non-predictive marker or marker set assayed is tumor specific (versus normal cells). Such a later use is particularly important in identifying whether a responsive or non-predictive marker or marker set can serve as a target marker or marker set. In addition, as more data is accumulated, the mean expression value can be revised, providing improved relative expression values based on accumulated data.

Still further, as outlined above, there are various methods available to examine the expression of the markers, including gene array/chip technology, RT-PCR, in-situ hybridization, immunohistochemistry, immunoblotting, FISH (flouresence in-situ hybridization), FACS analyses, northern blot, southern blot or cytogenetic analyses. A skilled artisan can select from these or other appropriate and available methods based on the nature of the marker(s), tissue sample and disease in question. Different methods or combinations of methods could be appropriate in different cases or, for instance in different solid or hematological tumor types.

## Detection Assays

An exemplary method for detecting the presence or absence of a polypeptide or nucleic acid corresponding to a marker described herein in a biological sample involves obtaining a biological sample *(e.g.* a tumor sample) from a test subject and contacting the biological sample with a compound or an agent capable of detecting the polypeptide or nucleic acid (*e.g*., mRNA, genomic DNA, or cDNA). The detection methods of the invention can thus be used to detect mRNA, protein, cDNA, or genomic DNA, for example, in a biological sample *in vitro* as well as *in vivo.* For example, *in vitro* techniques for detection of mRNA include Northern hybridizations. *in situ* hybridizations, and TaqMan assays (Applied Biosystems) under GLP approved laboratory conditions. *In vitro* techniques for detection of a polypeptide corresponding to a marker of the invention include enzyme linked immunosorbent assays (ELISAs), Western blots, immunoprecipitations and immunofluorescence. *In vitro* techniques for detection of genomic DNA include Southern hybridizations. Furthermore, *in vivo* techniques for detection of a polypeptide corresponding to a marker of the invention include introducing into a subject a labeled antibody directed against the polypeptide. For example, the antibody can be

labeled with a radioactive marker whose presence and location in a subject can be detected by standard imaging techniques.

A general principle of such diagnostic and prognostic assays involves preparing a sample or reaction mixture that may contain a marker, and a probe, under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one embodiment of such a method, a sample from a subject, which is to be assayed for presence and/or concentration of marker, can be anchored onto a carrier or solid phase support. In another embodiment, the reverse situation is possible, in which the probe can be anchored to a solid phase and a sample from a subject can be allowed to react as an unanchored component of the assay. One example of such an embodiment includes use of an array or chip which contains a predictive marker or marker set anchored for expression analysis of the sample.

There are many established methods for anchoring assay components to a solid phase. These include, without limitation, marker or probe molecules which are immobilized through conjugation of biotin and streptavidin. Such biotinylated assay components can be prepared from biotin-NHS (N-hydroxy-succinimide) using techniques known in the art (*e.g.*, biotinylation kit, Pierce Chemicals, Rockford, IL), and immobilized in the wells of streptavidin-coated 96 well plates (Pierce Chemical). In certain embodiments, the surfaces with immobilized assay components can be prepared in advance and stored.

Other suitable carriers or solid phase supports for such assays include any material capable of binding the class of molecule to which the marker or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilized component is added to the solid phase upon which the second component is anchored. After the reaction is complete, uncomplexed components may be removed (*e.g.,* by washing) under conditions such that any complexes formed will remain immobilized upon the solid phase. The detection of marker/probe complexes anchored to the solid phase can be accomplished in a number of methods outlined herein.

In a preferred embodiment, the probe, when it is the unanchored assay component, can be labeled for the purpose of detection and readout of the assay, either directly or indirectly, with detectable labels discussed herein and which are well-known to one skilled in the art.

It is also possible to directly detect marker/probe complex formation without further manipulation or labeling of either component (marker or probe), for example by utilizing the technique of fluorescence energy transfer (see, for example, Lakowicz et al., U.S. Patent No. 5,631,169; Stavrianopoulos, et al., U.S. Patent No. 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (*e.g.*, using a fluorimeter).

In another embodiment, determination of the ability of a probe to recognize a marker can be accomplished without labeling either assay component (probe or marker) by utilizing a technology such as real-time Biomolecular Interaction Analysis (BIA) (see, *e.g.,* Sjolander, S. and Urbaniczky, C., 1991, Anal. Chem. 63:2338-2345 and Szabo et al., 1995, Curr. Opin. Struct. Biol. 5:699-705). As used herein, "BIA" or "surface plasmon resonance" is a technology for studying biospecific interactions in real time, without labeling any of the interactants (*e.g.*, BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another embodiment, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas, G., and Minton, A.P., 1993, Trends Biochem Sci. 18(8):284-7). Standard chromatographic techniques may also be utilized to

separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, *e.g.,* Heegaard, N.H., 1998, J. Mol. Recognit. Winter 11(1-6):141-8; Hage, D.S., and Tweed, S.A. J Chromatogr B Biomed Sci Appl 1997 Oct 10;699(1-2):499-525). Gel electrophoresis may also be employed to separate complexed assay components from unbound components (see, *e.g.,* Ausubel et al., ed., Current Protocols in Molecular Biology, John Wiley & Sons, New York, 1987-1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

In a particular embodiment, the level of mRNA corresponding to the marker can be determined both by *in situ* and by *in vitro* formats in a biological sample using methods known in the art. The term "biological sample" is intended to include tissues, cells, biological fluids and isolates thereof, isolated from a subject, as well as tissues, cells and fluids present within a subject. Many expression detection methods use isolated RNA. For *in vitro* methods, any RNA isolation technique that does not select against the isolation of mRNA can be utilized for the purification of RNA from tumor cells (see, *e.g.,* Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Patent No. 4,843,155).

The isolated mRNA can be used in hybridization or amplification assays that include, but are not limited to, Southern or Northern analyses, polymerase chain reaction and TaqMan analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding a marker of the present invention. Other suitable probes for use in the diagnostic assays of the invention are described herein. Hybridization of an mRNA with the probe indicates that the marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers.

An alternative method for determining the level of mRNA corresponding to a marker in a sample involves the process of nucleic acid amplification, e.g., by rtPCR (the experimental embodiment set forth in Mullis, 1987, U.S. Patent No. 4,683,202), ligase chain reaction (Barany, 1991, Proc. Natl. Acad. Sci. USA, 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi *et al.*, U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated from the cancer cells prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a control gene that is not a marker, *e.g.,* a housekeeping gene that is constitutively expressed. Suitable genes for normalization include housekeeping genes such as the actin gene, or epithelial cell-specific genes. This normalization allows the comparison of the expression level in one sample, e.g., a patient sample, to another sample, *e.g.,* a non- cancer sample, or between samples from different sources.

Alternatively, the expression level can be provided as a relative expression level. To determine a relative

expression level of a marker, the level of expression of the marker is determined for 10 or more samples of normal versus cancer cell isolates, preferably 50 or more samples, prior to the determination of the expression level for the sample in question. The mean expression level of each of the markers and marker sets assayed in the larger number of samples is determined and this is used as a baseline expression level for the marker. The expression level of the marker determined for the test sample (absolute level of expression) is then divided by the mean expression value obtained for that marker. This provides a relative expression level.

In another embodiment, a polypeptide corresponding to a marker is detected. A preferred agent for detecting a polypeptide is an antibody capable of binding to a polypeptide corresponding to a marker of the invention, preferably an antibody with a detectable label. Antibodies can be polyclonal, or more preferably, monoclonal. An intact antibody, or a fragment thereof (*e.g.*, Fab or $F(ab')_2$) can be used. The term "labeled", with regard to the probe or antibody, is intended to encompass direct labeling of the probe or antibody by coupling (*i.e.*, physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

A variety of formats can be employed to determine whether a sample contains a protein that binds to a given antibody. Examples of such formats include, but are not limited to, enzyme immunoassay (EIA), radioimmunoassay (RIA), Western blot analysis and enzyme linked immunoabsorbant assay (ELISA). A skilled artisan can readily adapt known protein/antibody detection methods for use in determining whether cancer cells express a marker described herein.

In one format, antibodies, or antibody fragments, can be used in methods such as Western blots or immunofluorescence techniques to detect the expressed proteins. In such uses, it is generally preferable to immobilize either the antibody or proteins on a solid support. Suitable solid phase supports or carriers include any support capable of binding an antigen or an antibody. Well-known supports or carriers include glass, polystyrene, polypropylene, polyethylene, dextran, nylon, amylases, natural and modified celluloses, polyacrylamides, gabbros, and magnetite.

One skilled in the art will know many other suitable carriers for binding antibody or antigen, and will be able to adapt such support for use with the present invention. For example, protein isolated from tumor cells can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

The invention also encompasses kits for detecting the presence of a polypeptide or nucleic acid corresponding to a marker of the invention in a biological sample (*e.g.* an ovary-associated body fluid such as a urine sample). Such kits can be used to determine if a subject is suffering from or is at increased risk of developing cancer. For example, the kit can comprise a labeled compound or agent capable of detecting a polypeptide or an mRNA encoding a polypeptide corresponding to a marker of the invention in a biological sample and means for determining the amount of the polypeptide or mRNA in the sample (*e.g.*, an antibody which binds the polypeptide or an oligonucleotide probe which binds to DNA or mRNA encoding the polypeptide). Kits can also include instructions for interpreting the results obtained using the kit.

For antibody-based kits, the kit can comprise, for example: (1) a first antibody (*e.g.*, attached to a solid support) which binds to a polypeptide corresponding to a marker of the invention; and, optionally, (2) a second, different antibody which binds to either the polypeptide or the first antibody and is conjugated to a detectable label.

For oligonucleotide-based kits, the kit can comprise, for example: (1) an oligonucleotide, e.g., a detectably labeled oligonucleotide, which hybridizes to a nucleic acid sequence encoding a polypeptide corresponding to a marker of the invention; (2) a pair of primers useful for amplifying a nucleic acid molecule corresponding to a marker of the invention; or (3) a marker set comprising oligonucleotides which hybridize to at least two nucleic acid sequences encoding polypeptide predictive markers of the invention. The kit can also comprise, e.g., a buffering agent, a preservative, or a protein stabilizing agent. The kit can further comprise components necessary for detecting the detectable label (*e.g.,* an enzyme or a substrate). For marker sets, the kit can comprise a marker set array or chip for use in detecting the predictive markers. The kit can also contain a control sample or a series of control samples which can be assayed and compared to the test sample. Each component of the kit can be enclosed within an individual container and all of the various containers can be within a single package, along with instructions for interpreting the results of the assays performed using the kit.

Monitoring the Effectiveness of an Anti-Cancer Agent

As discussed above, the identified responsive and non-predictive markers can be used as pharmacodynamic markers to assess whether the tumor has become refractory to an ongoing treatment (*e.g.*, a proteasome inhibition therapy). When the cancer is not responding to a treatment the expression profile of the tumor cells will change: the level or relative expression of two or more of the predictive markers (e.g., those predictive markers identified in Table

1, Table 2, Table 3) such that the expression profile represents a non-responsive patient.

As used herein, a patient refers to any subject undergoing proteasome inhibition therapy for cancer treatment. In one embodiment, the subject will be a human patient undergoing proteasome inhibition using a sole proteasome inhibition agent (e.g., bortezomib or other related agent). In another embodiment, the subject is a human patient undergoing proteasome inhibition using a proteasome inhibition agent in conjunction with another agent (e.g., a chemotherapy treatment). This embodiment of the present invention can also include comparing two or more samples obtained from a patient undergoing anti-cancer treatment including proteasome inhibition therapy. In general, it is conceivable to obtain a first sample from the patient prior to beginning therapy and one or more samples during treatment. In such a use, a baseline of expression prior to therapy is determined, then changes in the baseline state of expression is monitored during the course of therapy. Alternatively, two or more successive samples obtained during treatment can be used without the need of a pre-treatment baseline sample. In such a use, the first sample obtained from the subject is used as a baseline for determining whether the expression of a particular marker or marker set is increasing or decreasing.

In general, when monitoring the effectiveness of a therapeutic treatment, two or more samples from a patient are examined. In another aspect, three or more successively obtained samples are used, including at least one pre-treatment sample.

Electronic Apparatus Readable Arrays

Electronic apparatus readable arrays are described herein comprising at least two predictive markers described herein. As used herein, "electronic apparatus readable media" refers to any suitable medium for storing, holding or containing data or information that can be read and accessed directly by an electronic apparatus. As used herein, the term "electronic apparatus" is intended to include any suitable computing or processing apparatus or other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as a personal digital assistants (PDAs), cellular phone, pager and the like; and local and distributed processing systems. As used herein, "recorded" refers to a process for storing or encoding information on the electronic apparatus readable medium. Those skilled in the art can readily adopt any of the presently known methods for recording information on known media to generate manufactures comprising the markers described herein.

The array can be used to assay expression of one or more predictive markers or predictive marker sets in the array. In one embodiment, the array can be used to assay predictive marker or marker set expression in a tissue to ascertain tissue specificity of markers in the array. In this manner, up to about 44,000 markers can be simultaneously assayed for expression. This allows a profile to be developed showing a battery of markers specifically expressed in one or more tissues.

The array is also useful for ascertaining differential expression patterns of two or more markers in normal and abnormal (e.g., tumor) cells. This provides a battery of predictive markers that could serve as a tool for ease of identification of responsive and non-responsive patients.

In addition to such qualitative determination, the invention allows the quantitation of marker expression. Thus, predictive markers can be grouped on the basis of marker sets or responsive and non-responsive indications by the level of expression in the sample. This is useful, for example, in ascertaining the responsive or non-responsive indication of the sample by virtue of scoring the expression levels according to the methods provided herein.

In another embodiment, the array can be used to monitor the time course of expression of two or more predictive markers in the array.

The array is also useful for ascertaining the effect of the expression of a marker on the expression of other predictive markers in the same cell or in different cells. This provides, for example, a selection of alternate molecular targets for therapeutic intervention if the proteasome inhibition regimen is non-responsive.

Therapeutic Agents

The markers of the present invention are shown to be predictive of patients who are responsive or non-responsive (sensitive or resistant) to proteasome inhibition therapy. Proteasome inhibition therapy can comprise treatment of a cancer patient with a proteasome inhibitor agent, alone or in combination with additional agents, such as chemotherapeutic agents.

The examples described herein entail use of the proteasome inhibitor N-pyrazinecarbonyl-L-phenylalanine-L-leucineboronic acid, bortezomib ((VELCADE™); formerly known as MLN341 or PS-341). The language "proteasome inhibitor" is intended to include bortezomib, compounds which are structurally similar to bortezomib and/or analogs of bortezomib. The language "proteasome inhibitor" can also include "mimics". "Mimics" is intended to include compounds which may not be structurally similar to bortezomib but mimic the therapeutic activity of bortezomib or structurally similar

compounds *in vivo.* Proteasome inhibitor compounds of this invention are those compounds which are useful for inhibiting tumor growth, (e.g., multiple myeloma tumor growth, other hematological or solid tumors as described in further detail herein) in patients. Proteasome inhibitor also is intended to include pharmaceutically acceptable salts of the compounds.

Proteasome inhibitors for use in the practice of the invention include additional peptide boronic acids such as those disclosed in Adams et al., U.S. Patent No. 5,780,454 (1998), U.S. Patent No. 6,066,730 (2000), U.S. Patent No. 6,083,903 (2000), U.S. Patent No. 6,548,668 (2003), and Siman et al. WO 91/13904, including all compounds and formulae disclosed therein. Preferably, a boronic acid compound for use in the present invention is selected from the group consisting of: N-(4-morpholine)carbonyl-.beta.-(1-naphthyl)-L-alanine-L-leucine boronic acid; N-(8-quinoline)sulfonyl-.beta.-(1-naphthyl)-L-alanine-L-alanine-L-leucine boronic acid; N-(2-pyrazine)carbonyl-L-phenylalanine-L-leucine boronic acid, and N-(4-morpholine)carbonyl-[O-(2-pyridylmethyl)]-L-tyrosine-L-leucine boronic acid.

Additionally, proteasome inhibitors include peptide aldehyde proteasome inhibitors such as those disclosed in Stein et al. U.S. Patent No. 5,693,617 (1997), and International patent publications WO 95/24914 published Sep. 21, 1995 and Siman et al. WO 91/13904 published Sep. 19, 1991; Iqbal et al. J. Med. Chem. 38:2276-2277 (1995), as well as Bouget et al. Bioorg Med Chem 17:4881-4889 (2003), including all compounds and formulae disclosed therein.

Further, proteasome inhibitors include lactacystin and lactacycstin analogs which have been disclosed in Fentany et al, U.S. Patent No. 5,756,764 (1998), and U.S.

Patent No. 6,147,223(2000), Schreiber et al U.S. Patent No. 6,645,999 (2003), and Fenteany et al. Proc. Natl. Acad. Sci. USA (1994) 91:3358, including all compounds and formulae disclosed therein.

Additionally, synthetic peptide vinyl sulfone proteasome inhibitors and epoxyketone proteasome inhibitors have been disclosed and are useful in the methods of the invention. See, e.g., Bogyo et al., Proc. Natl. Acad. Sci. 94:6629 (1997); Spaltensteinet al. Tetrahedron Lett. 37:1343 (1996); Meng L, Proc. Natl. Acad Sci 96: 10403 (1999); and Meng LH, Cancer Res 59: 2798 (1999).

Still further, natural compounds have been recently shown to have proteasome inhibition activity can be used in the present methods. For example, TMC-95A, a cyclic peptide, or Gliotoxin, both fungal metabolites or polyphenols compounds found in green tea have been identified as proteasome inhibitors. See, e.g., Koguchi Y, Antibiot (Tokyo) 53:105. (2000); Kroll M, Chem Biol 6:689 (1999); and Nam S, J. Biol Chem 276: 13322(2001).

Further to the above, the language, proteasome inhibition therapy can also include additional agents in addition to proteasome inhibition agents, including chemotherapeutic agents. A "chemotherapeutic agent" is intended to include chemical reagents which inhibit the growth of proliferating cells or tissues wherein the growth of such cells or tissues is undesirable. Chemotherapeutic agents such as anti-metabolic agents, *e.g.*, Ara AC, 5-FU and methotrexate, antimitotic agents, *e.g.*, taxane, vinblastine and vincristine, alkylating agents, *e.g.*, melphanlan, BCNU and nitrogen mustard, Topoisomerase II inhibitors, *e.g.,* VW-26, topotecan and Bleomycin, strand-breaking agents, *e.g.,* doxorubicin and DHAD, cross-linking agents, *e.g.*, cisplatin and CBDCA, radiation and ultraviolet light. In a preferred embodiment, the agent is a proteasome inhibitor (*e.g.*, bortezomib or other related compounds).are well known in the art (see *e.g.,* Gilman A.G., et al., The Pharmacological Basis of Therapeutics, 8th Ed., Sec 12:1202-1263 (1990)), and are typically used to treat neoplastic diseases. The chemotherapeutic agents generally employed in chemotherapy treatments are listed below in Table A.

**TABLE A**

| CLASS | TYPE OF AGENT | NONPROPRIETARY NAMES (OTHER NAMES) |
|---|---|---|
| Alkylating | Nitrogen Mustards | Mechlorethamine ($HN_2$)<br>Cyclophosphamide<br>Ifosfamide<br>Melphalan (L-sarcolysin)<br>Chlorambucil |
| | Ethylenimines And Methylmelamines | Hexamethylmelamine<br>Thiotepa |
| | Alkyl Sulfonates | Busulfan |
| Alkylating | Nitrosoureas | Carmustine (BCNU)<br>Lomustine (CCNU)<br>Semustine (methyl-CCNU)<br>Streptozocin (streptozotocin) |
| | Triazenes | Decarbazine (DTIC; dimethyltriazenoimidazolecarboxamide) |
| Alkylating | Alkylator | cis-diamminedichloroplatinum II (CDDP) |

(continued)

| CLASS | TYPE OF AGENT | NONPROPRIETARY NAMES (OTHER NAMES) |
|---|---|---|
| Antimetabolites | Folic Acid Analogs | Methotrexate (amethopterin) |
| | Pyrimidine Analogs | Fluorouracil ('5-fluorouracil; 5-FU)<br>Floxuridine (fluorode-oxyuridine; FUdR)<br>Cytarabine (cytosine arabinoside) |
| | Purine Analogs and Related Inhibitors | Mercaptopuine (6-mercaptopurine; 6-MP)<br>Thioguanine (6-thioguanine; TG)<br>Pentostatin (2' - deoxycoformycin) |
| Natural Products | Vinca Alkaloids | Vinblastin (VLB)<br>Vincristine |
| | Topoisomerase Inhibitors | Etoposide<br>Teniposide<br>Camptothecin<br>Topotecan<br>9-amino-campotothecin CPT-11 |
| | Antibiotics | Dactinomycin (actinomycin D)<br>Adriamycin<br>Daunorubicin (daunomycin; rubindomycin)<br><br>Doxorubicin<br>Bleomycin<br>Plicamycin (mithramycin)<br>Mitomycin (mitomycin C)<br>TAXOL<br>Taxotere |
| | Enzymes | L-Asparaginase |
| Natural Products | Biological Response Modifiers | Interfon alfa<br>Interleukin 2 |
| Miscellaneous Agents | Platinum Coordination Complexes | cis-diamminedichloroplatinum II (CDDP)<br>Carboplatin |
| | Anthracendione | Mitoxantrone |
| | Substituted Urea | Hydroxyurea |
| | Methyl Hydraxzine Derivative | Procarbazine (N-methylhydrazine,(MIH) |
| | Adrenocortical Suppressant | Mitotane (o,p'-DDD)<br>Aminoglutethimide |
| Hormones and Antagonists | Adrenocorticosteroids | Prednisone |
| | Progestins | Hydroxyprogesterone caproate<br>Medroxyprogesterone acetate<br>Megestrol acetate |
| | Estrogens | Diethylstilbestrol<br>Ethinyl estradiol |
| | Antiestrogen | Tamoxifen |
| | Androgens | Testosterone propionate<br>Fluoxymesterone |

(continued)

| CLASS | TYPE OF AGENT | NONPROPRIETARY NAMES (OTHER NAMES) |
|---|---|---|
| | Antiandrogen | Flutamide |
| | Gonadotropin-releasing Hormone analog | Leuprolide |

The agents tested in the present methods can be a single agent or a combination of agents. For example, the present methods can be used to determine whether a single chemotherapeutic agent, such as methotrexate, can be used to treat a cancer or whether a combination of two or more agents can be used in combination with a proteasome inhibitor. Preferred combinations will include agents that have different mechanisms of action, e.g., the use of an anti-mitotic agent in combination with an alkylating agent and a proteasome inhibitor.

The agents disclosed herein may be administered by any route, including intradermally, subcutaneously, orally, intraarterially or intravenously. Preferably, administration will be by the intravenous route. Preferably parenteral administration may be provided in a bolus or by infusion.

The concentration of a disclosed compound in a pharmaceutically acceptable mixture will vary depending on several factors, including the dosage of the compound to be administered, the pharmacokinetic characteristics of the compound(s) employed, and the route of administration. Effective amounts of agents for treating ischemia or reperfusion injury would broadly range between about 10 μg and about 50 mg per Kg of body weight of a recipient mammal. The agent may be administered in a single dose or in repeat doses. a recipient mammal. The agent may be administered in a single dose or in repeat doses. Treatments may be administered daily or more frequently depending upon a number of factors, including the overall health of a patient, and the formulation and route of administration of the selected compound(s).

Isolated Nucleic Acid Molecules, Vectors and Host Cells

One aspect of the invention pertains to isolated nucleic acid molecules that correspond to a predictive marker, including nucleic acids which encode a polypeptide corresponding to a predictive marker or a portion of such a polypeptide. Isolated nucleic acids also include nucleic acid molecules sufficient for use as hybridization probes to identify nucleic acid molecules that correspond to a predictive marker, including nucleic acids which encode a polypeptide corresponding to a predictive marker, and fragments of such nucleic acid molecules, e.g., those suitable for use as PCR primers for the amplification or mutation of nucleic acid molecules. As used herein, the term "nucleic acid molecule" is intended to include DNA molecules (*e.g.*, cDNA or genomic DNA) and RNA molecules (*e.g.*, mRNA) and analogs of the DNA or RNA generated using nucleotide analogs. The nucleic acid molecule can be single-stranded or double-stranded, but preferably is double-stranded DNA.

A nucleic acid molecule, *e.g.,* a nucleic acid encoding a protein corresponding to a marker listed in any one of Table 1, Table 2, and/or Table 3, can be isolated and manipulated (e.g., amplified, cloned, synthesized, etc.) using standard molecular biology techniques and the sequence information in the database records described herein. (*e.g.*, described in Sambrook et al., ed., Molecular Cloning: A Laboratory Manual, 2nd ed., Cold Spring Harbor Laboratory Press, Cold Spring Harbor, NY, 1989).

Moreover, a nucleic acid molecule can comprise only a portion of a nucleic acid sequence, wherein the full length nucleic acid sequence comprises a predictive marker of the invention or which encodes a polypeptide corresponding to a marker of the invention. Such nucleic acids can be used, for example, as a probe or primer. The probe/primer typically is used as one or more substantially purified oligonucleotides. The oligonucleotide typically comprises a region of nucleotide sequence that hybridizes under stringent conditions to at least about 7, preferably about 15, more preferably about 25, 50, 75, 100, 125, 150, 175, 200, 250, 300, 350, or 400 or more consecutive nucleotides.

Probes based on the sequence of a nucleic acid molecule can be used to detect transcripts or genomic sequences corresponding to one or more predictive markers. The probe comprises a label group attached thereto, e.g., a radioisotope, a fluorescent compound, an enzyme, or an enzyme co-factor. Such probes can be used as part of a diagnostic test kit for identifying cells or tissues which express the protein, such as by measuring levels of a nucleic acid molecule encoding the protein in a sample of cells from a subject, e.g., detecting mRNA levels or determining whether a gene encoding the protein has been mutated or deleted.

In addition to the nucleotide sequences described in the database records described herein, it will be appreciated by those skilled in the art that DNA sequence polymorphisms that lead to changes in the amino acid sequence can exist within a population (*e.g.*, the human population). Such genetic polymorphisms can exist among individuals within a

population due to natural allelic variation. An allele is one of a group of genes which occur alternatively at a given genetic locus. In addition, it will be appreciated that DNA polymorphisms that affect RNA expression levels can also exist that may affect the overall expression level of that gene (*e.g*., by affecting regulation or degradation).

As used herein, the terms "gene" and "recombinant gene" refer to nucleic acid molecules comprising an open reading frame encoding a polypeptide corresponding to a marker of the invention, including, e.g., sequences which differ, due to degeneracy of the genetic code, from the nucleotide sequence of nucleic acids encoding a protein which corresponds to a marker of the invention, and thus encode the same protein..

As used herein, the phrase "allelic variant" refers to a nucleotide sequence which occurs at a given locus or to a polypeptide encoded by the nucleotide sequence. Such natural allelic variations can typically result in 1-5% variance in the nucleotide sequence of a given gene. Alternative alleles can be identified by sequencing the gene of interest in a number of different individuals. This can be readily carried out by using hybridization probes to identify the same genetic locus in a variety of individuals. Any and all such nucleotide variations and resulting amino acid polymorphisms or variations that are the result of natural allelic variation and that do not alter the functional activity are intended to be within the scope of the invention.

Antisense nucleic acid molecules are molecules which are complementary to a sense nucleic acid, e.g., complementary to the coding strand of a double-stranded cDNA molecule corresponding to a marker or complementary to an mRNA sequence corresponding to a marker. Accordingly, an antisense nucleic acid can hydrogen bond to (*i.e.* anneal with) a sense nucleic acid. The antisense nucleic acid can be complementary to an entire coding strand, or to only a portion thereof, *e.g.,* all or part of the protein coding region (or open reading frame). An antisense nucleic acid molecule can also be antisense to all or part of a non-coding region of the coding strand of a nucleotide sequence encoding a polypeptide. The non-coding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45, or 50 or more nucleotides in length. An antisense nucleic acid of the invention can be constructed using chemical synthesis and enzymatic ligation reactions using procedures known in the art. For example, an antisense nucleic acid (*e.g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been sub-cloned in an antisense orientation (*i.e.,* RNA transcribed from the inserted nucleic acid will be of an antisense orientation to a target nucleic acid of interest, described further in the following subsection).

In various embodiments, the nucleic acid molecules can be modified at the base moiety, sugar moiety or phosphate backbone to improve, *e.g*., the stability, hybridization, or solubility of the molecule. For example, the deoxyribose phosphate backbone of the nucleic acids can be modified to generate peptide nucleic acids (see Hyrup et al., 1996, Bioorganic & Medicinal Chemistry 4(1): 5-23). As used herein, the terms "peptide nucleic acids" or "PNAs" refer to nucleic acid mimics, *e.g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup *et al.* (1996), *supra*; Perry-O'Keefe et al. (1996) Proc. Natl. Acad. Sci. USA 93:14670-675.

PNAs can be used in therapeutic and diagnostic applications. For example, PNAs can be used, *e.g.,* in the analysis of single base pair mutations in a gene by, *e.g.,* PNA directed PCR clamping; as artificial restriction enzymes when used in combination with other enzymes, *e.g.,* S1 nucleases (Hyrup (1996), *supra*; or as probes or primers for DNA sequence and hybridization (Hyrup, 1996, *supra*; Perry-O'Keefe et al., 1996, Proc. Natl. Acad. Sci. USA 93:14670-675).

In another aspect, PNAs can be modified, *e.g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which can combine the advantageous properties of PNA and DNA. Such chimeras allow DNA recognition enzymes, *e.g*., RNASE H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup, 1996, *supra*). The synthesis of PNA-DNA chimeras can be performed as described in Hyrup (1996), *supra,* and Finn et al. (1996) Nucleic Acids Res. 24(17):3357-63. For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al., 1989, Nucleic Acids Res. 17:5973-88). PNA monomers are then coupled in a step-wise manner to produce a chimeric molecule with a 5' PNA segment and a 3' DNA segment (Finn et al., 1996, Nucleic Acids Res. 24(17):3357-63). Alternatively, chimeric molecules can be synthesized with a 5' DNA segment and a 3' PNA segment (Peterser et al., 1975, Bioorganic Med. Chem. Lett. 5:1119-11124).

In other embodiments, the oligonucleotide can include other appended groups such as peptides (*e.g.,* for targeting host cell receptors *in vivo*), or agents facilitating transport across the cell membrane (see, *e.g.,* Letsinger et al., 1989, Proc. Natl. Acad. Sci. USA 86:6553-6556; Lemaitre et al., 1987, Proc. Natl. Acad. Sci. USA 84:648-652; PCT Publication No. WO 88/09810) or the blood-brain barrier (see, *e.g.,* PCT Publication No. WO 89/10134). In addition, oligonucleotides can be modified with hybridization-triggered cleavage agents (see, *e.g.,* Krol et al., 1988, Bio/Techniques 6:958-976) or intercalating agents (see, *e.g.,* Zon, 1988, Pharm. Res. 5:539-549). To this end, the oligonucleotide can be conjugated to another molecule, e.g., a peptide, hybridization triggered cross-linking agent, transport agent, hybridization-triggered cleavage agent, etc.

Molecular beacon nucleic acids having at least one region which is complementary to a marker are useful for quantitating the presence of the predictive marker in a sample. A "molecular beacon" nucleic acid is a nucleic acid comprising a pair of complementary regions and having a fluorophore and a fluorescent quencher associated therewith. The fluorophore and quencher are associated with different portions of the nucleic acid in such an orientation that when the complementary regions are annealed with one another, fluorescence of the fluorophore is quenched by the quencher. When the complementary regions of the nucleic acid are not annealed with one another, fluorescence of the fluorophore is quenched to a lesser degree. Molecular beacon nucleic acids are described, for example, in U.S. Patent 5,876,930.

Vectors, preferably expression vectors, containing a nucleic acid encoding a polypeptide corresponding to a predictive marker can be used for production of nucleic acid and proteins corresponding to predictive markers; as well as for production of compositions relating to the predictive markers. Useful vectors further comprise promoter and/or regulatory sequences for effective expression of the nucleic acid and/or protein corresponding to the predictive marker of interest. In certain instances, promoters can include constitutive promoter/regulatory sequences, inducible promoter/regulatory sequences, tissue specific promoter/regulatory sequences, or the natural endogenous promoter/regulatory sequences corresponding to the predictive marker of interest, as required. Various expression vectors are well known in the art and can be adapted to suit the particular system for expression. For example, recombinant expression vectors can be designed for expression of a polypeptide corresponding to a marker of the invention in prokaryotic (*e.g*., *E. coli*) or eukaryotic cells ( e.g., insect cells {using baculovirus expression vectors}, yeast cells or mammalian cells). Suitable host cells are discussed further in Goeddel, *supra.* Alternatively, the recombinant expression vector can be transcribed and translated *in vitro,* for example using T7 promoter regulatory sequences and T7 polymerase.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue-specific manner.

A "constitutive" promoter is a nucleotide sequence which, when operably linked with a polynucleotide which encodes or specifies a gene product, causes the gene product to be produced in a living human cell under most or all physiological conditions of the cell.

An "inducible" promoter is a nucleotide sequence which, when operably linked with a polynucleotide which encodes or specifies a gene product, causes the gene product to be produced in a living human cell substantially only when an inducer which corresponds to the promoter is present in the cell.

A "tissue-specific" promoter is a nucleotide sequence which, when operably linked with a polynucleotide which encodes or specifies a gene product, causes the gene product to be produced in a living human cell substantially only if the cell is a cell of the tissue type corresponding to the promoter.

Host cells into which a recombinant expression vector has been introduced are described herein. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. A host cell can be any prokaryotic (*e.g., E coli*) or eukaryotic cell (*e.g*., insect cells, yeast or mammalian cells).

Vector DNA can be introduced into prokaryotic or eukaryotic cells via conventional transformation or transfection

techniques. As used herein, the terms "transformation" and "transfection" are intended to refer to a variety of art-recognized techniques for introducing foreign nucleic acid into a host cell, including calcium phosphate or calcium chloride co-precipitation, DEAE-dextran-mediated transfection, lipofection, or electroporation. Suitable methods for transforming or transfecting host cells can be found in Sambrook, *et al.* (*supra*), and other laboratory manuals.

A host cell, such as a prokaryotic or eukaryotic host cell in culture, can be used to produce a polypeptide corresponding to a marker. Accordingly, methods for producing a polypeptide corresponding to a marker using the host cells are described herein. In one embodiment, the method comprises culturing the host cell (into which a recombinant expression vector encoding a polypeptide has been introduced) in a suitable medium such that the marker is produced. In another embodiment, the method further comprises isolating the marker polypeptide from the medium or the host cell.

<u>Isolated Proteins and Antibodies</u>

Isolated proteins which correspond to predictive markers, and biologically active portions thereof are described herein, as well as polypeptide fragments suitable for use as immunogens to raise antibodies directed against a polypeptide corresponding to a predictive marker. Polypeptides can be isolated, purified, or produced using the gene identification information provided herein in combination with routine molecular biology, protein purification and recombinant DNA techniques well known in the art.

Biologically active portions of a polypeptide corresponding to a marker include polypeptides comprising amino acid sequences sufficiently identical to or derived from the amino acid sequence of the protein corresponding to the predictive marker, which include fewer amino acids than the full length protein, and exhibit at least one activity of the corresponding full-length protein. Typically, biologically active portions comprise a domain or motif with at least one activity of the corresponding protein. A biologically active portion of a protein of the invention can be a polypeptide which is, for example, 10, 25, 50, 100 or more amino acids in length. Moreover, other biologically active portions, in which other regions of the protein are deleted, can be prepared by recombinant techniques and evaluated for one or more of the functional activities of the native form of a polypeptide of the invention.

Preferred polypeptides have the amino acid sequence listed in the one of the GenBank and NUC database records described herein. Other useful proteins are substantially identical (*e.g.*, at least about 50%, preferably 70%, 80%, 90%, 95%, or 99%) to one of these sequences and retain the functional activity of the protein of the corresponding naturally-occurring protein yet differ in amino acid sequence due to natural allelic variation or mutagenesis.

The determination of percent identity between two sequences can be accomplished using a mathematical algorithm determining the number of identical positions shared between two sequences. Determination can be carried out using any known method in the art for comparison of identity and similarity. Examples of methods used can include for example, a mathematical algorithm utilized for the comparison of two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. Such an algorithm is incorporated into the NBLAST and XBLAST programs of Altschul, et al. (1990) J. Mol. Biol. 215:403-410. BLAST nucleotide searches can be performed with the NBLAST program, score = 100, wordlength = 12 to obtain nucleotide sequences homologous to a nucleic acid molecules of the invention. BLAST protein searches can be performed with the XBLAST program, score = 50, wordlength = 3 to obtain amino acid sequences homologous to a protein molecules of the invention. To obtain gapped alignments for comparison purposes, Gapped BLAST can be utilized as described in Altschul et al. (1997) Nucleic Acids Res. 25:3389-3402. Alternatively, PSI-Blast can be used to perform an iterated search which detects distant relationships between molecules. When utilizing BLAST, Gapped BLAST, and PSI-Blast programs, the default parameters of the respective programs (*e.g.*, XBLAST and NBLAST) can be used. See http://www.ncbi.nlm.nih.gov. Another example of a mathematical algorithm utilized for the comparison of sequences is the algorithm of Myers and Miller, (1988) CABIOS 4:11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson and Lipman (1988) Proc. Natl. Acad. Sci. USA 85:2444-2448. When using the FASTA algorithm for comparing nucleotide or amino acid sequences, a PAM120 weight residue table can, for example, be used with a *k*-tuple value of 2. The percent identity between two sequences can be determined using techniques similar to those described above, with or without allowing gaps. In calculating percent identity, only exact matches are counted.

Chimeric or fusion proteins corresponding to a marker are described herein. As used herein, a "chimeric protein" or "fusion protein" comprises all or part (preferably a biologically active part) of a polypeptide corresponding to a marker of the invention operably linked to a heterologous polypeptide (*i.e.*, a polypeptide other than the polypeptide corresponding to the marker). Within the fusion protein, the term "operably linked" is intended to indicate that the polypeptide of the invention and the heterologous polypeptide are fused in-frame to each other. The heterologous polypeptide can be fused to the amino-terminus or the carboxyl-terminus of the polypeptide of the invention. Useful fusion proteins can include

GST, c-myc, FLAG, HA, and any other well known heterologous tag for use in fusion protein production. Such fusion proteins can facilitate the purification of a recombinant polypeptide of the invention.

In addition, fusion proteins can include a signal sequence from another protein such as gp67, melittin, human placental alkaline phosphatase, and phoA. In yet another aspect, the fusion protein is an immunoglobulin fusion protein in which all or part of a polypeptide corresponding to a predictive marker of the invention is fused to sequences derived from a member of the immunoglobulin protein family. The immunoglobulin fusion proteins of the invention can be used as immunogens to produce antibodies directed against a polypeptide of the invention in a subject, to purify ligands and in screening assays to identify molecules which inhibit the interaction of receptors with ligands.

An isolated polypeptide corresponding to a predictive marker, or a fragment thereof, can be used as an immunogen to generate antibodies using standard techniques for polyclonal and monoclonal antibody preparation. For example, an immunogen typically is used to prepare antibodies by immunizing a suitable (*i.e*. immunocompetent) subject such as a rabbit, goat, mouse, or other mammal or vertebrate. An appropriate immunogenic preparation can contain, for example, recombinantly-expressed or chemically-synthesized polypeptide. The preparation can further include an adjuvant, such as Freund's complete or incomplete adjuvant, or a similar immunostimulatory agent.

Accordingly, antibodies directed against a polypeptide are referred to herein. The terms "antibody" and "antibody substance" as used interchangeably herein refer to immunoglobulin molecules and immunologically active portions of immunoglobulin molecules, *i.e.,* molecules that contain an antigen binding site which specifically binds an antigen, such as a polypeptide of the invention, e.g., an epitope of a polypeptide of the invention. A molecule which specifically binds to a given polypeptide is a molecule which binds the polypeptide, but does not substantially bind other molecules in a sample, e.g., a biological sample, which naturally contains the polypeptide. Examples of immunologically active portions of immunoglobulin molecules include F(ab) and $F(ab')_2$ fragments which can be generated by treating the antibody with an enzyme such as pepsin. Polyclonal and monoclonal antibodies are referred to herein.

Polyclonal antibodies can be prepared as described above by immunizing a suitable subject with a polypeptide of the invention as an immunogen. Preferred polyclonal antibody compositions are ones that have been selected for antibodies directed against a predictive marker or markers. The antibody titer in the immunized subject can be monitored over time by standard techniques, such as with an enzyme linked immunosorbent assay (ELISA) using immobilized polypeptide. If desired, the antibody molecules can be harvested or isolated from the subject (e.g., from the blood or serum of the subject) and further purified by well-known techniques, such as protein A chromatography to obtain the IgG fraction.

Alternatively, antibodies specific for a protein or polypeptide can be selected or (*e.g*., partially purified) or purified by, *e.g.,* affinity chromatography to obtain substantially purified and purified antibody. By a substantially purified antibody composition is meant, in this context, that the antibody sample contains at most only 30% (by dry weight) of contaminating antibodies directed against epitopes other than those of the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

Additionally, monoclonal antibodies directed to the predictive markers can be prepared. Methods for generation of monoclonal antibodies are well known in the art and can be produced using any method. For example, at an appropriate time after immunization, *e.g*., when the specific antibody titers are highest, antibody-producing cells can be obtained from the subject and used to prepare monoclonal antibodies by standard techniques, such as the hybridoma technique originally described by Kohler and Milstein (1975) Nature 256:495-497, the human B cell hybridoma technique (see Kozbor et al., 1983, Immunol. Today 4:72), the EBV-hybridoma technique (see Cole et al., pp. 77-96 In Monoclonal Antibodies and Cancer Therapy, Alan R. Liss, Inc., 1985) or trioma techniques. The technology for producing hybridomas is well known (see generally Current Protocols in Immunology, Coligan et al. ed., John Wiley & Sons, New York, 1994). Hybridoma cells producing a monoclonal antibody of the invention are detected by screening the hybridoma culture supernatants for antibodies that bind the polypeptide of interest, *e.g*., using a standard ELISA assay.

Additionally, recombinant antibodies, such as chimeric and humanized monoclonal antibodies, comprising both human and non-human portions, can be made using standard recombinant DNA techniques. A chimeric antibody is a molecule in which different portions are derived from different animal species, such as those having a variable region derived from a murine mAb and a human immunoglobulin constant region. (See, *e.g.,* Cabilly et al., U.S. Patent No. 4,816,567; and Boss et al., U.S. Patent No. 4,816,397.) Humanized antibodies are antibody molecules from non-human species having one or more complementarily determining regions (CDRs) from the non-human species and a framework region from a human immunoglobulin molecule. (See, *e.g*., Queen, U.S. Patent No. 5,585,089, which is incorporated herein by reference in its entirety.) Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al. (1988) Science 240:1041-1043; Liu et al. (1987) Proc. Natl. Acad. Sci. USA 84:3439-3443; Liu et al. (1987) J. Immunol. 139:3521- 3526;

Sun et al. (1987) Proc. Natl. Acad. Sci. USA 84:214-218; Nishimura et al. (1987) Cancer Res. 47:999-1005; Wood et al. (1985) Nature 314:446-449; and Shaw et al. (1988) J. Natl. Cancer Inst. 80:1553-1559); Morrison (1985) Science 229:1202-1207; Oi et al. (1986) Bio/Techniques 4:214; U.S. Patent 5,225,539; Jones et al. (1986) Nature 321:552-525; Verhoeyan et al. (1988) Science 239:1534; and Beidler et al. (1988) J. Immunol. 141:4053-4060.

Human antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, e.g., all or a portion of a polypeptide corresponding to a marker of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar (1995) Int. Rev. Immunol. 13:65-93). For a detailed discussion of this technology for producing human antibodies and human monoclonal antibodies and protocols for producing such antibodies, see, *e.g.,* U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a murine antibody, is used to guide the selection of a completely human antibody recognizing the same epitope (Jespers et al., 1994, Bio/technology 12:899-903).

An antibody directed against a polypeptide corresponding to a predictive marker (*e.g.*, a monoclonal antibody) can be used to detect the predictive marker (*e.g.*, in a cellular sample) in order to evaluate the level and pattern of expression of the predictive marker. The antibodies can also be used diagnostically to monitor protein levels in tissues or body fluids (*e.g.* in an tumor sample) as part of a clinical testing procedure, *e.g.*, to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}I$, $^{131}I$, $^{35}S$ or $^{3}H$.

Further, an antibody (or fragment thereof) can be conjugated to a therapeutic moiety such as a cytotoxin, a therapeutic agent or a radioactive metal ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (*e.g.*, methotrexate, 6-mercaptopurine, 6-thioguanine, cytarabine, 5-fluorouracil decarbazine), alkylating agents (*e.g.*, mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.*, daunorubicin (formerly daunomycin) and doxorubicin), antibiotics *( e.g.,* dactinomycin (formerly actinomycin), bleomycin, mithramycin, and anthramycin (AMC)), and anti-mitotic agents *(e.g.,* vincristine and vinblastine).

Techniques for conjugating such therapeutic moiety to antibodies are well known, see, *e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described by Segal in U.S. Patent No. 4,676,980.

Accordingly, substantially purified antibodies or fragments thereof, and non-human antibodies or fragments thereof are referred to herein, which antibodies or fragments specifically bind to a polypeptide comprising an amino acid sequence encoded by a predictive marker identified herein. In various embodiments, the substantially purified antibodies, or fragments thereof, can be human, non-human, chimeric and/or humanized antibodies.

Non-human antibodies or fragments thereof are referred to herein, which antibodies or fragments specifically bind to a polypeptide comprising an amino acid sequence which is encoded by a nucleic acid molecule of a predictive marker. Such non-human antibodies can be goat, mouse, sheep, horse, chicken, rabbit, or rat antibodies. Alternatively, the non-human antibodies can be chimeric and/or humanized antibodies. In addition, the non-human antibodies can be polyclonal antibodies or monoclonal antibodies.

Monoclonal antibodies or fragments thereof are described herein, which antibodies or fragments specifically bind to a polypeptide comprising an amino acid sequence selected from the group consisting of the amino acid sequences of the present invention, an amino acid sequence encoded by the cDNA of the present invention, a fragment of at least 15 amino acid residues of an amino acid sequence of the present invention, an amino acid sequence which is at least 95% identical to an amino acid sequence of the present invention (wherein the percent identity is determined using the ALIGN program of the GCG software package with a PAM120 weight residue table, a gap length penalty of 12, and a gap penalty of 4) and an amino acid sequence which is encoded by a nucleic acid molecule which hybridizes to a nucleic acid molecule consisting of the nucleic acid molecules of the present invention, or a complement thereof, under conditions of hybridization of 6X SSC at 45°C and washing in 0.2 X SSC, 0.1% SDS at 65°C. The monoclonal antibodies can be human, humanized, chimeric and/or non-human antibodies.

The substantially purified antibodies or fragments thereof may specifically bind to a signal peptide, a secreted sequence, an extracellular domain, a transmembrane or a cytoplasmic domain or cytoplasmic membrane of a polypeptide of the invention. In a particularly preferred embodiment, the substantially purified antibodies or fragments thereof, the non-human antibodies or fragments thereof, and/or the monoclonal antibodies or fragments thereof, of the invention specifically bind to a secreted sequence or an extracellular domain of the amino acid sequences.

Screening Assays

Methods are described herein (also referred to herein as "screening assays") for identifying modulators, *i.e.,* candidate or test compounds or agents (*e.g.,* peptides, peptidomimetics, peptoids, small molecules or other drugs) which (a) bind to the marker, or (b) have a modulatory (e.g., stimulatory or inhibitory) effect on the activity of the marker or, more specifically, (c) have a modulatory effect on the interactions of the marker with one or more of its natural substrates (*e.g.*, peptide, protein, hormone, co-factor, or nucleic acid), or (d) have a modulatory effect on the expression of the marker. Such assays typically comprise a reaction between the marker and one or more assay components. The other components may be either the test compound itself, or a combination of test compound and a natural binding partner of the marker.

Test compounds may be obtained from any available source, including systematic libraries of natural and/or synthetic compounds. Test compounds may also be obtained by any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, *e.g.,* Zuckermann et al., 1994, J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, 1997, Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (*e.g.*, Houghten, 1992, Biotechniques 13:412-421), or on beads (Lam, 1991, Nature 354:82-84), chips (Fodor, 1993, Nature 364:555-556), bacteria and/or spores, (Ladner, USP 5,223,409), plasmids (Cull et al, 1992, Proc Natl Acad Sci USA 89:1865-1869) or on phage (Scott and Smith, 1990, Science 249:386-390; Devlin, 1990, Science 249:404-406; Cwirla et al, 1990, Proc. Natl. Acad. Sci. 87:6378-6382; Felici, 1991, J. Mol. Biol. 222:301-310; Ladner, *supra*.).

Assays are described herein for screening candidate or test compounds which are substrates of a marker or biologically active portion thereof. In addition assays are described herein for screening candidate or test compounds which bind to a marker or biologically active portion thereof. Determining the ability of the test compound to directly bind to a marker can be accomplished, for example, by coupling the compound with a radioisotope or enzymatic label such that binding of the compound to the marker can be determined by detecting the labeled marker compound in a complex. For example, compounds (*e.g.*, marker substrates) can be labeled with $^{125}I$, $^{35}S$, $^{14}C$, or $^{3}H$, either directly or indirectly, and the radioisotope detected by direct counting of radioemission or by scintillation counting. Alternatively, assay components can be enzymatically labeled with, for example, horseradish peroxidase, alkaline phosphatase, or luciferase, and the enzymatic label detected by determination of conversion of an appropriate substrate to product.

Assays are described herein for screening candidate or test compounds which modulate the activity of a marker or a biologically active portion thereof. In all likelihood, the marker can, *in vivo,* interact with one or more molecules, such as but not limited to, peptides, proteins, hormones, cofactors and nucleic acids. For the purposes of this discussion, such cellular and extracellular molecules are referred to herein as "binding partners" or marker "substrate". In order to facilitate such screening the marker is used to identify its natural *in vivo* binding partners. Many of the known binding partners or substrates of the identified predictive markers are either known in the art, or can be identified using standard methodologies known in the art (e.g., two hybrid screening, etc.).

Assays may be devised for the purpose of identifying compounds which modulate (*e.g.*, affect either positively or negatively) interactions between a marker and its substrates and/or binding partners. Such compounds can include, but are not limited to, molecules such as antibodies, peptides, hormones, oligonucleotides, nucleic acids, and analogs thereof. Such compounds may also be obtained from any available source, including systematic libraries of natural and/or synthetic compounds. The preferred assay components for use is an predictive marker identified herein, the known binding partner and/or substrate of same, and the test compound. Test compounds can be supplied from any source.

The basic principle of the assay systems used to identify compounds that interfere with the interaction between the marker and its binding partner involves preparing a reaction mixture containing the marker and its binding partner under conditions and for a time sufficient to allow the two products to interact and bind, thus forming a complex. In order to test an agent for inhibitory activity, the reaction mixture is prepared in the presence and absence of the test compound. The test compound can be initially included in the reaction mixture, or can be added at a time subsequent to the addition of the marker and its binding partner. Control reaction mixtures are incubated without the test compound or with a placebo. The formation of any complexes between the marker and its binding partner is then detected. The formation of a complex in the control reaction, but less or no such formation in the reaction mixture containing the test compound, indicates that the compound interferes with the interaction of the marker and its binding partner. Conversely, the formation of more complex in the presence of compound than in the control reaction indicates that the compound may enhance interaction of the marker and its binding partner.

The assay for compounds that interfere with the interaction of the marker with its binding partner may be conducted in a heterogeneous or homogeneous format. Heterogeneous assays involve anchoring either the marker or its binding partner onto a solid phase and detecting complexes anchored to the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the markers and the binding partners (*e.g.*, by competition) can be identified by conducting the reaction in the presence of the test substance, *i.e.,* by adding the test substance to the reaction mixture prior to or simultaneously with the marker and its interactive binding partner. Alternatively, test compounds that disrupt preformed complexes, *e.g.*, compounds with higher binding constants that displace one of the components from the complex, can be tested by adding the test compound to the reaction mixture after complexes have been formed. The various formats are briefly described below.

In a heterogeneous assay system, either the marker or its binding partner is anchored onto a solid surface or matrix, while the other corresponding non-anchored component may be labeled, either directly or indirectly. In practice, microtitre plates are often utilized for this approach. The anchored species can be immobilized by a number of methods, either non-covalent or covalent, that are typically well known to one who practices the art. Non-covalent attachment can often be accomplished simply by coating the solid surface with a solution of the marker or its binding partner and drying. Alternatively, an immobilized antibody specific for the assay component to be anchored can be used for this purpose. Such surfaces can often be prepared in advance and stored.

In related embodiments, a fusion protein can be provided which adds a domain that allows one or both of the assay components to be anchored to a matrix. For example, glutathione-S-transferase/marker fusion proteins or glutathione-S-transferase/binding partner can be adsorbed onto glutathione sepharose beads (Sigma Chemical, St. Louis, MO) or glutathione derivatized microtiter plates, which are then combined with the test compound or the test compound and either the non-adsorbed marker or its binding partner, and the mixture incubated under conditions conducive to complex formation (*e.g.*, physiological conditions). Following incubation, the beads or microtiter plate wells are washed to remove any unbound assay components, the immobilized complex assessed either directly or indirectly, for example, as described above. Alternatively, the complexes can be dissociated from the matrix, and the level of marker binding or activity determined using standard techniques.

Other techniques for immobilizing proteins on matrices can also be used in the screening assays. For example, either a marker or a marker binding partner can be immobilized utilizing conjugation of biotin and streptavidin. Biotinylated marker protein or target molecules can be prepared from biotin-NHS (N-hydroxy-succinimide) using techniques known in the art (*e.g.*, biotinylation kit, Pierce Chemicals, Rockford, IL), and immobilized in the wells of streptavidin-coated 96 well plates (Pierce Chemical). In certain embodiments, the protein-immobilized surfaces can be prepared in advance and stored.

In order to conduct the assay, the corresponding partner of the immobilized assay component is exposed to the coated surface with or without the test compound. After the reaction is complete, unreacted assay components are removed (*e.g.*, by washing) and any complexes formed will remain immobilized on the solid surface. The detection of complexes anchored on the solid surface can be accomplished in a number of ways. Where the non-immobilized component is pre-labeled, the detection of label immobilized on the surface indicates that complexes were formed. Where the non-immobilized component is not pre-labeled, an indirect label can be used to detect complexes anchored on the surface; *e.g.*, using a labeled antibody specific for the initially non-immobilized species (the antibody, in turn, can be directly labeled or indirectly labeled with, *e.g.*, a labeled anti-Ig antibody). Depending upon the order of addition of reaction components, test compounds which modulate (inhibit or enhance) complex formation or which disrupt preformed complexes can be detected.

A homogeneous assay may be used. This is typically a reaction, analogous to those mentioned above, which is conducted in a liquid phase in the presence or absence of the test compound. The formed complexes are then separated from unreacted components, and the amount of complex formed is determined. As mentioned for heterogeneous assay systems, the order of addition of reactants to the liquid phase can yield information about which test compounds modulate (inhibit or enhance) complex formation and which disrupt preformed complexes.

In such a homogeneous assay, the reaction products may be separated from unreacted assay components by any of a number of standard techniques, including but not limited to: differential centrifugation, chromatography, electrophoresis and immunoprecipitation. In differential centrifugation, complexes of molecules may be separated from uncomplexed molecules through a series of centrifugal steps, due to the different sedimentation equilibria of complexes based on their different sizes and densities (see, for example, Rivas, G., and Minton, A.P., Trends Biochem Sci 1993 Aug;18(8):284-7). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the complex as compared to the uncomplexed molecules may be exploited to differentially separate the complex from the remaining individual reactants, for example through the use of ion-exchange chromatography resins. Such resins and chromatographic techniques are well known to one skilled in the art (see, *e.g.,* Heegaard, 1998, J Mol. Recognit. 11:141-148; Hage and Tweed, 1997, J. Chromatogr. B. Biomed. Sci. Appl., 699:499-525). Gel electrophoresis may also be employed to separate complexed molecules from unbound species (see, *e.g.,* Ausubel et al (eds.), In: Current Protocols in Molecular Biology, J. Wiley & Sons, New York. 1999). In this technique, protein or nucleic acid complexes are separated based on size or charge, for example. In order to maintain the binding interaction during the electrophoretic process, nondenaturing gels in the absence of reducing agent are typically preferred, but conditions appropriate to the particular interactants will be well known to one skilled in the art. Immunoprecipitation is another common technique utilized for the isolation of a protein-protein complex from solution (see, *e.g.,* Ausubel et al (eds.), In: Current Protocols in Molecular Biology, J. Wiley & Sons, New York. 1999). In this technique, all proteins binding to an antibody specific to one of the binding molecules are precipitated from solution by conjugating the antibody to a polymer bead that may be readily collected by centrifugation. The bound assay components are released from the beads (through a specific proteolysis event or other technique well known in the art which will not disturb the protein-protein interaction in the complex), and a second immunoprecipitation step is performed, this time utilizing antibodies specific for the correspondingly different interacting assay component. In this manner, only formed complexes should remain attached to the beads. Variations in complex formation in both the presence and the absence of a test compound can be compared, thus offering information about the ability of the compound to modulate interactions between the marker and its binding partner.

Methods are described herein for direct detection of interactions between the marker and its natural binding partner and/or a test compound in a homogeneous or heterogeneous assay system without further sample manipulation. For example, the technique of fluorescence energy transfer may be utilized (see, *e.g.,* Lakowicz et al, U.S. Patent No. 5,631,169; Stavrianopoulos et al, U.S. Patent No. 4,868,103). Generally, this technique involves the addition of a fluorophore label on a first 'donor' molecule (*e.g.,* marker or test compound) such that its emitted fluorescent energy will be absorbed by a fluorescent label on a second, 'acceptor' molecule (*e.g.*, marker or test compound), which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (*e.g.*, using a fluorimeter). A test substance which either enhances or hinders participation of one of the species in the preformed complex will result in the generation of a signal variant to that of background. In this way, test substances that modulate interactions between a marker and its binding partner can be identified in controlled assays.

Modulators of marker expression are identified in a method wherein a cell is contacted with a candidate compound and the expression of mRNA or protein, corresponding to a marker in the cell, is determined. The level of expression of mRNA or protein in the presence of the candidate compound is compared to the level of expression of mRNA or protein in the absence of the candidate compound. The candidate compound can then be identified as a modulator of marker expression based on this comparison. For example, when expression of marker mRNA or protein is greater (statistically significantly greater) in the presence of the candidate compound than in its absence, the candidate compound is identified as a stimulator of marker mRNA or protein expression. Conversely, when expression of marker mRNA or protein is less (statistically significantly less) in the presence of the candidate compound than in its absence, the candidate compound is identified as an inhibitor of marker mRNA or protein expression. The level of marker mRNA or protein expression in the cells can be determined by methods described herein for detecting marker mRNA or protein.

Still futher, in cell based assays, where a cell expressing a predictive marker of interest is used for screening therapeutic candidate agents, the activity or viability of the cell is monitored to determine the ability of the test compound to alter the activity of the predictive marker or markers. Such assays are carried in tandem with a control assay utilizing similar or identical cell lines which do not express the predictive marker or markers of interest, in order to determine specificity of the action of the test compound.

Two or more of the assays described herein can be used. For example, a modulating agent can be identified using a cell-based or a cell free assay, and the ability of the agent to modulate the activity of a marker protein can be further confirmed *in vivo, e.g.,* in a whole animal model for cellular transformation and/or tumorigenesis.

An agent identified as described herein (*e.g.*, an marker modulating agent, an antisense marker nucleic acid molecule, an marker-specific antibody, or an marker-binding partner) can be used in an animal model to determine the efficacy, toxicity, or side effects of treatment with such an agent. Alternatively, an agent identified as described herein can be used in an animal model to determine the mechanism of action of such an agent.

**SPECIFIC EXAMPLES**

<u>Treatment Dosage and Administration</u>

Drug Supply and Storage

Bortezomib for injection (VELCADE™ Millennium Pharmaceuticals, Inc., Cambridge, MA), a sterile lyophilized powder for reconstitution, was supplied in vials containing 2.5 mg bortezomib and 25 mg mannitol USP. Each vial was reconstituted with 2.5 mL of normal (0.9%) saline, Sodium Chloride Injection USP, such that the reconstituted solution contained bortezomib at a concentration of 1 mg/mL. The reconstituted solution was clear and colorless with a final pH between 5 and 6. Vials containing lyophilized bortezomib for Injection were stored refrigerated at 2 to 8°C.

**TABLE B Drug Information**

| | |
|---|---|
| Chemical Name | N-Pyrazinecarbonyl-L-phenylalanine-L-leucineboronic acid |
| Research Name | MLN341 or PS-341 |
| Generic Name | bortezomib |
| Proprietary Name | VELCADE™ |
| CAS Registry Number | 179324-69-7 |
| U.S. Patent Number | 5,780,454 |
| Classification | Proteasome Inhibitor |
| Molecular Formula | $C_{19}H_{25}BN_4O_4$ |
| Molecular Weight | 384.25 |
| Structure | Boronic acid derivative of a leucine phenylalanine dipeptide |

<u>An Open-Label Phase II Study of Bortezomib in Patients with Myeloma Who Have Relapsed Following Front-line Therapy and are Refractory to their Most Recent Therapy</u>

**Pharmacodynamic/pharmacogenomic/pharmacokinetic data collected**

A multicenter, open-label, non-randomized Phase 2 trial was conducted, wherein enrolled were patients with relapsed myeloma that was refractory to therapy. Patients were treated with 1.3 mg of bortezomib per square meter of

body surface area, twice weekly for two weeks, followed by one week without treatment, for up to eight cycles (24 weeks).

The following evaluations were conducted to assess the pharmacodynamics and pharmacogenomics of bortezomib:.

Proteasome inhibition assay (blood for this *ex vivo* assay was collected before and one hour after dosing on Day 1 and Day 11 of Cycles 1, 7, and, if applicable, the cycle in which dexamethasone was started and one hour after dosing on Day 11 of Cycle 8). Some patients had an additional sample collected for the proteasome inhibition assay at 24 hours after dosing on Day 1, Cycle 1.

Pharmacogenomic data (blood and bone marrow samples for evaluation of the expression of global mRNA levels; these procedures were conducted only in patients who consented to participate via a separate consent form).

Population pharmacokinetics (blood for determination of population pharmacokinetics was collected from all patients before and one to six hours after study drug administration on Day 1, Cycle 1, and before and one to six hours after study drug administration on Day 11 of Cycles 1, 2, 7, and 8 and, if applicable, the cycle in which dexamethasone was started). Pre-dose blood samples were collected at the same time as those for clinical laboratory evaluations.

Individual pharmacokinetics: blood for determination of plasma bortezomib levels was collected immediately before and at 2, 5, 10, 15, 30, 60, and 120 minutes and 24 hours after bortezomib administration on Day 1, Cycle 1.

Statistical procedures

Statistical analysis focused on the need to estimate response rates within specified limits of accuracy in order to determine if either of the two dose levels 1.0 or 1.3 $mg/m^2$/dose alone or in combination with dexamethasone are sufficiently efficacious to warrant further clinical study. This study was noncomparative in nature; therefore efficacy comparisons between the two doses of bortezomib were not performed. In addition, this study provided safety data that helped to characterize the potential toxicity of treatment at the two evaluated dose levels for up to eight cycles of therapy.

Summary tabulations were presented that displayed the number of observations, mean, standard deviation, median, minimum, and maximum for continuous variables, and the number and percent per category for categorical data. The categories for summarization were the two assigned dose groups.

A formal statistical analysis plan was developed and finalized prior to database lock. The primary efficacy analyses were performed on the intent-to-treat (ITT) population. The primary efficacy analysis were performed on the rates of responders, where a responder was defined as a CR, PR, or MR using the criteria prospectively established in Table C. Two-sided 90% confidence limits on proportions of responders in each dose group were established, corresponding to a 95% one-sided lower limit.

**Table C Disease Response Criteria[1]**

| Response | Criteria for response |
|---|---|
| Complete response (CR)[2] | Requires all of the following:<br>Disappearance of the original monoclonal protein from the blood and urine on at least two determinations for a minimum of six weeks by immunofixation studies.<br>< 5% plasma cells in the bone marrow on at least two determinations<br>for a minimum of six weeks.<br>No increase in the size or number of lytic bone lesions (development of a compression fracture does not exclude response).<br>Disappearance of soft tissue plasmacytomas for at least six weeks. |
| Partial response (PR)[3] | PR includes patients in whom some, but not all, criteria for CR are fulfilled providing the remaining criteria satisfy the requirements for PR.<br>Requires all of the following:<br>≥50% reduction in the level of serum monoclonal protein for at least two determinations six weeks apart.<br>If present, reduction in 24-hour urinary light chain excretion by either ≥90% or to < 200 mg for at least two determinations six weeks apart.<br>≥ 50% reduction in the size of soft tissue plasmacytomas (by clinical or radiographic examination) for at least six weeks.<br>No increase in size or number of lytic bone lesions (development of compression fracture does not exclude response). |

(continued)

| Response | Criteria for response |
|---|---|
| Minimal response (MR) | MR includes patients in whom some, but not all, criteria for PR are fulfilled providing the remaining criteria satisfy the requirements for MR.<br>Requires all of the following:<br>≥25% to ≤ 49% reduction in the level of serum monoclonal protein for at least two determinations six weeks apart.<br>If present, a 50 to 89% reduction in 24-hour light chain excretion, which still exceeds 200 mg/24 h, for at least two determinations six weeks apart.<br>For patients with non-secretory myeloma only, a 25 to 49% reduction in plasma cells in the bone marrow for a minimum of six weeks. 25-49% reduction in the size of plasmacytomas (by clinical or radiographic examination) for at least six weeks.<br>No increase in size or number of lytic bone lesions (development of compression fracture does not exclude response). |
| No change (NC) | Not meeting the criteria for MR or PD. |
| Progressive disease (PD) (for patients not in CR) | Requires one or more of the following:<br>>25% increase in the level of serum monoclonal paraprotein, which must also be an absolute increase of at least 5 g/L and confirmed on a repeat investigation.<br>>25% increase in 24-hour urinary light chain excretion, which must also be an absolute increase of at least 200 mg/24 h and confirmed on a repeat investigation.<br>>25% increase in plasma cells in a bone marrow aspirate or on trephine biopsy, which must also be an absolute increase of at least 10%.<br>Definite increase in the size of existing lytic bone lesions or soft tissue plasmacytomas.<br>Development of new bone lesions or soft tissue plasmacytomas (not including compression fracture).<br>Development of hypercalcemia (corrected serum calcium >11.5 mg/dL or 2.8 mmol/L not attributable to any other cause). |
| Relapse from CR | Requires at least one of the following:<br>Reappearance of serum or urinary paraprotein on immunofixation or routine electrophoresis confirmed by at least one follow-up and excluding oligoclonal immune reconstitution.<br>≥5% plasma cells in the bone marrow aspirate or biopsy.<br>Development of new lytic bone lesions or soft tissue plasmacytomas or definite increase in the size of residual bone lesions (not including compression fracture).<br>Development of hypercalcemia (corrected serum calcium<br>>11.5 mg/dL or 2.8 mmol/L not attributable to any other cause). |

Based on the criteria reported by Kraut et al., J. Clin. Oncol. 16(2): 589-592 (1998) and Blade et al., Br. J. Haematol. 102(5): 1115-1123 (1998). In patients with CR, bone marrow was analyzed using PCR for verification of CR at the molecular level. Patients who met all criteria for PR but who exhibit a ≥75% reduction in the level of serum monoclonal protein for at least two determinations six weeks apart were termed in 'Remission' (R).

Quality of Life assessment was analyzed to determine if response to therapy was accompanied by measurable improvement in quality of life. Analysis was performed on summary scores as well as individual items, with specific analytical methods outlined in a formal statistical analysis plan developed prior to database lock.

Pharmacodynamic data (20S proteasome) were descriptively analyzed in order to characterize the degree of proteasome inhibition, and to investigate any correlation between degree of inhibition and therapeutic response and toxicity.

For those patients who participated in the pharmacogenomic portion of the study, correlation between RNA expression levels and response to therapy were evaluated descriptively. In addition, duration of response, time to disease progression, and overall patient survival may be analyzed using RNA expression as a factor.

A total of 202 patients were enrolled in the study. The overall response rate to PS-341 alone was 35% (CR+PR rate of 27%) prior to any patients receiving added dexamethasone for non-optimal response. These patients had all

received at least two prior treatment regimens for their disease and their disease had progressed on their most recent therapy. This patient population has a very poor prognosis and no available standard therapy. Karnofsky Performance Status (KPS) was ≤70 in 25% of patients, and Durie-Salmon stage was reported as IIA or IIIB in 79% of patients. Approximately 39% of the patients had $\beta_2$ microglobulin ≥4 mg/L at Baseline, with 22% of patients having this indicator of disease severity ≥6 mg/L. The majority of the patients had relapsed after all conventional, high-dose, and novel therapies, with 74% progressing despite prior treatment with thalidomide.

The dose of 1.3 mg/$m^2$ twice weekly for two weeks followed by a 10-day rest was well tolerated. Over 80% of the 78 patients completed 2 or more cycles of treatment, 62% completed 4 or more cycles, and 27% completed 8 cycles.

The Independent Review Committee (IRC) evaluation of confirmed response to treatment with bortezomib alone is provided in Table D; further categorization of response for those patients who experienced partial remission is provided in Table E. This independent panel panel of three medical oncologists reviewed all data for 193 evaluable patients in the trial and assigned response using Blade criteria (Table C). The IRC determined that 35% of these 193 patients with relapsed/refractory multiple myeloma had a response to treatment (CR + PR + MR) with bortezomib alone, with 53 (27%) of the 193 patients experiencing a complete or partial remission to therapy and an additional 14 patients with a minimal response. An additional 46 (24%) of patients had evidence for stable disease (NC, no change) in response to bortezomib alone, which reflects an improvement in status for these patients who were progressing at the time of study entry. Based on the IRC assessment, 38 (20%) of the 193 patients had progressive disease and an additional 42 patients (22%) were considered not evaluable for response by the IRC. These data have been published. See Richardson PG, et al., New Eng. J. Med.;348: 2609-17 (2003).

All pharmacogenomic analyses relied on the Independent Review Committee's judgement of response category.

**Table D: Summary of IRC Confirmed Response to Treatment with bortezomib Alone (N = 193)**

| Confirmed Response Category | Response to bortezomib[a] |
|---|---|
| Complete + Partial + Minor Responses | 67 (35%) |
| Complete + Partial Remissions | 53 (27%) |
| Complete + Near Complete Remissions (NCR) | 19 (10%) |
| Complete Remission (CR) | 19 (4%) |
| Partial Remission (PR) | 34 (23%) |
| Minor Response (MR) | 14 (5%) |
| No Change | 46 (27%) |
| Progressive Disease | 38 (20%) |
| Not Evaluable | 42 (22%) |

a Response to treatment while patients were receiving bortezomib alone. (N = 193) <u>Identification Of Responsive and Non-Predictive markers</u>

44 multiple myeloma patients had high quality gene expression data.

Candidate markers that are correlated with the outcome of multiple myeloma patients to a proteasome inhibition (e.g., bortezomib) therapy were selected by using a combination of marker ranking algorithms. Supervised learning and feature selection algorithms were then used to identify the markers of the present invention.

<u>Data Analysis</u>

A data set, comprised of 44 discovery samples, was classified as responders ($N_R$=17), stable disease ($N_S$ = 12), or progressive disease ($N_P$ = 15), based on the assignments of the IRC. For marker identification, the three response classes were further grouped into responders ($N_R$=17) vs non-responders ($N_{NR}$ = 27), or refractory/progressive disease ($N_P$ = 15) vs others (N = 29). For each sample, 44,928 gene transcripts (Affymetrix probe sets) were profiled on the two Affymetrix U133 microarrays according to manufacturer's directions. Total RNA was isolated from homogenized tissue by TriazolTM (Life Technologies, Inc.) following the manufacturer's recommendations. RNA was stored at 80 °C in diethyl pyrocarbonate-treated deionized water. Detailed methods for labeling the samples and subsequent hybridization to the arrays are available from Affymetrix (Santa Clara, CA). Briefly, 5.0 $\mu$g of total RNA was converted to double-stranded cDNA (Superscript; Life Technologies, Inc.) priming the first-strand synthesis with a T7-(dT)24 primer containing a T7 polymerase promoter (Affymetrix Inc.). All of the double-stranded cDNA was subsequently used as a template to generate biotinylated cRNA using the incorporated T7 promoter sequence in an in vitro transcription system (Megascript kit; Ambion and Bio-11-CTP and Bio-16-UTP; Enzo). Control oligonucleotides and spikes were added to 10 $\mu$g of cRNA, which was then hybridized to U133 oligonucleotide arrays for 16 h at 45 °C with constant rotation. The arrays were then

washed and stained on an Affymetrix fluidics station using the EUKGE-WS1 protocol and scanned on an Affymetrix GeneArray scanner.

Normalization and Logarithmic Transformation.

Expression values for all markers on each microarray were normalized to a trimmed mean of 150. Expression values were determined using MAS5 gene expression analysis data processing software (Affymetrix, Santa Clara, CA). These values will be referred to as the "normalized expression" in the remainder of this section. In a further processing step, each normalized expression value was divided by 150, and added to 1. The natural logarithm was taken of the resulting number, and this value will be referred to as the "log expression" in the remainder of this section.

Single Marker Selection.

Single gene transcripts that appear associated with sample classes can be identified using the feature ranking and filtering methodology described below. Single marker identification of Predictive Markers using the methodology described herein are set forth in Table 1 Table 2 and Table 3.

Model Selection.

A set of one or more gene transcripts that together classify samples into sensitive and resistant groups (or responsive and non-responsive), in the context if a particular classifier algorithm, is referred to as a "model." The gene transcripts are referred to as "features." Determining which combination of gene transcript(s) best classifies samples into sensitive and resistant groups is referred to as "model selection." The following section describes the process of how the models of the present invention were identified. Exemplary models are set forth in Table 4, Table 5, and Table 6. The methods provided herein along with the single marker identification or Predictive markers can be used to identify additional models comprising markers of the invention.

<u>Summary Of The Data Provided In The Tables</u>

The following terms are used throughout the Tables:

"No." or "Number" corresponds to an identification number for the markers.
"Probeset ID" corresponds to the Affymetrix (Santa Clara, CA) identifier from the Human Genome U133 set oligonucleotide arrays which were used;
"Sequence Derived from" or "Genbank" or "RefSeq" corresponds to the public database accession information for the markers.
"RefSeq" corresponds to the Reference Sequence Nucleic Accession Number;
"Genbank" corresponds to the GenBank accession number assigned to the particular sequence.
"Title" corresponds to a common description, where available;
"Gene symbol" corresponds to a symbol the gene is commonly known by;
"Unigene" corresponds to the unique gene identifier;
"Rank__" corresponds to the process of determining which individual markers may be used in combination to group or classify a sample, for example, as responsive(R) or non-responsive(NR). Rank and the relative scoring method used for various ranking is indicated, as is the lowest rank score identified among all the methods for each of the predictive markers. Four different feature selection methods were utilized for determining the best classifier: (1) Signal-to-Noise Ratio ("SNR"), (2) Class-Based Threshold ("CBT"), (3) Pooled Fold Change ("PFC"), and (4) the Wilcoxon Rank-Sum Test;
Additional titles correspond to scored and parameters used in each of the methods described in the following exemplification, including "Hazard," "Decision Boundary," "Weight," "Vote Weight," "Vote," "Confidence," "Expression," "Gene Expression," "Log Gene Expression," "Normalized Expression," and "Normalization Factor;" "Supplemental Annotation" and "Biological Category" correspond to additional characterization and categorization not set forth in the title;
For Table 8, cell lines were designated as Sensitive "S" or Resistant "R;" and
"Ratio of Sensitive/Resistant" indicates relative expression of marker indicated.

Feature ranking and filtering

The first step in model selection is to filter the 44,928 features down to a smaller number which show a correspondence with the sample classifications. Filtering involves first ranking the features by a scoring method, and then taking only the highest ranking features for forther analysis. The filtering algorithms used in the present invention were: (1) Signal-to-Noise Ratio ("SNR"), (2) Class-Based Threshold ("CBT"), (3) Pooled Fold Change ("PFC"), and (4) the Wilcoxon Rank-Sum Test. In preferred embodiments, SNR was used to identify genes showing a small but consistent change in levels, and CBT was used to identify genes that were "off" in one class, but "on" in a fraction of the other class.

SNR is computed from the log expression values as absolute value of the difference in class means divided by the sum of the class standard deviations, and has been used to analyze expression data before; for example, see the definition of P(g,c), a measure of correlation between expression of gene g and class vector c, in Golub et al., "Molecular Classification of Cancer: Class discovery and class prediction by marker expression monitoring," Science, 286:531-537 (1999). To use SNR for filtering, the features with the top 100 SNR scores were retained and the remainder discarded from consideration.

CBT is computed from the normalized expression values, and defines one class ("class A") as the "off" class, and the other class ("class B") as the "on" class. In the present studies, the "off" class, class A is Responders; and the "on" class, class B, is Non-Responders. The CBT score may be computed in one of two ways: (1) Threshold each class B value to the average class A expression value for that feature. CBT is the difference between the average thresholded class B expression and the average class A expression, divided by the standard deviation of the class A expression:

$$CBT = \frac{\frac{1}{N_B}[\sum_{i=1}^{N_B} \max(x_i, \mu_A)] - \mu_A}{\sigma_A}$$

where $\mu_A$ is the average class A expression value, $\sigma_A$ is the standard deviation of the class A expression values, and $x_i$ represent the $N_B$ individual class B expression values. (2) CBT is the percentage of class B samples which exceed a fixed multiple of the maximum (or other percentile value) of expression values in class A. In either method, a constant value may be added to the class A threshold value to compensate for noise. In preferred embodiments, method 1 was utilized, and the top 100 features were selected.

The Pooled Fold Change ("PFC") method is a measure of differential expression between two groups of samples, arbitrarily designated "control" and "tester." PFC finds genes with higher expression in the tester than in the control samples. The analysis was performed looking at both Responders as "tester" (PFC-R) and Non-Responders as "tester" (PFC-NR). To qualify as having higher expression, tester samples must be above the $k^{th}$ percentile control sample. The fold-change values of tester samples are subjected to a nonlinear transformation that rises to a user-specified asymptote, in order to distinguish moderate levels of fold-change, but not make distinctions between very large fold-changes. The squashed fold-change values of the over-expressed tester samples are averaged to get the POOF score. In particular, PFC for gene g is computed as the average across tester samples of the compressed tester:control ratio R(s,g). For a given tester sample s and gene g, $R(s,g) = C(x_{gs}/(k+x_g^Q))$, where

C(x) is the compression function $C(z) = A(1-e^{-z/A})$ for $z \geq T$, and $C(z) = 0$ for $z<T$, where T is a threshold value no less than 1.0.

A is an upper asymptote on the fold-change value (we used 5),

k is a constant reflecting the additive noise in the data, i.e., the fixed component of the variance in repeated measurements. We derived a value of 30 for this parameter from calibration experiments.

$x_{gs}$ is the expression value of gene g in sample s,

$x_g^Q$ is the Qth percentile of the control samples' expression value.

Also, a minimum fraction f of the tester samples must have R(s,g) greater than 0; if this does not hold true, then the value of R(s,g) is set to 0.

We used the following parameters in two runs of this algorithm:

| Parameter | Value in run 1 | Value in run 2 |
|---|---|---|
| Q | 1.0 | 0.8 |
| f | 0.2 | 0.4 |
| T | 1.25 | 1.25 |

The Wilcoxon Rank-Sum test is a standard statistical technique. See, for example, Conover, W. J. 1980. Practical Nonparametric Statistics. 2nd ed. New York: John Wiley & Sons. This test is also known as the Mann-Whitney U test. The goal is to test the null hypothesis that the population distributions corresponding to two random samples are identical against the alternative hypothesis that they are different. Only the rank of the samples' expression values is

examined, not the values themselves.

Markers using the 44,928 probe sets were analyzed for differential expression across the 44 patient samples using the methods described in the above. In particular, we applied PFC (run 1), PFC (run 2), SNR, the Wilcoxon rank-sum test and the Class-Based Threshold as described above. The first three methods were run in each direction, to look for genes up in responders and then up in non-responders. The Wilcoxon rank-sum test was bidirectional and identified genes up in either responders or non-responders. Thus, there were 7 runs of the methods. In each case, the probe sets were sorted based on their score, and ranked. The top 100 ranked probe sets from each method were selected for Table 1. The last column in the table identifies the minimum rank across the methods.

**TABLE 1. PREDICTIVE MARKER IDENTIFICATION**

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 1 | 204298 _s_at | NM_0023 17.1 | lysyl oxidase | LOX | 44928 | 44928 | 44928 | 44928 | 44855 | 74 | 112 | >100 | 74 |
| 2 | 205884 _at | NM_0008 85.2 | integrin, alpha 4 (antigen CD49D, alpha 4 subunit of VLA-4 receptor) | ITGA4 | 44928 | 44928 | 86 | 44928 | 949 | 43980 | 2675 | >100 | 86 |
| 3 | 228841 _at | AW29925 0 | Homo sapiens cDNA FLJ32429 fis, clone SKMUS2001014. | --- | 44928 | 44928 | 91 | 44928 | 95 | 44834 | 197 | >100 | 91 |
| 4 | 243366 _s_at | AI936034 | integrin, alpha 4 (antigen CD49D, alpha 4 subunit of VLA-4 receptor) | ITGA4 | 44928 | 44928 | 98 | 44928 | 1896 | 43033 | 6343 | >100 | 98 |
| 5 | 214265 _at | AI193623 | integrin, alpha 8 | ITGA8 | 14 | 44928 | 25 | 44928 | 924 | 44005 | 4689 | 16 | 14 |
| 6 | 203949 _at | NM_0002 50.1 | myeloperoxidase | MPO | 44928 | 2 | 44928 | 25 | 44178 | 751 | 2599 | >100 | 2 |
| 7 | 207341 _at | NM_0027 77.2 | proteinase 3 (serine proteinase, neutrophil, Wegener granulomatosis autoantigen) | PRTN3 | 44928 | 4 | 44928 | 44928 | 43054 | 1875 | 17751 | >100 | 4 |
| 8 | 203948 _s_at | J02694.1 | myeloperoxidase | MPO | 44928 | 11 | 44928 | 44928 | 42466 | 2463 | 17515 | >100 | 11 |
| 9 | 224461 _s_at | BC006121 .1 | apoptosis-inducing factor (AIF)-homologous mitochondrion-associated inducer of death | AMID | 59 | 44928 | 44928 | 44928 | 360 | 44569 | 2121 | >100 | 59 |
| 10 | 206056_x_at | X52075 | sialophorin (gpL115, leukosialin, CD43) | SPN | 44928 | 44928 | 44928 | 82 | 44735 | 194 | 304 | >100 | 82 |
| 11 | 203489 _at | NM_0064 27.2 | CD27-binding (Siva) protein | SIVA | 44928 | 44928 | 44928 | 44928 | 86 | 44843 | 281 | >100 | 86 |
| 12 | 226507 _at | AU15440 8 | p21/Cdc42/Rac1-activated kinase 1 (STE20 homolog, yeast) | PAK1 | 90 | 44928 | 44928 | 44928 | 974 | 43955 | 3521 | >100 | 90 |
| 13 | 216055 _at | AK02292 0.1 | platelet-derived growth factor beta polypeptide (simian sarcoma viral (v-sis) oncogene homolog) | PDGFB | 44928 | 44928 | 44928 | 44928 | 44829 | 100 | 224 | >100 | 100 |
| 14 | 209942 _x_at | BC000340 .1 | melanoma antigen, family A, 3 | MAGEA 3 | 44928 | 44928 | 2 | 44928 | 217 | 44712 | 602 | >100 | 2 |
| 15 | 214612 _x_at | U10691 | --- | - | 44928 | 44928 | 4 | 44928 | 357 | 44572 | 2061 | >100 | 4 |
| 16 | 217969 _at | NM_0132 65.2 | melanoma antigen, family D, 1 | MAGED 1 | 8 | 44928 | 55 | 44928 | 197 | 44732 | 2165 | 4 | 4 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 17 | 215733 _x_at | AJ012833 .1 | cancer/testis antigen 2 | CTAG2 | 18 | 44928 | 5 | 44928 | 922 | 44007 | 28547 | 36 | 5 |
| 18 | 210546 _x_at | U87459.1 | cancer/testis antigen 1 | CTAG1 | 13 | 44928 | 7 | 44928 | 1278 | 43651 | 12645 | 32 | 7 |
| 19 | 211674 _x_at | AF038567 .1 | cancer/testis antigen 1 | CTAG1 | 21 | 44928 | 8 | 44928 | 1185 | 43744 | 27104 | 25 | 8 |
| 20 | 223313 _s_at | BC001207 .1 | MAGE-E1 protein | MAGE-E1 | 44928 | 44928 | 44928 | 12 | 42615 | 2314 | 9805 | >100 | 12 |
| 21 | 210467 _x_at | BC003408 .1 | melanoma antigen, family A, 12 | MAGEA 12 | 44928 | 44928 | 21 | 44928 | 2258 | 42671 | 10757 | >100 | 21 |
| 22 | 220057 _at | NM_0204 11.1 | G antigen, family D, 2 | GAGED 2 | 44928 | 44928 | 24 | 44928 | 2785 | 42144 | 10634 | >100 | 24 |
| 23 | 236152 _at | AW13533 0 | PAGE-5 protein | PAGE-5 | 40 | 44928 | 44928 | 44928 | 908 | 44021 | 8811 | >100 | 40 |
| 24 | 233831 _at | AI246052 | Homo sapiens serologically defined breast cancer antigen NY-BR-40 mRNA, partial cds | --- | 44928 | 44928 | 44928 | 44928 | 44874 | 55 | 142 | >100 | 55 |
| 25 | 206427 _s_at | U06654.1 | melan-A | MLANA | 44928 | 44928 | 44928 | 44928 | 44873 | 56 | 159 | >100 | 56 |
| 26 | 206218 _at | NM_0023 64.1 | melanoma antigen, family B, 2 | MAGEB 2 | 63 | 44928 | 44928 | 44928 | 3637 | 41292 | 38186 | >100 | 63 |
| 27 | 203386 _at | AI650848 | TBC1 domain family, member 4 | TBC1D4 | 44928 | 44928 | 44928 | 44928 | 44844 | 85 | 439 | >100 | 85 |
| 28 | 201457 _x_at | AF081496 .1 | BUB3 budding uninhibited by benzimidazoles 3 homolog (yeast) | BUB3 | 44928 | 44928 | 61 | 44928 | 62 | 44867 | 113 | 14 | 14 |
| 29 | 213348 _at | N33167 | cyclin-dependent kinase inhibitor 1C (p57, Kip2) | CDKN1 C | 44928 | 31 | 44928 | 44928 | 44846 | 83 | 147 | >100 | 31 |
| 30 | 204170 _s_at | NM_0018 27.1 | CDC28 protein kinase regulatory subunit 2 | CKS2 | 44928 | 44928 | 34 | 44928 | 464 | 44465 | 828 | >100 | 34 |
| 31 | 206205 _at | NM_0227 82.1 | M-phase phosphoprotein 9 | MPHOS PH9 | 44928 | 44928 | 44928 | 44928 | 40 | 44889 | 72 | >100 | 40 |
| 32 | 208796 _s_at | BC000196 .1 | cyclin G1 | CCNG1 | 44928 | 44928 | 68 | 44928 | 250 | 44679 | 517 | >100 | 68 |
| 33 | 204460 _s_at | AF074717 .1 | RAD1 homolog (S. pombe) | RAD1 | 44928 | 44928 | 44928 | 44928 | 71 | 44858 | 128 | >100 | 71 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 34 | 224918 _x_at | AI220117 | microsomal glutathione S-transferase 1 | MGST1 | 28 | 44928 | 44928 | 44928 | 10617 | 34312 | 19002 | >100 | 28 |
| 35 | 205998 _x_at | NM_0174 60.2 | cytochrome P450, subfamily IIIA (niphedipine oxidase), polypeptide 4 | CYP3A4 | 44928 | 44928 | 44928 | 44928 | 44852 | 77 | 87 | >100 | 77 |
| 36 | 239476 _at | AW15216 6 | Homo sapiens cDNA FLJ36491 fis, clone THYMU2018197. | --- | 44928 | 44928 | 44928 | 44928 | 44925 | 4 | 9 | >100 | 4 |
| 37 | 211298 _s_at | AF116645 .1 | albumin | ALB | 44928 | 44928 | 44928 | 44928 | 44914 | 15 | 95 | >100 | 15 |
| 38 | 216835_s_at | AF035299.1 | docking protein 1, 62kDa (downstream of tyrosine kinase 1) | DOK1 | 44928 | 44928 | 44928 | 44928 | 44921 | 8 | 42 | >100 | 8 |
| 39 | 213891 _s_at | AI927067 | Homo sapiens cDNA FLJ11918 fis, clone HEMBB1000272. | --- | 44928 | 44928 | 44928 | 20 | 43578 | 1351 | 1063 | >100 | 20 |
| 40 | 212387 _at | AK02198 0.1 | Homo sapiens cDNA FLJ11918 fis, clone HEMBB1000272. | --- | 44928 | 44928 | 44928 | 31 | 43365 | 1564 | 393 | >100 | 31 |
| 41 | 212382 _at | AK02198 0.1 | Homo sapiens cDNA FLJ11918 fis, clone HEMBB 1000272. | --- | 44928 | 40 | 44928 | 44928 | 37843 | 7086 | 9000 | >100 | 40 |
| 42 | 203753 _at | NM_0031 99.1 | transcription factor 4 | TCF4 | 44928 | 44928 | 44928 | 42 | 43376 | 1553 | 1580 | >100 | 42 |
| 43 | 212386 _at | AK02198 0.1 | Homo sapiens cDNA FLJ11918 fis, clone HEMBB 1000272. | --- | 44928 | 44928 | 44928 | 64 | 42346 | 2583 | 1261 | >100 | 64 |
| 44 | 211709 _s_at | BC005810 .1 | stem cell growth factor; lymphocyte secreted C-type lectin | SCGF | 44928 | 44928 | 44928 | 99 | 44282 | 647 | 1192 | >100 | 99 |
| 45 | 217020 _at | X04014 | - | --- | 44928 | 44928 | 44928 | 44928 | 44917 | 12 | 71 | >100 | 12 |
| 46 | 217786 _at | NM_0061 09.1 | SKB1 homolog (S. pombe) | SKB1 | 44928 | 44928 | 44928 | 44928 | 34 | 44895 | 17 | >100 | 17 |
| 47 | 206109 _at | NM_0001 48.1 | fucosyltransferase 1 (galactoside 2-alpha-L-fucosyltransferase, Bombay phenotype included) | FUT1 | 44928 | 44928 | 44928 | 44928 | 44907 | 22 | 41 | >100 | 22 |
| 48 | 227798 _at | AU14689 1 | ESTs | --- | 44928 | 44928 | 23 | 44928 | 2520 | 42409 | 6771 | >100 | 23 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 49 | 208743 _s_at | BC001359 .1 | tyrosine 3-monooxygenase/tryptop han 5-monooxygenase activation protein, beta polypeptide | YWHAB | 44928 | 44928 | 44928 | 44928 | 51 | 44878 | 100 | >100 | 51 |
| 50 | 225239 _at | AI355441 | ESTs, Moderately similar to hypothetical protein FLJ20958 [Homo sapiens] [H.sapiens] | --- | 44928 | 44928 | 44928 | 57 | 44845 | 84 | 226 | >100 | 57 |
| 51 | 215551 _at | AI073549 | estrogen receptor 1 | ESR1 | 44928 | 44928 | 44928 | 44928 | 44868 | 61 | 109 | >100 | 61 |
| 52 | 215067 _x_at | AU14794 2 | Homo sapiens cDNA FLJ12333 fis, clone MAMMA1002198, highly similar to THIOREDOXIN PEROXIDASE 1. | --- | 44928 | 44928 | 44928 | 72 | 43871 | 1058 | 2063 | >100 | 72 |
| 53 | 210993 _s_at | U54826.1 | MAD, mothers against decapentaplegic homolog 1 (Drosophila) | MADH1 | 44928 | 44928 | 100 | 44928 | 3077 | 41852 | 5470 | >100 | 100 |
| 54 | 209374 _s_at | BC001872 .1 | immunoglobulin heavy constant mu | IGHM | 2 | 44928 | 44928 | 44928 | 1769 | 43160 | 31220 | 66 | 2 |
| 55 | 224342 _x_at | L14452.1 | immunoglobulin lambda locus | IGL@ | 4 | 44928 | 44928 | 44928 | 2837 | 42092 | 28929 | 29 | 4 |
| 56 | 212827 _at | X17115.1 | immunoglobulin heavy constant mu | IGHM | 6 | 44928 | 44928 | 44928 | 3364 | 41565 | 36442 | >100 | 6 |
| 57 | 234366 _x_at | AF103591 .1 | immunoglobulin lambda locus | IGL@ | 44928 | 44928 | 44928 | 26 | 30154 | 14775 | 21162 | >100 | 26 |
| 58 | 216986 _s_at | D78261.1 | interferon regulatory factor 4 | IRF4 | 44928 | 44928 | 44928 | 44928 | 43 | 44886 | 129 | >100 | 43 |
| 59 | 205098 _at | AI421071 | chemokine (C-C motif) receptor 1 | CCR1 | 46 | 44928 | 44928 | 44928 | 2037 | 42892 | 13544 | >100 | 46 |
| 60 | 239237 _at | AI798822 | ESTs | --- | 120 | 44928 | 79 | 44928 | 4324 | 40605 | 22488 | >100 | 79 |
| 61 | 205099 _s_at | NM_0012 95.1 | chemokine (C-C motif) receptor 1 | CCR1 | 85 | 44928 | 44928 | 44928 | 3294 | 41635 | 13545 | >100 | 85 |
| 62 | 223472 | AF071594.1_at | Wolf-Hirschhorn syndrome candidate 1 | WHSC1 | 44928 | 44928 | 44928 | 2 | 43897 | 1032 | 6635 | >100 | 2 |
| 63 | 222778 _s_at | AI770166 | Wolf-Hirschhorn syndrome candidate 1 | WHSC1 | 44928 | 44928 | 44928 | 3 | 42704 | 2225 | 7936 | >100 | 3 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 64 | 209054 _s_at | AF083389 .1 | Wolf-Hirschhorn syndrome candidate 1 | WHSC1 | 44928 | 44928 | 44928 | 4 | 44524 | 405 | 444 | >100 | 4 |
| 65 | 222777 _s_at | AI770166 | Wolf-Hirschhorn syndrome candidate 1 | WHSC1 | 44928 | 44928 | 44928 | 5 | 41834 | 3095 | 13244 | >100 | 5 |
| 66 | 209053 _s_at | AF083389 .1 | Wolf-Hirschhorn syndrome candidate 1 | WHSC1 | 44928 | 44928 | 44928 | 7 | 42426 | 2503 | 10341 | >100 | 7 |
| 67 | 200921 _s_at | NM_0017 31.1 | B-cell translocation gene 1, anti-proliferative | BTG1 | 75 | 44928 | 27 | 44928 | 260 | 44669 | 787 | 24 | 24 |
| 68 | 209052 _s_at | AF083389 .1 | Wolf-Hirschhorn syndrome candidate 1 | WHSC1 | 44928 | 44928 | 44928 | 24 | 42989 | 1940 | 4673 | >100 | 24 |
| 69 | 213940 _s_at | AU14505 3 | formin binding protein 1 | FNBP1 | 44928 | 44928 | 43 | 44928 | 7005 | 37924 | 11991 | >100 | 43 |
| 70 | 213732 _at | BE962186 | transcription factor 3 (E2A immunoglobulin enhancer binding factors E12/E47) | TCF3 | 44928 | 44928 | 44928 | 44928 | 44876 | 53 | 200 | >100 | 53 |
| 71 | 213047 _x_at | AI278616 | SET translocation (myeloid leukemiaassociated) | SET | 44928 | 44928 | 74 | 44928 | 85 | 44844 | 207 | >100 | 74 |
| 72 | 200631 _s_at | NM_0030 11.1 | SET translocation (myeloid leukemia-associated) | SET | 130 | 44928 | 44928 | 44928 | 175 | 44754 | 642 | 81 | 81 |
| 73 | 205068 _s_at | BE671084 | GTPase regulator associated with focal adhesion kinase pp125(FAK) | GRAF | 44928 | 44928 | 44928 | 44928 | 44830 | 99 | 190 | >100 | 99 |
| 74 | 220146 _at | NM_0165 62.1 | toll-like receptor 7 | TLR7 | 10 | 44928 | 44928 | 44928 | 961 | 43968 | 9515 | >100 | 10 |
| 75 | 232304 _at | AK02671 4.1 | pellino homolog 1 (Drosophila) | PELI1 | 44928 | 44928 | 44928 | 13 | 44623 | 306 | 766 | >100 | 13 |
| 76 | 232213_at | AU147506 | pellino homolog 1 (Drosophila) | PELI1 | 44928 | 44928 | 44928 | 18 | 44653 | 276 | 1025 | >100 | 18 |
| 77 | 218319 _at | NM_0206 51.2 | pellino homolog 1 (Drosophila) | PELI1 | 44928 | 44928 | 44928 | 38 | 41381 | 3548 | 3985 | >100 | 38 |
| 78 | 215744 _at | AW51414 0 | fusion, derived from t(12;16) malignant liposarcoma | FUS | 44928 | 44928 | 44928 | 44928 | 44853 | 76 | 158 | >100 | 76 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 79 | 206363 _at | NM_0053 60.2 | v-maf musculoaponeurotic fibrosarcoma oncogene homolog (avian) | MAF | 44928 | 44928 | 44928 | 8 | 34192 | 10737 | 7331 | >100 | 8 |
| 80 | 202768 _at | NM_0067 32.1 | FBJ murine osteosarcoma viral oncogene homolog B | FOSB | 44928 | 44928 | 44928 | 51 | 43123 | 1806 | 2597 | >100 | 51 |
| 81 | 202647 _s_at | NM_0025 24.2 | neuroblastoma RAS viral (v-ras) oncogene homolog | NRAS | 78 | 44928 | 52 | 44928 | 169 | 44760 | 691 | >100 | 52 |
| 82 | 209640 _at | M79462.1 | promyelocytic leukemia | PML | 44928 | 44928 | 44928 | 44928 | 44851 | 78 | 115 | >100 | 78 |
| 140 | 232231 _at | AL353944 .1 | Runt domain transcription factor 2 | RUNX2 | 1 | 44928 | 1 | 44928 | 17 | 44912 | 212 | 1 | 1 |
| 83 | 201575 _at | NM_0122 45.1 | SKI-interacting protein | SNW1 | 44928 | 44928 | 44928 | 44928 | 3 | 44926 | 12 | >100 | 3 |
| 84 | 224985 _at | BE964484 | Homo sapiens, clone IMAGE:3446533, mRNA | --- | 31 | 44928 | 13 | 44928 | 54 | 44875 | 130 | 6 | 6 |
| 85 | 204602 _at | NM_0122 42.1 | dickkopf homolog 1 (Xenopus laevis) | DKK1 | 44928 | 44928 | 10 | 44928 | 2757 | 42172 | 9868 | > 100 | 10 |
| 86 | 201653 _at | NM_0057 76.1 | cornichon homolog (Drosophila) | CNIH | 44928 | 44928 | 45 | 44928 | 16 | 44913 | 26 | 94 | 16 |
| 87 | 234021 _at | AK02498 4.1 | Homo sapiens cDNA: FLJ21331 fis, clone COL02520. | --- | 44928 | 44928 | 44928 | 44928 | 44909 | 20 | 16 | >100 | 16 |
| 88 | 212063 _at | BE903880 | CD44 antigen (homing function and Indian blood group system) | CD44 | 44928 | 44928 | 18 | 44928 | 2720 | 42209 | 8726 | 62 | 18 |
| 89 | 204489 _s_at | NM_0006 10.1 | CD44 antigen (homing function and Indian blood group system) | CD44 | 34 | 44928 | 54 | 44928 | 3784 | 41145 | 21033 | >100 | 34 |
| 90 | 227167 _s_at | AW51131 9 | Homo sapiens mesenchymal stem cell protein DSC96 mRNA, partial cds | --- | 44928 | 44928 | 37 | 44928 | 155 | 44774 | 430 | >100 | 37 |
| 91 | 202290 _at | NM_0148 91.1 | PDGFA associated protein 1 | PDAP1 | 44928 | 44928 | 44928 | 44928 | 78 | 44851 | 108 | >100 | 78 |
| 92 | 215499 _at | AA78038 1 | mitogen-activated protein kinase kinase 3 | MAP2K3 | 44928 | 44928 | 44928 | 78 | 44259 | 670 | 1433 | >100 | 78 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 93 | 200047 _s_at | NM_0034 03.2 | YY1 transcription factor | YY1 | 44928 | 44928 | 44928 | 44928 | 135 | 44794 | 193 | 95 | 95 |
| 94 | 222555 _s_at | AI338045 | mitochondrial ribosomal protein L44 | MRPL44 | 44928 | 44928 | 44928 | 44928 | 4 | 44925 | 11 | >100 | 4 |
| 95 | 212694 _s_at | NM_0005 32.1 | propionyl Coenzyme A carboxylase, beta polypeptide | PCCB | 44928 | 44928 | 44928 | 44928 | 7 | 44922 | 19 | >100 | 7 |
| 96 | 222530 _s_at | AF275813 .1 | McKusick-Kaufman syndrome | MKKS | 69 | 44928 | 129 | 44928 | 13 | 44916 | 15 | 42 | 13 |
| 97 | 200869 _at | NM_0009 80.1 | ribosomal protein L18a | RPL18A | 20 | 44928 | 97 | 44928 | 723 | 44206 | 2697 | 76 | 20 |
| 98 | 200023 _s_at | NM_0037 54.1 | eukaryotic translation initiation factor 3, subunit 5 epsilon, 47kDa | EIF3S5 | 29 | 44928 | 65 | 44928 | 178 | 44751 | 992 | 21 | 21 |
| 99 | 200812 _at | NM_0064 29.1 | chaperonin containing TCP1, subunit 7 (eta) | CCT7 | 44928 | 44928 | 44928 | 44928 | 22 | 44907 | 25 | >100 | 22 |
| 100 | 225190 _x_at | AW40266 0 | ribosomal protein L35a | RPL35A | 27 | 44928 | 44928 | 44928 | 423 | 44506 | 1445 | 27 | 27 |
| 101 | 200023 _s_at | NM_0037 54.1 | eukaryotic translation initiation factor 3, subunit 5 epsilon, 47kDa | EIF3S5 | 58 | 44928 | 51 | 44928 | 182 | 44747 | 332 | 31 | 31 |
| 102 | 217919_s_at | BE782148 | mitochondrial ribosomal protein L42 | MRPL42 | 44928 | 44928 | 82 | 44928 | 60 | 44869 | 34 | >100 | 34 |
| 103 | 211972 _x_at | AI953822 | ribosomal protein, large, P0 | RPLP0 | 92 | 44928 | 44928 | 44928 | 378 | 44551 | 420 | 38 | 38 |
| 104 | 200024 _at | NM_0010 09.1 | ribosomal protein S5 | RPS5 | 118 | 44928 | 93 | 44928 | 122 | 44807 | 333 | 41 | 41 |
| 105 | 200715 _x_at | BC000514 .1 | ribosomal protein L13a | RPL13A | 47 | 44928 | 114 | 44928 | 2857 | 42072 | 9548 | >100 | 47 |
| 106 | 201258 _at | NM_0010 20.1 | ribosomal protein S16 | RPS16 | 99 | 44928 | 99 | 44928 | 185 | 44744 | 738 | 51 | 51 |
| 107 | 200003 _s_at | NM_0009 91.1 | ribosomal protein L28 | RPL28 | 56 | 44928 | 44928 | 44928 | 2488 | 42441 | 9320 | >100 | 56 |
| 108 | 221726 _at | BE250348 | ribosomal protein L22 | RPL22 | 44928 | 44928 | 115 | 44928 | 206 | 44723 | 657 | 64 | 64 |
| 109 | 200041 _s_at | NM_0046 40.1 | HLA-B associated transcript 1 | BAT1 | 44928 | 44928 | 44928 | 70 | 33237 | 11692 | 18501 | >100 | 70 |
| 110 | 211937 _at | NM_0014 17.1 | eukaryotic translation initiation factor 4B | EIF4B | 44928 | 44928 | 71 | 44928 | 794 | 44135 | 2480 | >100 | 71 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 111 | 200082 _s_at | AI805587 | ribosomal protein S7 | RPS7 | 72 | 44928 | 84 | 44928 | 468 | 44461 | 1272 | 85 | 72 |
| 112 | 214167 _s_at | AA55511 3 | ribosomal protein, large, P0 | RPLPO | 44928 | 44928 | 107 | 44928 | 239 | 44690 | 326 | 73 | 73 |
| 113 | 200024 _at | NM_0010 09.1 | ribosomal protein S5 | RPS5 | 152 | 44928 | 44928 | 44928 | 156 | 44773 | 546 | 77 | 77 |
| 114 | 217719 _at | NM_0160 91.1 | eukaryotic translation initiation factor 3, subunit 6 interacting protein | EIF3S6I P | 44928 | 44928 | 44928 | 44928 | 532 | 44397 | 951 | 78 | 78 |
| 115 | 225797 _at | AV70756 8 | mitochondrial ribosomal protein L54 | MRPL54 | 166 | 44928 | 138 | 44928 | 108 | 44821 | 312 | 83 | 83 |
| 116 | 200937 _s_at | NM_0009 69.1 | ribosomal protein L5 | RPL5 | 44928 | 44928 | 89 | 44928 | 1188 | 43741 | 3462 | >100 | 89 |
| 117 | 208985 _s_at | BC002719 .1 | eukaryotic translation initiation factor 3, subunit 1 alpha, 35kDa | EIF3S1 | 105 | 44928 | 44928 | 44928 | 90 | 44839 | 199 | >100 | 90 |
| 118 | 200834 _s_at | NM_0010 24.1 | ribosomal protein S21 | RPS21 | 109 | 44928 | 136 | 44928 | 870 | 44059 | 4275 | 98 | 98 |
| 119 | 216153 _x_at | AK02289 7.1 | reversion-inducing-cysteine-rich protein with kazal motifs | RECK | 44928 | 3 | 44928 | 9 | 44724 | 205 | 1125 | >100 | 3 |
| 120 | 217687 _at | AA22444 6 | adenylate cyclase 2 (brain) | ADCY2 | 44928 | 44928 | 44928 | 44928 | 44923 | 6 | 28 | >100 | 6 |
| 121 | 222632 _s_at | AA84313 2 | leucine zipper transcription factor-like 1 | LZTFL1 | 44928 | 44928 | 22 | 44928 | 559 | 44370 | 962 | >100 | 22 |
| 122 | 236623 _at | AI367432 | hypothetical protein MGC16179 | MGC161 79 | 44928 | 33 | 44928 | 44928 | 43090 | 1839 | 11437 | >100 | 33 |
| 123 | 221899 _at | AI809961 | hypothetical protein from BCRA2 region | CG005 | 44928 | 41 | 44928 | 44928 | 40910 | 4019 | 11859 | >100 | 41 |
| 124 | 221691 _x_at | AB04227 8.1 | nucleophosmin (nucleolar phosphoprotein B23, numatrin) | NPM1 | 43 | 44928 | 44928 | 44928 | 926 | 44003 | 3231 | >100 | 43 |
| 125 | 209030 _s_at | NM_0143 33.1 | immunoglobulin superfamily, member 4 | IGSF4 | 44928 | 44928 | 44 | 44928 | 2842 | 42087 | 9276 | >100 | 44 |
| 126 | 222762 _x_at | AU14425 9 | LIM domains containing 1 | LIMD1 | 44928 | 44928 | 57 | 44928 | 1570 | 43359 | 4714 | >100 | 57 |
| 127 | 240983 _s_at | AW29227 3 | cysteinyl-tRNA synthetase | CARS | 44928 | 44928 | 80 | 44928 | 1536 | 43393 | 2413 | >100 | 80 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 128 | 200713 _s_at | NM_0123 25.1 | microtubule-associated protein, RP/EB family, member 1 | MAPRE 1 | 44928 | 44928 | 44928 | 44928 | 96 | 44833 | 300 | >100 | 96 |
| 129 | 200814 _at | NM_0062 63.1 | proteasome (prosome, macropain) activator subunit 1 (PA28 alpha) | PSME1 | 44928 | 44928 | 130 | 44928 | 14 | 44915 | 31 | 44 | 14 |
| 130 | 201532 _at | NM_0027 88.1 | proteasome (prosome, macropain) subunit, alpha type, 3 | PSMA3 | 76 | 44928 | 30 | 44928 | 19 | 44910 | 22 | 26 | 19 |
| 131 | 218011_at | NM_024292.1 | ubiquitin-like 5 | UBL5 | 44928 | 44928 | 94 | 44928 | 39 | 44890 | 90 | 47 | 39 |
| 132 | 224747 _at | AK00061 7.1 | hypothetical protein LOC92912 | LOC929 12 | 44928 | 44928 | 44928 | 44928 | 391 | 44538 | 706 | 45 | 45 |
| 133 | 201758 _at | NM_0062 92.1 | tumor susceptibility gene 101 | TSG101 | 44928 | 44928 | 44928 | 44928 | 65 | 44864 | 171 | >100 | 65 |
| 134 | 200019 _s_at | NM_0019 97.1 | Finkel-Biskis-Reilly murine sarcoma virus (FBR-MuSV) ubiquitously expressed (fox derived); ribosomal protein S30 | FAU | 156 | 44928 | 44928 | 44928 | 220 | 44709 | 640 | 68 | 68 |
| 135 | 202346 _at | NM_0053 39.2 | huntingtin interacting protein 2 | HIP2 | 44928 | 44928 | 44928 | 44928 | 79 | 44850 | 255 | >100 | 79 |
| 136 | 201177 _s_at | NM_0054 99.1 | SUMO-1 activating enzyme subunit 2 | UBA2 | 44928 | 44928 | 143 | 44928 | 81 | 44848 | 170 | 87 | 81 |
| 137 | 200043 _at | NM_0044 50.1 | enhancer of rudimentary homolog (Drosophila) | ERH | 44928 | 44928 | 140 | 44928 | 1 | 44928 | 7 | 22 | 1 |
| 138 | 212109 _at | AK02315 4.1 | HN1 like | HN1L | 44928 | 44928 | 44928 | 44928 | 44928 | 1 | 4 | >100 | 1 |
| 139 | 212190 _at | AL541302 | serine (or cysteine) proteinase inhibitor, clade E (nexin, plasminogen activator inhibitor type 1), member 2 | SERPIN E2 | 44928 | 44928 | 44928 | 1 | 44650 | 279 | 325 | >100 | 1 |
| 141 | 234428 _at | AL110127 .1 | Homo sapiens mRNA; cDNA DKFZp564I1316 (from clone DKFZp564I1316) | --- | 44928 | 44928 | 44928 | 44928 | 44927 | 2 | 1 | >100 | 1 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 142 | 235102 _x_at | AI684439 | phenylalanine hydroxylase | PAH | 44928 | 1 | 44928 | 6 | 44469 | 460 | 4356 | >100 | 1 |
| 143 | 200965 _s_at | NM_0067 20.1 | actin binding LIM protein 1 | ABLIM1 | 44928 | 44928 | 44928 | 44928 | 44919 | 10 | 2 | >100 | 2 |
| 144 | 222783_s_at | NM_022137.1 | SPARC related modular calcium binding 1 | SMOC1 | 22 | 44928 | 3 | 44928 | 72 | 44857 | 117 | 2 | 2 |
| 145 | 232075 _at | BF791874 | recombination protein REC14 | REC14 | 5 | 44928 | 31 | 44928 | 2 | 44927 | 8 | 3 | 2 |
| 146 | 220565 _at | NM_0166 02.1 | G protein-coupled receptor 2 | GPR2 | 3 | 44928 | 14 | 44928 | 304 | 44625 | 851 | 5 | 3 |
| 147 | 220572 _at | NM_0187 05.1 | hypothetical protein DKFZp547G183 | DKFZp5 47G183 | 44928 | 44928 | 44928 | 44928 | 44926 | 3 | 3 | >100 | 3 |
| 148 | 208263 _at | NM_0185 81.1 | --- | --- | 44928 | 44928 | 44928 | 44928 | 44903 | 26 | 5 | >100 | 5 |
| 149 | 221569 _at | AL136797 .1 | hypothetical protein FLJ20069 | FLJ2006 9 | 44928 | 9 | 44928 | 48 | 44924 | 5 | 13 | >100 | 5 |
| 150 | 222427 _s_at | AK02141 3.1 | leucyl-tRNA synthetase | LARS | 12 | 44928 | 76 | 44928 | 5 | 44924 | 36 | 9 | 5 |
| 151 | 230941 _at | AI651340 | Homo sapiens, clone IMAGE:5271446, mRNA | --- | 44928 | 5 | 44928 | 44928 | 44738 | 191 | 96 | >100 | 5 |
| 152 | 201682 _at | NM_0042 79.1 | peptidase (mitochondrial processing) beta | PMPCB | 38 | 44928 | 73 | 44928 | 6 | 44923 | 10 | 20 | 6 |
| 153 | 210258 _at | AF030107 .1 | regulator of G-protein signalling 13 | RGS13 | 44928 | 44928 | 6 | 44928 | 3847 | 41082 | 26318 | >100 | 6 |
| 154 | 218438 _s_at | NM_0252 05.1 | endothelial-derived gene 1 | EG1 | 60 | 44928 | 44928 | 44928 | 10 | 44919 | 6 | >100 | 6 |
| 155 | 227341 _at | AW19540 7 | Homo sapiens mRNA; cDNA DKFZp686C072 (from clone DKFZp686C072) | --- | 44928 | 6 | 44928 | 44928 | 43167 | 1762 | 10075 | >100 | 6 |
| 156 | 202075 _s_at | NM_0062 27.1 | phospholipid transfer protein | PLTP | 44928 | 7 | 44928 | 44928 | 39569 | 5360 | 20579 | >100 | 7 |
| 157 | 216288 _at | AU15927 6 | cysteinyl leukotriene receptor 1 | CYSLTR 1 | 44928 | 44928 | 44928 | 44928 | 44922 | 7 | 46 | >100 | 7 |
| 158 | 217915 _s_at | NM_0163 04.1 | chromosome 15 open reading frame 15 | C15orf15 | 33 | 44928 | 35 | 44928 | 11 | 44918 | 14 | 7 | 7 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 159 | 222968 _at | NM_0169 47.1 | chromosome 6 open reading frame 48 | C6orf48 | 7 | 44928 | 11 | 44928 | 107 | 44822 | 481 | 43 | 7 |
| 160 | 202567 _at | NM_0041 75.1 | small nuclear ribonucleoprotein D3 polypeptide 18kDa | SNRPD3 | 44928 | 44928 | 28 | 44928 | 8 | 44921 | 32 | 28 | 8 |
| 161 | 213510 _x_at | AW 19454 3 | TL132 protein | LOC220 594 | 44928 | 8 | 44928 | 34 | 44098 | 831 | 2375 | >100 | 8 |
| 162 | 225065 _x_at | AI826279 | hypotheticalprotein MGC40157 | MGC401 57 | 41 | 44928 | 33 | 44928 | 68 | 44861 | 92 | 8 | 8 |
| 163 | 204287 _at | NM_0047 11.1 | synaptogyrin 1 | SYNGR1 | 44928 | 44928 | 44928 | 44928 | 44920 | 9 | 24 | >100 | 9 |
| 164 | 206762 _at | NM_0022 34.1 | potassium voltage-gated channel, shaker-related subfamily, member 5 | KCNA5 | 9 | 44928 | 44928 | 44928 | 1038 | 43891 | 20489 | >100 | 9 |
| 165 | 210250 _x_at | AF067854 .1 | adenylosuccinatelyase | ADSL | 44928 | 44928 | 44928 | 44928 | 9 | 44920 | 27 | >100 | 9 |
| 166 | 210497 _x_at | BC002818 .1 | synovial sarcoma, X breakpoint 2 | SSX2 | 44928 | 44928 | 9 | 44928 | 651 | 44278 | 3927 | >100 | 9 |
| 167 | 223358 _s_at | AW26983 4 | Homo sapiens cDNA FLJ33024 fis, clone THYMU1000532, moderately similar to HIGH-AFFINITY CAMP-SPECIFIC 3',5'-CYCLIC PHOSPHODIESTERAS E (EC 3.1.4.17). | --- | 54 | 44928 | 39 | 44928 | 99 | 44830 | 366 | 10 | 10 |
| 168 | 225767 _at | AL531684 | ESTs, Weakly similar to T02345 hypothetical protein KIAA0324 - human (fragment) [H.sapiens] | --- | 44928 | 10 | 44928 | 44928 | 31271 | 13658 | 34008 | >100 | 10 |
| 169 | 232169 _x_at | AK00211 0.1 | NADH dehydrogenase (ubiquinone) Fe-S protein 8, 23kDa (NADH-coenzyme Q reductase) | NDUFS8 | 44928 | 44928 | 44928 | 10 | 44849 | 80 | 245 | >100 | 10 |
| 170 | 216287 _at | AK02193 0.1 | --- | --- | 44928 | 44928 | 44928 | 44928 | 44918 | 11 | 52 | >100 | 11 |
| 171 | 228332 _s_at | AA52693 9 | selenoprotein H | SELH | 55 | 44928 | 149 | 44928 | 38 | 44891 | 67 | 11 | 11 |
| 172 | 242903 _at | AI458949 | ESTs | --- | 44928 | 44928 | 44928 | 11 | 44599 | 330 | 1363 | >100 | 11 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 173 | 244114 _x_at | AI003508 | ESTs | --- | 11 | 44928 | 44928 | 44928 | 3539 | 41390 | 33890 | >100 | 11 |
| 174 | 223490 _s_at | AF281132 .1 | exosome component Rrp40 | RRP40 | 44928 | 44928 | 44928 | 44928 | 12 | 44917 | 29 | >100 | 12 |
| 175 | 224496 _s_at | BC006292 .1 | hypothetical protein MGC10744 | MGC107 44 | 44928 | 12 | 44928 | 44 | 40920 | 4009 | 11871 | >100 | 12 |
| 176 | 226243 _at | BF590958 | hypothetical protein MGC11266 | MGC112 66 | 44928 | 44928 | 12 | 44928 | 97 | 44832 | 49 | 49 | 12 |
| 177 | 231045 _x_at | H29876 | selenoprotein H | SELH | 44928 | 44928 | 121 | 44928 | 28 | 44901 | 39 | 12 | 12 |
| 178 | 206978 _at | NM_0006 47.2 | chemokine (C-C motif) receptor 2 | CCR2 | 82 | 44928 | 20 | 44928 | 818 | 44111 | 2153 | 13 | 13 |
| 179 | 212062 _at | AB01451 1.1 | ATPase, Class II, type 9A | ATP9A | 44928 | 13 | 44928 | 44928 | 44776 | 153 | 45 | >100 | 13 |
| 180 | 227692 _at | AU15386 6 | guanine nucleotide binding protein (G protein), alpha inhibiting activity polypeptide 1 | GNAI1 | 44928 | 44928 | 44928 | 44928 | 44916 | 13 | 21 | >100 | 13 |
| 181 | 200710 _at | NM_0000 18.1 | acyl-Coenzyme A dehydrogenase, very long chain | ACADV L | 44928 | 14 | 44928 | 69 | 44212 | 717 | 2804 | >100 | 14 |
| 182 | 216529 _at | AL049244 .1 | Homo sapiens mRNA; cDNA DKFZp564C163 (from clone DKFZp564C163) | --- | 44928 | 44928 | 44928 | 44928 | 44915 | 14 | 75 | >100 | 14 |
| 183 | 233437 _at | AF238869 .1 | gamma-aminobutyric acid (GABA) A receptor, alpha 4 | GABRA 4 | 44928 | 36 | 44928 | 14 | 44817 | 112 | 455 | >100 | 14 |
| 184 | 202591 _s_at | NM_0031 43.1 | single-stranded DNA binding protein | SSBP1 | 44928 | 44928 | 44928 | 44928 | 15 | 44914 | 69 | 75 | 15 |
| 185 | 206632 _s_at | NM_0049 00.1 | apolipoprotein B mRNA editing enzyme, catalytic polypeptide-like 3B | APOBE C3B | 61 | 44928 | 15 | 44928 | 386 | 44543 | 1554 | 65 | 15 |
| 186 | 213975 _s_at | AV71190 4 | lysozyme (renal amyloidosis) | LYZ | 44928 | 44928 | 44928 | 15 | 39536 | 5393 | 16729 | >100 | 15 |
| 187 | 224493 _x_at | BC006280 .1 | hypothetical protein MGC11386 | MGC113 86 | 44928 | 15 | 44928 | 44928 | 44792 | 137 | 450 | >100 | 15 |
| 188 | 226392 _at | AI888503 | Homo sapiens cDNA: FLJ21652 fis, clone COL08582. | --- | 112 | 44928 | 69 | 44928 | 80 | 44849 | 94 | 15 | 15 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 189 | 235666 _at | AA90347 3 | ESTs, Weakly similar to hypothetical protein FLJ20489 [Homo sapiens] [H.sapiens] | --- | 15 | 44928 | 44928 | 44928 | 2414 | 42515 | 6329 | 58 | 15 |
| 190 | 205807 _s_at | NM_0201 27.1 | tuftelin 1 | TUFT1 | 44928 | 44928 | 44928 | 44928 | 44913 | 16 | 44 | >100 | 16 |
| 191 | 206121 _at | NM_0000 36.1 | adenosine monophosphate deaminase 1 (isoform M) | AMPD1 | 44928 | 44928 | 16 | 44928 | 236 | 44693 | 516 | 23 | 16 |
| 192 | 207697 _x_at | NM_0058 74.1 | leukocyte immunoglobulin-like receptor, subfamily B (with TM and ITIM domains), member 2 | LILRB2 | 44928 | 16 | 44928 | 44928 | 43348 | 1581 | 11408 | >100 | 16 |
| 193 | 207912 _s_at | NM_0040 81.2 | deleted in azoospermia | DAZ | 16 | 44928 | 44928 | 44928 | 1052 | 43877 | 10620 | >100 | 16 |
| 194 | 222315 _at | AW97285 5 | ESTs | --- | 44928 | 44928 | 44928 | 16 | 40968 | 3961 | 5887 | >100 | 16 |
| 195 | 58367_ s_at | AA42961 5 | hypothetical protein FLJ23233 | FLJ2323 3 | 44928 | 44928 | 44928 | 44928 | 44912 | 17 | 53 | >100 | 17 |
| 196 | 214657 _s_at | AU13497 7 | Human clone 137308 mRNA, partial cds. | --- | 44928 | 17 | 44928 | 21 | 44515 | 414 | 1432 | >100 | 17 |
| 197 | 217466 _x_at | L48784 | --- | --- | 44928 | 44928 | 17 | 44928 | 527 | 44402 | 1267 | 18 | 17 |
| 198 | 220232 _at | NM_0249 06.1 | hypothetical protein FLJ21032 | FLJ2103 2 | 44928 | 44928 | 44928 | 17 | 44432 | 497 | 1066 | >100 | 17 |
| 199 | 225698 _at | BF314746 | TIGA1 | TIGA1 | 53 | 44928 | 46 | 44928 | 342 | 44587 | 1351 | 17 | 17 |
| 200 | 232010 _at | AA12944 4 | hypothetical protein DKFZp566D234 | DKFZp5 66D234 | 17 | 44928 | 44928 | 44928 | 614 | 44315 | 6850 | 86 | 17 |
| 201 | 219429 _at | NM_0243 06.1 | fatty acid hydroxylase | FAAH | 44928 | 44928 | 44928 | 44928 | 44863 | 66 | 18 | >100 | 18 |
| 202 | 225981 _at | AW13954 9 | chromosome 17 open reading frame 28 | C17orf28 | 44928 | 44928 | 44928 | 44928 | 44911 | 18 | 83 | >100 | 18 |
| 203 | 229483 _at | AA76073 8 | ESTs | --- | 44928 | 18 | 44928 | 44928 | 44712 | 217 | 612 | >100 | 18 |
| 204 | 235940 _at | AW98369 1 | hypothetical protein MGC10999 | MGC109 99 | 71 | 44928 | 66 | 44928 | 18 | 44911 | 40 | 84 | 18 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 205 | 204836 _at | NM_0001 70.1 | glycine dehydrogenase (decarboxylating; glycine decarboxylase, glycine cleavage system protein P) | GLDC | 19 | 44928 | 44928 | 44928 | 2228 | 42701 | 23086 | 99 | 19 |
| 206 | 210800 _at | BC005236 .1 | hypothetical protein MGC12262 | MGC122 62 | 44928 | 44928 | 44928 | 44928 | 44910 | 19 | 62 | >100 | 19 |
| 207 | 222465 _at | AF165521 .1 | chromosome 15 open reading frame 15 | C15orf15 | 44928 | 44928 | 83 | 44928 | 46 | 44883 | 82 | 19 | 19 |
| 208 | 222784 _at | NM_0221 37.1 | SPARC related modular calcium binding 1 | SMOC1 | 44928 | 44928 | 19 | 44928 | 1100 | 43829 | 4324 | >100 | 19 |
| 209 | 225710 _at | H99792 | Homo sapiens cDNA FLJ34013 fis, clone FCBBF2002111. | - | 44928 | 44928 | 44928 | 19 | 44375 | 554 | 688 | >100 | 19 |
| 210 | 229170 _s_at | AW02443 7 | tetratricopeptide repeat-containing protein | LOC118 491 | 44928 | 19 | 44928 | 92 | 43950 | 979 | 5702 | >100 | 19 |
| 211 | 219373 _at | NM_0189 73.1 | dolichyl-phosphate mannosyltransferase polypeptide 3 | DPM3 | 44928 | 20 | 44928 | 44928 | 38207 | 6722 | 15777 | >100 | 20 |
| 212 | 221532 _s_at | AF309553 .1 | recombination protein REC14 | REC14 | 44928 | 44928 | 132 | 44928 | 25 | 44904 | 20 | 88 | 20 |
| 213 | 226882 _x_at | AI861913 | WD repeat domain 4 | WDR4 | 44928 | 44928 | 26 | 44928 | 20 | 44909 | 38 | >100 | 20 |
| 214 | 222410 _s_at | AF121856 .1 | sorting nexin 6 | SNX6 | 173 | 44928 | 50 | 44928 | 21 | 44908 | 35 | 39 | 21 |
| 215 | 225177 _at | AA14379 3 | Rab coupling protein | RCP | 44928 | 21 | 44928 | 44928 | 43188 | 1741 | 4334 | >100 | 21 |
| 216 | 243178 _at | AW96970 3 | ESTs, Weakly similar to hypothetical protein FLJ20489 [Homo sapiens] [H.sapiens] | --- | 44928 | 44928 | 44928 | 44928 | 44908 | 21 | 50 | >100 | 21 |
| 217 | 205671 _s_at | NM_0021 20.1 | major histocompatibility complex, class II, DO beta | HLA-DOB | 44928 | 25 | 44928 | 22 | 44677 | 252 | 596 | >100 | 22 |
| 218 | 232538 _at | AK02722 6.1 | Homo sapiens cDNA: FLJ23573 fis, clone LNG12520. | --- | 44928 | 22 | 44928 | 29 | 44459 | 470 | 2019 | >100 | 22 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 219 | 208151 _x_at | NM_0308 81.1 | DEAD/H (Asp-Glu-Ala-Asp/His) box polypeptide 17, 72kDa | DDX17 | 44928 | 44928 | 44928 | 23 | 42362 | 2567 | 8455 | >100 | 23 |
| 220 | 214246 _x_at | AI859060 | misshapen/NIK-related kinase | MINK | 44928 | 23 | 44928 | 93 | 44744 | 185 | 1197 | >100 | 23 |
| 221 | 223996 _s_at | AF151083 .1 | mitochondrial ribosomal protein L30 | MRPL30 | 44928 | 44928 | 44928 | 44928 | 23 | 44906 | 37 | >100 | 23 |
| 222 | 224330 _s_at | AB04964 7.1 | mitochondrial ribosomal protein L27 | MRPL27 | 44928 | 44928 | 59 | 44928 | 31 | 44898 | 23 | >100 | 23 |
| 223 | 227174 _at | Z98443 | ESTs | --- | 23 | 44928 | 44928 | 44928 | 1433 | 43496 | 8774 | >100 | 23 |
| 224 | 235875 _at | BF510711 | ESTs | --- | 44928 | 44928 | 44928 | 44928 | 44906 | 23 | 65 | >100 | 23 |
| 225 | 201520 _s_at | NM_0020 92.1 | G-rich RNA sequence binding factor 1 | GRSF1 | 44928 | 44928 | 102 | 44928 | 24 | 44905 | 61 | >100 | 24 |
| 226 | 211276 _at | AF063606 .1 | my048 protein | my048 | 44928 | 24 | 44928 | 44928 | 44693 | 236 | 186 | >100 | 24 |
| 227 | 223395 _at | AB05610 6.1 | DKFZP586L2024 protein | NESHBP | 24 | 44928 | 44928 | 44928 | 4177 | 40752 | 26522 | >100 | 24 |
| 228 | 237429 _at | AI677858 | ESTs | --- | 44928 | 44928 | 44928 | 44928 | 44905 | 24 | 99 | >100 | 24 |
| 229 | 215604 _x_at | AK02378 3.1 | --- | --- | 44928 | 44928 | 44928 | 44928 | 44904 | 25 | 148 | >100 | 25 |
| 230 | 239092 _at | BF939224 | ESTs, Highly similar to ITA8_HUMAN Integrin alpha-8 [H.sapiens] | --- | 25 | 44928 | 44928 | 44928 | 151 | 44778 | 1162 | >100 | 25 |
| 231 | 211747 _s_at | BC005938 .1 | LSM5 homolog, U6 small nuclear RNA associated (S. cerevisiae) | LSM5 | 122 | 44928 | 44928 | 44928 | 26 | 44903 | 54 | 50 | 26 |
| 232 | 216274 _s_at | N99438 | signal peptidase complex (18kD) | SPC18 | 26 | 44928 | 44928 | 44928 | 102 | 44827 | 359 | 34 | 26 |
| 233 | 236427 _at | BF830560 | ESTs | --- | 44928 | 26 | 44928 | 44928 | 44074 | 855 | 2194 | >100 | 26 |
| 234 | 203058 _s_at | AW29995 8 | 3'-phosphoadenosine 5'-phosphosulfate synthase 2 | PAPSS2 | 44928 | 27 | 44928 | 44928 | 44761 | 168 | 593 | >100 | 27 |
| 235 | 200043 _at | NM_0044 50.1 | enhancer of rudimentary homolog (Drosophila) | ERH | 44928 | 44928 | 47 | 44928 | 27 | 44902 | 63 | 40 | 27 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 236 | 234087 _at | AK02234 3.1 | EST, Moderately similar to hypothetical protein FLJ20294 [Homo sapiens] [H.sapiens] | --- | 44928 | 29 | 44928 | 44928 | 44902 | 27 | 79 | >100 | 27 |
| 237 | 242311 _x_at | H37943 | ESTs, Weakly similar to hypothetical protein FLJ20489 [Homo sapiens] [H. sapiens] | --- | 44928 | 44928 | 44928 | 27 | 44590 | 339 | 667 | >100 | 27 |
| 238 | 213307 _at | AB02894 5.1 | SH3 and multiple ankyrin repeat domains 2 | SHANK 2 | 44928 | 44928 | 44928 | 44928 | 44901 | 28 | 43 | >100 | 28 |
| 239 | 237414 _at | H70477 | coagulation factor VII (serum prothrombin conversion accelerator) | F7 | 44928 | 44928 | 44928 | 28 | 44539 | 390 | 2002 | >100 | 28 |
| 240 | 239555 _at | W87626 | ESTs | --- | 44928 | 28 | 44928 | 44928 | 40008 | 4921 | 12979 | >100 | 28 |
| 241 | 222893 _s_at | AI609064 | hypothetical protein FLJ13150 | FLJ1315 0 | 44928 | 44928 | 44928 | 44928 | 29 | 44900 | 47 | >100 | 29 |
| 242 | 225647 _s_at | AI246687 | cathepsin C | CTSC | 44928 | 44928 | 29 | 44928 | 56 | 44873 | 30 | >100 | 29 |
| 243 | 233876 _at | AK00067 7.1 | Homo sapiens cDNA FLJ20670 fis, clone KAIA4743. | --- | 44928 | 44928 | 44928 | 44928 | 44900 | 29 | 105 | >100 | 29 |
| 244 | 201554 _x_at | NM_0041 30.1 | glycogenin | GYG | 128 | 44928 | 40 | 44928 | 67 | 44862 | 387 | 30 | 30 |
| 245 | 203561 _at | NM_0216 42.1 | Fc fragment of IgG, low affinity IIa, receptor for (CD32) | FCGR2A | 44928 | 44928 | 44928 | 97 | 44899 | 30 | 74 | >100 | 30 |
| 246 | 214594 _x_at | BG25266 6 | ATPase, Class I, type 8B, member 1 | ATP8B1 | 44928 | 44928 | 44928 | 30 | 44816 | 113 | 236 | >100 | 30 |
| 247 | 219030 _at | NM_0160 58.1 | CGI-121 protein | CGI-121 | 44928 | 44928 | 44928 | 44928 | 30 | 44899 | 56 | >100 | 30 |
| 248 | 219233 _s_at | NM_0185 30.1 | hypothetical protein PRO2521 | PRO252 1 | 44928 | 30 | 44928 | 44928 | 44418 | 511 | 1342 | >100 | 30 |
| 249 | 242135 _at | AA92753 3 | Homo sapiens cDNA FLJ32537 fis, clone SMINT2000400, highly similar to Homo sapiens FRG1 mRNA. | --- | 30 | 44928 | 44928 | 44928 | 661 | 44268 | 3000 | >100 | 30 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 250 | 228726 _at | AW51219 6 | ESTs, Weakly similar to hypothetical protein FLJ20489 [Homosapiens] [H.sapiens] | --- | 44928 | 42 | 44928 | 44928 | 44898 | 31 | 84 | >100 | 31 |
| 251 | 208642 _s_at | AA20583 4 | X-ray repair complementing defective repair in Chinese hamster cells 5 (double-strand-break rejoining; Ku autoantigen, 80kDa) | XRCC5 | 44928 | 44928 | 161 | 44928 | 32 | 44897 | 70 | 74 | 32 |
| 252 | 220725 _x_at | NM_0250 95.1 | hypothetical protein FLJ23558 | FLJ2355 8 | 44928 | 32 | 44928 | 44928 | 44060 | 869 | 2613 | >100 | 32 |
| 253 | 220755 _s_at | NM_0169 47.1 | chromosome 6 open reading frame 48 | C6orf48 | 32 | 44928 | 64 | 44928 | 431 | 44498 | 1780 | 35 | 32 |
| 254 | 229269 _x_at | BF976372 | myo-inositol 1-phosphate synthase A1 | ISYNA1 | 44928 | 44928 | 32 | 44928 | 809 | 44120 | 3681 | >100 | 32 |
| 255 | 232659 _at | AU14686 4 | Homo sapiens cDNA FLJ12017 fis, clone HEMBB1001735. | --- | 44928 | 44928 | 44928 | 44928 | 44897 | 32 | 178 | >100 | 32 |
| 256 | 244042 _x_at | AA88383 1 | ESTs | --- | 44928 | 44928 | 44928 | 32 | 44833 | 96 | 120 | >100 | 32 |
| 257 | 204518 _s_at | NM_0009 43.1 | peptidylprolyl isomerase C (cyclophilin C) | PPIC | 44928 | 44928 | 44928 | 33 | 44763 | 166 | 841 | >100 | 33 |
| 258 | 205500 _at | NM_0017 35.1 | complement component 5 | C5 | 44928 | 44928 | 44928 | 44928 | 44896 | 33 | 86 | >100 | 33 |
| 259 | 209345 _s_at | AL561930 | phosphatidylinositol 4-kinase type II | PI4KII | 44928 | 44928 | 44928 | 44928 | 44890 | 39 | 33 | >100 | 33 |
| 260 | 222531 _s_at | AW13752 6 | chromosome 14 open reading frame 108 | C14orf10 8 | 44928 | 44928 | 41 | 44928 | 33 | 44896 | 111 | 54 | 33 |
| 261 | 224709 _s_at | AF131831 .1 | non-kinase Cdc42 effector protein SPEC2 | SPEC2 | 143 | 44928 | 62 | 44928 | 280 | 44649 | 857 | 33 | 33 |
| 262 | 209427 _at | AF064238 .3 | smoothelin | SMTN | 44928 | 44928 | 44928 | 44928 | 44895 | 34 | 59 | >100 | 34 |
| 263 | 236254 _at | BE048857 | hypothetical protein MGC45726 | MGC457 26 | 44928 | 34 | 44928 | 44928 | 44254 | 675 | 2739 | >100 | 34 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 264 | 201056_at | N53479 | Homo sapiens cDNAFLJ37232 fis, clone BRAMY2001114. | --- | 44928 | 44928 | 44928 | 44928 | 44894 | 35 | 66 | >100 | 35 |
| 265 | 205644 _s_at | NM_0030 96.1 | small nuclear ribonucleoprotein polypeptide G | SNRPG | 155 | 44928 | 44928 | 44928 | 35 | 44894 | 77 | 37 | 35 |
| 266 | 228919 _at | AA60103 1 | ESTs, Highly similar to cell division cycle 2-like 1, isoform 1; Cell division cycle 2-like 1; PITSLRE protein kinase alpha; p58/GTA protein kinase; galactosyltransferase associated protein kinase; CDC-related protein kinase p58; PITSLRE B [Homo sapiens] [H.sapiens] | --- | 44928 | 44928 | 44928 | 35 | 41176 | 3753 | 12711 | >100 | 35 |
| 267 | 231131 _at | AA90933 0 | hypothetical protein FLJ37659 | FLJ3765 9 | 35 | 44928 | 44928 | 44928 | 1469 | 43460 | 6555 | 71 | 35 |
| 268 | 240587 _x_at | AI478814 | ESTs | --- | 44928 | 35 | 44928 | 44928 | 36474 | 8455 | 27078 | > 100 | 35 |
| 269 | AFFX-HUMR GE/M1 0098_ M_at | M10098 | --- | --- | 44928 | 44928 | 44928 | 36 | 25931 | 18998 | 37580 | >100 | 36 |
| 270 | 212238 _at | AL117518 .1 | additional sex combs like 1 (Drosophila) | ASXL1 | 44928 | 44928 | 44928 | 44928 | 44893 | 36 | 80 | >100 | 36 |
| 271 | 221434 _s_at | NM_0312 10.1 | hypothetical protein DC50 | DC50 | 44928 | 44928 | 44928 | 44928 | 36 | 44893 | 103 | >100 | 36 |
| 272 | 223029 _s_at | AL136921 .1 | ring finger and WD repeat domain 1 | RFWD1 | 39 | 44928 | 36 | 44928 | 104 | 44825 | 1374 | >100 | 36 |
| 273 | 227641_at | AI613010 | hypothetical protein MGC33974 | MGC33974 | 36 | 44928 | 105 | 44928 | 124 | 44805 | 313 | >100 | 36 |
| 274 | 206323 _x_at | NM_0025 47.1 | oligophrenin 1 | OPHN1 | 44928 | 44928 | 44928 | 37 | 44545 | 384 | 324 | >100 | 37 |
| 275 | 211424 _x_at | AF113007 .1 | DKFZP586A0522 protein | DKFZP5 86A0522 | 44928 | 37 | 44928 | 77 | 44775 | 154 | 575 | >100 | 37 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 276 | 215322 _at | AL080190 .1 | Homo sapiens mRNA; cDNA DKFZp434A202 (from clone DKFZp434A202) | --- | 44928 | 44928 | 44928 | 44928 | 44892 | 37 | 116 | >100 | 37 |
| 277 | 222713 _s_at | AF181995 .1 | Fanconi anemia, complementation group F | FANCF | 160 | 44928 | 154 | 44928 | 37 | 44892 | 151 | >100 | 37 |
| 278 | 228496 _s_at | AW24308 1 | cysteine-rich motor neuron 1 | CRIM1 | 37 | 44928 | 44928 | 44928 | 5459 | 39470 | 29457 | >100 | 37 |
| 279 | 221223 _x_at | NM_0133 24.2 | cytokine inducible SH2-containing protein | CISH | 44928 | 44928 | 44928 | 44928 | 44891 | 38 | 57 | >100 | 38 |
| 280 | 224673 _at | AI613244 | --- | --- | 44928 | 38 | 44928 | 67 | 44728 | 201 | 561 | >100 | 38 |
| 281 | 224841 _x_at | BF316352 | Homo sapiens mRNA; cDNA DKFZp564D0164 (from clone DKFZp564D0164) | --- | 104 | 44928 | 38 | 44928 | 1040 | 43889 | 3386 | 46 | 38 |
| 282 | 237266 _at | BE552347 | Kv channel interacting protein 2 | KCNIP2 | 44928 | 39 | 44928 | 44928 | 43140 | 1789 | 11320 | >100 | 39 |
| 283 | 244357 _at | T90760 | ESTs | --- | 44928 | 44928 | 44928 | 39 | 43992 | 937 | 3272 | >100 | 39 |
| 284 | 228434 _at | AA80696 5 | Homo sapiens, Similar to hypothetical protein B430208I01, clone IMAGE:5181522, mRNA, partial cds | --- | 44928 | 44928 | 44928 | 40 | 44467 | 462 | 1357 | >100 | 40 |
| 285 | 232746 _at | BE552368 | Homo sapiens cDNA FLJ13445 fis, clonePLACE1002962. | --- | 44928 | 44928 | 44928 | 44928 | 44889 | 40 | 64 | >100 | 40 |
| 286 | 37793_ r_at | AF034956 | RAD51-like 3 (S. cerevisiae) | RAD51L 3 | 44928 | 44928 | 44928 | 44928 | 44888 | 41 | 126 | >100 | 41 |
| 287 | 203408 _s_at | NM_0029 71.1 | special AT-rich sequence binding protein 1 (binds to nuclear matrix/scaffold-associating DNA's) | SATB1 | 44928 | 44928 | 44928 | 41 | 43257 | 1672 | 1941 | >100 | 41 |
| 288 | 207124 _s_at | NM_0065 78.1 | guanine nucleotide binding protein (G protein), beta 5 | GNB5 | 44928 | 44928 | 44928 | 44928 | 41 | 44888 | 184 | >100 | 41 |
| 289 | 208844 _at | BC002456 .1 | --- | --- | 44928 | 44928 | 44928 | 44928 | 44887 | 42 | 137 | >100 | 42 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 290 | 218139 _s_at | NM_0182 29.1 | chromosome 14 open reading frame 108 | C14orf10 8 | 44928 | 44928 | 44928 | 44928 | 42 | 44887 | 55 | >100 | 42 |
| 291 | 224579 _at | AK02426 3.1 | Homo sapiens cDNA FLJ14201 fis, clone NT2RP3002955. | --- | 44928 | 44928 | 42 | 44928 | 400 | 44529 | 757 | 52 | 42 |
| 292 | 244359 _s_at | H28915 | ESTs | --- | 42 | 44928 | 44928 | 44928 | 3802 | 41127 | 28000 | >100 | 42 |
| 293 | 53987_ at | AL041852 | KIAA1464 protein | KIAA14 64 | 44928 | 44928 | 44928 | 44928 | 44886 | 43 | 127 | >100 | 43 |
| 294 | 212307 _s_at | BF001665 | O-linked N-acetylglucosamine (GlcNAc) transferase (UDP-N-acetylglucosamine:polyp eptide-N-acetylglucosaminyl transferase) | OGT | 44928 | 43 | 44928 | 44928 | 33355 | 11574 | 18158 | >100 | 43 |
| 295 | 232098 _at | AK02514 2.1 | ESTs | --- | 44928 | 44928 | 44928 | 43 | 42790 | 2139 | 2890 | >100 | 43 |
| 296 | 215908 _at | AF009267 .1 | Homo sapiens full length insert cDNA YU79F10 | --- | 44928 | 44 | 44928 | 44928 | 44462 | 467 | 1470 | >100 | 44 |
| 297 | 217294 _s_at | U88968.1 | enolase 1, (alpha) | ENO1 | 44 | 44928 | 44928 | 44928 | 47 | 44882 | 135 | >100 | 44 |
| 298 | 220852 _at | NM_0140 99.1 | PRO1768 protein | PRO176 8 | 44928 | 44928 | 44928 | 44928 | 44885 | 44 | 102 | >100 | 44 |
| 299 | 225402 _at | BG33945 0 | chromosome 20 open reading frame 64 | C20orf64 | 44928 | 44928 | 44928 | 44928 | 44 | 44885 | 78 | >100 | 44 |
| 300 | 212923 _s_at | AK02482 8.1 | hypothetical protein LOC221749 | LOC221 749 | 44928 | 44928 | 44928 | 44928 | 44884 | 45 | 123 | >100 | 45 |
| 301 | 222714 _s_at | BC000878 .1 | CGI-83 protein | CGI-83 | 44928 | 44928 | 44928 | 44928 | 45 | 44884 | 104 | >100 | 45 |
| 302 | 229050 _s_at | AL533103 | Homo sapiens cDNA FLJ30346 fis, clone BRACE2007527. | --- | 45 | 44928 | 44928 | 44928 | 2495 | 42434 | 6112 | >100 | 45 |
| 303 | 240593 _x_at | R98767 | ESTs, Weakly similar to hypothetical protein FLJ20378 [Homo sapiens] [H. sapiens] | --- | 44928 | 45 | 44928 | 44928 | 39771 | 5158 | 14507 | >100 | 45 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 304 | 241722 _x_at | BF724558 | ESTs, Moderately similar to T02670 probable thromboxane A2 receptor isoform beta - human [H.sapiens] | --- | 44928 | 44928 | 44928 | 45 | 43069 | 1860 | 3871 | >100 | 45 |
| 305 | 212110 _at | D31887.1 | KIAA0062 protein | KIAA00 62 | 44928 | 46 | 44928 | 44928 | 27676 | 17253 | 28338 | >100 | 46 |
| 306 | 215628 _x_at | AL049285 .1 | Homo sapiens mRNA; cDNA DKFZp564M193 (from clone DKFZp564M193) | --- | 44928 | 44928 | 44928 | 46 | 44499 | 430 | 654 | >100 | 46 |
| 307 | 236946 _at | AI220134 | ESTs | --- | 44928 | 44928 | 44928 | 44928 | 44883 | 46 | 204 | >100 | 46 |
| 308 | 210992 _x_at | U90939.1 | Fc fragment of IgG, low affinity IIa, receptor for (CD32) | FCGR2A | 44928 | 44928 | 44928 | 47 | 43239 | 1690 | 3640 | >100 | 47 |
| 309 | 217527 _s_at | AI478300 | Homo sapiens, clone IMAGE:3659798, mRNA | --- | 44928 | 47 | 44928 | 44928 | 40926 | 4003 | 14691 | >100 | 47 |
| 310 | 219183 _s_at | NM_0133 85.2 | pleckstrin homology, Sec7 and coiled/coil domains 4 | PSCD4 | 44928 | 44928 | 44928 | 44928 | 44882 | 47 | 101 | >100 | 47 |
| 311 | 200826 _at | NM_0045 97.3 | small nuclear ribonucleoprotein D2 polypeptide 16.5kDa | SNRPD2 | 165 | 44928 | 44928 | 44928 | 48 | 44881 | 221 | 89 | 48 |
| 312 | 203663 _s_at | NM_0042 55.1 | cytochrome c oxidase subunit Va | COX5A | 44928 | 44928 | 110 | 44928 | 52 | 44877 | 48 | >100 | 48 |
| 313 | 209049 _s_at | BC001004 .1 | protein kinase C binding protein 1 | PRKCBP 1 | 44928 | 48 | 44928 | 44928 | 39921 | 5008 | 15023 | >100 | 48 |
| 314 | 209486 _at | BC004546 .1 | disrupter of silencing 10 | SAS10 | 79 | 44928 | 48 | 44928 | 144 | 44785 | 600 | 57 | 48 |
| 315 | 213345 _at | AI624015 | nuclear factor of activated T-cells, cytoplasmic, calcineurin-dependent 4 | NFATC4 | 44928 | 44928 | 44928 | 44928 | 44881 | 48 | 51 | >100 | 48 |
| 316 | 223076 _s_at | BC001041 .1 | hypothetical protein FLJ20303 | FLJ2030 3 | 48 | 44928 | 44928 | 44928 | 566 | 44363 | 2838 | 69 | 48 |
| 317 | 224364 _s_at | AF251049 .1 | peptidylprolyl isomerase (cyclophilin)-like 3 | PPIL3 | 139 | 44928 | 44928 | 44928 | 121 | 44808 | 368 | 48 | 48 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 318 | 212750 _at | AB02063 0.1 | protein phosphatase 1, regulatory (inhibitor) subunit 16B | PPP1R16 B | 44928 | 44928 | 49 | 44928 | 953 | 43976 | 2373 | >100 | 49 |
| 319 | 219203 _at | NM_0160 49.1 | CGI-112 protein | CGI-112 | 44928 | 44928 | 44928 | 44928 | 49 | 44880 | 271 | >100 | 49 |
| 320 | 224741 _x_at | BG32917 5 | Homo sapiens mRNA; cDNA DKFZp564D0164 (from clone DKFZp564D0164) | --- | 49 | 44928 | 70 | 44928 | 1470 | 43459 | 5688 | 53 | 49 |
| 321 | 227062_at | AU15536 1 | plectin 1, intermediate filament binding protein 500kDa | PLEC1 | 44928 | 44928 | 44928 | 49 | 44613 | 316 | 708 | >100 | 49 |
| 322 | 232516 _x_at | AU15038 5 | YY1 associated protein | YAP | 44928 | 44928 | 44928 | 101 | 44880 | 49 | 153 | >100 | 49 |
| 323 | 207573 _x_at | NM_0064 76.1 | ATP synthase, H+ transporting, mitochondrial F0 complex, subunit g | ATP5L | 50 | 44928 | 44928 | 44928 | 168 | 44761 | 305 | 56 | 50 |
| 324 | 212644 _s_at | AI671747 | chromosome 14 open reading frame 32 | C14orf32 | 44928 | 44928 | 44928 | 44928 | 50 | 44879 | 89 | >100 | 50 |
| 325 | 231825 _x_at | AK02506 0.1 | activating transcription factor 7 interacting protein | ATF7IP | 44928 | 44928 | 44928 | 44928 | 44879 | 50 | 152 | >100 | 50 |
| 326 | 239331 _at | AW95419 9 | ESTs | --- | 44928 | 44928 | 44928 | 50 | 42943 | 1986 | 4181 | >100 | 50 |
| 327 | 209733 _at | AL034399 | hypothetical protein LOC286440 | LOC286 440 | 44928 | 44928 | 44928 | 44928 | 44878 | 51 | 283 | >100 | 51 |
| 328 | 230876 _at | AI827906 | hypothetical protein LOC169834 | LOC169 834 | 51 | 44928 | 44928 | 44928 | 658 | 44271 | 3954 | >100 | 51 |
| 329 | 216750 _at | AK02487 1.1 | amyloid beta (A4) precursor protein-binding, family B, member 2 (Fe65-like) | APBB2 | 44928 | 44928 | 44928 | 44928 | 44877 | 52 | 277 | >100 | 52 |
| 330 | 228728 _at | BF724137 | hypothetical protein FLJ21986 | FLJ2198 6 | 52 | 44928 | 85 | 44928 | 215 | 44714 | 1139 | >100 | 52 |
| 331 | 230014 _at | BF515592 | ESTs | --- | 44928 | 44928 | 44928 | 52 | 41139 | 3790 | 8523 | >100 | 52 |
| 332 | 210715 _s_at | AF027205 .1 | serine protease inhibitor, Kunitz type, 2 | SPINT2 | 44928 | 44928 | 44928 | 53 | 40070 | 4859 | 8720 | >100 | 53 |

(continued)

| No. | Probes et ID | Sequence De-rived From | Title | Gene Sym-bol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 333 | 218467 _at | NM_0202 32.1 | hepatocellular carcinoma susceptibility protein | HCCA3 | 44928 | 44928 | 44928 | 44928 | 53 | 44876 | 149 | 100 | 53 |
| 334 | AFFX-HUMI SGF3A /M979 35_M A_at | M97935 | --- | --- | 44928 | 44928 | 53 | 44928 | 708 | 44221 | 1068 | >100 | 53 |
| 335 | 204227 _s_at | NM_0046 14.1 | thymidine kinase 2, mitochondrial | TK2 | 44928 | 44928 | 44928 | 44928 | 44875 | 54 | 114 | >100 | 54 |
| 336 | 232138 _at | AW27691 4 | Homo sapiens clone IMAGE:713177, mRNA sequence | --- | 44928 | 44928 | 44928 | 54 | 44534 | 395 | 1280 | >100 | 54 |
| 337 | 204517 _at | BE962749 | peptidylprolyl isomerase C (cyclophilin C) | PPIC | 44928 | 44928 | 44928 | 55 | 44402 | 527 | 978 | >100 | 55 |
| 338 | 211275 _s_at | AF087942 .1 | glycogenin | GYG | 131 | 44928 | 44928 | 44928 | 369 | 44560 | 1427 | 55 | 55 |
| 339 | 226888 _at | BG10486 0 | casein kinase 1, gamma 1 | CSNK1G 1 | 44928 | 44928 | 44928 | 44928 | 55 | 44874 | 58 | >100 | 55 |
| 340 | AFFX-HUMI SGF3A /M979 35_MR _at | M97935 | --- | --- | 44928 | 44928 | 56 | 44928 | 454 | 44475 | 523 | >100 | 56 |
| 341 | 225373 _at | BE271644 | PP2135 protein | PP2135 | 44928 | 44928 | 44928 | 56 | 44814 | 115 | 372 | >100 | 56 |
| 342 | 205618 _at | NM_0009 50.1 | proline-rich Gla (G- carboxyglutamic acid) polypeptide 1 | PRRG1 | 44928 | 44928 | 44928 | 44928 | 44872 | 57 | 81 | >100 | 57 |
| 343 | 200030 _s_at | NM_0026 35.1 | solute carrier family 25 (mitochondrial carrier; phosphate carrier), member 3 | SLC25A 3 | 44928 | 44928 | 44928 | 44928 | 57 | 44872 | 91 | 67 | 57 |
| 344 | 228400 _at | AW02514 1 | ESTs | --- | 57 | 44928 | 44928 | 44928 | 223 | 44706 | 1047 | >100 | 57 |
| 345 | 201491 _at | NM_0121 11.1 | chromosome 14 open reading frame 3 | C14orf3 | 44928 | 44928 | 44928 | 44928 | 58 | 44871 | 107 | >100 | 58 |
| 346 | 209031 _at | NM_0143 33.1 | immunoglobulin superfamily, member 4 | IGSF4 | 44928 | 44928 | 58 | 44928 | 2854 | 42075 | 8458 | >100 | 58 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 347 | 222529 _at | BG25146 7 | mitochondrial solute carrier protein | MSCP | 44928 | 44928 | 44928 | 58 | 27388 | 17541 | 33137 | >100 | 58 |
| 348 | 244142 _at | D60329 | ESTs | --- | 44928 | 44928 | 44928 | 44928 | 44871 | 58 | 125 | >100 | 58 |
| 349 | 226227 _x_at | BF185165 | Homo sapiens, clone IMAGE:5285034, mRNA | --- | 73 | 44928 | 44928 | 44928 | 675 | 44254 | 1792 | 59 | 59 |
| 350 | 226830 _x_at | BG33924 5 | Homo sapiens cDNA FLJ14030 fis, clone HEMBA1004086. | --- | 44928 | 44928 | 44928 | 44928 | 59 | 44870 | 166 | >100 | 59 |
| 351 | 233234 _at | AB03773 8.1 | KIAA1317 protein | KIAA13 17 | 44928 | 44928 | 44928 | 59 | 44197 | 732 | 15108 | >100 | 59 |
| 352 | 243147 _x_at | AW11870 7 | ESTs, Weakly similar to YYY1_HUMAN Very very hypothetical protein RMSA-1 [H.sapiens] | --- | 44928 | 44928 | 44928 | 44928 | 44870 | 59 | 68 | >100 | 59 |
| 353 | 221458 _at | NM_0008 66.1 | 5-hydroxytryptamine (serotonin) receptor 1F | HTR1F | 44928 | 44928 | 44928 | 44928 | 44869 | 60 | 106 | >100 | 60 |
| 354 | 225084 _at | BG17074 3 | SEC10-like 1 (S. cerevisiae) | SEC10L 1 | 44928 | 44928 | 122 | 44928 | 69 | 44860 | 141 | 60 | 60 |
| 355 | 227598 _at | AI762857 | hypothetical protein BC011406 | LOC113 763 | 44928 | 44928 | 44928 | 44928 | 76 | 44853 | 60 | >100 | 60 |
| 356 | 235113 _at | AA74224 4 | peptidylprolyl isomerase (cyclophilin) like 5 | PPIL5 | 44928 | 44928 | 60 | 44928 | 200 | 44729 | 456 | >100 | 60 |
| 357 | 242749 _at | AI022173 | ESTs | --- | 44928 | 44928 | 44928 | 60 | 43605 | 1324 | 4746 | >100 | 60 |
| 358 | AFFX-HUMRGE/M1 0098_M_at | M10098 | --- | --- | 44928 | 44928 | 44928 | 61 | 24464 | 20465 | 33430 | >100 | 61 |
| 359 | 225281_at | AL117573.1 | DKFZP434F2021 protein | DKFZP4 34F2021 | 44928 | 44928 | 44928 | 44928 | 132 | 44797 | 194 | 61 | 61 |
| 360 | 234942 _s_at | AK02522 0.1 | --- | --- | 44928 | 44928 | 44928 | 44928 | 61 | 44868 | 248 | >100 | 61 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 361 | 213873 _at | D29810.1 | endothelial and smooth muscle cell-derived neuropilin-like protein | ESDN | 44928 | 44928 | 44928 | 44928 | 44867 | 62 | 73 | >100 | 62 |
| 362 | 216524 _x_at | AL049260 .1 | Homo sapiens mRNA; cDNA DKFZp564E233 (from clone DKFZp564E233) | --- | 44928 | 44928 | 44928 | 62 | 44161 | 768 | 1958 | >100 | 62 |
| 363 | 231265 _at | AI126453 | cytochrome c oxidase subunit VIIb2 | COX7B2 | 62 | 44928 | 44928 | 44928 | 2009 | 42920 | 21140 | >100 | 62 |
| 364 | 201264 _at | NM_0072 63.1 | coatomer protein complex, subunit epsilon | COPE | 80 | 44928 | 96 | 44928 | 176 | 44753 | 739 | 63 | 63 |
| 365 | 222510 _s_at | AI809203 | makorin, ring finger protein, 2 | MKRN2 | 44928 | 44928 | 44928 | 44928 | 63 | 44866 | 110 | >100 | 63 |
| 366 | 226179 _at | N63920 | Homo sapiens, clone IMAGE:5294823, mRNA | --- | 44928 | 44928 | 44928 | 63 | 27539 | 17390 | 31921 | >100 | 63 |
| 367 | 226835 _s_at | BG33052 0 | Homo sapiens, clone IMAGE:5285034, mRNA | --- | 44928 | 44928 | 63 | 44928 | 1324 | 43605 | 4164 | >100 | 63 |
| 368 | 228159 _at | N45312 | Homo sapiens cDNA FLJ38039 fis, clone CTONG2013934. | --- | 44928 | 44928 | 44928 | 44928 | 44866 | 63 | 290 | >100 | 63 |
| 369 | 202026 _at | NM_0030 02.1 | succinate dehydrogenase complex, subunit D, integral membrane protein | SDHD | 44928 | 44928 | 44928 | 44928 | 64 | 44865 | 189 | >100 | 64 |
| 370 | 220534 _at | NM_0241 14.1 | tripartite motif- containing 48 | TRIM48 | 44928 | 44928 | 44928 | 44928 | 44865 | 64 | 124 | >100 | 64 |
| 371 | 239294 _at | AA81026 5 | ESTs | --- | 64 | 44928 | 44928 | 44928 | 867 | 44062 | 3303 | 82 | 64 |
| 372 | 224298_s_at | BC004528.1 | phosphoglyceratedehydrogenase like 1 | PHGDH L1 | 65 | 44928 | 44928 | 44928 | 1198 | 43731 | 15433 | >100 | 65 |
| 373 | 224558 _s_at | BG48393 9 | PRO1073 protein | PRO107 3 | 44928 | 44928 | 44928 | 65 | 40007 | 4922 | 10881 | >100 | 65 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 374 | 244172 _at | AA93156 2 | ESTs, Weakly similar to hypothetical protein FLJ20489 [Homo sapiens] [H.sapiens] | --- | 44928 | 44928 | 44928 | 85 | 44864 | 65 | 143 | >100 | 65 |
| 375 | 205370 _x_at | NM_0019 18.1 | dihydrolipoamide branched chain transacylase (E2 component of branched chain keto acid dehydrogenase complex; maple syrup urine disease) | DBT | 44928 | 44928 | 44928 | 66 | 44434 | 495 | 1851 | >100 | 66 |
| 376 | 222789 _at | BE888593 | hypothetical protein FLJ11220 | FLJ1122 0 | 44928 | 44928 | 44928 | 44928 | 66 | 44863 | 76 | >100 | 66 |
| 377 | 226558 _at | BE856637 | ESTs | --- | 66 | 44928 | 44928 | 44928 | 751 | 44178 | 2501 | >100 | 66 |
| 378 | 215109 _at | R02172 | ESTs, Moderately similar to hypothetical protein FLJ20234 [Homo sapiens] [H.sapiens] | --- | 44928 | 44928 | 44928 | 44928 | 44862 | 67 | 203 | >100 | 67 |
| 379 | 224740 _at | BE613001 | Homo sapiens, clone IMAGE:4620009, mRNA | --- | 44928 | 44928 | 67 | 44928 | 426 | 44503 | 263 | 70 | 67 |
| 380 | 226265 _at | AW29489 4 | hypothetical protein FLJ21924 | FLJ2192 4 | 67 | 44928 | 44928 | 44928 | 145 | 44784 | 397 | >100 | 67 |
| 381 | 217188 _s_at | AC00718 2 | chromosome 14 open reading frame 1 | C14orf1 | 68 | 44928 | 44928 | 44928 | 245 | 44684 | 508 | >100 | 68 |
| 382 | 229466 _at | AU14418 7 | hypothetical protein LOC256273 | LOC256 273 | 44928 | 44928 | 44928 | 44928 | 44861 | 68 | 139 | >100 | 68 |
| 383 | 242619_x_at | H82831 | ESTs | --- | 44928 | 44928 | 44928 | 68 | 44810 | 119 | 408 | >100 | 68 |
| 384 | 220073 _s_at | NM_0181 73.1 | hypothetical protein FLJ10665 | FLJ1066 5 | 44928 | 44928 | 44928 | 44928 | 44860 | 69 | 361 | >100 | 69 |
| 385 | 210092 _at | AF067173 .1 | mago-nashi homolog, proliferation-associated (Drosophila) | MAGOH | 44928 | 44928 | 44928 | 44928 | 70 | 44859 | 157 | >100 | 70 |
| 386 | 213371 _at | AI803302 | LIM domain binding 3 | LDB3 | 44928 | 44928 | 44928 | 44928 | 44859 | 70 | 132 | >100 | 70 |
| 387 | 229655 _at | N66656 | hypothetical protein CLONE25003 | CLONE2 5003 | 70 | 44928 | 44928 | 44928 | 4007 | 40922 | 24679 | >100 | 70 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 388 | 228866 _at | BF514864 | Homo sapiens cDNA FLJ13825 fis, clone THYRO1000558. | --- | 44928 | 44928 | 44928 | 71 | 43995 | 934 | 494 | >100 | 71 |
| 389 | 244795 _at | AV69398 6 | ESTs | --- | 44928 | 44928 | 44928 | 44928 | 44858 | 71 | 273 | >100 | 71 |
| 390 | 204610 _s_at | NM_0068 48.1 | hepatitis delta antigen-interacting protein A | DIPA | 44928 | 44928 | 72 | 44928 | 1914 | 43015 | 8164 | >100 | 72 |
| 391 | 225218 _at | AA20575 4 | hypothetical protein FLJ32919 | FLJ3291 9 | 44928 | 44928 | 44928 | 44928 | 44857 | 72 | 169 | >100 | 72 |
| 392 | 225904 _at | N64686 | Homo sapiens cDNA FLJ25935 fis, clone JTH06710. | --- | 87 | 44928 | 78 | 44928 | 1309 | 43620 | 4215 | 72 | 72 |
| 393 | 206992 _s_at | NM_0156 84.1 | ATP synthase, H+ transporting, mitochondrial F0 complex, subunits (factor B) | ATP5S | 44928 | 44928 | 44928 | 44928 | 73 | 44856 | 145 | >100 | 73 |
| 394 | 226944 _at | AW51872 8 | serine protease HTRA3 | HTRA3 | 44928 | 44928 | 44928 | 44928 | 44856 | 73 | 196 | >100 | 73 |
| 395 | 227084 _at | AW33931 0 | dystrobrevin, alpha | DTNA | 44928 | 44928 | 44928 | 73 | 44615 | 314 | 833 | >100 | 73 |
| 396 | 209703 _x_at | BC004492 .1 | DKFZP586A0522 protein | DKFZP5 86A0522 | 44928 | 44928 | 44928 | 74 | 42035 | 2894 | 1118 | >100 | 74 |
| 397 | 210154_at | M55905.1 | malic enzyme 2, NAD(+)-dependent, mitochondrial | ME2 | 44928 | 44928 | 44928 | 44928 | 74 | 44855 | 98 | >100 | 74 |
| 398 | 226050 _at | AL576117 | chromosome 13 open reading frame 11 | C13orf11 | 74 | 44928 | 44928 | 44928 | 1168 | 43761 | 5900 | >100 | 74 |
| 399 | 209340 _at | S73498.1 | UDP-N-acteylglucosamine pyrophosphorylase 1 | UAP1 | 124 | 44928 | 75 | 44928 | 2926 | 42003 | 12143 | 79 | 75 |
| 400 | 215504 _x_at | AF131777 .1 | Homo sapiens clone 25061 mRNA sequence | --- | 44928 | 44928 | 44928 | 75 | 44199 | 730 | 1434 | >100 | 75 |
| 401 | 219878 _s_at | NM_0159 95.1 | Kruppel-like factor 13 | KLF13 | 44928 | 44928 | 44928 | 44928 | 75 | 44854 | 175 | >100 | 75 |
| 402 | 221978 _at | BE138825 | major histocompatibility complex, class I, F | HLA-F | 44928 | 44928 | 44928 | 44928 | 44854 | 75 | 176 | >100 | 75 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 403 | 226051 _at | BF973568 | selenoprotein SelM | SELM | 44928 | 44928 | 44928 | 76 | 43355 | 1574 | 2394 | >100 | 76 |
| 404 | 208690 _s_at | BC000915 .1 | PDZ and LIM domain 1 (elfin) | PDLIM1 | 77 | 44928 | 124 | 44928 | 1120 | 43809 | 3441 | >100 | 77 |
| 405 | 213738 _s_at | AI587323 | ATP synthase, H+ transporting, mitochondrial F1 complex, alpha subunit, isoform 1, cardiac muscle | ATP5A1 | 44928 | 44928 | 44928 | 44928 | 77 | 44852 | 191 | >100 | 77 |
| 406 | 226276 _at | BF439522 | hypothetical protein LOC153339 | LOC153 339 | 44928 | 44928 | 77 | 44928 | 781 | 44148 | 909 | >100 | 77 |
| 407 | 39313_ at | AB00234 2 | protein kinase, lysine deficient 1 | PRKWN K1 | 44928 | 44928 | 44928 | 44928 | 44850 | 79 | 343 | >100 | 79 |
| 408 | 222109 _at | AA55858 3 | hypothetical protein FLJ10613 | FLJ1061 3 | 44928 | 44928 | 44928 | 79 | 44834 | 95 | 310 | >100 | 79 |
| 409 | 211474 _s_at | BC004948 .1 | serine (or cysteine) proteinase inhibitor, clade B (ovalbumin), member 6 | SERPIN B6 | 44928 | 44928 | 44928 | 80 | 44692 | 237 | 648 | >100 | 80 |
| 410 | 224915_x_at | AV75613 1 | Homo sapiens, clone IMAGE:5285034, mRNA | --- | 89 | 44928 | 44928 | 44928 | 726 | 44203 | 1875 | 80 | 80 |
| 411 | 215528 _at | AL049390 .1 | Homo sapiens mRNA; cDNA DKFZp58601318 (from clone DKFZp586O1318) | --- | 44928 | 44928 | 44928 | 44928 | 44848 | 81 | 223 | >100 | 81 |
| 412 | 222428 _s_at | D84223.1 | leucyl-tRNA synthetase | LARS | 44928 | 44928 | 81 | 44928 | 598 | 44331 | 1689 | >100 | 81 |
| 413 | 232369 _at | AF339768 .1 | Homo sapiens clone IMAGE:119716, mRNA sequence | --- | 44928 | 44928 | 44928 | 81 | 44430 | 499 | 864 | >100 | 81 |
| 414 | 233849 _s_at | AK02301 4.1 | Rho GTPase activating protein 5 | ARHGA P5 | 81 | 44928 | 44928 | 44928 | 577 | 44352 | 1929 | >100 | 81 |
| 415 | 204173 _at | NM_0024 75.1 | myosin light chain 1 slow a | MLC1S A | 44928 | 44928 | 44928 | 44928 | 82 | 44847 | 146 | >100 | 82 |
| 416 | 213632 _at | M94065.1 | dihydroorotate dehydrogenase | DHODH | 44928 | 44928 | 44928 | 44928 | 44847 | 82 | 155 | >100 | 82 |
| 417 | 225086 _at | BF679966 | hypothetical protein FLJ38426 | FLJ3842 6 | 83 | 44928 | 123 | 44928 | 408 | 44521 | 610 | >100 | 83 |
| 418 | 225468 _at | AI761804 | tripartite motif- containing 14 | TRIM14 | 44928 | 44928 | 44928 | 44928 | 83 | 44846 | 136 | >100 | 83 |
| 419 | 236617 _at | AW66308 3 | Homo sapiens, clone IMAGE:5285945, mRNA | --- | 44928 | 44928 | 44928 | 83 | 44770 | 159 | 217 | >100 | 83 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 420 | 210453 _x_at | AL050277 .1 | ATP synthase, H+ transporting, mitochondrial F0 complex, subunit g | ATP5L | 84 | 44928 | 44928 | 44928 | 531 | 44398 | 1585 | >100 | 84 |
| 421 | 216977 _x_at | AJ130972 .1 | small nuclear ribonucleoprotein polypeptide A' | SNRPA1 | 44928 | 44928 | 44928 | 44928 | 84 | 44845 | 187 | >100 | 84 |
| 422 | 237475 _x_at | AI151104 | selenoprotein P, plasma, 1 | SEPP1 | 44928 | 44928 | 44928 | 84 | 43126 | 1803 | 2926 | >100 | 84 |
| 423 | 211794 _at | AF198052 .1 | FYN binding protein (FYB-120/130) | FYB | 44928 | 44928 | 44928 | 44928 | 44160 | 769 | 85 | >100 | 85 |
| 424 | 201892 _s_at | NM_0008 84.1 | IMP (inosine monophosphate) dehydrogenase 2 | IMPDH2 | 86 | 44928 | 44928 | 44928 | 3337 | 41592 | 14262 | >100 | 86 |
| 425 | 218901 _at | NM_0203 53.1 | phospholipid scramblase 4 | PLSCR4 | 44928 | 44928 | 44928 | 44928 | 44843 | 86 | 121 | >100 | 86 |
| 426 | 241997 _at | AA70081 7 | ESTs, Weakly similar to hypothetical protein FLJ20234 [Homo sapiens] [H.sapiens] | --- | 44928 | 44928 | 44928 | 86 | 42689 | 2240 | 6135 | >100 | 86 |
| 427 | 208463 _at | NM_0008 09.1 | gamma-aminobutyric acid (GABA) A receptor, alpha 4 | GABRA 4 | 44928 | 44928 | 44928 | 87 | 44731 | 198 | 377 | >100 | 87 |
| 428 | 220071 _x_at | NM_0180 97.1 | hypothetical protein FLJ10460 | FLJ1046 0 | 44928 | 44928 | 44928 | 91 | 44842 | 87 | 322 | >100 | 87 |
| 429 | 222646 _s_at | AW26836 5 | ERO1-like (S. cerevisiae) | ERO1L | 44928 | 44928 | 44928 | 44928 | 87 | 44842 | 150 | >100 | 87 |
| 430 | 234875 _at | AJ224082 | --- | --- | 44928 | 44928 | 87 | 44928 | 845 | 44084 | 2407 | >100 | 87 |
| 431 | 207300 _s_at | NM_0001 31.2 | coagulation factor VII (serum prothrombin conversion accelerator) | F7 | 44928 | 44928 | 44928 | 44928 | 44782 | 147 | 88 | >100 | 88 |
| 432 | 209083 _at | U34690.1 | coronin, actin binding protein, 1A | CORO1 A | 88 | 44928 | 44928 | 44928 | 7864 | 37065 | 30105 | >100 | 88 |
| 433 | 216644 _at | AK00018 5.1 | Homo sapiens cDNA FLJ20178 fis, clone COL09990. | --- | 44928 | 44928 | 44928 | 44928 | 44841 | 88 | 270 | >100 | 88 |
| 434 | 218920 _at | NM_0190 57.1 | hypothetical protein FLJ10404 | FLJ1040 4 | 44928 | 44928 | 44928 | 88 | 44757 | 172 | 446 | >100 | 88 |
| 435 | 224518 _s_at | BC006436 .1 | hypothetical protein MGC13105 | MGC131 05 | 44928 | 44928 | 88 | 44928 | 450 | 44479 | 1018 | >100 | 88 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 436 | 227916 _x_at | AA74730 3 | exosome component Rrp40 | RRP40 | 44928 | 44928 | 44928 | 44928 | 88 | 44841 | 227 | >100 | 88 |
| 437 | 202232 _s_at | NM_0063 60.1 | dendritic cell protein | GA17 | 44928 | 44928 | 44928 | 44928 | 89 | 44840 | 254 | >100 | 89 |
| 438 | 215916 _at | AL157418 .1 | misshapen/NIK-related kinase | MINK | 44928 | 44928 | 44928 | 44928 | 44840 | 89 | 402 | >100 | 89 |
| 439 | 228818 _at | BF110792 | Homo sapiens cDNA FLJ12727 fis, clone NT2RP2000027. | --- | 44928 | 44928 | 44928 | 89 | 43849 | 1080 | 3023 | >100 | 89 |
| 440 | 200903 _s_at | NM_0006 87.1 | S-adenosylhomocysteine hydrolase | AHCY | 44928 | 44928 | 90 | 44928 | 142 | 44787 | 237 | 97 | 90 |
| 441 | 206790 _s_at | NM_0045 45.1 | NADH dehydrogenase (ubiquinone) 1 beta subcomplex, 1, 7kDa | NDUFB1 | 126 | 44928 | 92 | 44928 | 352 | 44577 | 1766 | 90 | 90 |
| 442 | 208013 _s_at | NM_0201 15.1 | acrosomal vesicle protein 1 | ACRYL | 44928 | 44928 | 44928 | 44928 | 44839 | 90 | 162 | >100 | 90 |
| 443 | 224254 _x_at | AF116695 .1 | --- | --- | 44928 | 44928 | 44928 | 90 | 42695 | 2234 | 2842 | >100 | 90 |
| 444 | 201825 _s_at | AL572542 | CGI-49 protein | CGI-49 | 91 | 44928 | 44928 | 44928 | 921 | 44008 | 4114 | >100 | 91 |
| 445 | 204795 _at | NM_0252 63.1 | CAT56 protein | CAT56 | 44928 | 44928 | 44928 | 44928 | 91 | 44838 | 256 | >100 | 91 |
| 446 | 218332 _at | NM_0184 76.1 | brain expressed, X-linked 1 | BEX1 | 44928 | 44928 | 44928 | 44928 | 44838 | 91 | 201 | >100 | 91 |
| 447 | 222975 _s_at | AB02069 2.1 | NRAS-related gene | D1S155E | 44928 | 44928 | 113 | 44928 | 119 | 44810 | 177 | 91 | 91 |
| 448 | 215806 _x_at | M13231.1 | T cell receptor gamma constant 2 | TRGC2 | 44928 | 44928 | 44928 | 44928 | 44837 | 92 | 321 | >100 | 92 |
| 449 | 200037 _s_at | NM_0165 87.1 | chromobox homolog 3 (HP1 gamma homolog, Drosophila) | CBX3 | 44928 | 44928 | 135 | 44928 | 233 | 44696 | 448 | 92 | 92 |
| 450 | 225892 _at | BF438417 | Homo sapiens mRNA; cDNA DKFZp564D1164 (from clone DKFZp564D1164) | --- | 44928 | 44928 | 108 | 44928 | 92 | 44837 | 164 | >100 | 92 |
| 451 | 209786 _at | BC001282 .1 | high mobility group nucleosomal binding domain 4 | HMGN4 | 44928 | 44928 | 44928 | 44928 | 267 | 44662 | 484 | 93 | 93 |
| 452 | 215056 _at | A1267546 | ESTs | --- | 44928 | 44928 | 44928 | 44928 | 44836 | 93 | 160 | >100 | 93 |
| 453 | 223433 _at | AF226046 .1 | GK003 protein | GK003 | 44928 | 44928 | 44928 | 44928 | 93 | 44836 | 122 | >100 | 93 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 454 | 225304 _s_at | BE741920 | NADH-ubiquinone oxidoreductase subunit B14.7 | NDUFA 11 | 44928 | 44928 | 152 | 44928 | 146 | 44783 | 93 | >100 | 93 |
| 455 | 234462 _at | S51397 | --- | --- | 93 | 44928 | . 44928 | 44928 | 4340 | 40589 | 28484 | >100 | 93 |
| 456 | 205119 _s_at | NM_0020 29.1 | formyl peptide receptor 1 | FPR1 | 44928 | 44928 | 44928 | 44928 | 44835 | 94 | 257 | >100 | 94 |
| 457 | 224872 _at | AB04089 6.1 | KIAA1463 protein | KIAA14 63 | 44928 | 44928 | 44928 | 44928 | 94 | 44835 | 451 | >100 | 94 |
| 458 | 224952 _at | B115054 | putative ankyrin-repeat containing protein | DKFZP5 64D166 | 44928 | 44928 | 44928 | 94 | 43286 | 1643 | 7694 | >100 | 94 |
| 459 | 226756 _at | AA19174 1 | Homo sapiens cDNA FLJ11436 fis, clone HEMBA1001213. | --- | 94 | 44928 | 44928 | 44928 | 776 | 44153 | 2397 | >100 | 94 |
| 460 | 202250 _s_at | NM_0157 26.1 | H326 | H326 | 44928 | 44928 | 44928 | 95 | 42923 | 2006 | 6207 | >100 | 95 |
| 461 | 223334 _at | AL136941 .1 | hypothetical protein DKFZp586C1924 | DKFZp5 86C1924 | 44928 | 44928 | 95 | 44928 | 240 | 44689 | 704 | >100 | 95 |
| 462 | 226789 _at | W84421 | Human S6 H-8 mRNA expressed in chromosome 6-suppressed melanoma cells. | --- | 95 | 44928 | 44928 | 44928 | 2994 | 41935 | 15082 | >100 | 95 |
| 463 | 208742 _s_at | U78303.1 | sin3-associated polypeptide, 18kDa | SAP18 | 44928 | 44928 | 44928 | 44928 | 242 | 44687 | 599 | 96 | 96 |
| 464 | 231810 _at | BG10691 9 | BRI3 binding protein | BRI3BP | 96 | 44928 | 44928 | 44928 | 929 | 44000 | 3396 | >100 | 96 |
| 465 | 244495 _x_at | AL521157 | hypothetical protein MGC11386 | MGC113 86 | 44928 | 44928 | 44928 | 96 | 41892 | 3037 | 4559 | >100 | 96 |
| 466 | 205260 _s_at | NM_0011 07.1 | acylphosphatase 1, erythrocyte (common) type | ACYP1 | 44928 | 44928 | 44928 | 44928 | 136 | 44793 | 97 | >100 | 97 |
| 467 | 213746 _s_at | AW05185 6 | filamin A, alpha (actin binding protein 280) | FLNA | 97 | 44928 | 44928 | 44928 | 4383 | 40546 | 25901 | >100 | 97 |
| 468 | 215601 _at | AK02389 5.1 | --- | --- | 44928 | 44928 | 44928 | 44928 | 44832 | 97 | 932 | >100 | 97 |
| 469 | 202565 _s_at | NM_0031 74.2 | supervillin | SVIL | 98 | 44928 | 44928 | 44928 | 8543 | 36386 | 44011 | >100 | 98 |

(continued)

| No. | Probes et ID | Sequence Derived From | Title | Gene Symbol | Rank NR PFC-1 | Rank R PFC-1 | Rank NR PFC-2 | Rank R PFC-1 | Rank NR SNR | Rank R SNR | Rank Wilcoxon rank-sum test | Rank CBT | Minimum rank |
|---|---|---|---|---|---|---|---|---|---|---|---|---|---|
| 470 | 209596 _at | AF245505 .1 | adlican | DKFZp5 6411922 | 44928 | 44928 | 44928 | 44928 | 44831 | 98 | 239 | >100 | 98 |
| 471 | 225470 _at | AL529634 | mitotic phosphoprotein 44 | LOC129 401 | 44928 | 44928 | 44928 | 44928 | 98 | 44831 | 265 | >100 | 98 |
| 472 | 243450 _at | T40707 | ESTs | --- | 44928 | 44928 | 44928 | 98 | 36175 | 8754 | 15508 | >100 | 98 |
| 473 | 209036 _s_at | BC001917 .1 | malate dehydrogenase 2, NAD (mitochondrial) | MDH2 | 44928 | 44928 | 44928 | 44928 | 100 | 44829 | 258 | >100 | 100 |
| 474 | 216380 _x_at | AC00501 1 | --- | --- | 100 | 44928 | 131 | 44928 | 1371 | 43558 | 4699 | >100 | 100 |
| 475 | 236646 _at | BE301029 | hypothetical protein FLJ31166 | FLJ3116 6 | 44928 | 44928 | 44928 | 100 | 40827 | 4102 | 1539 | >100 | 100 |

A Cox proportional hazard analysis was performed to determine predictors of time until disease progression (TTP) in patients with relapsed and refractory multiple myeloma after treatment with bortezomib. This methodology is designed to analyze time to event data where some of the data may be censored (see E.T. Lee, Statistical Methods for Survival Data Analysis, 2nd ed. 1992, John Wiley& Sons, Inc.). The statistical package SAS was used to perform the analysis. We first examined clinical and prognostic factors to identify which combination of factors showed the greatest association with TTP. This was accomplished by use of the score method for best subset selection. This method provides score chi-squared statistics for all possible model sizes ranging from one predictor to the total number of explanatory variables under consideration. Thus, the method first provides the best single predictor models in order of the highest chi-squared statistics. If there are significant single predictor models ($p < 0.05$), the procedure goes on to the next step of estimating all two predictor models and ranking them by the highest chi-squared statistic.

To assess if a 2 predictor model is a better fit than a single predictor model, the difference in the chi-squared statistics is calculated. This is a one degree of freedom chi-square test and can be assessed for statistical significance. If the difference proves to be significant at $p < 0.05$, we conclude the two predictor model is a better fit, the second variable is significantly associated with TTP after taking into account the first variable, and the process continues by estimating all three predictor models. The three predictor model is compared to the two predictor model in the same way as the two predictor model was assessed against the single predictor model. This process is continued until the difference chi-square test fails, that is $p > 0.05$ for adding in an additional variable to the model. By using this process, we found that the best model contained 3 significant prognostic or clinical factors, abnormal cytogentics, $\beta$2-microglobulin, and c-reactive protein. We defined this as our best prognostic variable model.

The next step was to determine if there were any genomic markers that were significantly associated with TTP after accounting for the prognostic factors. We first filtered the genomic data set, made up of some 44,000 transcripts from the Affymetrics U133A and U133B human array chips, to those genes which had at least one present call using the Affymetrix detection system for determining if a transcript is reliably detected or not. This left 13,529 transcripts for analysis. We then estimated Cox proportional hazard models for each of the 13,529 transcripts where each model also contained the 3 prognostic factors discussed above. That is, 13,529 models were estimated where each model contained 1 transcript and the three prognostic factors. From each model, we obtained estimates of relative risk, 95% confidence intervals and p values for the association of each transcript to TTP. From the 13,529 models, we found 834 transcripts which had p values of less than 0.05. That is, we found 834 transcripts that were significantly and independently, from the prognostic factors, associated with TTP. These are listed in Table 2

**TABLE 2 Predictive markers Associated with Time to Disease Progression (TTP)**

| No. | Probe set ID | Seq. Derived From (RefSeq/Genbank Accession) | Title | Gene Symbol | Hazard |
|---|---|---|---|---|---|
| 83 | 201575_at | NM_012245.1 | SKI-interacting protein | SNW1 | >1 |
| 81 | 202647_s_at | NM_002524.2 | neuroblastoma RAS viral (v-ras) oncogene homolog | NRAS | >1 |
| 234 | 203058_s_at | AW299958 | 3'-phosphoadenosine 5-phosphosulfate synthase 2 | PAPSS2 | <1 |
| 42 | 203753_at | NM_003199.1 | transcription factor 4 | TCF4 | <1 |
| 415 | 204173_at | NM_002475.1 | myosin light chain 1 slow a | MLC1SA | >1 |
| 191 | 206121_at | NM_000036.1 | adenosine monophosphate deaminase 1 (isoform M) | AMPD1 | >1 |
| 404 | 208690_s_at | BC000915.1 | PDZ and LIM domain 1 (elfin) | PDL1M1 | >1 |
| 53 | 210993_s_at | U54826.1 | MAD, mothers against decapentaplegic homolog 1 (Drosophila) | MADH1 | >1 |
| 305 | 212110_at | D31887.1 | KIAA0062 protein | KIAA0062 | <1 |
| 41 | 212382_at | AK021980.1 | Homo sapiens cDNA FLJ11918 fis, clone HEMBB1000272. | --- | <1 |
| 43 | 212386_at | AK021980.1 | Homo sapiens cDNA FLJ11918 fis, clone HEMBB1000272. | --- | <1 |

(continued)

| No. | Probe set ID | Seq. Derived From (RefSeq/Genbank Accession) | Title | Gene Symbol | Hazard |
|---|---|---|---|---|---|
| 40 | 212387_at | AK021980.1 | Homo sapiens cDNA FLJ11918 fis, clone HEMBB1000272. | --- | <1 |
| 467 | 213746_s_at | AW051856 | filamin A, alpha (actin binding protein 280) | FLNA | >1 |
| 39 | 213891_s_at | AI927067 | Homo sapiens cDNA FLJ11918 fis, clone HEMBB1000272. | --- | <1 |
| 78 | 215744_at | AW514140 | fusion, derived from t(12;16) malignant liposarcoma | FUS | <1 |
| 77 | 218319_at | NM_020651.2 | pellino homolog 1 (Drosophila) | PELI1 | <1 |
| 201 | 219429_at | NM_024306.1 | fatty acid hydroxylase | FAAH | <1 |
| 126 | 222762_x_at | AU144259 | LIM domains containing 1 | LIMD1 | >1 |
| 376 | 222789_at | BE888593 | hypothetical protein FLJ11220 | FLJ11220 | >1 |
| 341 | 225373_at | BE271644 | PP2135 protein | PP2135 | <1 |
| 209 | 225710_at | H99792 | Homo sapiens cDNA FLJ34013 fis, clone FCBBF2002111. | --- | <1 |
| 48 | 227798_at | AU146891 | EST | --- | >1 |
| 464 | 231810_at | BG106919 | BRI3 binding protein | BRI3BP | >1 |
| 76 | 232213_at | AU147506 | pellino homolog 1 (Drosophila) | PELI1 | <1 |
| 75 | 232304_at | AK026714.1 | pellino homolog 1 (Drosophila) | PELI1 | <1 |
| 224 | 235875_at | BF510711 | EST | --- | <1 |
| 172 | 242903_at | AI458949 | EST | --- | <1 |
| 476 | 222788_s_at | BE888593 | hypothetical protein FLJ11220 | FLJ11220 | >1 |
| 477 | 213305_s_at | L42375.1 | protein phosphatase 2, regulatory subunit B (B56), gamma isoform | PPP2R5C | >1 |
| 478 | 204774_at | NM_014210.1 | ecotropic viral integration site 2A | EVI2A | <1 |
| 479 | 200984_s_at | NM_000611.1 | CD59 antigen p18-20 (antigen identified by monoclonal antibodies 16.3A5, EJ16, EJ30, EL32 and G344) | CD59 | <1 |
| 480 | 208956_x_at | U62891.1 | dUTP pyrophosphatase | DUT | >1 |
| 481 | 216326_s_at | AF059650 | histone deacetylase 3 | HDAC3 | <1 |
| 482 | 203845_at | AV727449 | p300/CBP-associated factor | PCAF | <1 |
| 483 | 214349_at | AV764378 | Homo sapiens cDNA: FLJ23438 fis, clone HRC13275. | --- | >1 |
| 484 | 202332_at | NM_001894.1 | casein kinase 1, epsilon | CSNK1E | >1 |
| 485 | 201020_at | NM_003405.1 | tyrosine 3-monooxygenase/tryptophan 5-monooxygenase activation protein, eta polypeptide | YWHAH | <1 |
| 486 | 200612_s_at | NM_001282.1 | adaptor-related protein complex 2, beta 1 subunit | AP2B1 | <1 |
| 487 | 212612_at | D31888.1 | REST corepressor | RCOR | >1 |

(continued)

| No. | Probe set ID | Seq. Derived From (RefSeq/Genbank Accession) | Title | Gene Symbol | Hazard |
|---|---|---|---|---|---|
| 488 | 202963_at | AW027312 | regulatory factor X, 5 (influences HLA class II expression) | RFX5 | <1 |
| 489 | 212463_at | BE379006 | Homo sapiens mRNA; cDNA DKFZp564J0323 (from clone DKFZp564J0323) | --- | <1 |
| 490 | 202453_s_at | NM_005316.1 | general transcription factor IIH, polypeptide 1, 62kDa | GTF2H1 | <1 |
| 491 | 209239_at | M55643.1 | nuclear factor of kappa light polypeptide gene enhancer in B-cells 1 (p105) | NFKB1 | <1 |
| 492 | 213405_at | N95443 | Homo sapiens, clone IMAGE:4831050, mRNA | --- | <1 |
| 493 | 200679_x_at | BE311760 | high-mobility group box 1 | HMGB1 | >1 |
| 494 | 205981_s_at | NM_001564.1 | inhibitor of growth family, member 1-like | ING1L | >1 |
| 495 | 211783_s_at | BC006177.1 | metastasis associated 1 | MTA1 | >1 |
| 496 | 227482_at | AI097656 | hypothetical protein LOC57143 | LOC57143 | >1 |
| 497 | 214943_s_at | D38491.1 | KIAA0117 protein | KIAA0117 | >1 |
| 498 | 205504_at | NM_000061.1 | Bruton agammaglobulinemia tyrosine kinase | BTK | <1 |
| 499 | 218216_x_at | NM_016638.1 | ADP-ribosylation-like factor 6 interacting protein 4 | ARL6IP4 | >1 |
| 500 | 221014_s_at | NM_031296.1 | RAB33B, member RAS oncogene family | RAB33B | <1 |
| 501 | 202408_s_at | NM_015629.1 | PRP31 pre-mRNA processing factor 31 homolog (yeast) | PRPF31 | >1 |
| 502 | 217996_at | AA576961 | pleckstrin homology-like domain, family A, member 1 | PHLDA1 | >1 |
| 503 | 229723_at | BF591040 | T-cell activation GTPase activating protein | TAGAP | <1 |
| 504 | 227112_at | AW270037 | KIAA0779 protein | KIAA0779 | <1 |
| 505 | 218224_at | NM_006029.2 | paraneoplastic antigen MA1 | PNMA1 | >1 |
| 506 | 213415_at | AI768628 | chloride intracellular channel 2 | CLIC2 | <1 |
| 507 | 225251_at | AK021761.1 | Homo sapiens cDNA FLJ11699 fis, clone HEMBA1005047, highly similar to RAS-RELATED PROTEIN RAB-24. | RAB24 | <1 |
| 508 | 219228_at | NM_018555.2 | zinc finger protein 463 | ZNF463 | <1 |
| 509 | 226979_at | AI125541 | mitogen-activated protein kinase kinase kinase 2 | MAP3K2 | <1 |
| 510 | 227179_at | AK002152.1 | staufen, RNA binding protein, homolog 2 (Drosophila) | STAU2 | >1 |
| 511 | 205621_at | NM_006020.1 | alkB, alkylation repair homolog (E. coli) | ALKBH | >1 |
| 512 | 226421_at | AA707320 | hypothetical protein LOC286505 | LOC286505 | <1 |
| 513 | 219709_x_at | NM_023933.1 | hypothetical protein MGC2494 | MGC2494 | >1 |

(continued)

| No. | Probe set ID | Seq. Derived From (RefSeq/Genbank Accession) | Title | Gene Symbol | Hazard |
|---|---|---|---|---|---|
| 514 | 217803_at | NM_022130.1 | golgi phosphoprotein 3 (coat-protein) | GOLPH3 | <1 |
| 515 | 228980_at | AI760772 | fring | LOC117584 | <1 |
| 516 | 243020_at | R06738 | EST | --- | >1 |
| 517 | 211289_x_at | AF067524.1 | cell division cycle 2-like 2 | CDC2L2 | >1 |
| 518 | 213137_s_at | AI828880 | protein tyrosine phosphatase, non-receptor type 2 | PTPN2 | >1 |
| 519 | 204407_at | AF080255.1 | transcription termination factor, RNA polymerase II | TTF2 | >1 |
| 520 | 224938_at | AU144387 | EST | --- | <1 |
| 521 | 225466_at | AI761804 | tripartite motif-containing 14 | TRIM 14 | <1 |
| 522 | 208908_s_at | AF327443.1 | calpastatin | CAST | <1 |
| 523 | 222343_at | AA629050 | Homo sapiens full length insert cDNA clone ZA94C02 | --- | >1 |
| 524 | 224566_at | AK027191.1 | Homo sapiens cDNA: FLJ23538 fis, clone LNG08010, highly similar to BETA2 Human MEN1 region clone epsilon/beta mRNA. | --- | <1 |
| 525 | 208297_s_at | NM_005665.1 | - | --- | >1 |
| 526 | 213923_at | AW005535 | RAP2B, member of RAS oncogene family | RAP2B | <1 |
| 527 | 228680_at | AW340096 | EST, Moderately similar to hypothetical protein FLJ20489 [Homo sapiens] [H.sapiens] | --- | <1 |
| 528 | 209204_at | AI824831 | LIM domain only 4 | LMO4 | >1 |
| 529 | 208093_s_at | NM_030808.1 | LIS1-interacting protein NUDEL; endooligopeptidase A | NUDEL | <1 |
| 530 | 200982_s_at | NM_001155.2 | annexin A6 | ANXA6 | <1 |
| 531 | 218249_at | NM_022494.1 | zinc finger, DHHC domain containing 6 | ZDHHC6 | <1 |
| 532 | 203345_s_at | AI566096 | likely ortholog of mouse metal response element binding transcription factor 2 | M96 | >1 |
| 533 | 223141_at | AK022317.1 | uridine-cytidine kinase 1 | UCK1 | >1 |
| 534 | 222444_at | AL121883 | ALEX3 protein | ALEX3 | <1 |
| 535 | 217853_at | NM_022748.1 | tumor endothelial marker 6 | TEM6 | <1 |
| 536 | 220244_at | NM_013343.1 | NAG-7 protein | NAG-7 | <1 |
| 537 | 213995_at | AW195882 | ATP synthase, H+ transporting, mitochondrial F0 complex, subunit s (factor B) | ATP5S | >1 |
| 538 | 214072_x_at | AA679297 | secreted protein of unknown function | SPUF | >1 |
| 539 | 200950_at | NM_006409.1 | actin related protein 2/3 complex, subunit 1A, 41kDa | ARPC1A | <1 |
| 540 | 224878_at | N63936 | similar to ubiquitin binding protein | UBPH | >1 |

(continued)

| No. | Probe set ID | Seq. Derived From (RefSeq/Genbank Accession) | Title | Gene Symbol | Hazard |
|---|---|---|---|---|---|
| 541 | 227294_at | A1474448 | hypothetical protein BC014000 | LOC115509 | >1 |
| 542 | 214334_x_at | N34846 | DAZ associated protein 2 | DAZAP2 | >1 |
| 543 | 214659_x_at | AC007956 | ZAP3 protein | ZAP3 | >1 |
| 544 | 36499_at | D87469 | cadherin, EGF LAG seven-pass G-type receptor 2 (flamingo homolog, Drosophila) | CELSR2 | >1 |
| 545 | 229512_at | BE464337 | EST | --- | >1 |
| 546 | 206662_at | NM_002064.1 | glutaredoxin (thioltransferase) | GLRX | <1 |
| 547 | 200914_x_at | BF589024 | kinectin 1 (kinesin receptor) | KTN1 | >1 |
| 548 | 214938_x_at | AF283771.2 | high-mobility group box 1 | HMGB1 | >1 |
| 549 | 203243_s_at | NM_006457.1 | LIM protein (similar to rat protein kinase C-binding enigma) | LIM | <1 |
| 550 | 214395_x_at | AI335509 | eukaryotic translation elongation factor 1 delta (guanine nucleotide exchange protein) | EEF1D | >1 |
| 551 | 217208_s_at | AL121981 | discs, large (Drosophila) homolog 1 | DLG1 | >1 |
| 552 | 224180_x_at | AF131737.1 | hypothetical protein LOC51057 | LOC51057 | >1 |
| 553 | 218724_s_at | NM_021809.1 | TGFB-induced factor 2 (TALE family homeobox) | TGIF2 | <1 |
| 554 | 210387_at | BC001131.1 | histone 1, H2bg | HIST1H2BG | >1 |
| 555 | 208898_at | AF077614.1 | ATPase, H+ transporting, lysosomal 34kDa, V1 subunit D | ATP6V1D | >1 |
| 556 | 200645_at | NM_007278.1 | GABA(A) receptor-associated protein | GABARAP | <1 |
| 557 | 200985_s_at | NM_000611.1 | CD59 antigen p18-20 (antigen identified by monoclonal antibodies 16.3A5, EJ16, EJ30, EL32 and G344) | CD59 | <1 |
| 558 | 220595_at | NM_013377.1 | hypothetical protein DKFZp434B0417 | DKFZp434B 0417 | >1 |
| 559 | 236550_s_at | BF508689 | Homo sapiens mRNA; cDNA DKFZp686I2118 (from clone DKFZp686I2118) | ZNF311 | >1 |
| 560 | 202279_at | NM_004894.1 | chromosome 14 open reading frame 2 | C14orf2 | >1 |
| 561 | 234312_s_at | AK000162.1 | acetyl-Coenzyme A synthetase 2 (ADP forming) | ACAS2 | >1 |
| 562 | 213945_s_at | AI867102 | nucleoporin 210 | NUP210 | >1 |
| 563 | 228380_at | BE551193 | EST, Weakly similar to hypothetical protein FLJ20378 [Homo sapiens] [H.sapiens] | --- | <1 |
| 564 | 203574_at | NM_005384.1 | nuclear factor, interleukin 3 regulated | NFIL3 | >1 |
| 565 | 222146_s_at | AK026674.1 | transcription factor 4 | TCF4 | <1 |
| 566 | 227665_at | BE968576 | Homo sapiens, clone IMAGE:4152387, mRNA | --- | <1 |

(continued)

| No. | Probe set ID | Seq. Derived From (RefSeq/Genbank Accession) | Title | Gene Symbol | Hazard |
|---|---|---|---|---|---|
| 567 | 207995_s_at | NM_014257.1 | CD209 antigen-like | CD209L | <1 |
| 568 | 201097_s_at | NM_001660.2 | ADP-ribosylation factor 4 | ARF4 | <1 |
| 569 | 203975_s_at | BF000239 | chromatin assembly factor 1, subunit A (p150) | CHAF1A | >1 |
| 570 | 209136_s_at | BG390445 | ubiquitin specific protease 10 | USP10 | >1 |
| 571 | 238086_at | AI288372 | EST | --- | >1 |
| 572 | 242388_x_at | AW576600 | EST | --- | <1 |
| 573 | 241876_at | AW663060 | EST | --- | <1 |
| 574 | 228195_at | BE645119 | EST | --- | <1 |
| 575 | 202334_s_at | AA877765 | ubiquitin-conjugating enzyme E2B (RAD6 homolog) | UBE2B | <1 |
| 576 | 201472_at | NM_003372.2 | von Hippel-Lindau binding protein 1 | VBP1 | <1 |
| 577 | 217092_x_at | AL031589 | --- | --- | >1 |
| 578 | 208744_x_at | BG403660 | heat shock 105kDa/110kDa protein 1 | HSPH1 | >1 |
| 579 | 212412_at | AV715767 | Homo sapiens mRNA; cDNA DKFZp564A072 (from clone DKFZp564A072) | --- | <1 |
| 580 | 217995_at | NM_021199.1 | sulfide quinone reductase-like (yeast) | SQRDL | <1 |
| 581 | 203275_at | NM_002199.2 | interferon regulatory factor 2 | IRF2 | <1 |
| 582 | 207335_x_at | NM_007100.1 | ATP synthase, H+ transporting, mitochondrial F0 complex, subunit e | ATP5I | >1 |
| 583 | 218130_at | NM_024510.1 | hypothetical protein MGC4368 | MGC4368 | >1 |
| 584 | 208914_at | NM_015044.1 | golgi associated, gamma adaptin ear containing, ARF binding protein 2 | GGA2 | <1 |
| 585 | 202985_s_at | NM_004873.1 | BCL2-associated athanogene 5 | BAG5 | >1 |
| 586 | 206587_at | NM_006584.1 | chaperonin containing TCP1, subunit 6B (zeta 2) | CCT6B | <1 |
| 587 | 223419_at | BC004290.1 | hypothetical protein MGC10870 | MGC10870 | >1 |
| 588 | 213102_at | Z78330 | ARP3 actin-related protein 3 homolog (yeast) | ACTR3 | <1 |
| 589 | 226520_at | AI831506 | EST | --- | <1 |
| 590 | 201366_at | NM_004034.1 | annexin A7 | ANXA7 | <1 |
| 591 | 213021_at | AI741876 | Homo sapiens mRNA; cDNA DKFZp566B213 (from clone DKFZp566B213) | --- | <1 |
| 592 | 201172_x_at | NM_003945.1 | ATPase, H+ transporting, lysosomal 9kDa, V0 subunit e | ATP6V0E | <1 |
| 593 | 213295_at | AA555096 | Homo sapiens mRNA; cDNA DKFZp586D1122 (from clone DKFZp586D1122) | --- | <1 |

(continued)

| No. | Probe set ID | Seq. Derived From (RefSeq/Genbank Accession) | Title | Gene Symbol | Hazard |
|---|---|---|---|---|---|
| 594 | 226406_at | AI823360 | hypothetical protein MGC12909 | MGC12909 | <1 |
| 595 | 210564_x_at | AF009619.1 | CASP8 and FADD-like apoptosis regulator | CFLAR | <1 |
| 596 | 242606_at | AL043482 | EST | --- | <1 |
| 597 | 203292_s_at | NM_021729.2 | vacuolar protein sorting 11 (yeast) | VPS11 | >1 |
| 598 | 202579_x_at | NM_006353.1 | high mobility group nucleosomal binding domain 4 | HMGN4 | <1 |
| 599 | 229113_s_at | W16779 | protein kinase C, zeta | PRKCZ | >1 |
| 600 | 244743_x_at | AA114243 | zinc finger protein 138 (clone pHZ-32) | ZNF138 | <1 |
| 601 | 222622_at | BG284709 | hypothetical protein LOC283871 | LOC283871 | >1 |
| 602 | 210312_s_at | BC002640.1 | hypothetical protein LOC90410 | LOC90410 | <1 |
| 603 | 221530_s_at | A044088.1 | basic helix-loop-helix domain containing, class B, 3 | BHLHB3 | <1 |
| 604 | 201994_at | NM_012286.1 | mortality factor 4 like 2 | MORF4L2 | <1 |
| 605 | 227262_at | BE348293 | Homo sapiens proteoglycan link protein mRNA, complete cds. | --- | >1 |
| 606 | 203693_s_at | NM_001949.2 | E2F transcription factor 3 | E2F3 | <1 |
| 607 | 221750_at | BG035985 | 3-hydroxy-3-methylglutaryl-Coenzyme A synthase 1 (soluble) | HMGCS1 | <1 |
| 608 | 214789_x_at | AA524274 | Splicing factor, arginine/serine-rich, 46kD | SRP46 | <1 |
| 609 | 200761_s_at | NM_006407.2 | vitamin A responsive; cytoskeleton related | JWA | <1 |
| 610 | 212233_at | AL523076 | Homo sapiens cDNA FLJ30550 fis, clone BRAWH2001502. | --- | <1 |
| 611 | 209300_s_at | BC002888.1 | DKFZP566B 183 protein | DKFZP566B 183 | <1 |
| 612 | 213708_s_at | N40555 | transcription factor-like 4 | TCFL4 | <1 |
| 613 | 207467_x_at | NM_001750.2 | calpastatin | CAST | <1 |
| 614 | 225414_at | AL558987 | hypothetical protein LOC284996 | LOC284996 | <1 |
| 615 | 235104_at | BG292389 | EST | --- | <1 |
| 616 | 214003_x_at | BF184532 | ribosomal protein S20 | RPS20 | >1 |
| 617 | 201342_at | AY008268.1 | SAR1 protein | SAR1 | <1 |
| 618 | 211316_x_at | AF009616.1 | CASP8 and FADD-like apoptosis regulator | CFLAR | <1 |
| 619 | 221522_at | AL136784.1 | hypothetical protein DKFZp434L0718 | DKFZP434L 0718 | <1 |
| 620 | 210844_x_at | D14705.1 | catenin (cadherin-associated protein), alpha 1, 102kDa | CTNNA1 | <1 |
| 621 | 210448_s_at | U49396.1 | purinergic receptor P2X, ligand-gated ion channel, 5 | P2RX5 | <1 |
| 622 | 212843_at | AA126505 | neural cell adhesion molecule 1 | NCAM1 | <1 |

(continued)

| No. | Probe set ID | Seq. Derived From (RefSeq/Genbank Accession) | Title | Gene Symbol | Hazard |
|---|---|---|---|---|---|
| 623 | 224284_x_at | AF338193.1 | --- | --- | >1 |
| 624 | 222650_s_at | BE898559 | SLC2A4 regulator | SLC2A4RG | >1 |
| 625 | 212719_at | AB011178.1 | pleckstrin homology domain containing, family E (with leucine rich repeats) member 1 | PTEKHE1 | >1 |
| 626 | 38069_at | Z67743 | chloride channel 7 | CLCN7 | >1 |
| 627 | 233625_x_at | AK021939.1 | hypothetical protein FLJ20542 | FLJ20542 | >1 |
| 628 | 205053_at | NM_000946.1 | primase, polypeptide 1, 49kDa | PRIM1 | >1 |
| 629 | 239749_at | AW205090 | EST | --- | >1 |
| 630 | 34764_at | D21851 | leucyl-tRNA synthetase, mitochondrial | LARS2 | >1 |
| 631 | 205659_at | NM_014707.1 | histone deacetylase 9 | HDAC9 | <1 |
| 632 | 242092_at | AA019300 | EST, Moderately similar to hypothetical protein FLJ20097 [Homo sapiens] [H. sapiens] | --- | >1 |
| 633 | 203575_at | NM_001896.1 | casein kinase 2, alpha prime polypeptide | CSNK2A2 | >1 |
| 634 | 221297_at | NM_018654.1 | G protein-coupled receptor, family C, group 5, member D | GPRC5D | <1 |
| 635 | 212900_at | BE645231 | SEC24 related gene family, member A (S. cerevisiae) | SEC24A | <1 |
| 636 | 230036_at | BE669858 | hypothetical protein FLJ39885 | FLJ39885 | <1 |
| 637 | 213101_s_at | Z78330 | ARP3 actin-related protein 3 homolog (yeast) | ACTR3 | <1 |
| 638 | 222846_at | AB038995.1 | RAB-8b protein | LOC51762 | <1 |
| 639 | 213455_at | W87466 | pleckstrin homology domain containing, family B (evectins) member 2 | PLEKHB2 | <1 |
| 640 | 242613_at | AI809536 | EST | --- | >1 |
| 641 | 218206_x_at | NM_016558.1 | SCAN domain containing 1 | SCAND1 | >1 |
| 642 | 222014_x_at | AI249752 | MTO1 protein | MTO1 | <1 |
| 643 | 212219_at | D38521.1 | proteasome activator 200 kDa | PA200 | <1 |
| 644 | 219806_s_at | NM_020179.1 | FN5 protein | FN5 | <1 |
| 645 | 218875_s_at | NRM_012177.1 | F-box only protein 5 | FBXO5 | >1 |
| 646 | 208485_x_at | NM_003879.1 | CASP8 and FADD-like apoptosis regulator | CFLAR | <1 |
| 647 | 218233_s_at | NM_017601.1 | chromosome 6 open reading frame 49 | C6orf49 | >1 |
| 648 | 214130_s_at | AI821791 | phosphodiesterase 4D interacting protein (myomegalin) | PDE4DIP | <1 |
| 649 | 208723_at | BC000350.1 | ubiquitin specific protease 11 | USP11 | >1 |
| 650 | 217814_at | NM_020198.1 | GK001 protein | GK001 | <1 |
| 651 | 208809_s_at | AL136632.1 | hypothetical protein FLJ12619 | FLJ12619 | >1 |

(continued)

| No. | Probe set ID | Seq. Derived From (RefSeq/Genbank Accession) | Title | Gene Symbol | Hazard |
|---|---|---|---|---|---|
| 652 | 201199_s_at | NM_002807.1 | proteasome (prosome, macropain) 26S subunit, non-ATPase, 1 | PSMD1 | <1 |
| 653 | 242937_at | AV763408 | EST, Moderately similar to ILF1_HUMAN Interleukin enhancer-binding factor 1 (Cellular transcription factor ILF-1) [H.sapiens] | --- | >1 |
| 654 | 212333_at | AL049943.1 | DKFZP564F0522 protein | DKFZP564F0522 | <1 |
| 655 | 210817_s_at | BC004130.1 | nuclear domain 10 protein | NDP52 | <1 |
| 656 | 212508_at | AK024029.1 | modulator of apoptosis 1 | MOAP1 | >1 |
| 657 | 213603_s_at | BE138888 | ras-related C3 botulinum toxin substrate 2 (rho family, small GTP binding protein Rac2) | RAC2 | <1 |
| 658 | 233274_at | AU145144 | --- | --- | >1 |
| 659 | 218557_at | NM_020202.1 | Nit protein 2 | NIT2 | <1 |
| 660 | 231428_at | BE502947 | EST | --- | <1 |
| 661 | 201810_s_at | AL562152 | SH3-domain binding protein 5 (BTK-associated) | SH3BP5 | <1 |
| 662 | 209970_x_at | M87507.1 | caspase 1, apoptosis-related cysteine protease (interleukin 1, beta, convertase) | CASP1 | <1 |
| 663 | 208965_s_at | BG256677 | interferon, gamma-inducible protein 16 | IFI16 | >1 |
| 664 | 203038_at | NM_002844.1 | protein tyrosine phosphatase, receptor type, K | PTPRK | <1 |
| 665 | 202442_at | NM_001284.1 | adaptor-related protein complex 3, sigma 1 subunit | AP3S1 | <1 |
| 666 | 209515_s_at | U38654.3 | RAB27A, member RAS oncogene family | RAB27A | <1 |
| 667 | 201865_x_at | AI432196 | nuclear receptor subfamily 3, group C, member 1 (glucocorticoid receptor) | NR3C1 | <1 |
| 668 | 204786_s_at | L41944.1 | interferon (alpha, beta and omega) receptor 2 | IFNAR2 | >1 |
| 669 | 209508_x_at | AF005774.1 | CASP8 and FADD-like apoptosis regulator | CFLAR | <1 |
| 670 | 200822_x_at | NM_000365.1 | triosephosphate isomerase 1 | TPI1 | >1 |
| 671 | 217322_x_at | AL024509 | --- | --- | >1 |
| 672 | 203505_at | AF285167.1 | ATP-binding cassette, sub-family A (ABC1), member 1 | ABCA1 | >1 |
| 673 | 223347_at | AL360266.1 | hypothetical protein FLJ22283 | FLJ22283 | >1 |
| 674 | 209765_at | Y13786.2 | a disintegrin and metalloproteinase domain 19 (meltrin beta) | ADAM19 | <1 |
| 675 | 202972_s_at | AW450403 | family with sequence similarity 13, member A1 | FAM13A1 | >1 |

(continued)

| No. | Probe set ID | Seq. Derived From (RefSeq/Genbank Accession) | Title | Gene Symbol | Hazard |
|---|---|---|---|---|---|
| 676 | 203380_x_at | NM_006925.1 | splicing factor, arginine/serine-rich 5 | SFRS5 | >1 |
| 677 | 212211_at | AI986295 | gene trap ankyrin repeat | GTAR | <1 |
| 678 | 218326_s_at | NM_018490.1 | G protein-coupled receptor 48 | GPR48 | >1 |
| 679 | 217994_x_at | NM_017871.1 | hypothetical protein FLJ20542 | FLJ20542 | >1 |
| 680 | 239835_at | AA669114 | T-cell activation kelch repeat protein | TA-KRP | <1 |
| 681 | 213353_at | BF693921 | ATP-binding cassette, sub-family A (ABC1), member 5 | ABCA5 | <1 |
| 682 | 208710_s_at | AI424923 | adaptor-related protein complex 3, delta 1 subunit | AP3D1 | >1 |
| 683 | 205011_at | NM_014622.1 | loss of heterozygosity, 11, chromosomal region 2, gene A | LOH11CR2 A | <1 |
| 684 | 202027_at | NM_012264.1 | chromosome 22 open reading frame 5 | C22orf5 | >1 |
| 685 | 203642_s_at | NM_014900.1 | KIAA0977 protein | KIAA0977 | <1 |
| 686 | 212266_s_at | AW084582 | splicing factor, arginine/serine-rich 5 | SFRS5 | >1 |
| 687 | 238693_at | AA165136 | EST | --- | <1 |
| 688 | 219342_at | NM_022900.1 | O-acetyltransferase | CAS1 | <1 |
| 689 | 201769_at | NM_014666.1 | enthoprotin | ENTH | <1 |
| 690 | 243982_at | AA455180 | EST, Weakly similar to KHLX_HUMAN Kelch-like protein X [H.sapiens] | --- | >1 |
| 691 | 230490_x_at | AI866717 | hypothetical protein FLJ31034 | FLJ31034 | <1 |
| 692 | 227073_at | N50665 | Homo sapiens cDNA FLJ36574 fis, clone TRACH2012376. | --- | <1 |
| 693 | 226858_at | T51255 | chromosome 1 open reading frame 28 | C1orf28 | >1 |
| 694 | 219759_at | NM_022350.1 | aminopeptidase | LOC64167 | <1 |
| 695 | 208325_s_at | NM_006738.1 | A kinase (PRKA) anchor protein 13 | AKAP13 | >1 |
| 696 | 212053_at | AK025504.1 | KIAA0251 protein | KIAA0251 | <1 |
| 697 | 222715_s_at | BE856321 | AP1 gamma subunit binding protein 1 | AP1GBP1 | <1 |
| 698 | 235456_at | AI810266 | Homo sapiens, clone IMAGE:4819084, mRNA | --- | >1 |
| 699 | 235424_at | N66727 | EST | --- | <1 |
| 700 | 212407_at | AL049669.1 | CGI-01 protein | CGI-01 | <1 |
| 701 | 227565_at | BE501881 | EST | --- | <1 |
| 702 | 228091_at | AI800609 | EST, Weakly similar to D29149 proline-rich protein - mouse (fragment) [M.musculus] | --- | >1 |
| 703 | 209258_s_at | NM_005445.1 | chondroitin sulfate proteoglycan 6 (bamacan) | CSPG6 | >1 |
| 704 | 222590_s_at | AF180819.1 | nemo-like kinase | NLK | <1 |

(continued)

| No. | Probe set ID | Seq. Derived From (RefSeq/Genbank Accession) | Title | Gene Symbol | Hazard |
|---|---|---|---|---|---|
| 705 | 212528_at | AL023553 | Homo sapiens, clone IMAGE:3605655, mRNA | --- | <1 |
| 706 | 203981_s_at | AL574660 | ATP-binding cassette, sub-family D (ALD), member 4 | ABCD4 | >1 |
| 707 | 201011_at | NM_002950.1 | ribophorin I | RPN1 | <1 |
| 708 | 244268_x_at | BF435769 | EST, Weakly similar to hypothetical protein FLJ20378 [Homo sapiens] [H.sapiens] | --- | <1 |
| 709 | 202315_s_at | NM_004327.2 | breakpoint cluster region | BCR | <1 |
| 710 | 227698_s_at | AW007215 | RAB40C, member RAS oncogene family | RAB40C | >1 |
| 711 | 218311_at | NM_003618.1 | mitogen-activated protein kinase kinase kinase kinase 3 | MAP4K3 | <1 |
| 712 | 213931_at | AI819238 | inhibitor of DNA binding 2, dominant negative helix-loop-helix protein | ID2 | >1 |
| 713 | 217997_at | AA576961 | pleckstrin homology-like domain, family A, member 1 | PHLDA1 | >1 |
| 714 | 208951_at | BC002515.1 | aldehyde dehydrogenase 7 family, member A1 | ALDH7A1 | >1 |
| 715 | 225847_at | AB037784.1 | KIAA1363 protein | KIAA1363 | <1 |
| 716 | 202846_s_at | NM_002642.1 | phosphatidylinositol glycan, class C | PIGC | <1 |
| 717 | 200681_at | NM_006708.1 | glyoxalase I | GLO1 | <1 |
| 718 | 202727_s_at | NM_000416.1 | interferon gamma receptor 1 | IFNGR1 | <1 |
| 719 | 222231_s_at | AK025328.1 | hypothetical protein PRO1855 | PRO1855 | <1 |
| 720 | 228482_at | AV702789 | hypothetical protein FLJ36674 | FLJ36674 | >1 |
| 721 | 235056_at | AV722693 | EST | --- | <1 |
| 722 | 202010_s_at | NRM_021188.1 | likely ortholog of mouse another partner for ARF 1 | APA1 | >1 |
| 723 | 226556_at | BF431260 | Homo sapiens, clone IMAGE:4815204, mRNA | --- | <1 |
| 724 | 215088_s_at | BG110532 | EST, Highly similar to succinate dehydrogenase complex, subunit C precursor; Succinate dehydrogenase complex, subunit C, integral membrane protein,; succinate-ubiquinone oxidoreducatase cytochrome B large subunit [Homo sapiens] [H.sapiens] | --- | >1 |
| 725 | 209492_x_at | BC003679.1 | ATP synthase, H+ transporting, mitochondrial F0 complex, subunit e | ATP5I | >1 |
| 726 | 211075_s_at | Z25521.1 | CD47 antigen (Rh-related antigen, integrin-associated signal transducer) | CD47 | <1 |
| 727 | 204552_at | AA355179 | Homo sapiens cDNA FLJ34214 fis, clone FCBBF3021807. | --- | <1 |

(continued)

| No. | Probe set ID | Seq. Derived From (RefSeq/Genbank Accession) | Title | Gene Symbol | Hazard |
|---|---|---|---|---|---|
| 728 | 211862_x_at | AF015451.1 | CASP8 and FADD-like apoptosis regulator | CFLAR | <1 |
| 729 | 201403_s_at | NM_004528.1 | microsomal glutathione S-transferase 3 | MGST3 | <1 |
| 730 | 209899_s_at | AF217197.1 | fuse-binding protein-interacting repressor | SIAHBP1 | >1 |
| 731 | 219023_at | NM_018569.1 | hypothetical protein PRO0971 | PRO0971 | >1 |
| 732 | 236506_at | BF507371 | EST | --- | >1 |
| 733 | 205191_at | NRM_006915.1 | retinitis pigmentosa 2 (X-linked recessive) | RP2 | <1 |
| 734 | 202146_at | AA747426 | interferon-related developmental regulator 1 | IFRD1 | <1 |
| 735 | 243304_at | AI733824 | hypothetical protein LOC286109 | LOC286109 | >1 |
| 736 | 223658_at | AF134149.1 | potassium channel, subfamily K, member 6 | KCNK6 | <1 |
| 737 | 202074_s_at | NM_021980.1 | optineurin | OPTN | <1 |
| 738 | 203162_s_at | NM_005886.1 | katanin p80 (WD40-containing) subunit B 1 | KATNB1 | >1 |
| 739 | 208841_s_at | AB014560.1 | Ras-GTPase activating protein SH3 domain-binding protein 2 | G3BP2 | <1 |
| 740 | 230128_at | AK025231.1 | Homo sapiens cDNA: FLJ21578 fis, clone COL06726. | --- | <1 |
| 741 | 214394_x_at | AI613383 | eukaryotic translation elongation factor 1 delta (guanine nucleotide exchange protein) | EEF1D | >1 |
| 742 | 242969_at | AI288679 | EST | --- | <1 |
| 743 | 210251_s_at | AF112221.1 | rap2 interacting protein x | RIPX | >1 |
| 744 | 209894_at | U50748.1 | leptin receptor | LEPR | <1 |
| 745 | 204190_at | NM_005800.1 | highly charged protein | D13S106E | >1 |
| 746 | 202438_x_at | BF346014 | Homo sapiens, clone IMAGE:5278680, mRNA | --- | <1 |
| 747 | 211968_s_at | NM_005348.1 | heat shock 90kDa protein 1, alpha | HSPCA | >1 |
| 748 | 222424_s_at | BC000805.1 | similar to rat nuclear ubiquitous casein kinase 2 | NUCKS | >1 |
| 749 | 226445_s_at | AI743109 | tripartite motif-containing 41 | TRIM41 | >1 |
| 750 | 235061_at | AV706522 | hypothetical protein DKFZp761G058 | DKFZp761G058 | <1 |
| 751 | 34031_i_at | U90268 | cerebral cavernous malformations 1 | CCM1 | <1 |
| 752 | 213160_at | D86964.1 | dedicator of cyto-kinesis 2 | DOCK2 | <1 |
| 753 | 209194_at | BC005334.1 | centrin, EF-hand protein, 2 | CETN2 | <1 |
| 754 | 209240_at | AF070560.1 | O-linked N-acetylglucosamine (GlcNAc) transferase (UDP-N-acetylglucosamine:polypeptide-N-acetylglucosaminyl transferase) | OGT | <1 |

(continued)

| No. | Probe set ID | Seq. Derived From (RefSeq/Genbank Accession) | Title | Gene Symbol | Hazard |
|---|---|---|---|---|---|
| 755 | 218962_s_at | NM_022484.1 | hypothetical protein FLJ13576 | FLJ13576 | <1 |
| 756 | 203525_s_at | AI375486 | adenomatosis polyposis coli | APC | <1 |
| 757 | 219904_at | NM_024303.1 | hypothetical protein MGC4161 | MGC4161 | >1 |
| 758 | 205550_s_at | NM_004899.1 | brain and reproductive organ-expressed (TNFRSF1A modulator) | BRE | <1 |
| 759 | 209932_s_at | U90223.1 | dUTP pyrophosphatase | DUT | >1 |
| 760 | AFFX-M27830_M_at | M27830 | --- | --- | >1 |
| 761 | 205297_s_at | NM_000626.1 | CD79B antigen (immunoglobulin-associated beta) | CD79B | <1 |
| 762 | 232297_at | AL049385.1 | Homo sapiens mRNA; cDNA DKFZp586M1418 (from clone DKFZp586M1418) | --- | <1 |
| 763 | 204019_s_at | NM_015677.1 | likely ortholog of mouse Sh3 domain YSC-like 1 | SH3YL1 | <1 |
| 764 | 230769_at | AI916261 | EST, Weakly similar to PRP1_HUMAN Salivary proline-rich protein precursor (Clones CP3, CP4 and CP5) [Contains: Basic peptide IB-6; Peptide P-H] [H.sapiens] | --- | >1 |
| 765 | 217501_at | AI339732 | Homo sapiens, clone IMAGE:5268928, mRNA | --- | <1 |
| 766 | 205105_at | NM_002372.1 | mannosidase, alpha, class 2A, member 1 | MAN2A1 | <1 |
| 767 | 209514_s_at | BE502030 | RAB27A, member RAS oncogene family | RAB27A | <1 |
| 768 | 203217_s_at | NM_003896.1 | sialyltransferase 9 (CMP-NeuAc:lactosylceramide alpha-2,3-sialyltransferase; GM3 synthase) | SIAT9 | <1 |
| 769 | 203176_s_at | BE552470 | transcription factor A, mitochondrial | TFAM | >1 |
| 770 | 208988_at | AK024505.1 | F-box and leucine-rich repeat protein 11 | FBXL11 | <1 |
| 771 | 221500_s_at | AF008936.1 | aminopeptidase-like 1 | NPEPL1 | >1 |
| 772 | 229236_s_at | AI346445 | eukaryotic translation initiation factor 3, subunit 10 theta, 150/170kDa | EIF3S10 | <1 |
| 773 | 218267_at | N_016550.1 | cyclin-dependent kinase 2-interacting protein | CINP | >1 |
| 774 | 208129_x_at | NM_001754.1 | runt-related transcription factor 1 (acute myeloid leukemia 1; aml1 oncogene) | RUNX1 | >1 |
| 775 | 208764_s_at | D13119.1 | ATP synthase, H+ transporting, mitochondrial F0 complex, subunit c (subunit 9), isoform 2 | ATP5G2 | >1 |
| 776 | 225498_at | AV713673 | chromosome 20 open reading frame 178 | C20orf178 | <1 |

(continued)

| No. | Probe set ID | Seq. Derived From (RefSeq/Genbank Accession) | Title | Gene Symbol | Hazard |
|---|---|---|---|---|---|
| 777 | 211317_s_at | AF041461.1 | CASP8 and FADD-like apoptosis regulator | CFLAR | <1 |
| 778 | 200760_s_at | N92494 | vitamin A responsive; cytoskeleton related | JWA | <1 |
| 779 | 215483_at | AK000270.1 | A kinase (PRKA) anchor protein (yotiao) 9 | AKAP9 | <1 |
| 780 | 218194_at | NRM_015523.1 | small fragment nuclease | DKFZP566E144 | <1 |
| 781 | 201388_at | NM_002809.1 | proteasome (prosome, macropain) 26S subunit, non-ATPase, 3 | PSMD3 | <1 |
| 782 | 34406_at | AB011174 | KIAA0602 protein | KIAA0602 | >1 |
| 783 | 208386_x_at | NM_007068.1 | DMC1 dosage suppressor of mck1 homolog, meiosis-specific homologous recombination (yeast) | DMC1 | >1 |
| 784 | 244481_at | BF196523 | EST | --- | >1 |
| 785 | 239673_at | AW080999 | EST | --- | <1 |
| 786 | 208773_s_at | AL136943.1 | FLJ20288 protein | FLJ20288 | <1 |
| 787 | 222206_s_at | AA781143 | hypothetical protein from EUROIMAGE 2021883 | LOC56926 | >1 |
| 788 | 228658_at | R54042 | Homo sapiens cDNA FLJ25887 fis, clone CBR02996. | --- | <1 |
| 789 | 212586_at | BG111635 | type 1 tumor necrosis factor receptor shedding aminopeptidase regulator | ARTS-1 | <1 |
| 790 | 238011_at | BF668314 | Homo sapiens cDNA FLJ37032 fis, clone BRACE2011265. | --- | >1 |
| 791 | 204659_s_at | AF124604.1 | growth factor, augmenter of liver regeneration (ERV1 homolog, S. cerevisiae) | GFER | >1 |
| 792 | 200096_s_at | AI862255 | ATPase, H+ transporting, lysosomal 9kDa, V0 subunit e | ATP6V0E | <1 |
| 793 | 227293_at | AI264003 | Homo sapiens cDNA FLJ34052 fis, clone FCBBF3000175. | --- | <1 |
| 794 | 228454_at | AW663968 | KIAA1795 protein | MLR2 | <1 |
| 795 | 209576_at | AL049933.1 | guanine nucleotide binding protein (G protein), alpha inhibiting activity polypeptide 1 | GNAI1 | <1 |
| 796 | 201684_s_at | BE783632 | chromosome 14 open reading frame 92 | C14orf92 | >1 |
| 797 | 233068_at | AK023264.1 | EST, Weakly similar to POL2_MOUSE Retrovirus-related POL polyprotein [Contains: Reverse transcriptase ; Endonuclease] [M.musculus] | --- | <1 |
| 798 | 210532_s_at | AF116639.1 | chromosome 14 open reading frame 2 | C14orf2 | >1 |
| 799 | 211911_x_at | L07950.1 | major histocompatibility complex, class I, B | HLA-B | <1 |

(continued)

| No. | Probe set ID | Seq. Derived From (RefSeq/Genbank Accession) | Title | Gene Symbol | Hazard |
|---|---|---|---|---|---|
| 800 | 208991_at | AA634272 | Homo sapiens cDNA FLJ35646 fis, clone SPLEN2012743. | --- | <1 |
| 801 | 226612_at | AW572911 | Homo sapiens cDNA FLJ25076 fis, clone CBL06117. | --- | <1 |
| 802 | 223068_at | AV707345 | echinoderm microtubule associated protein like 4 | EML4 | <1 |
| 803 | 227462_at | BE889628 | EST | --- | <1 |
| 804 | 224680_at | AL539253 | Homo sapiens, clone IMAGE:3866125, mRNA | --- | <1 |
| 805 | 244075_at | BF224218 | EST | --- | >1 |
| 806 | 228220_at | AI627666 | hypothetical protein BC014311 | LOC115548 | <1 |
| 807 | 225729_at | AI870857 | Homo sapiens cDNA: FLJ21560 fis, clone COL06410. | --- | <1 |
| 808 | 222771_s_at | NM_016132.1 | myelin gene expression factor 2 | MEF-2 | <1 |
| 809 | 209944_at | BC000330.1 | likely ortholog of mouse another partner for ARF 1 | APA1 | >1 |
| 810 | 224565_at | AK027191.1 | Homo sapiens cDNA: FLJ23538 fis, clone LNG08010, highly similar to BETA2 Human MEN1 region clone epsilon/beta mRNA. | --- | <1 |
| 811 | 202439_s_at | NM_000202.2 | iduronate 2-sulfatase (Hunter syndrome) | IDS | <1 |
| 812 | 212051_at | AK026913.1 | Homo sapiens cDNA FLJ30463 fis, clone BRACE2009517. | --- | <1 |
| 813 | 211969_at | NM_005348.1 | heat shock 90kDa protein 1, alpha | HSPCA | >1 |
| 814 | 218209_s_at | NRM_018170.1 | hypothetical protein FLJ10656 | P15RS | <1 |
| 815 | 208877_at | AF092132.1 | Homo sapiens, clone IMAGE:6058556, mRNA | --- | <1 |
| 816 | 202043_s_at | N_004595.1 | spermine synthase | SMS | <1 |
| 817 | 209092_s_at | AF061730.1 | CGI-150 protein | CGI-150 | <1 |
| 818 | 225412_at | AA761169 | hypothetical protein FLJ14681 | FLJ14681 | <1 |
| 819 | 201173_x_at | NM_006600.1 | nuclear distribution gene C homolog (A. nidulans) | NUDC | >1 |
| 820 | 201409_s_at | NM_002709.1 | protein phosphatase 1, catalytic subunit, beta isoform | PPP1CB | <1 |
| 821 | 235594_at | AL542578 | EST, Weakly similar to cytokine receptor-like factor 2; cytokine receptor CRL2 precusor [Homo sapiens] [H.sapiens] | --- | >1 |
| 822 | 218269_at | NM_013235.1 | putative ribonuclease III | RNASE3L | >1 |
| 823 | 213892_s_at | AA927724 | adenine phosphoribosyltransferase | APRT | >1 |

(continued)

| No. | Probe set ID | Seq. Derived From (RefSeq/Genbank Accession) | Title | Gene Symbol | Hazard |
|---|---|---|---|---|---|
| 824 | 209715_at | L07515.1 | chromobox homolog 5 (HP1 alpha homolog, Drosophila) | CBX5 | >1 |
| 825 | 215001_s_at | AL161952.1 | glutamate-ammonia ligase (glutamine synthase) | GLUL | <1 |
| 826 | 230011_at | AW195720 | hypothetical protein MGC40042 | MGC40042 | <1 |
| 827 | 202623_at | NM_018453.1 | chromosome 14 open reading frame 11 | C14orf11 | >1 |
| 828 | 226749_at | AL582429 | Homo sapiens, clone IMAGE:4791565, mRNA | --- | <1 |
| 829 | 209337_at | AF063020.1 | PC4 and SFRS1 interacting protein 2 | PSIP2 | <1 |
| 830 | 216526_x_at | AK024836.1 | major histocompatibility complex, class I, C | HLA-C | <1 |
| 831 | 212428_at | AB002366.1 | KIAA0368 protein | PLAA0368 | <1 |
| 832 | 222035_s_at | AI984479 | poly(A) polymerase alpha | PAPOLA | >1 |
| 833 | 223277_at | BC000623.1 | hypothetical protein FLJ20211 | FLJ20211 | >1 |
| 834 | 212807 _s_at | BE742268 | sortilin 1 | SORT1 | >1 |
| 835 | 212193_s_at | BE881529 | likely ortholog of mouse la related protein | LARP | <1 |
| 836 | 238642_at | AW367571 | Homo sapiens full length insert cDNA clone YB31A06 | --- | >1 |
| 837 | 216607_s_at | U40053 | --- | --- | <1 |
| 838 | 224851_at | AW274756 | Homo sapiens cDNA FLJ31360 fis, clone MESAN2000572. | --- | <1 |
| 839 | 53202_at | AA402435 | hypothetical protein MGC2821 | MGC2821 | <1 |
| 840 | 224435_at | BC005871.1 | hypothetical protein MGC4248 | MGC4248 | <1 |
| 841 | 200953_s_at | NM_001759.1 | cyclin D2 | CCND2 | <1 |
| 842 | 240237_at | H23230 | EST, Moderately similar to hypothetical protein FLJ20489 [Homo sapiens] [H.sapiens] | --- | <1 |
| 843 | 227801_at | N90779 | EST, Weakly similar to hypothetical protein FLJ20378 [Homo sapiens] [H. sapiens] | --- | <1 |
| 844 | 243217_at | AI681312 | EST | --- | <1 |
| 845 | 217742_s_at | NM_016628.1 | WW domain-containing adapter with a coiled-coil region | WAC | <1 |
| 846 | 206472_s_at | NM_005078.1 | transducin-like enhancer of split 3 (E(sp1) homolog, Drosophila) | TLE3 | <1 |
| 847 | 219100_at | NM_024928.1 | hypothetical protein FLJ22559 | FLJ22559 | <1 |
| 848 | 41856_at | AL049370 | Homo sapiens mRNA; cDNA DKFZp586D0918 (from clone DKFZp586D0918) | --- | >1 |
| 849 | 211921_x_at | AF348514.1 | prothymosin, alpha (gene sequence 28) | PTMA | >1 |

(continued)

| No. | Probe set ID | Seq. Derived From (RefSeq/Genbank Accession) | Title | Gene Symbol | Hazard |
|---|---|---|---|---|---|
| 850 | 220597_s_at | NM_018694.1 | ADP-ribosylation-like factor 6 interacting protein 4 | ARL6IP4 | >1 |
| 851 | 202461_at | NM_014239.1 | eukaryotic translation initiation factor 2B, subunit 2 beta, 39kDa | EIF2B2 | >1 |
| 852 | 201734_at | NRM_001829.1 | Homo sapiens mRNA; cDNA DKFZp564I0463 (from clone DKFZp564I0463) | --- | <1 |
| 853 | 200644_at | NM_023009.1 | MARCKS-like protein | MLP | >1 |
| 854 | 223459_s_at | BE222214 | hypothetical protein FLJ20519 | FLJ20519 | >1 |
| 855 | 219215_s_at | NM_017767.1 | solute carrier family 39 (zinc transporter), member 4 | SLC39A4 | >1 |
| 856 | 201811_x_at | NM_004844.1 | SH3-domain binding protein 5 (BTK-associated) | SH3BP5 | <1 |
| 857 | 212264_s_at | D87450.1 | friend of EBNA2 | FOE | <1 |
| 858 | 218668_s_at | NM_021183.1 | hypothetical protein similar to small G proteins, especially RAP-2A | LOC57826 | <1 |
| 859 | 209418_s_at | BC003615.1 | chromosome 22 open reading frame 19 | C22orf19 | >1 |
| 860 | 203028_s_at | NRM_000101.1 | cytochrome b-245, alpha polypeptide | CYBA | >1 |
| 861 | 219410_at | NM_018004.1 | hypothetical protein FLJ10134 | FLJ10134 | <1 |
| 862 | 218220_at | NM_021640.1 | chromosome 12 open reading frame 10 | C12orf10 | >1 |
| 863 | 213154_s_at | AB014599.1 | coiled-coil protein BICD2 | BICD2 | >1 |
| 864 | 200920_s_at | AL535380 | B-cell translocation gene 1, anti-proliferative | BTG1 | >1 |
| 865 | 214459_x_at | M12679.1 | Cw1 antigen | HUMMHCW 1A | <1 |
| 866 | 205955_at | NM_018336.1 | hypothetical protein FLJ11136 | FLJ11136 | >1 |
| 867 | 218482_at | NM_020189.1 | DC6 protein | DC6 | >1 |
| 868 | 203159_at | NM_014905.1 | glutaminase | GLS | <1 |
| 869 | 217823_s_at | NM_016021.1 | ubiquitin-conjugating enzyme E2, J1 (UBC6 homolog, yeast) | UBE2J1 | <1 |
| 870 | 225445_at | AI332346 | EST | --- | <1 |
| 871 | 211368_s_at | U13700.1 | caspase 1, apoptosis-related cysteine protease (interleukin 1, beta, convertase) | CASP1 | <1 |
| 872 | 227811_at | AK000004.1 | FGD1 family, member 3 | FGD3 | >1 |
| 873 | 204116_at | NM_000206.1 | interleukin 2 receptor, gamma (severe combined immunodeficiency) | IL2RG | <1 |
| 874 | 212120_at | BF348067 | ras-like protein TC10 | TC10 | <1 |
| 875 | 37986_at | M60459 | erythropoietin receptor | EPOR | <1 |
| 876 | 242692_at | AI798758 | EST | --- | >1 |

(continued)

| No. | Probe set ID | Seq. Derived From (RefSeq/Genbank Accession) | Title | Gene Symbol | Hazard |
|---|---|---|---|---|---|
| 877 | 209644_x_at | U38945.1 | cyclin-dependent kinase inhibitor 2A (melanoma, p16, inhibits CDK4) | CDKN2A | >1 |
| 878 | 228545_at | AI016784 | EST | --- | <1 |
| 879 | 201858_s_at | J03223.1 | proteoglycan 1, secretory granule | PRG1 | <1 |
| 880 | 215823_x_at | U64661 | EST, Highly similar to PAB1_HUMAN Polyadenylate-binding protein 1 (Poly(A)-binding protein 1) (PABP 1) (PABP1) [H.sapiens] | --- | >1 |
| 881 | 201972_at | AF113129.1 | ATPase, H+ transporting, lysosomal 70kDa, V1 subunit A, isoform 1 | ATP6V1A1 | <1 |
| 882 | 201951_at | NM_001627.1 | activated leukocyte cell adhesion molecule | ALCAM | <1 |
| 883 | 201986_at | NM_005121.1 | thyroid hormone receptor-associated protein, 240 kDa subunit | TRAP240 | <1 |
| 884 | 202393_s_at | NM_005655.1 | TGFB inducible early growth response | TIEG | >1 |
| 885 | 212118_at | NM_006510.1 | ret finger protein | RFP | <1 |
| 886 | 225910_at | BF514723 | hypothetical protein LOC284019 | LOC284019 | <1 |
| 887 | 218795_at | NM_016361.1 | lysophosphatidic acid phosphatase | ACP6 | >1 |
| 888 | 204985_s_at | NM_024108.1 | hypothetical protein MGC2650 | MGC2650 | >1 |
| 889 | 217436_x_at | M80469 | --- | --- | <1 |
| 890 | 215690_x_at | AL157437.1 | GPAA1P anchor attachment protein 1 homolog (yeast) | GPAA1 | >1 |
| 891 | 208683_at | M23254.1 | calpain 2, (m/II) large subunit | CAPN2 | <1 |
| 892 | 223638_at | AL136890.1 | hypothetical protein DKFZp434D177 | DKFZp434D 177 | <1 |
| 893 | 218079_s_at | NM_024835.1 | C3HC4-type zinc finger protein | LZK1 | <1 |
| 894 | 209250_at | BC000961.2 | degenerative spermatocyte homolog, lipid desaturase (Drosophila) | DEGS | <1 |
| 895 | 238724_at | R63824 | EST | --- | >1 |
| 896 | 212809_at | AA152202 | hypothetical protein FLJ14639 | FLJ14639 | >1 |
| 897 | 222391_at | AL080250 | hypothetical protein FLJ10856 | FLJ10856 | <1 |
| 898 | 209533_s_at | AF145020.1 | phospholipase A2-activating protein | PLAA | <1 |
| 899 | 218205_s_at | NM_017572.1 | MAP kinase-interacting serine/threonine kinase 2 | MKNK2 | >1 |
| 900 | 232174_at | AA480392 | Homo sapiens clone 24838 mRNA sequence | --- | >1 |
| 901 | 201068_s_at | NM_002803.1 | proteasome (prosome, macropain) 26S subunit, ATPase, 2 | PSMC2 | <1 |
| 902 | 218573_at | NM_0 14061.1 | APR-1 protein | MAGEH1 | <1 |
| 903 | 216272_x_at | AF209931.1 | hypothetical protein FLJ13511 | 7h3 | >1 |

(continued)

| No. | Probe set ID | Seq. Derived From (RefSeq/Genbank Accession) | Title | Gene Symbol | Hazard |
|---|---|---|---|---|---|
| 904 | 222309_at | AW972292 | EST | --- | >1 |
| 905 | 226461_at | AA204719 | homeo box B9 | HOXB9 | >1 |
| 906 | 214449_s_at | NM_012249.1 | ras-like protein TC10 | TC10 | <1 |
| 907 | 217880_at | AI203880 | cell division cycle 27 | CDC27 | <1 |
| 908 | 213238_at | AI478147 | ATPase, Class V, type 10D | ATP10D | <1 |
| 909 | 228464_at | AI651510 | EST, Weakly similar to T12486 hypothetical protein DKFZp566H033.1 - human [H.sapiens] | --- | <1 |
| 910 | 203157_s_at | AB020645.1 | glutaminase | GLS | <1 |
| 911 | 204547_at | NM_006822.1 | RAB40B, member RAS oncogene family | RAB40B | >1 |
| 912 | 203067_at | NM_003477.1 | E3-binding protein | PDX1 | <1 |
| 913 | 228289_at | AI131537 | adenylate cyclase 7 | ADCY7 | <1 |
| 914 | 217955_at | NM_015367.1 | BCL2-like 13 (apoptosis facilitator) | BCL2L13 | <1 |
| 915 | 201768_s_at | BC004467.1 | enthoprotin | ENTH | <1 |
| 916 | 217832_at | NM_006372.1 | NS1-associated protein 1 | NSAP1 | <1 |
| 917 | 226923_at | AW205790 | hypothetical protein FLJ39514 | FLJ39514 | <1 |
| 918 | 217939_s_at | NM_017657.1 | hypothetical protein FLJ20080 | FLJ20080 | <1 |
| 919 | 244732_at | R06827 | Homo sapiens, clone IMAGE:5276307, mRNA | --- | >1 |
| 920 | 221718_s_at | M90360.1 | A kinase (PRKA) anchor protein 13 | AKAP13 | >1 |
| 921 | 218970_s_at | NM_015960.1 | CGI-32 protein | CGI-32 | <1 |
| 922 | 214259_s_at | AW074911 | aldo-keto reductase family 7, member A2 (aflatoxin aldehyde reductase) | AKR7A2 | >1 |
| 923 | 204020_at | BF739943 | purine-rich element binding protein A | PURA | <1 |
| 924 | 205565_s_at | NM_000144.1 | Friedreich ataxia | FRDA | <1 |
| 925 | 218768_at | NM_020401.1 | nuclear pore complex protein | NUP107 | >1 |
| 926 | 202011_at | NM_003257.1 | tight junction protein 1 (zona occludens 1) | TJP1 | <1 |
| 927 | 211423_s_at | D85181.1 | sterol-C5-desaturase (ERG3 delta-5-desaturase homolog, fungal)-like | SC5DL | <1 |
| 928 | 202738_s_at | BG149218 | phosphorylase kinase, beta | PHKB | <1 |
| 929 | 228697_at | AW731710 | histidine triad nucleotide binding protein 3 | HINT3 | <1 |
| 930 | 225317_at | AL574669 | hypothetical protein MGC2404 | MGC2404 | >1 |
| 931 | 217368_at | X69909 | --- | --- | >1 |
| 932 | 201393_s_at | NM_000876.1 | insulin-like growth factor 2 receptor | IGF2R | <1 |
| 933 | 205158_at | NM_002937.1 | ribonuclease, RNase A family, 4 | RNASE4 | <1 |
| 934 | 200734_s_at | BG341906 | ADP-ribosylation factor 3 | ARF3 | >1 |
| 935 | 239586_at | AA085776 | hypothetical protein MGC14128 | MGC14128 | >1 |

(continued)

| No. | Probe set ID | Seq. Derived From (RefSeq/Genbank Accession) | Title | Gene Symbol | Hazard |
|---|---|---|---|---|---|
| 936 | 225216_at | AI590719 | Homo sapiens cDNA: FLJ21191 fis, clone COL00104. | --- | <1 |
| 937 | 203373_at | NM_003877.1 | suppressor of cytokine signaling 2 | SOCS2 | >1 |
| 938 | 218003_s_at | NM_002013.1 | FK506 binding protein 3, 25kDa | FKBP3 | >1 |
| 939 | 208296_x_at | NM_014350.1 | TNF-induced protein | GG2-1 | <1 |
| 940 | 217716_s_at | NM_013336.1 | protein transport protein SEC61 alpha subunit isoform 1 | SEC61A1 | <1 |
| 941 | 202028_s_at | BC000603.1 | ribosomal protein L38 | RPL38 | >1 |
| 942 | 218231_at | NM_017567.1 | N-acetylglucosamine kinase | NAGK | <1 |
| 943 | 211528_x_at | M90685.1 | HLA-G histocompatibility antigen, class I, G | HLA-G | <1 |
| 944 | 203142_s_at | NM_003664.1 | adaptor-related protein complex 3, beta 1 subunit | AP3B1 | <1 |
| 945 | 230597_at | AI963203 | solute carrier family 7 (cationic amino acid transporter, y+ system), member 3 | SLC7A3 | >1 |
| 946 | 200864_s_at | NM_004663.1 | RAB11A, member RAS oncogene family | RAB11A | <1 |
| 947 | 205541_s_at | NM_018094.1 | G1 to S phase transition 2 | GSPT2 | <1 |
| 948 | 209267_s_at | AB040120.1 | BCG-induced gene in monocytes, clone 103 | BIGM103 | <1 |
| 949 | 207428_x_at | NM_001787.1 | cell division cycle 2-like 1 (PITSLRE proteins) | CDC2L1 | >1 |
| 950 | 205801_s_at | NM_015376.1 | guanine nucleotide exchange factor for Rap 1 | GRP3 | <1 |
| 951 | 228614_at | AW182614 | hypothetical protein LOC205251 | LOC205251 | <1 |
| 952 | 230261_at | AA552969 | Homo sapiens, clone IMAGE:4816784, mRNA | --- | <1 |
| 953 | 229194_at | AL045882 | Homo sapiens, clone IMAGE:5273745, mRNA | --- | <1 |
| 954 | 224951_at | BE348305 | hypothetical protein MGC45411 | LOC91012 | >1 |
| 955 | 230026_at | N74662 | mitochondrial ribosomal protein L43 | MRPL43 | >1 |
| 956 | 217975_at | NM_016303.1 | pp21 homolog | LOC51186 | <1 |
| 957 | 212714_at | AL050205.1 | c-Mpl binding protein | LOC113251 | <1 |
| 958 | 212990_at | AB020717.1 | synaptojanin 1 | SYNJ1 | <1 |
| 959 | 211356_x_at | U66495.1 | leptin receptor | LEPR | <1 |
| 960 | 241342_at | BG288115 | hypothetical protein BC017881 | LOC157378 | >1 |
| 961 | 239891_x_at | AA001052 | EST, Weakly similar to RB10_HUMAN Ras-related protein Rab-10 [H.sapiens] | --- | <1 |
| 962 | 214672_at | AB023215.1 | KIAA0998 protein | KIAA0998 | >1 |
| 963 | 201628_s_at | NM_006570.1 | Ras-related GTP-binding protein | RAGA | <1 |

(continued)

| No. | Probe set ID | Seq. Derived From (RefSeq/Genbank Accession) | Title | Gene Symbol | Hazard |
|---|---|---|---|---|---|
| 964 | 232761_at | AL117381 | cytochrome c oxidase subunit IV isoform 2 | COX4I2 | >1 |
| 965 | 233164_x_at | AK026955.1 | hypothetical protein DKFZp547E052 | DKFZp547E052 | <1 |
| 966 | 200077_s_at | D87914.1 | ornithine decarboxylase antizyme 1 | OAZ1 | >1 |
| 967 | 219549_s_at | NM_006054.1 | reticulon 3 | RTN3 | <1 |
| 968 | 203560_at | NM_003878.1 | gamma-glutamyl hydrolase (conjugase, folylpolygammaglutamyl hydrolase) | GGH | >1 |
| 969 | 217923_at | NM_012392.1 | PEF protein with a long N-terminal hydrophobic domain (peflin) | PEF | <1 |
| 970 | 201862_s_at | NM_004735.1 | leucine rich repeat (in FLII) interacting protein 1 | LRRFIP1 | <1 |
| 971 | 223400_s_at | AF197569.1 | polybromo 1 | PB1 | <1 |
| 972 | AFFX-M27830_M_at | M27830 | --- | --- | >1 |
| 973 | 41220_at | AB023208 | MLL septin-like fusion | MSF | >1 |
| 974 | 209276_s_at | AF162769.1 | glutaredoxin (thioltransferase) | GLRX | <1 |
| 975 | 207627_s_at | NM_005653.1 | transcription factor CP2 | TFCP2 | <1 |
| 976 | 204785_x_at | NM_000874.1 | interferon (alpha, beta and omega) receptor 2 | IFNAR2 | >1 |
| 977 | 222615_s_at | AW206812 | hypothetical protein FLJ13902 | FLJ13902 | >1 |
| 978 | 200949_x_at | NM_001023.1 | ribosomal protein S20 | RPS20 | >1 |
| 979 | 217192_s_at | AL022067 | PR domain containing 1, with ZNF domain | PRDM1 | >1 |
| 980 | 235792_x_at | AU154663 | Homo sapiens mRNA; cDNA DKFZp564L222 (from clone DKFZp564L222) | --- | <1 |
| 981 | 213857_s_at | BG230614 | Homo sapiens, clone IMAGE:4822825, mRNA | --- | <1 |
| 982 | 235507_at | AA461195 | similar to hypothetical protein FLJ10883 | LOC115294 | >1 |
| 983 | 218191_s_at | NM_018368.1 | hypothetical protein FLJ11240 | FLJ11240 | <1 |
| 984 | 200649_at | BC002356.1 | nucleobindin 1 | NUCB1 | <1 |
| 985 | 210260_s_at | BC005352.1 | TNF-induced protein | GG2-1 | <1 |
| 986 | 209513_s_at | BC004331.1 | hypothetical protein MGC10940 | MGC10940 | <1 |
| 987 | 211801_x_at | AF329637.1 | mitofusin 1 | MFN1 | <1 |
| 988 | 206875_s_at | NM_014720.1 | Ste20-related serine/threonine kinase | SLK | <1 |
| 989 | 39705_at | AB014600 | SIN3 homolog B, transcriptional regulator (yeast) | SIN3B | <1 |
| 990 | 203658_at | BC001689.1 | solute carrier family 25 (carnitine/acylcarnitine translocase), member 20 | SLC25A20 | <1 |

(continued)

| No. | Probe set ID | Seq. Derived From (RefSeq/Genbank Accession) | Title | Gene Symbol | Hazard |
|---|---|---|---|---|---|
| 991 | 235566_at | AW591660 | Homo sapiens cDNA FLJ39046 fis, clone NT2RP7010612. | --- | <1 |
| 992 | 205089_at | NM_003416.1 | zinc finger protein 7 (KOX 4, clone HF.16) | ZNF7 | >1 |
| 993 | 212040_at | AK025557.1 | Homo sapiens, clone IMAGE:6057297, mRNA | --- | <1 |
| 994 | 210962_s_at | AB019691.1 | A kinase (PRKA) anchor protein (yotiao) 9 | AKAP9 | <1 |
| 995 | 203053_at | NM_005872.1 | breast carcinoma amplified sequence 2 | BCAS2 | >1 |
| 996 | 233867_at | AK000119.1 | EST, Moderately similar to KIAA0737 gene product [Homo sapiens] [H.sapiens] | --- | >1 |
| 997 | 200993_at | AL137335.1 | EST | --- | <1 |
| 998 | 204328_at | NM_007267.2 | epidermodysplasia verruciformis 1 | EVER1 | >1 |
| 999 | 212926_at | AB011166.1 | SMC5 structural maintenance of chromosomes 5-like 1 (yeast) | SMC5L1 | >1 |
| 1000 | 229353_s_at | AW515443 | similar to rat nuclear ubiquitous casein kinase 2 | NUCKS | >1 |
| 1001 | 212455_at | N36997 | KIAA1966 protein | KIAA1966 | <1 |
| 1002 | 202025_x_at | NM_001607.2 | acetyl-Coenzyme A acyltransferase 1 (peroxisomal 3-oxoacyl-Coenzyme A thiolase) | ACAA1 | >1 |
| 1003 | 235009_at | AI049791 | hypothetical protein FLJ33215 | FLJ33215 | >1 |
| 1004 | 218306_s_at | NM_003922.1 | hect (homologous to the E6-AP (UBE3A) carboxyl terminus) domain and RCC1 (CHC1)-like domain (RLD) 1 | HERC1 | <1 |
| 1005 | 225592_at | D81048 | nurim (nuclear envelope membrane protein) | NRM | >1 |
| 1006 | 238604_at | AA768884 | Homo sapiens cDNA FLJ25559 fis, clone JTH02834. | --- | <1 |
| 1007 | 202264_s_at | NM_006114.1 | translocase of outer mitochondrial membrane 40 homolog (yeast) | TOMM40 | >1 |
| 1008 | 239258_at | BE551407 | EST, Moderately similar to hypothetical protein FLJ20234 [Homo sapiens] [H.sapiens] | --- | <1 |
| 1009 | 210538_s_at | U37546.1 | baculoviral IAP repeat-containing 3 | BIRC3 | <1 |
| 1010 | 202545_at | NM_006254.1 | protein kinase C, delta | PRKCD | <1 |
| 1011 | 212622_at | D26067.1 | KIAA0033 protein | KIAA0033 | <1 |
| 1012 | 207431_s_at | NM_003676.1 | degenerative spermatocyte homolog, lipid desaturase (Drosophila) | DEGS | <1 |
| 1013 | 218549_s_at | NM_016033.1 | CGI-90 protein | CGI-90 | >1 |
| 1014 | 225058_at | AL365404.1 | G protein-coupled receptor 108 | GPR108 | <1 |

(continued)

| No. | Probe set ID | Seq. Derived From (RefSeq/Genbank Accession) | Title | Gene Symbol | Hazard |
|---|---|---|---|---|---|
| 1015 | 224847_at | AW274756 | Homo sapiens cDNA FLJ20653 fis, clone KAT01739. | --- | <1 |
| 1016 | 222024_s_at | AK022014.1 | A kinase (PRKA) anchor protein 13 | AKAP13 | >1 |
| 1017 | 208882_s_at | U69567 | progestin induced protein | DD5 | >1 |
| 1018 | 208937_s_at | D13889.1 | inhibitor of DNA binding 1, dominant negative helix-loop-helix protein | ID1 | >1 |
| 1019 | 200857_s_at | NM_006311.1 | nuclear receptor co-repressor 1 | NCOR1 | <1 |
| 1020 | 219972_s_at | NM_022495.1 | chromosome 14 open reading frame 135 | C14orf135 | >1 |
| 1021 | 226191_at | AW139538 | EST, Highly similar to SMD1_HUMAN Small nuclear ribonucleoprotein Sm D1 (snRNP core protein D1) (Sm-D1) (Sm-D autoantigen) [H.sapiens] | --- | <1 |
| 1022 | 222129_at | AK026155.1 | hypothetical protein MGC3035 | MGC3035 | <1 |
| 1023 | 201668_x_at | AW163148 | myristoylated alanine-rich protein kinase C substrate | MARCKS | >1 |
| 1024 | 208549_x_at | NRM_016171.1 | prothymosin a14 | LOC51685 | >1 |
| 1025 | 242241_x_at | R66713 | EST | --- | >1 |
| 1026 | 211671_s_at | U01351.1 | nuclear receptor subfamily 3, group C, member 1 (glucocorticoid receptor) | NR3C1 | <1 |
| 1027 | 221787_at | AF055030.1 | PHD zinc finger protein XAP135 | XAP135 | <1 |
| 1028 | 228600_x_at | BE220330 | Homo sapiens mRNA; cDNA DKFZp686F0810 (from clone DKFZp686F0810) | --- | <1 |
| 1029 | 213620_s_at | AA126728 | intercellular adhesion molecule 2 | ICAM2 | <1 |
| 1030 | 204267_x_at | NM_004203.1 | membrane-associated tyrosine- and threonine-specific cdc2-inhibitory kinase | PKMYT1 | >1 |
| 1031 | 205443_at | NM_003082.1 | small nuclear RNA activating complex, polypeptide 1, 43kDa | SNAPC1 | >1 |
| 1032 | 218408_at | NM_012456.1 | translocase of inner mitochondrial membrane 10 homolog (yeast) | TIMM10 | >1 |
| 1033 | 221897_at | AA205660 | tripartite motif-containing 52 | TRIM52 | <1 |
| 1034 | 201970_s_at | NM_002482.1 | nuclear autoantigenic sperm protein (histone-binding) | NASP | >1 |
| 1035 | 227701_at | AK024739.1 | CTCL tumor antigen L14-2 | FLJ10188 | <1 |
| 1036 | 228549_at | AI491983 | EST, Moderately similar to hypothetical protein FLJ20378 [Homo sapiens] [H. sapiens] | --- | <1 |
| 1037 | 211404_s_at | BC004371.1 | amyloid beta (A4) precursor-like protein 2 | APLP2 | >1 |
| 1038 | 218905_at | NM_017864.1 | hypothetical protein FLJ20530 | FLJ20530 | >1 |

(continued)

| No. | Probe set ID | Seq. Derived From (RefSeq/Genbank Accession) | Title | Gene Symbol | Hazard |
|---|---|---|---|---|---|
| 1039 | 203774_at | NM_000254.1 | 5-methyltetrahydrofolate-homocysteine methyltransferase | MTR | <1 |
| 1040 | 200759_x_at | NM_003204.1 | nuclear factor (erythroid-derived 2)-like 1 | NFE2L1 | <1 |
| 1041 | 242674_at | T82467 | Homo sapiens cDNA FLJ41014 fis, clone UTERU2018674. | --- | >1 |
| 1042 | AFFX-HSAC07/XO0351_M_at | X00351 | actin, beta | ACTB | <1 |
| 1043 | 201025_at | NM_015904.1 | translation initiation factor IF2 | IF2 | <1 |
| 1044 | 226344_at | AI741051 | KIAA1789 protein | KIAA1789 | <1 |
| 1045 | 227854_at | BE620258 | hypothetical protein FLJ10335 | FLJ10335 | <1 |
| 1046 | 220202_s_at | NM_018835.1 | membrane-associated nucleic acid binding protein | MNAB | <1 |
| 1047 | 203158_s_at | AF097493.1 | glutaminase | GLS | <1 |
| 1048 | 233186_s_at | AK001039.1 | BTG3 associated nuclear protein | BANP | >1 |
| 1049 | 205569_at | NM_014398.1 | lysosomal-associated membrane protein 3 | LAMP3 | <1 |
| 1050 | 222680_s_at | AK001261.1 | RA-regulated nuclear matrix-associated protein | RAMP | >1 |
| 1051 | 208523_x_at | NM_003525.1 | histone 1, H2bi | HIST1H2BI | >1 |
| 1052 | 207761_s_at | NM_014033.1 | DKFZP586A0522 protein | DKFZP586A0522 | <1 |
| 1053 | 220547_s_at | NM_019054.1 | hypothetical protein MGC5560 | MGC5560 | <1 |
| 1054 | 224912_at | BE205790 | tetratricopeptide repeat domain 7 | TTC7 | <1 |
| 1055 | 211367_s_at | U13699.1 | caspase 1, apoptosis-related cysteine protease (interleukin 1, beta, convertase) | CASP1 | <1 |
| 1056 | 209376_x_at | AW084759 | splicing factor, arginine/serine-rich 2, interacting protein | SFRS2IP | >1 |
| 1057 | 213932_x_at | AI923492 | major histocompatibility complex, class I, A | HLA-A | <1 |
| 1058 | 202261_at | NM_005997.1 | transcription factor-like 1 | TCFL1 | >1 |
| 1059 | 213811_x_at | BG393795 | transcription factor 3 (E2A immunoglobulin enhancer binding factors E12/E47) | TCF3 | >1 |
| 1060 | 212833_at | M74089.1 | hypothetical protein BC017169 | LOC91137 | <1 |
| 1061 | 216540_at | X61072.1 | T cell receptor alpha locus | TRA@ | >1 |
| 1062 | 215284_at | AF070575.1 | Homo sapiens clone 24407 mRNA sequence | --- | <1 |
| 1063 | 239395_at | AA835887 | Homo sapiens, clone IMAGE:5286379, mRNA | --- | >1 |
| 1064 | 209388_at | BC000927.1 | poly(A) polymerase alpha | PAPOLA | >1 |
| 1065 | 235038_at | BF665176 | HIV-1 rev binding protein 2 | HRB2 | >1 |

(continued)

| No. | Probe set ID | Seq. Derived From (RefSeq/Genbank Accession) | Title | Gene Symbol | Hazard |
|---|---|---|---|---|---|
| 1066 | 235745_at | AV704183 | hypothetical protein FLJ30999 | FLJ30999 | <1 |
| 1067 | 242048_at | BE905316 | EST | --- | >1 |
| 1068 | 239250_at | BE966038 | hypothetical protein LOC147947 | LOC147947 | >1 |
| 1069 | 213828_x_at | AA477655 | H3 histone, family 3A | H3F3A | >1 |
| 1070 | 222593_s_at | AA584308 | hypothetical protein FLJ13117 | FLJ13117 | >1 |
| 1071 | 229075_at | AI754871 | EST | --- | <1 |
| 1072 | 219978_s_at | NM_018454.1 | nucleolar protein ANKT | ANKT | >1 |
| 1073 | 211676_s_at | AF056979.1 | interferon gamma receptor 1 | IFNGR1 | <1 |
| 1074 | 234347_s_at | AF038554.1 | density-regulated protein | DENR | >1 |
| 1075 | 209066_x_at | M26700.1 | ubiquinol-cytochrome c reductase binding protein | UQCRB | >1 |
| 1076 | 241435_at | AA702930 | EST | --- | >1 |
| 1077 | 219507_at | NM_016625.1 | hypothetical protein LOC51319 | LOC51319 | >1 |
| 1078 | 202284_s_at | NM_000389.1 | cyclin-dependent kinase inhibitor 1A (p21, Cip1) | CDKN1A | <1 |
| 1079 | 218732_at | NM_016077.1 | CGI-147 protein | CGI-147 | <1 |
| 1080 | 207654_x_at | NM_001938.1 | down-regulator of transcription 1, TBP-binding (negative cofactor 2) | DR1 | >1 |
| 1081 | 226671_at | AI150000 | Homo sapiens, clone IMAGE:4797120, mRNA | --- | <1 |
| 1082 | 227637_at | AV712694 | transcription factor CP2 | TFCP2 | >1 |
| 1083 | 201580_s_at | AL544094 | hypothetical protein Dr971N18.2 | DJ971N18.2 | <1 |
| 1084 | 226580_at | AA779684 | breast cancer metastasis-suppressor 1 | BRMS1 | >1 |
| 1085 | 224312_x_at | BC000675.1 | hypothetical protein FLJ20542 | FLJ20542 | >1 |
| 1086 | 227425_at | AI984607 | Homo sapiens cDNA FLJ40165 fis, clone TESTI2015962. | --- | <1 |
| 1087 | 202643_s_at | AI738896 | tumor necrosis factor, alpha-induced protein 3 | TNFAIP3 | <1 |
| 1088 | 227080_at | AW003092 | Homo sapiens cDNA: FLJ23366 fis, clone HEP15665. | --- | >1 |
| 1089 | 235353_at | AI887866 | KIAA0746 protein | KIAA0746 | >1 |
| 1090 | 209534_x_at | BF222823 | A kinase (PRKA) anchor protein 13 | AKAP13 | >1 |
| 1091 | 235103_at | AA029155 | Homo sapiens mRNA; cDNA DKFZp686H1529 (from clone DKFZp686H1529) | --- | <1 |
| 1092 | 235474_at | AI241810 | EST, Weakly similar to T31613 hypothetical protein Y50E8A.i - Caenorhabditis elegans [C.elegans] | --- | <1 |
| 1093 | 218662_s_at | NM_022346.1 | chromosome condensation protein G | HCAP-G | >1 |

(continued)

| No. | Probe set ID | Seq. Derived From (RefSeq/Genbank Accession) | Title | Gene Symbol | Hazard |
|---|---|---|---|---|---|
| 1094 | 208668_x_at | BC003689.1 | high-mobility group nucleosomal binding domain 2 | HMGN2 | >1 |
| 1095 | 214919_s_at | R39094 | Homo sapiens, clone IMAGE:3866125, mRNA | --- | <1 |
| 1096 | 218976_at | NM_021800.1 | J domain containing protein 1 | JDP1 | <1 |
| 1097 | 241955_at | BE243270 | EST, Weakly similar to C34D4.14.p [Caenorhabditis elegans] [C.elegans] | --- | >1 |
| 1098 | 201138_s_at | BG532929 | Sjogren syndrome antigen B (autoantigen La) | SSB | >1 |
| 1099 | 209056_s_at | AW268817 | CDC5 cell division cycle 5-like (S. pombe) | CDC5L | >1 |
| 1100 | 219384_s_at | NM_012091.2 | adenosine deaminase, tRNA-specific 1 | ADAT1 | <1 |
| 1101 | 212886_at | AL080169.1 | DKFZP434C171 protein | DKFZP434C171 | <1 |
| 1102 | 226773_at | AW290940 | Homo sapiens cDNA FLJ35131 fis, clone PLACE6008824. | --- | <1 |
| 1103 | 215756_at | AU153979 | Homo sapiens cDNA FLJ14231 fis, clone NT2RP3004470. | --- | >1 |
| 1104 | 227994_x_at | AA548838 | chromosome 20 open reading frame 149 | C20orf149 | >1 |
| 1105 | 218120_s_at | D21243.1 | heme oxygenase (decycling) 2 | HMOX2 | <1 |
| 1106 | 225092_at | AL550977 | rabaptin-5 | RAB5EP | <1 |
| 1107 | 220696_at | NM_014129.1 | PRO0478 protein | PRO0478 | >1 |
| 1108 | 210170_at | BC001017.1 | alpha-actinin-2-associated LIM protein | ALP | >1 |
| 1109 | 224648_at | AI860946 | vasculin | DKFZp761C169 | <1 |
| 1110 | 212830_at | BF110421 | EGF-like-domain, multiple 5 | EGFL5 | <1 |
| 1111 | 213410_at | AL050102.1 | DKFZp586F1019 protein | DKFZp586F1019 | >1 |
| 1112 | 212718_at | BG110231 | poly(A) polymerase alpha | PAPOLA | >1 |
| 1113 | 203173_s_at | AW080196 | esophageal cancer associated protein | MGC16824 | >1 |
| 1114 | 229520_s_at | BF060678 | chromosome 14 open reading frame 118 | C14orf118 | >1 |
| 1115 | 203974_at | NM_012080.1 | family with sequence similarity 16, member A, X-linked | FAM16AX | <1 |
| 1116 | 230075_at | AV724323 | RAB39B, member RAS oncogene family | RAB39B | <1 |
| 1117 | 225880_at | BF676081 | Homo sapiens cDNA FLJ11174 fis, clone PLACE1007367. | --- | <1 |
| 1118 | 222891_s_at | AI912275 | B-cell CLL/lymphoma 11A (zinc finger protein) | BCL11A | <1 |
| 1119 | 213494_s_at | AA748649 | YY1 transcription factor | YY1 | >1 |
| 1120 | 211366_x_at | U13698.1 | caspase 1, apoptosis-related cysteine protease (interleukin 1, beta, convertase) | CASP1 | <1 |

(continued)

| No. | Probe set ID | Seq. Derived From (RefSeq/Genbank Accession) | Title | Gene Symbol | Hazard |
|---|---|---|---|---|---|
| 1121 | 221995_s_at | BF195165 | mitochondrial ribosomal protein 63 | MRP63 | >1 |
| 1122 | 203322_at | NM_014913.1 | KIA.A0863 protein | KIAA0863 | <1 |
| 1123 | 243051_at | AW135412 | EST | --- | >1 |
| 1124 | 207245_at | NM_001077.1 | UDP glycosyltransferase 2 family, polypeptide B 17 | UGT2B17 | <1 |
| 1125 | 225651_at | BF431962 | hypothetical protein FLJ25157 | FLJ25157 | <1 |
| 1126 | 232288_at | AK026209.1 | Homo sapiens cDNA: FLJ22556 fis, clone HSI01326. | --- | <1 |
| 1127 | 218701_at | NM_016027.1 | CGI-83 protein | CGI-83 | >1 |
| 1128 | 201102_s_at | NM_002626.1 | phosphofructokinase, liver | PFKL | >1 |
| 1129 | 210458_s_at | BC003388.1 | TRAF family member-associated NFKB activator | TANK | <1 |
| 1130 | 226787_at | BF966015 | zinc finger protein 18 (KOX 11) | ZNF18 | <1 |
| 1131 | 218679_s_at | NM_016208.1 | vacuolar protein sorting 28 (yeast) | VPS28 | >1 |
| 1132 | 212232_at | AB023231.1 | formin binding protein 4 | FNBP4 | <1 |
| 1133 | 212221_x_at | AL117536.1 | Homo sapiens, clone IMAGE:5278680, mRNA | --- | <1 |
| 1134 | 200995_at | AL137335.1 | importin 7 | IPO7 | <1 |
| 1135 | 229549_at | AA868461 | calumenin | CALU | <1 |
| 1136 | 227239_at | AV734839 | down-regulated by Ctnnb1, a | DRCTNNB1 A | <1 |
| 1137 | 210716_s_at | M97501.1 | restin (Reed-Steinberg cell-expressed intermediate filament-associated protein) | RSN | <1 |
| 1138 | 235170_at | T52999 | hypothetical protein FLJ34299 | FLJ34299 | >1 |
| 1139 | 216841_s_at | X15132.1 | superoxide dismutase 2, mitochondrial | SOD2 | >1 |
| 1140 | 204683_at | NM_000873.2 | intercellular adhesion molecule 2 | ICAM2 | <1 |
| 1141 | 228829_at | AI279868 | activating transcription factor 7 | ATF7 | >1 |
| 1142 | 212902_at | BE645231 | SEC24 related gene family, member A (S. cerevisiae) | SEC24A | <1 |
| 1143 | 212542_s_at | BF224151 | pleckstrin homology domain interacting protein | PHIP | >1 |
| 1144 | 201971_s_at | NM_001690.1 | ATPase, H+ transporting, lysosomal 70kDa, V1 subunit A, isoform 1 | ATP6V1A1 | <1 |
| 1145 | 210266_s_at | AF220137.1 | tripartite motif-containing 33 | TRIM33 | >1 |
| 1146 | 222426_at | BG499947 | mitogen-activated protein kinase associated protein 1 | MAPKAP1 | >1 |
| 1147 | 201840_at | NM_006156.1 | neural precursor cell expressed, developmentally down-regulated 8 | NEDD8 | >1 |
| 1148 | 225282_at | AL137764.1 | hypothetical protein AL133206 | LOC64744 | <1 |

(continued)

| No. | Probe set ID | Seq. Derived From (RefSeq/Genbank Accession) | Title | Gene Symbol | Hazard |
|---|---|---|---|---|---|
| 1149 | 231931_at | AL355710.1 | Homo sapiens EST from clone 112590, full insert | --- | >1 |
| 1150 | 202271_at | AB007952.1 | KIAA0483 protein | KIAA0483 | <1 |
| 1151 | 204215_at | NM_024315.1 | hypothetical protein MGC4175 | MGC4175 | <1 |
| 1152 | 213127_s_at | BG230758 | mediator of RNA polymerase II transcription, subunit 8 homolog (yeast) | MED8 | <1 |
| 1153 | 217826_s_at | NM_016021.1 | ubiquitin-conjugating enzyme E2, J1 (UBC6 homolog, yeast) | UBE2J1 | <1 |
| 1154 | 203943_at | NM_004798.1 | kinesin family member 3B | KIF3B | <1 |
| 1155 | 209384_at | AA176833 | proline synthetase co-transcribed homolog (bacterial) | PROSC | <1 |
| 1156 | 228469_at | BF431902 | peptidylprolyl isomerase D (cyclophilin D) | PPID | <1 |
| 1157 | 209093_s_at | K02920.1 | glucosidase, beta; acid (includes glucosylceramidase) | GBA | >1 |
| 1158 | 239714_at | AA780063 | EST | --- | >1 |
| 1159 | 239487_at | AI743261 | EST | --- | <1 |
| 1160 | 204565_at | NM_018473.1 | uncharacterized hypothalamus protein HT012 | HT012 | <1 |
| 1161 | 201311_s_at | AL515318 | SH3 domain binding glutamic acid-rich protein like | SH3BGRL | <1 |
| 1162 | 235606_at | AA417117 | Homo sapiens cDNA FLJ31372 fis, clone NB9N42000281. | --- | <1 |
| 1163 | 201952_at | NM_001627.1 | activated leukocyte cell adhesion molecule | ALCAM | <1 |
| 1164 | 212223_at | AL117536.1 | Homo sapiens, clone IMAGE:5278680, mRNA | --- | <1 |
| 1165 | 218084_x_at | NM_014164.2 | FXYD domain containing ion transport regulator 5 | FXYD5 | <1 |
| 1166 | 223559_s_at | AF161411.2 | HSPC043 protein | HSPC043 | <1 |
| 1167 | 208445_s_at | NM_023005.1 | bromodomain adjacent to zinc finger domain, 1B | BAZ1B | <1 |
| 1168 | 218423_x_at | NM_016516.1 | tumor antigen SLP-8p | HCC8 | <1 |
| 1169 | 203320_at | NM_005475.1 | lymphocyte adaptor protein | LNK | <1 |
| 1170 | 201618_x_at | NM_003801.2 | GPAA1P anchor attachment protein 1 homolog (yeast) | GPAA1 | >1 |
| 1171 | 229861_at | N66669 | general transcription factor IIH, polypeptide 3, 34kDa | GTF2H3 | <1 |
| 1172 | 203420_at | NM_016255.1 | family with sequence similarity 8, member A1 | FAM8A1 | <1 |

(continued)

| No. | Probe set ID | Seq. Derived From (RefSeq/Genbank Accession) | Title | Gene Symbol | Hazard |
|---|---|---|---|---|---|
| 1173 | 239209_at | AA826931 | regenerating islet-derived 1 alpha (pancreatic stone protein, pancreatic thread protein) | REG1A | >1 |
| 1174 | 206974_s_at | AL138761 | Ste20-related serine/threonine kinase | SLK | <1 |
| 1175 | 227988_s_at | AW629014 | chorea acanthocytosis | CHAC | <1 |
| 1176 | 238346_s_at | AW973003 | nuclear receptor coactivator 6 interacting protein | NCOA6IP | >1 |
| 1177 | 203707_at | NM_005741.1 | zinc finger protein 263 | ZNF263 | >1 |
| 1178 | 222790_s_at | BE888593 | hypothetical protein FLJ11220 | FLJ11220 | >1 |
| 1179 | 207734_at | NM_017773.1 | hypothetical protein FLJ20340 | LAX | <1 |
| 1180 | 201859_at | NM_002727.1 | proteoglycan 1, secretory granule | PRG1 | <1 |
| 1181 | 216250_s_at | X77598.1 | leupaxin | LPXN | <1 |
| 1182 | 217846_at | NM_005051.1 | glutaminyl-tRNA synthetase | QARS | >1 |
| 1183 | 202862_at | NM_000137.1 | fumarylacetoacetate hydrolase (fumarylacetoacetase) | FAH | <1 |
| 1184 | 209061_at | AF012108.1 | similar to glucosamine-6-sulfatases | SULF2 | <1 |
| 1185 | 203970_s_at | NM_003630.1 | peroxisomal biogenesis factor 3 | PEX3 | <1 |
| 1186 | 235067_at | D81987 | Homo sapiens, clone MGC:27281 IMAGE:4656464, mRNA, complete cds | --- | <1 |
| 1187 | 228528_at | AI927692 | EST | --- | <1 |
| 1188 | 218577_at | NM_017768.1 | hypothetical protein FLJ20331 | FLJ20331 | <1 |
| 1189 | 211089_s_at | Z25434.1 | NIMA (never in mitosis gene a)-related kinase 3 | NEK3 | <1 |
| 1190 | 221778_at | BE217882 | KIAA1718 protein | KIAA1718 | <1 |
| 1191 | 207981_s_at | NM_001438.1 | estrogen-related receptor gamma | ESRRG | <1 |
| 1192 | 219939_s_at | NM_007158.1 | NRAS-related gene | D1S155E | >1 |
| 1193 | 201084_s_at | NM_014739.1 | Bcl-2-associated transcription factor | BTF | <1 |
| 1194 | 209452_s_at | AF035824.1 | vesicle transport through interaction with t-SNAREs homolog 1B (yeast) | VTI1B | >1 |
| 1195 | 214527_s_at | AB041836.1 | polyglutamine binding protein 1 | PQBP1 | <1 |
| 1196 | 222243_s_at | AB051450.1 | transducer of ERBB2, 2 | TOB2 | >1 |
| 1197 | 204192_at | NM_001774.1 | CD37 antigen | CD37 | <1 |
| 1198 | 217775_s_at | NM_016026.1 | retinol dehydrogenase 11 (all-trans and 9-cis) | RDH11 | >1 |
| 1199 | 227685_at | AI767750 | Homo sapiens cDNA FLJ39046 fis, clone NT2RP7010612. | --- | <1 |
| 1200 | 225731_at | AB033049.1 | KIAA1223 protein | KIAA1223 | <1 |
| 1201 | 209475_at | AF106069.1 | ubiquitin specific protease 15 | USP15 | <1 |

(continued)

| No. | Probe set ID | Seq. Derived From (RefSeq/Genbank Accession) | Title | Gene Symbol | Hazard |
|---|---|---|---|---|---|
| 1202 | 213024_at | BF593908 | TATA element modulatory factor 1 | TMF1 | <1 |
| 1203 | 221508_at | AF181985.1 | STE20-like kinase | JIK | <1 |
| 1204 | 212242_at | AL565074 | tubulin, alpha 1 (testis specific) | TUBA1 | <1 |
| 1205 | 200607_s_at | BG289967 | RAD21 homolog (S. pombe) | RAD21 | >1 |
| 1206 | 213671_s_at | AA621558 | methionine-tRNA synthetase | MARS | >1 |
| 1207 | 201697_s_at | NM_001379.1 | DNA (cytosine-5-)-methyltransferase 1 | DNMT1 | >1 |
| 1208 | 202105_at | NM_001551.1 | immunoglobulin (CD79A) binding protein 1 | IGBP1 | >1 |
| 1209 | 241370_at | AA278233 | Homo sapiens cDNA FLJ37785 fis, clone BRHIP2028330. | --- | >1 |
| 1210 | 220368_s_at | NM_017936.1 | hypothetical protein FLJ20707 | FLJ20707 | >1 |
| 1211 | 226710_at | AI199072 | ribosomal protein S3A | RPS3A | >1 |
| 1212 | 214317_x_at | BE348997 | ribosomal protein S9 | RPS9 | >1 |
| 1213 | 228341_at | AI809108 | Homo sapiens cDNA FLJ36248 fis, clone THYMU2001989. | --- | <1 |
| 1214 | 204523_at | NM_003440.1 | zinc finger protein 140 (clone pHZ-39) | ZNF140 | <1 |
| 1215 | 212465_at | AA524500 | hypothetical protein FLJ23027 | FLJ23027 | >1 |
| 1216 | 203606_at | NM_004553.1 | NADH dehydrogenase (ubiquinone) Fe-S protein 6, 13kDa (NADH-coenzyme Q reductase) | NDUFS6 | >1 |
| 1217 | 211529_x_at | M90684.1 | HLA-G histocompatibility antigen, class I, G | HLA-G | <1 |
| 1218 | 211517_s_at | M96651.1 | interleukin 5 receptor, alpha | IL5RA | <1 |
| 1219 | 220946_s_at | NM_014159.1 | huntingtin interacting protein B | HYPB | >1 |
| 1220 | 204350_s_at | NM_004270.1 | cofactor required for Sp1 transcriptional activation, subunit 9, 33kDa | CRSP9 | <1 |
| 1221 | 39582_at | AL050166 | Homo sapiens mRNA; cDNA DKFZp586D1122 (from clone DKFZp586D1122) | --- | <1 |
| 1222 | 204645_at | NM_001241.1 | cyclin T2 | CCNT2 | <1 |
| 1223 | 211136_s_at | BC004865.1 | cleft lip and palate associated transmembrane protein 1 | CLPTM1 | <1 |
| 1224 | 229312_s_at | BF434321 | protein kinase anchoring protein GKAP42 | GKAP42 | >1 |
| 1225 | 226504_at | AA522720 | Homo sapiens, similar to CG12393 gene product, clone IMAGE:5188623, mRNA, partial cds | --- | >1 |
| 1226 | 221547_at | BC000794.1 | PRP18 pre-mRNA processing factor 18 homolog (yeast) | PRPF18 | <1 |
| 1227 | 238035_at | N66313 | EST | --- | <1 |
| 1228 | 213011_s_at | BF116254 | triosephosphate isomerase 1 | TPI1 | >1 |

(continued)

| No. | Probe set ID | Seq. Derived From (RefSeq/Genbank Accession) | Title | Gene Symbol | Hazard |
|---|---|---|---|---|---|
| 1229 | 208718_at | Z97056 | Homo sapiens, clone IMAGE:5264473, mRNA | --- | <1 |
| 1230 | 204686_at | NM_005544.1 | insulin receptor substrate 1 | IRS1 | >1 |
| 1231 | 225763_at | AI659418 | hypothetical protein MGC21854 | MGC21854 | <1 |
| 1232 | 212643_at | AI671747 | chromosome 14 open reading frame 32 | C14orf32 | >1 |
| 1233 | 203060_s_at | AF074331.1 | 3'-phosphoadenosine 5'-phosphosulfate synthase 2 | PAPSS2 | <1 |
| 1234 | 206900_x_at | NM_021047.1 | zinc finger protein 253 | ZNF253 | <1 |
| 1235 | 225798_at | AI990891 | hypothetical protein DKFZp761K2222 | DKFZp761K 2222 | <1 |
| 1236 | 209619_at | K01144.1 | CD74 antigen (invariant polypeptide of major histocompatibility complex, class II antigen-associated) | CD74 | <1 |
| 1237 | 200996_at | NM_005721.2 | ARP3 actin-related protein 3 homolog (yeast) | ACTR3 | <1 |
| 1238 | 228150_at | AI807478 | regucalcin gene promotor region related protein | RGPR | <1 |
| 1239 | 218152_at | NM_018200.1 | high-mobility group 20A | HMG20A | >1 |
| 1240 | 202546_at | NM_003761.1 | vesicle-associated membrane protein 8 (endobrevin) | VAMP8 | <1 |
| 1241 | 218603_at | NM_016217.1 | hHDC for homolog of Drosophila headcase | HDCL | <1 |
| 1242 | 213793_s_at | BE550452 | homer homolog 1 (Drosophila) | HOMER1 | >1 |
| 1243 | 205917_at | NM_003417.1 | - | --- | <1 |
| 1244 | 218669_at | NM_021183.1 | hypothetical protein similar to small G proteins, especially RAP-2A | LOC57826 | <1 |
| 1245 | 226381_at | AW450329 | hypothetical protein FLJ20366 | FLJ20366 | <1 |
| 1246 | 211065_x_at | BC006422.1 | phosphofructokinase, liver | PFKL | >1 |
| 1247 | 224848_at | AW274756 | Homo sapiens cDNA FLJ20653 fis, clone KAT01739. | --- | <1 |
| 1248 | 212616_at | AB002306.1 | hypothetical protein MGC17528 | MGC17528 | <1 |
| 1249 | 232171_x_at | AK001742.1 | hypothetical protein DKFZp434G0522 | DKFZp434G 0522 | >1 |
| 1250 | 237181_at | AI478850 | EST | --- | >1 |
| 1251 | 204171_at | NM_003161.1 | ribosomal protein S6 kinase, 70kDa, polypeptide 1 | RPS6KB1 | <1 |
| 1252 | 201780_s_at | NM_007282.1 | ring finger protein 13 | RNF13 | <1 |
| 1253 | 215148_s_at | AI141541 | amyloid beta (A4) precursor protein-binding, family A, member 3 (X11-like 2) | APBA3 | <1 |
| 1254 | 203359_s_at | AL525412 | c-myc binding protein | MYCBP | <1 |

(continued)

| No. | Probe set ID | Seq. Derived From (RefSeq/Genbank Accession) | Title | Gene Symbol | Hazard |
|---|---|---|---|---|---|
| 1255 | 201788_at | NM_007372.1 | RNA helicase-related protein | RNAHP | <1 |
| 1256 | 235661_at | T99553 | EST | --- | <1 |
| 1257 | 202375_at | NM_014822.1 | SEC24 related gene family, member D (S. cerevisiae) | SEC24D | <1 |
| 1258 | 203491_s_at | AI123527 | KIAA0092 gene product | KIAA0092 | >1 |
| 1259 | 221989_at | AW057781 | ribosomal protein L10 | RPL10 | <1 |
| 1260 | 65630_at | AI742455 | SIPL protein | SIPL | <1 |
| 1261 | 214030_at | BE501352 | hypothetical protein DKFZp667G2110 | DKFZp667G 2110 | <1 |
| 1262 | 243552_at | AW008914 | EST | --- | >1 |
| 1263 | 214615_at | NM_014499.1 | purinergic receptor P2Y, G-protein coupled, 10 | P2RY10 | <1 |
| 1264 | 203404_at | NM_014782.1 | armadillo repeat protein ALEX2 | ALEX2 | <1 |
| 1265 | 212877_at | AA284075 | kinesin 2 60/70kDa | KNS2 | >1 |
| 1266 | 231059_x_at | AI744643 | SCAN domain containing 1 | SCAND1 | >1 |
| 1267 | 225681_at | AA584310 | collagen triple helix repeat containing 1 | CTHRC1 | >1 |
| 1268 | 227946_at | AI955239 | oxysterol binding protein-like 7 | OSBPL7 | >1 |
| 1269 | 221323_at | NM_025218.1 | UL16 binding protein 1 | ULBP1 | >1 |
| 1270 | 232431_at | AI934556 | Human glucocorticoid receptor alpha mRNA, variant 3'UTR | --- | <1 |
| 1271 | 32209_at | AF052151 | Mouse Mammary Turmor Virus Receptor homolog 1 | MTVR1 | <1 |
| 1272 | 201980_s_at | NM_012425.2 | Ras suppressor protein 1 | RSU1 | <1 |
| 1273 | 201558_at | NM_003610.1 | RAE1 RNA export 1 homolog (S. pombe) | RAE1 | >1 |
| 1274 | 221613_s_at | AL136598.1 | protein associated with PRK1 | AWP1 | <1 |
| 1275 | 243570_at | AA921960 | EST, Moderately similar to T12486 hypothetical protein DKFZp566H033.1 - human [H.sapiens] | --- | <1 |
| 1276 | 214179_s_at | H93013 | nuclear factor (erythroid-derived 2)-like 1 | NFE2L1 | <1 |
| 1277 | 224768_at | AW451291 | hypothetical protein FLJ10006 | FLJ10006 | <1 |
| 1278 | 227518_at | AW051365 | EST, Moderately similar to hypothetical protein FLJ20378 [Homo sapiens] [H. sapiens] | --- | <1 |
| 1279 | 218850_s_at | NM_014240.1 | LIM domains containing 1 | LIMD1 | >1 |
| 1280 | 201408_at | AI186712 | protein phosphatase 1, catalytic subunit, beta isoform | PPP1CB | <1 |
| 1281 | 214097_at | AW024383 | ribosomal protein S21 | RPS21 | >1 |
| 1282 | 242208_at | AI634543 | EST, Weakly similar to hypothetical protein FLJ20489 [Homo sapiens] [H.sapiens] | --- | <1 |

Still further, Table 3 sets forth markers which are significantly expressed in myeloma samples from non-responder patients whose disease is refractory (i.e. progressive disease) to treatment with bortezomib. The markers identified in Table 3 were identified similar to the methods described above for Table 1. These markers will serve to distinguish refractory patients from those who will be either stable or responsive to treatment.

**TABLE 3 Predictive Markers in Progressive Disease**

| No. | Probeset _ID | RefSeq/ Genbank Accession | Title | Gene Symbol | Unigene |
|---|---|---|---|---|---|
| 1283 | 205124_ at | NM_005919.1 | MADS box transcription enhancer factor 2, polypeptide B (myocyte enhancer factor 2B) | MEF2B | Hs.78881 |
| 1284 | 206626_x_at | BC001003.2 | synovial sarcoma, X breakpoint 1 | SSX1 | Hs.194759 |
| 34 | 224918_x_at | AI220117 | microsomal glutathione S-transferase 1 | MGST1 | Hs.355733 |
| 1285 | 206640_x_at | NM_001477.1 | G antigen 7B | GAGE7 B | Hs.251677 |
| 223 | 227174_at | Z98443 | | | Hs.86366 |
| 1286 | 227617_at | BF315093 | Weakly similar to MUC2_HUMAN Mucin 2 precursor | | Hs.22293 |
| 1287 | 207086_x_at | NM_001474.1 | G antigen 4 | GAGE4 | Hs.183199 |
| 1288 | 209732_at | BC005254.1 | Similar to C-type (calcium dependent, carbohydrate-recognition domain) lectin, superfamily member 2 (activation-induced) | CLECS F2 | Hs.85201 |
| 1289 | 214596_at | T15991 | cholinergic receptor, muscarinic 3 | CHRM3 | Hs.7138 |
| 1290 | 202779_s_at | NM_014501.1 | ubiquitin carrier protein (E2-EPF) | E2-EPF | Hs.174070 |
| 1291 | 231568_at | AI200804 | similar to Proliferation-associated protein 2G4 (Cell cycle protein p38-2G4 homolog) | | Hs.98612 |
| 1292 | 207480_s_at | NM_020149.1 | TALE homeobox protein Meis2e | MEIS2 | Hs.283312 |
| 1293 | 230352_at | AI392908 | phosphoribosyl pyrophosphate synthetase 2 | PRPS2 | Hs.2910 |
| 1294 | 202411_at | NM_005532.1 | interferon, alpha-inducible protein 27 | IFI27 | Hs.278613 |
| 17 | 215733_ x_at | AJ012833.1 | CTL-recognized antigen on melanoma (CAMEL) | CTAG2 | Hs.87225 |
| 1295 | 243030_at | AA211369 | | | Hs.269493 |
| 18 | 210546_ x_at | U87459.1 | autoimmunogenic cancertestis antigen NY-ESO-1 | CTAG1 | Hs.167379 |
| 1296 | 202044_ at | AU159484 | glucocorticoid receptor DNA binding factor 1 | GRLF1 | Hs.102548 |
| 1297 | 217977_ at | NM_016332. 1 | selenoprotein X, 1 | SEPX1 | Hs.279623 |
| 1298 | 231000_ at | BE350315 | receptor tyrosine kinase-like orphan receptor 2 | ROR2 | Hs.155585 |
| 1299 | 238587_ at | AI927919 | Nm23-phosphorylated unknown substrate | | Hs.187625 |
| 1300 | 239119_ at | AW014374 | | | Hs.144849 |
| 1301 | 236741_ at | AW299463 | | | Hs.208067 |
| 223 | 227174_ at | Z98443 | | | Hs.86366 |

(continued)

| No. | Probeset _ID | RefSeq/ Genbank Accession | Title | Gene Symbol | Unigene |
|---|---|---|---|---|---|
| 1302 | 206897_ at | NM_003785. 2 | G antigen, family B, 1 (prostate associated) | GAGEB 1 | Hs.128231 |
| 205 | 204836_ at | NM_000170. 1 | glycine dehydrogenase (decarboxylating; glycine decarboxylase, glycine cleavage system protein P) | GLDC | Hs.27 |
| 1303 | 208282_ x_at | NM_020363. 1 | deleted in azoospermia 2 | DAZ2 | Hs.283813 |
| 1304 | 216922_ x_at | AF271088.1 | deleted in azoospermia | DAZ | Hs.70936 |
| 1305 | 231771_ at | AI694073 | gap junction protein, beta 6 (connexin 30) | GJB6 | Hs.48956 |
| 267 | 231131_ at | AA909330 | weakly similar to GAR2 PROTEIN | | Hs.112765 |
| 1306 | 217007_s _at | AK000667.1 | a disintegrin and metalloproteinase domain 15 (metargidin) | | Hs.92208 |
| 1307 | 220445_s _at | NM_004909. 1 | taxol resistance associated gene 3 | TRAG3 | Hs.251377 |
| 1308 | 233216_ at | AV741116 | | | Hs.283933 |
| 1309 | 211323_s _at | L38019.1 | inositol 1,4,5-trisphosphate receptor type 1 | ITPR1 | Hs.198443 |
| 1310 | 224188_s _at | BC001208.1 | Similar to hypothetical protein LOC63929 | | Hs.182061 |
| 1311 | 213222_ at | KIAA0581 | 1-phosphatidylinositol-4,5-bisphosphate phosphodiesterase beta 1 | PLCB1 | Hs.41143 |
| 1312 | 201897_s _at | AF274941.1 | CDC28 protein kinase 1 | CKS1 | Hs.77550 |
| 1313 | 206012_ at | NM_003240. 1 | endometrial bleeding associated factor (left-right determination, factor A; transforming growth factor beta superfamily) | LEFTB | Hs.25195 |

-Classifiers

Various algorithms are currently available that can be used to classify patient samples into prior defined groups using a given set of features. Therefore, the combination of markers selected through the feature selection process may be used in one of the following classifying algorithms in order to derive a prediction equation as to whether the patient sample is sensitive or resistant. The classifiers used in the present invention were: 1) Weighted Voting ("WV"); and 2) Combination of Thresholded Features ("CTF").

The Weighted Voting classifier was implemented as described by Golub et al., "Molecular Classification of Cancer: Class discovery and class prediction by marker expression monitoring." Science, 286:531-537 (1999). For weighted voting, the classification criterion for each feature used the following formula for the weighted vote of feature *j*:

$$V_j = \frac{(\bar{x}_R - \bar{x}_S)}{S_S + S_R}\left[z_j - \left(\frac{\bar{x}_R + \bar{x}_S}{2}\right)_j\right]$$

where $z_j$ represents the log expression value for the $j^{th}$ feature in the set. For the class indicated by the subscript, $\bar{x}$ represents the mean log expression value of the jth feature, and S represents the standard deviation. The first term on the right hand side of the equation is signal-to-noise ratio (the weight given to this feature in the weighted voting), while the subtracted term is called the decision boundary. To determine the class prediction, the weighted votes for all the features in the set are summed. If the result is greater than 0, then the prediction is class R; otherwise, the prediction is class S. For each prediction, a confidence is also computed. To compute the confidence, each feature in the set is

labeled as being in agreement or disagreement with the class prediction. Let $\nu_a$ be the sum of the absolute values of the votes of the features in agreement with the class prediction, and let $\nu_d$ be the sum of absolute values of the votes in disagreement with the class prediction. Then the prediction confidence is defined as:

$$C = \frac{\nu_a}{\nu_a + \nu_d}$$

The CTF classifier first chooses a threshold on the normalized expression value for each feature. The CTF threshold is the CBT threshold divided by the CBT feature filtering score, each of which are described above. Expression values are then divided by this threshold, resulting in a "threshold-normalized expression value." The threshold-normalized expression values of the features in the marker set or model are then combined into a "combined value" using one of these methods: (1) average, (2) maximum. In preferred embodiments, the first approach, average, is used. Finally, a threshold on the combined value is determined as the average value of the combined values in class A, plus some number of standard deviations of the combined values in class A. In preferred embodiments, the number of standard deviations is 2. Using the terminology introduced in the description of the CBT feature filtering method, samples with a combined value below this threshold are classified into class A, and samples with a combined value above this threshold are classified into class B.

Feature Selection

Feature selection is the process of determining the best subset of the 44,928 available features in the dataset, resulting in a combination of features, that form a marker set or model, to classify patients into sensitive and resistant groups. The first step is filtering to the top 100 markers, as described above. Next, for building Weighted Voting (WV) marker sets, a standard feature selection method, sequential forward feature selection, is used >Dash and Liu, "Feature Selection for Classification," Intelligent Data Analysis 1:131-156, 1997). For building CTF marker sets, two methods were utilized: selection of the top N CBT scored markers (N<=100), and exhaustive search of all one- and two-feature models. We now describe how each of these is applied to our dataset to select features.

For the WV models, the top 100 SNR markers were determined. Sequential forward selection starts with no markers in the set.

At each iteration, a new feature set is formed by adding a feature selected by an evaluation function. Iteration terminates when no feature can be added that improves the evaluation function. The evaluation function has two parts. The first part is the number of samples correctly predicted either (1) by the model built on all of the samples, or (2) in leave-one-out cross-validation (Dash and Liu, 1997). Ties in the first part of the evaluation function are broken by a value equal to the sum of the confidences of the correct predictions less the sum of the confidences of the incorrect predictions. This second part of the evaluation function favors sets that have higher confidence and more correct predictions.

Each probe set was used as a single-marker model to predict bortezomib response. Multiple marker sets were generated by repeated rounds of feature selection, each time removing the features already selected. The score of each model was determined. The probe set comprising the highest-scoring model was selected.

The remaining probe sets were each used one at a time in a model along with the already-selected probe set(s). Each of these models was given a score. If the score of the new model was no higher than the score of the already-selected markers, then marker selection stopped, and the algorithm goes on to final selection by setting aside and continuing with selection of additional set(s) (see below). Otherwise, the probe set that was added to the already-selected markers to obtain the model with the highest score was added to the list of selected markers, and the algorithm returns to selection of additional markers to improve the score.

Upon final selection where no additional marker improves the score, the selected markers are set aside. Marker selection is then initiated as described above. This process is repeated until there are 5 sets of selected markers. These are combined into one complete predictive marker set.

For building CTF marker sets, the top 100 CBT features are considered for use in sets, and all one- and two-feature sets are evaluated exhaustively. The score for a given set is the number of class B samples which are above the CTF threshold (described above) for that set. Ties between CTF marker sets are broken by the best CBT score (described above) of any of the constituent markers in a set.

An example of a weighted voting predictive marker set identified using the WV and SNR scored markers is set forth in Table 4. This procedure is one of many described herein as well as others known in the art, which can be used to identify and select markers for sets predicting proteasome inhibition response in cancer patients. This procedure is the same as the procedure used in cross-validation to determine the predictive accuracy of the method (see Classification Accuracy below:

**TABLE 4: Weighted Voting Predictive Marker Set**

| No. | Decision boundary | Weight | Probe set ID | Title | Gene Symbol |
|---|---|---|---|---|---|
| 143 | 0.5177 | 0.8165 | 200965_s_at | actin binding LIM protein 1 | ABLIM1 |
| 141 | 0.3222 | 0.9174 | 234428_at | Homo sapiens mRNA; cDNA DKFZp564I1316 (from clone DKFZp564I1316) | --- |
| 221 | 1.1666 | -0.8281 | 223996_s_at | mitochondrial ribosomal protein L30 | MRPL30 |
| 94 | 0.9622 | -0.8998 | 222555_s_at | mitochondrial ribosomal protein L44 | MRPL44 |
| 147 | 0.29 | 0.9019 | 220572_at | hypothetical protein DKFZp547G183 | DKFZp547 G183 |
| 242 | 0.8798 | -0.739 | 225647_s_at | cathepsin C | CTSC |
| 180 | 0.3451 | 0.8046 | 227692_at | guanine nucleotide binding protein (G protein), alpha inhibiting activity polypeptide 1 | GNAI1 |
| 279 | 0.8811 | 0.7428 | 221223_x_at | cytokine inducible SH2-containing protein | CISH |
| 163 | 0.4398 | 0.8189 | 204287_at | synaptogyrin 1 | SYNGR1 |
| 38 | 0.4805 | 0.8322 | 216835_s_at | docking protein 1, 62kDa (downstream of tyrosine kinase 1) | DOK1 |
| 277 | 1.0222 | -0.7718 | 222713_s_at | Fanconi anemia, complementation group F | FANCF |
| 138 | 0.3196 | 0.9477 | 212109_at | HN1 like | HN1L |
| 36 | 0.4335 | 0.897 | 239476_at | Homo sapiens cDNA FLJ36491 fis, clone THYMU2018197. | --- |
| 154 | 0.5779 | -0.8579 | 218438_s_at | endothelial-derived gene 1 | EG1 |
| 83 | 0.9308 | -0.9007 | 201575_at | SKI-interacting protein | SNW1 |
| 137 | 2.121 | -0.9414 | 200043_at | enhancer of rudimentary homolog (Drosophila) | ERH |
| 165 | 0.8934 | -0.8614 | 210250_x_at | adenylosuccinate lyase | ADSL |
| 251 | 1.5602 | -0.7928 | 208642_s_at | X-ray repair complementing defective repair in Chinese hamster cells 5 (double-strand-break rejoining; Ku autoantigen, 80kDa) | XRCC5 |
| 120 | 0.3485 | 0.8612 | 217687_at | adenylate cyclase 2 (brain) | ADCY2 |
| 152 | 1.3737 | -0.8783 | 201682_at | peptidase (mitochondrial processing) beta | PMPCB |
| 96 | 1.2482 | -0.8447 | 222530_s_at | McKusick-Kaufman syndrome | MKKS |
| 245 | 0.3578 | 0.7543 | 203561_at | Fc fragment of IgG, low affinity IIa, receptor for (CD32) | FCGR2A |
| 241 | 0.9737 | -0.8018 | 222893_s_at | hypothetical protein FLJ13150 | FLJ13150 |
| 260 | 1.5048 | -0.792 | 222531_s_at | chromosome 14 open reading frame 108 | C14orf108 |
| 311 | 2.3688 | -0.7505 | 200826_at | small nuclear ribonucleoprotein D2 polypeptide 16.5kDa | SNRPD2 |
| 213 | 0.3054 | -0.834 | 226882_x_at | WD repeat domain 4 | WDR4 |
| 224 | 1.2833 | 0.7725 | 235875_at | ESTs | --- |
| 290 | 0.8235 | -0.7645 | 218139_s_at | chromosome 14 open reading frame 108 | C14orf108 |
| 145 | 1.6774 | -0.9194 | 232075_at | recombination protein REC14 | REC14 |
| 312 | 2.2771 | -0.7446 | 203663_s_at | cytochrome c oxidase subunit Va | COX5A |

(continued)

| No. | Decision boundary | Weight | Probe set ID | Title | Gene Symbol |
|---|---|---|---|---|---|
| 49 | 1.0533 | -0.7456 | 208743_s_at | tyrosine 3-monooxygenase/tryptophan 5-monooxygenase activation protein, beta polypeptide | YWHAB |
| 160 | 1.1116 | -0.8655 | 202567_at | small nuclear ribonucleoprotein D3 polypeptide 18kDa | SNRPD3 |
| 289 | 0.577 | 0.7398 | 208844_at | --- | --- |
| 87 | 0.7265 | 0.7845 | 234021_at | Homo sapiens cDNA: FLJ21331 fis, clone COL02520. | --- |
| 170 | 0.4024 | 0.8105 | 216287_at | --- | --- |
| 129 | 2.216 | -0.8395 | 200814_at | proteasome (prosome, macropain) activator subunit 1 (PA28 alpha) | PSME1 |
| 149 | 0.7958 | 0.8846 | 221569_at | hypothetical protein FLJ20069 | FLJ20069 |
| 243 | 0.7858 | 0.7564 | 233876_at | Homo sapiens cDNA FLJ20670 fis, clone KAIA4743. | --- |
| 195 | 1.1291 | 0.7902 | 58367_s_at | hypothetical protein FLJ23233 | FLJ23233 |
| 190 | 0.7554 | 0.7919 | 205807_s_at | tuftelin 1 | TUFT1 |

Classification Accuracy

To determine the ability of the selected model to predict sensitivity or resistance in an independent group of tumors, five-fold cross-validation was applied. For more information on cross-validation, see for example Kohavi and John, "Wrappers for Feature Subset Selection," Artificial Intelligence 97 (1-2) (1997) pp. 273-324. Cross-validation provides for repeated division of the data set into training and test sets, building the model each time using only the training set, then evaluating its accuracy on the withheld test set. Five-fold cross-validation means that the training set contains 80% and the test set 20% of the original data set. The filtering, feature selection and model building operations are performed only on the training set, and the resulting models are then applied to the test set. Classification accuracy is measured only on the test sets, across multiple runs of cross-validation.

To determine if the most highly predictive models could be obtained by chance alone, a permutation test was performed. The labels were permuted on the 44 discovery samples 10 times; the entire marker selection procedure was repeated. Using Weighted Voting on the responders vs others comparison, for example, the overall error rate for the permuted models was 50%, compared to 29% for the observed labels. These results suggest that it is unlikely that those models could be identified by chance alone. In the refractory vs others comparisons, we did not see clear improvement of prediction accuracy when compared to permuted sample labels. However, we report here individual markers that have relatively high single-marker SNR or CBT scores.

It will be appreciated that additional marker sets may thus be obtained by employing the methods described herein for identifying models. There are many highly correlated features that could be substituted for each other in the models; these are not all listed.

Specific Application of Class Prediction

Weighted Voting (WV)

Here we illustrate how to apply a Weighted Voting model to obtain a prediction of Response or Non-response for a given patient, using the algorithm described herein. Using the 44 patients classified into Responsive or Nonresponsive groups, the table below shows the SNR scores and decision boundaries for each of the markers in a Weighted Voting predictive set built from the data set. Also indicated is whether the marker is more highly expressed in Responsive (R) or in Non-responsive (NR) patients. For one illustrative Non-responsive patient in the data set, the votes contributed by each marker are shown in Table 5. The sum of the vote weights is less than 0, indicating a prediction of Non-responsive. The confidence in the predicted class (Non-responsive) is 0.8431.

**TABLE 5 Weighted Voting Predictive Marker Set**

| No. | Probe Set ID | Gene Symbol | SNR scores | Decision boundary | Ex. patient log expression | Vote weight | Vote | Confidence |
|---|---|---|---|---|---|---|---|---|
| 143 | 200965_s_at | ABLIM1 | 0.8165 | 0.5177 | 0.3085 | -0.1708 | NR | |
| 141 | 234428_at | --- | 0.9174 | 0.3222 | 0.201 | -0.1112 | NR | |
| 221 | 223996_s_at | MRPL30 | -0.8281 | 1.1666 | 1.0436 | 0.1019 | R | |
| 94 | 222555_s_at | MRPL44 | -0.8998 | 0.9622 | 1.2401 | -0.2501 | NR | |
| 147 | 220572_at | DKFZp547G183 | 0.9019 | 0.29 | 0.2731 | -0.0153 | NR | |
| | Total | | | | | -0.4454 | NR | 0.8431 |

It will be appreciated that similar methods may be employed utilizing the marker sets of the present invention.

Combination of Threshold Features (CTF)

Using the 44 patients classified into Responsive or Nonresponsive groups, the normalization threshold for each of the up-in-Nonpredictive markers in a CTF predictive set was built from our data set. Each marker value for a patient expression is scaled by dividing by a factor which is the mean of the Responsive class divided by the CBT score for that marker. Normalized expression values are summed to determine the combined predictive value for that patient. The threshold above which patients are predicted to be Nonresponsive was determined to be 59.15, by the CTF method described above. Because the average scaled expression value for this patient is 46.81, which is less than 59.15, the patient is predicted to be responsive. See Table 6.

It will be appreciated that similar methods may be employed utilizing two or more markers from the identified marker sets described herein in order to generate similar Predictive Marker Sets.

**TABLE 6 CTF Predictive Marker Set**

| No. | Probeset ID | RefSeq/ Genbank Accession | Title | Gene Symbol | Normalization factor | gene expr. | Normalized gene expression |
|---|---|---|---|---|---|---|---|
| 28 | 201457_x_at | AF081496.1 | BUB3 budding uninhibited by benzimidazoles 3 homolog (yeast) | BUB3 | 250.785036 | 549.1 | 2.18952458 |
| 152 | 201682_at | NM_004279.1 | peptidase (mitochondrial processing) beta | PMPCB | 181.94166 | 373 | 2.05010771 |
| 178 | 206978_at | NM_000647.2 | chemokine (C-C motif) receptor 2 | CCR2 | 248.903364 | 263 | 1.05663498 |
| 5 | 214265_at | AI193623 | integrin, alpha 8 | ITGA8 | 141.445138 | 176.5 | 1.24783363 |
| 197 | 217466_x_at | L48784 | --- | --- | 197.537832 | 833.4 | 4.21893868 |
| 158 | 217915_s_at | NM_016304.1 | chromosome 15 open reading frame 15 | C15orf15 | 218.690016 | 629.7 | 2.87941814 |
| 16 | 217969_at | NM_013265.2 | melanoma antigen, family D, 1 | MAGED1 | 206.919392 | 426.4 | 2.06070584 |
| 146 | 220565_at | NM_016602.1 | G protein-coupled receptor 2 | GPR2 | 70.449873 | 53.1 | 0.75372741 |
| 150 | 222427_s_at | AK021413.1 | leucyl-tRNA synthetase | LARS | 247.606604 | 721.1 | 2.91228097 |
| 207 | 222465_at | AF165521.1 | chromosome 15 open reading frame 15 | C15orf15 | 404.384832 | 1167.7 | 2.88759594 |
| 144 | 222783_s_at | NM_022137.1 | SPARC related modular calcium binding 1 | SMOC1 | 103.896695 | 119.9 | 1.15403093 |
| 167 | 223358_s_at | AW269834 | Homo sapiens cDNA FLJ33024 fis, clone THYMU1000532. | --- | 131.346515 | 296.2 | 2.25510361 |
| 84 | 224985_at | BE964484 | Homo sapiens, clone IMAGE:3446533, mRNA | --- | 304.941586 | 860.4 | 2.82152399 |
| 162 | 225065_x_at | AI826279 | hypothetical protein MGC40157 | MGC40157 | 386.788155 | 943.5 | 2.43931979 |
| 199 | 225698_at | BF314746 | TIGA1 | TIGA1 | 285.001406 | 1317.3 | 4.62208246 |
| 188 | 226392_at | AI888503 | Homo sapiens cDNA: FLJ21652 fis, clone COL08582. | --- | 249.877029 | 421.8 | 1.68803032 |
| 171 | 228332_s_at | AA526939 | selenoprotein H | SELH | 869.698724 | 1647.4 | 1.89421918 |
| 177 | 231045_x_at | H29876 | selenoprotein H | SELH | 620.98954 | 1078.1 | 1.7361001 |
| 145 | 232075_at | BF791874 | recombination protein REC14 | REC14 | 179.443992 | 540.9 | 3.01431101 |
| 140 | 232231_at | AL353944.1 | Runt domain transcription factor 2 | RUNX2 | 32.563013 | 95.4 | 2.92970432 |
| | | | | sum of normalized expression values | | | 46.8111936 |
| | | | | threshold of control values | | | 59.15 |

(continued)

| No. | Probeset ID | RefSeq/ Genbank Accession | Title | Gene Symbol | Normalization factor | gene expr. | Normalized gene expression |
|---|---|---|---|---|---|---|---|
| | | | (> threshold = nonresponder; <threshold = responder) | Responder or nonresponder? | | | Responder |

Biological Annotation of Predictive markers

Among the response genes identified in Table 1 and Table 2, are a subset of genes whose putative biological function or functions are particularly interesting, including function(s) particularly relevant to the use of proteasome inhibitors for the treatment of cancers, including myeloma. Some of the genes are known to be involved in the initiation or progression of myeloma, the growth, survival or signaling of lymphoid cells, the regulation of drug metabolism or apoptotic pathways or encode components of the ubiquitin/proteasome pathway that is directly targeted by proteasome inhibitors. For example, this analysis identified genes in Table 1 that are associated with cellular adhesion (No. 1 to 5), apoptotic signalling (6 to 13), cancer antigen (14 to 27), cell cycle(28 to 33), drug metabolism(34 to 35), drug resistance(36 to 37), growth control, hematopoesis(38 to 44), mitogenic signaling (45-53), myeloma signaling(53 to 61), myeloma translocation(62-73), NFkB pathway(74-77), oncogenes(78 to 82), oncogenic signaling(83 to 93), protein homeostasis(94 to 118), tumor suppressor pathway(119 to 128), and the ubiquitin/proteasome pathway(129 to 136). Additionally, the genes identified in this exercise also correspond to genes also correspond to the predictive markers associated with progressive disease in Table 2. See Table 7.

The identification of such genes strengthens the hypothesis that the genes identified with these methodologies are indeed related to cancer biology and the potential sensitivity of a hematological tumor to the anti-cancer actions of a proteasome inhibitor (e.g., bortezomib). Further, the description of such functional molecules as markers of response could facilitate selection of the most appropriate markers for inclusion in a diagnostic tool. In cases where 2 distinct probesets provide equal predictive information, the inclusion of these or other markers known to be biologically relevant could facilitate uptake and implementation of the diagnostic method. Finally, characterization of these functional molecules and pathways may enable the identification of new and possibly improved markers that act in the same or similar biological pathways.

Further, this analysis indicates additional genomic markers of response may be found in these biological pathways. For example, the "oncogenic signaling" category contains several components of the Wnt signaling pathway. Thus, other genes or proteins that function in the Wnt pathway that may also be employed as response markers. Additional markers in these identified pathways may also function alone or in conjunction with markers shown in Table 1 and Table 2 to effectively predict response to treatment with bortezomib.

**TABLE 7: Biological Annotation**

| No. | Probes etID | Title | Gene Symbol | R/ NR | supplemental annotation | Biological Category |
|---|---|---|---|---|---|---|
| 1 | 204298 _s_at | lysyl oxidase | LOX | R | lysyl oxidase may play an important role in metastasis of colon, espohageal, cardiac, and gastric carcinomas | Adhesion |
| 2 | 205884 _at | integrin, alpha 4 (antigen CD49D, alpha 4 subunit of VLA-4 receptor) | ITGA4 | NR | Alpha 4 combines with beta 1 (ITGB1) on T-cells to form the integrin very late (activation) antigen 4 ('VLA-4') that can bind to the extracellular matrix molecules fibronectin or thrombospondin, and is also a ligand for the cell surface molecule vascular cell adhesion molecule 1 ('VCAM-1'). In addition, alpha 4 combines with beta 7 to form the lymphocyte homing receptor known as 'LPAM-1' (lymphocyte Peyer Patch adhesion molecule 1). Integrins are also known to participate in cell-surface mediated signalling. | Adhesion |
| 3 | 228841 _at | Homo sapiens cDNA FLJ32429 fis, clone SKMUS2001014. | --- | NR | An inhibitor of matrix metalloproteinases. Prohibit the degradation of the extracellualr matrix which is often a key step in the metastasis of tumor cells | Adhesion |
| 4 | 243366 _s_at | integrin, alpha 4 (antigen CD49D, alpha 4 subunit of VLA-4 receptor) | ITGA4 | NR | Alpha 4 combines with beta 1 (ITGB 1) on T-cells to form the integrin very late (activation) antigen 4 (VLA-4') that can bind to the extracellular matrix molecules fibronectin or thrombospondin, and is also a ligand for the cell surface molecule vascular cell adhesion molecule 1 ('VCAM-1'). In addition, alpha 4 combines with beta 7 to form the lymphocyte homing receptor known as 'LPAM-1' (lymphocyte Peyer Patch adhesion molecule 1). Integrins are also known to participate in cell-surface mediated signalling. | Adhesion |
| 5 | 214265 _at | integrin, alpha 8 | ITGA8 | NR | | Adhesion |
| 6 | 203949 _at | myeloperoxidase | MPO | R | MPO derived oxidants are involved in caspase-3 activation and apoptosis, also translocations invoving this gene are often found in leukemia | Apoptotic signalling |
| 7 | 207341 _at | proteinase 3 (serine proteinase, neutrophil, Wegener granulomatosis autoantigen) | PRTN3 | R | Cleavage of p21waf1 by proteinase-3, a myeloid-specific serine protease, potentiates cell proliferation. Also proteinase-3 mediates doxorubicin-induced apoptosis in the HL-60 leukemia cell line, and is downregulated in its doxorubicin-resistant variant | Apoptotic signalling |
| 8 | 203948 _s_at | myeloperoxidase | MPO | R | MPO derived oxidants are involved in caspase-3 activation and apoptosis, also translocations invoving this gene are often found in leukemia | Apoptotic signalling |

(continued)

| No. | Probes etID | Title | Gene Symbol | R/ NR | supplemental annotation | Biological Category |
|---|---|---|---|---|---|---|
| 9 | 224461 _s_at | apoptosis-inducing factor (AIF)-homologous mitochondrion-associated inducer of death | AMID | NR | Overexpression of this gene has been shown to induce apoptosis. The expression of this gene is found to be induced by tumor suppressor protein p53 in colon caner cells. | Apoptotic signalling |
| 10 | 206056 _x_at | sialophorin (gpL115, leukosialin, CD43) | SPN | R | engagement of CD43 may, presumably through the repressing transcription, initiate a Bad-dependent apoptotic pathway. | Apoptotic signalling |
| 11 | 203489 _at | CD27-binding (Siva) protein | SIVA | NR | This protein seems to have an important role in the apoptotic (programmed cell death) pathway induced by the CD27 antigen, a member of the tumor necrosis factor receptor (TFNR) superfamily, and it also binds to the CD27 antigen cytoplasmic tail. | Apoptotic signalling |
| 12 | 226507 _at | p21/Cdc42/Rac1-activated kinase 1 (STE20 homolog, yeast) | PAK1 | NR | (Pak1, Pak2, Pak3) have been studied in greater detail and shown to be involved in the regulation of cellular processes such as gene transcription, cell morphology, motility, and apoptosis. | Apoptotic signalling |
| 13 | 216055 _at | platelet-derived growth factor beta polypeptide (simian sarcoma viral (v-sis) oncogene homolog) | PDGFB | R | Most proliferating cells are programmed to undergo apoptosis unless specific survival signals are provided. Platelet-derived growth factor promotes cellular proliferation and inhibits apoptosis. Romashkova and Makarov (1999) showed that PDGF activates the RAS/PIK3/AKT1/IKK/NFKB1 pathway. In this pathway, NFKB1 (164011) does not induce c-myc and apoptosis, but instead induces putative antiapoptotic genes. In response to PDGF, AKT1 (164730) transiently associates with IKK (see 600664) and induces IKK activation. The authors suggested that under certain conditions PIK3 (see 171834) may activate NFKB1 without the involvement of NFKBIA (164008) or NFKBIB (604495) degradation. | Apoptotic signalling |
| 14 | 209942 _x_at | melanoma antigen, family A, 3 | MAGEA 3 | NR | A cancer antigen that binds to pro-caspase 12 and prevents its cleavage, therby preventing apoptosis reulting from ER stress , including the unfolded protein response | Cancer Antigen |
| 15 | 214612 _x_at | Human MAGE-6 antigen (MAGE6) gene | - | NR | A cancer/testis antigen | Cancer Antigen |
| 16 | 217969 _at | melanoma antigen, family D, 1 | MAGED 1 | NR | A cancer/testis antigen | Cancer Antigen |

(continued)

| No. | Probes etID | Title | Gene Symbol | R/ NR | supplemental annotation | Biological Category |
|---|---|---|---|---|---|---|
| 17 | 215733 _x_at | cancer/testis antigen 2 | CTAG2 | NR | A cancer/testis antigen | Cancer Antigen |
| 18 | 210546 _x_at | cancer/testis antigen 1 | CTAG1 | NR | A cancer/testis antigen | Cancer Antigen |
| 19 | 211674 _x_at | cancer/testis antigen 1 | CTAG1 | NR | A cancer/testis antigen | Cancer Antigen |
| 20 | 223313 _s_at | MAGE-E1 protein | MAGE-E1 | R | A cancer/testis antigen | Cancer Antigen |
| 21 | 210467 _x_at | melanoma antigen, family A, 12 | MAGEA 12 | NR | A cancer/testis antigen | Cancer Antigen |
| 22 | 220057 _at | GAGED2: G antigen, family D, 2 | GAGED 2 | NR | A cancer/testis antigen | Cancer Antigen |
| 23 | 236152 _at | PAGE-5 protein | PAGE-5 | NR | A cancer/testis antigen | Cancer Antigen |
| 24 | 233831 _at | Homo sapiens serologically defined breast cancer antigen NY-BR-40 mRNA, partial cds | --- | R | A breast cancer antigen | Cancer Antigen |
| 25 | 206427 _s_at | melan-A | MLANA | R | A cancer/testis antigen recognized by cytotoxic T-lympohocytes | Cancer Antigen |
| 26 | 206218 _at | melanoma antigen, family B, 2 | MAGEB 2 | NR | A cancer/testis antigen | Cancer Antigen |
| 27 | 203386 _at | TBC1 domain family, member 4 | TBC1D4 | R | cancer antigen detected first in human sarcoma | Cancer Antigen |
| 28 | 201457 _x_at | BUB3 budding uninhibited by benzimidazoles 3 homolog (yeast) | BUB3 | NR | mitotic spindle checkpoint component | Cell cycle |
| 29 | 213348 _at | cyclin-dependent kinase inhibitor 1C (p57, Kip2) | CDKN1 C | R | Cyclin-dependent kinase inhibitor 1C is a tight-binding inhibitor of several G1 cyclin/Cdk complexes and a negative regulator of cell proliferation. Mutations of CDKN1C are implicated in sporadic cancers and Beckwith-Wiedemann syndorome suggesting that it is a tumor suppressor candidate. | Cell cycle |

(continued)

| No. | Probes etID | Title | Gene Symbol | R/ NR | supplemental annotation | Biological Category |
|---|---|---|---|---|---|---|
| 30 | 204170 _s_at | CDC28 protein kinase regulatory subunit 2 | CKS2 | NR | CKS2 protein binds to the catalytic subunit of the cyclin dependent kinases and is essential for their biological function. The CKS2 mRNA is found to be expressed in different patterns through the cell cycle in HeLa cells, which reflects specialized role for the encoded protein. | Cell cycle |
| 31 | 206205 _at | M-phase phosphoprotein 9 | MPHOS PH9 | NR | May be involveded in the progression from G2 to M phase in the cell cycle | Cell cycle |
| 32 | 208796 _s_at | cyclin G1 | CCNG1 | NR | The cyclin G1 gene has been identified as a target for transcriptional activation by the p53 tumor suppressor protein. | Cell cycle |
| 33 | 204460 _s_at | RAD1 homolog (S. pombe) | RAD1 | NR | Has strong sequence homology to cell cycle checkpoint gene required for cell cycle arrest and DNA damage repair in response to DNA damage | Cell cycle |
| 34 | 224918 _x_at | microsomal glutathione S-transferase 1 | MGST1 | NR | MGST1 is a drug metabolizing enzyme involved in cellular defense against toxic electrophilic compounds. Localized to the endoplasmic reticulum and outer mitochondrial membrane where it is thought to protect these membranes from oxidative stress. | Drug metabolism |
| 35 | 205998 _x_at | cytochrome P450, subfamily IIIA (niphedipine oxidase), polypeptide 4 | CYP3A4 | R | Expression is induced by glucocorticoids and some pharmacological agents. This enzyme is involved in the metabolism of approximately half the drugs which are are used today, including acetaminophen, codeine, cyclosporin A, diazepam and erythromycin. | Drug metabolism |
| 36 | 239476 _at | phosphoinositide-3-kinase, regulatory subunit, polypeptide 1 (p85 alpha) | PIK3R1 | R | PIK3R1: phosphoinositide-3-kinase, regulatory subunit, polypeptide 1 (p85 alpha);pro-apoptotic activity via suppression of the AKT survival pathway that is frequently activated in myeloma | Drug Resistance |
| 37 | 211298 _s_at | albumin | ALB | R | Albumin has been shown to acitivate the AKT signalling pathway and protect B-chronic lymphocytic leukemia patients from chlorambucil- and radiation-induced apoptosis | Drug Resistance |
| 38 | 216835 _s_at | docking protein 1, 62kDa (downstream of tyrosine kinase 1) | DOK1 | R | Docking protein 1 is constitutively tyrosine phosphorylated in hematopoietic progenitors isolated from chronic myelogenous leukemia (CML) patients in the chronic phase. It may be a critical substrate for p210(bcr/abl), a chimeric protein whose presence is associated with CML. | Hematopoiesi s |
| 39 | 213891 _s_at | TCF4 | --- | R | TCF4 is expressed predominantly in pre-B-cells, it is activated upon Wnt signalling | Hematopoiesi s |

(continued)

| No. | Probes etID | Title | Gene Symbol | R/ NR | supplemental annotation | Biological Category |
|---|---|---|---|---|---|---|
| 40 | 212387 _at | TCF4 | --- | R | TCF4 is expressed predominantly in pre-B-cells, it is activated upon Wnt signalling | Hematopoiesi s |
| 41 | 212382 _at | TCF4: Transcription factor 4 | --- | R | TCF4 is expressed predominantly in pre-B-cells, it is activated upon Wnt signalling | Hematopoiesi s |
| 42 | 203753 _at | transcription factor 4 | TCF4 | R | TCF4 is expressed predominantly in pre-B-cells, it is activated upon Wnt signalling | Hematopoiesi s |
| 43 | 212386 _at | transcription factor 4 | TCF4 | R | TCF4 is expressed predominantly in pre-B-cells, it is activated upon Wnt signalling | Hematopoiesi s |
| 44 | 211709 _s_at | stem cell growth factor; lymphocyte secreted C-type lectin | SCGF | R | SCGF is selectively produced by osseous and hematopoietic stromal cells, and can mediate their proliferative activity on primitive hematopoietic progenitor cells. | Hematopoiesi s |
| 45 | 217020 _at | --- | --- | R | Binds retinoic acid, the biologically active form of vitamin A which mediates cellular signalling in embryonic morphogenesis, cell growth and differentiation. | Mitogenic Signalling |
| 46 | 217786 _at | SKB1 homolog (S. pombe) | SKB1 | NR | may regulate mitosis through binding SHK1 | Mitogenic Signalling |
| 47 | 206109 _at | fucosyltransferase 1 (galactoside 2-alpha-L- fucosyltransferase, Bombay phenotype included) | FUT1 | R | an essential component of Notch signalling pathway that regulate cell growth and differentiation | Mitogenic Signalling |
| 48 | 227798 _at | MADH1 MAD, mothers against decapentaplegic homolog 1 (Drosophila) | --- | NR | Involved in the TGF-beta signalling pathway, an important pathway that regulates cell growth, differentiation and apoptosis and is often disrupted in cancer. | Mitogenic Signalling |
| 49 | 208743 _s_at | tyrosine 3-monooxygenase/tryptophan 5-monooxygenase activation protein, beta polypeptide | YWHAB | NR | This gene encodes a protein belonging to the 14-3-3 family of proteins. It has been shown to interact with RAF1 and CDC25 phosphatases, suggesting that it may play a role in linking mitogenic signaling and the cell cycle machinery. | Mitogenic Signalling |
| 50 | 225239 _at | ESTs, Moderately similar to hypothetical protein FLJ20958 [Homo sapiens] [H.sapiens] | --- | R | SPRY4 is an inhibitor of the receptor-transduced mitogen-activated protein kinase (MAPK) signaling pathway, an important growth signalling pathway in cancer. | Mitogenic Signalling |

(continued)

| No. | Probes etID | Title | Gene Symbol | R/ NR | supplemental annotation | Biological Category |
|---|---|---|---|---|---|---|
| 51 | 215551 _at | estrogen receptor 1 | ESR1 | R | Estrogen receptor 1 alpha overexpression is implicated in breast and ovarian cancers, and activates the cyclin D1 pathway | Mitogenic Signalling |
| 52 | 215067 _x_at | PRDX2: peroxiredoxin 2 | --- | R | PRDX2 may have a proliferative effect and play a role in cancer development or progression. | Mitogenic Signalling |
| 53 | 210993 _s_at | MAD, mothers against decapentaplegic homolog 1 (Drosophila) | MADH1 | NR | TGFB1 is the prototype of a large family of cytokines that also includes the activins (e.g., 147290), inhibins (e.g., 147380), bone morphogenetic proteins, and Mullerian-inhibiting substance (600957). Members of the TGF-beta family exert a wide range of biologic effects on a large variety of cell types; for example, they regulate cell growth, differentiation, matrix production, and apoptosis. | Mitogenic Signalling |
| 54 | 209374 _s_at | immunoglobulin heavy constant mu | IGHM | NR | A surrogate marker of some types of multiple myeloma | Myeloma signalling |
| 55 | 224342 _x_at | immunoglobulin lambda locus | IGL@ | NR | A surrogate marker of some types of multiple myeloma | Myeloma signalling |
| 56 | 212827 _at | immunoglobulin heavy constant mu | IGHM | NR | A surrogate marker of some types of multiple myeloma | Myeloma signalling |
| 57 | 234366 _x_at | immunoglobulin lambda locus | IGL@ | R | A surrogate marker of some types of multiple myeloma | Myeloma signalling |
| 58 | 216986 _s_at | interferon regulatory factor 4 | IRF4 | NR | A mutliple myeloma oncogene, has been shown to regualte lymphocyte apoptosis by modulating the efficiency of the Fas signal | Myeloma signalling |
| 59 | 205098 _at | chemokine (C-C motif) receptor 1 | CCR1 | NR | studies suggest that chemokine receptor expression and the migratory capacity of MM cells to their ligands are relevant for the compartmentalization of MM cells in the bone marrow | Myeloma signalling |
| 60 | 239237 _at | ESTs | --- | NR | Strong sequence similarity to Ig heavy chain, a surrogate marker for some types of multiple myeloma | Myeloma signalling |
| 61 | 205099 _s_at | chemokine (C-C motif) receptor 1 | CCR1 | NR | studies suggest that chemokine receptor expression and the migratory capacity of multiple myeloma cells to their ligands are relevant for the compartmentalization of multiple myeloma cells in the bone marrow | . Myeloma signalling |
| 62 | 223472 _at | Wolf-Hirschhorn syndrome candidate 1 | WHSC1 | R | WHSC1 is involved in a chromosomal translocation t(4;14)(p16.3;q32.3) in multiple myelomas. | Myeloma translocation |

(continued)

| No. | Probes etID | Title | Gene Symbol | R/ NR | supplemental annotation | Biological Category |
|---|---|---|---|---|---|---|
| 63 | 222778 _s_at | Wolf-Hirschhorn syndrome candidate 1 | WHSC1 | R | WHSC1 is involved in a chromosomal translocation t(4;14)(p16.3;q32.3) in multiple myelomas. Also, vv | Myeloma translocation |
| 64 | 209054 _s_at | Wolf-Hirschhorn syndrome candidate 1 | WHSC1 | R | WHSC1 is involved in a chromosomal translocation t(4;14)(p16.3;q32.3) in multiple myelomas. | Myeloma translocation |
| 65 | 222777 _s_at | Wolf-Hirschhorn syndrome candidate 1 | WHSC1 | R | WHSC1 is involved in a chromosomal translocation t(4;14)(p16.3;q32.3) in multiple myelomas. Also, vv | Myeloma translocation |
| 66 | 209053 _s_at | Wolf-Hirschhorn syndrome candidate 1 | WHSC1 | R | WHSC1 is involved in a chromosomal translocation t(4;14)(p16.3;q32.3) in multiple myelomas. Also, vv | Myeloma translocation |
| 67 | 200921 _s_at | B-cell translocation gene 1, anti-proliferative | BTG1 | NR | The BTG1 gene locus has been shown to be involved in a t(8;12)(q24;q22) chromosomal translocation in a case of B-cell chronic lymphocytic leukemia. It is a member of a family of antiproliferative genes. BTG1 expression is maximal in the G0/G1 phases of the cell cycle and downregulated when cells progressed through G1. It negatively regulates cell proliferation. | Myeloma translocation |
| 68 | 209052 _s_at | Wolf-Hirschhorn syndrome candidate 1 | WHSC1 | R | WHSC1 is involved in a chromosomal translocation t(4;14)(p16.3;q32.3) in multiple myelomas. | Myeloma translocation |
| 69 | 213940 _s_at | formin binding protein 1(FBP17) | FNBP1 | NR | The human formin-binding protein 17 (FBP17) interacts with sorting nexin, SNX2, and is an MLL-fusion partner in acute myelogeneous leukemia | Myeloma translocation |
| 70 | 213732 _at | transcription factor 3 (E2A' immunoglobulin enhancer binding factors E12/E47) | TCF3 | R | The E2A gene maps to 19p13.3-p13.2, a site associated with nonrandom translocations in acute lymphoblastic leukemias. | Myeloma translocation |
| 71 | 213047 _x_at | SET translocation (myeloid leukemia-associated) | SET | NR | The SET translocation (6;9)(p23q34) is the hallmark of a specific subtype of acute myeloid leukemia (AML) characterized by a poor prognosis and a young age of onset. SET protein regulates G(2)/M transition by modulating cyclin B-CDK1 activity. | Myeloma translocation |
| 72 | 200631 _s_at | SET translocation (myeloid leukemia-associated) | SET | NR | The SET translocation (6;9)(p23q34) is the hallmark of a specific subtype of acute myeloid leukemia (AML) characterized by a poor prognosis and a young age of onset. SET protein regulates G(2)/M transition by modulating cyclin B-CDK1 activity. | Myeloma translocation |

(continued)

| No. | Probes etID | Title | Gene Symbol | R/ NR | supplemental annotation | Biological Category |
|---|---|---|---|---|---|---|
| 73 | 205068 _s_at | GTPase regulator associated with focal adhesion kinase pp125(FAK) | GRAF | R | GTPase regulator associated with the focal adhesion kinase pp125(FAK) is often involved in a translocations with the MLL gene in hematologic malignancies | Myeloma translocation |
| 74 | 220146 _at | toll-like receptor 7 | TLR7 | NR | Expression of TLR7 may activate NF-kB, an important mediator of cell survival, and possible downstream target of proteasome inhibition | NFkB pathway |
| 75 | 232304 _at | pellino homolog 1 (Drosophila) | PELI1 | R | Pellino 1 is required for NF kappa B activation and IL-8 gene expression in response to IL-1 | NFkB pathway |
| 76 | 232213 _at | pellino homolog 1 (Drosophila) | PELI1 | R | Pellino 1 is required for NF kappa B activation and IL-8 gene expression in response to IL-1 | NFkB pathway |
| 77 | 218319 _at | pellino homolog 1 (Drosophila) | PELI1 | R | Pellino 1 is required for NF kappa B activation and IL-8 gene expression in response to IL-1 | NFkB pathway |
| 78 | 215744 _at | fusion, derived from t(12;16) malignant liposarcoma | FUS | R | Proto-oncoprotein resulting from fusion gene in myxoid liposarcoma; derived from t(12;16) malignant liposarcoma. | Oncogene |
| 79 | 206363 _at | v-maf musculoaponeurotic fibrosarcoma oncogene homolog (avian) | MAF | R | MAF is a protooncogene | Oncogene |
| 80 | 202768 _at | FBJ murine osteosarcoma viral oncogene homolog B | FOSB | R | The fos genes encode leucine zipper proteins that can dimerize with proteins of the JUN family, thereby forming the transcription factor complex AP-1. Thus, the FOS proteins have been implicated as regulators of cell proliferation, differentiation, and oncogenic transformation. | Oncogene |
| 81 | 202647 _s_at | neuroblastoma RAS viral (v-ras) oncogene homolog | NRAS | NR | The N-ras oncogene is a member of the RAS gene family. It is mapped on chromosome 1, and it is activated in HL60, a promyelocytic leukemia line. | Oncogene |
| 82 | 209640 _at | promyelocytic leukemia | PML | R | The expression of PML is cell-cycle related and it regulates the p53 response to oncogenic signals. The gene is often involved in the translocation with the retinoic acid receptor alpha gene associated with acute promyelocytic leukemia (APL). | Oncogene |
| 140 | 232231 _at | Runt domain transcription factor | RUNX2 | NR | Runt domain transcription factor AML3/RUNX2 is essential for the generation and differentiation of osteoblasts, and has been associated with the survival of several types of metastases in bone. | Oncogene |

(continued)

| No. | Probes etID | Title | Gene Symbol | R/ NR | supplemental annotation | Biological Category |
|---|---|---|---|---|---|---|
| 83 | 201575 _at | SKI-interacting protein | SNW1 | NR | may be involved in oncogenesis since it interacts with a region of SKI oncoproteins that is required for transforming activity;overcomes the growth-suppressive activities of pRb | Oncogenic signalling |
| 84 | 224985 _at | Homo sapiens, clone IMAGE:3446533, mRNA | --- | NR | An oncogene involved in numerous cancers. A member of the RAS gene family. | Oncogenic signalling |
| 85 | 204602 _at | dickkopf homolog 1 (Xenopus laevis) | DKK1 | NR | A secreted inhibitor of WNT signalling, a pathway known to be important to oncogenesis | Oncogenic signalling |
| 86 | 201653 _at | cornichon homolog (Drosophila) | CNIH | NR | may regulate EGF signalling, a pathway known to be involved in oncogenesis | Oncogenic signalling |
| 87 | 234021 _at | Homo sapiens cDNA: FLJ21331 fis, clone COL02520. | --- | R | highly similar to plakophilin 2 which associates with beta-catenin and up-regulates the oncogenic beta-catenin/T cell factor-signaling activity | Oncogenic signalling |
| 88 | 212063 _at | CD44 antigen (homing function and Indian blood group system) | CD44 | NR | The wide prevalence of CD44 cleavage suggests that it plays an important role in the pathogenesis of human tumors. | Oncogenic signalling |
| 89 | 204489 _s_at | CD44 antigen (homing function and Indian blood group system) | CD44 | NR | The wide prevalence of CD44 cleavage suggests that it plays an important role in the pathogenesis of human tumors. | Oncogenic signalling |
| 90 | 227167 _s_at | Homo sapiens mesenchymal stem cell protein DSC96 mRNA, partial cds | --- | NR | The RAS oncogene (MIM 190020) is mutated in nearly one-third of all human cancers. Members of the RAS superfamily are plasma membrane GTP-binding proteins that modulate intracellular signal transduction pathways. A subfamily of RAS effectors, including RASSF3, share a RAS association (RA) domain | Oncogenic signalling |
| 91 | 202290 _at | PDGFA associated protein 1 | PDAP1 | NR | stimulates the inherent ATPase activity of Hsp90, a molecular chaperone that plays a key role in the conformational maturation of oncogenic signaling proteins | Oncogenic signalling |
| 92 | 215499 _at | mitogen-activated protein kinase kinase 3 (MAP2K3) | MAP2K3 | R | Expression of RAS oncogene is found to result in the accumulation of the active form of MAP2K3, which thus leads to the constitutive activation of MAPK14, and confers oncogenic transformation of primary cells. | Oncogenic signalling |
| 93 | 200047 _s_at | YY1 transcription factor | YY1 | NR | Some AML patients showed significantly elevated YY1 transcript levels in bone marrow cells. Taken together with mouse data, this suggests involvement in the pathogenesis of AML. | Oncogenic signalling |

(continued)

| No. | Probes etID | Title | Gene Symbol | R/ NR | supplemental annotation | Biological Category |
|---|---|---|---|---|---|---|
| 94 | 222555 _s_at | mitochondrial ribosomal protein L44 | MRPL44 | NR | involved in mitochondrial protein synthesis | Protein homeostasis |
| 95 | 212694 _s_at | propionyl Coenzyme A carboxylase, beta polypeptide | PCCB | NR | may function in protein homeostasis via degradation of brached chain amino acids | Protein homeostasis |
| 96 | 222530 _s_at | McKusick-Kaufman syndrome | MKKS | NR | similarity to the chaperonin family of proteins, suggesting a role for protein processing | Protein homeostasis |
| 97 | 200869 _at | ribosomal protein L18a | RPL18A | NR | Ribosomes are involved in protein synthesis and thus contribute to protein homeostasis | Protein homeostasis |
| 98 | 200023 _s_at | eukaryotic translation initiation factor 3, subunit 5 epsilon, 47kDa | EIF3S5 | NR | Regulates initiation of protein translation and thus is involved in protein homeostasis | Protein homeostasis |
| 99 | 200812 _at | chaperonin containing TCP1, subunit 7 (eta) | CCT7 | NR | CCT regulates protein homeostasis via the folding of newly translated polypeptide substrates, including cyclin E | Protein homeostasis |
| 100 | 225190 _x_at | ribosomal protein L35a | RPL35A | NR | Ribosomes are involved in protein synthesis and thus contribute to protein homeostasis | Protein homeostasis |
| 101 | 200023 _s_at | eukaryotic translation initiation factor 3, subunit 5 epsilon, 47kDa | EIF3S5 | NR | Regulates initiation of protein translation and thus is involved in protein homeostasis | Protein homeostasis |
| 102 | 217919 _s_at | mitochondrial ribosomal protein L42 | MRPL42 | NR | involved in mitochondrial protein synthesis | Protein homeostasis |
| 103 | 211972 _x_at | ribosomal protein, large, P0 | RPLP0 | NR | Ribosomes are involved in protein synthesis and thus contribute to protein homeostasis | Protein homeostasis |
| 104 | 200024 _at | ribosomal protein S5 | RPS5 | NR | Ribosomes are involved in protein synthesis and thus contribute to protein homeostasis | Protein homeostasis |
| 105 | 200715 _x_at | ribosomal protein L13a | RPL13A | NR | Ribosomes are involved in protein synthesis and thus contribute to protein homeostasis | Protein homeostasis |
| 106 | 201258 _at | ribosomal protein S16 | RPS16 | NR | Ribosomes are involved in protein synthesis and thus contribute to protein homeostasis | Protein homeostasis |
| 107 | 200003 _s_at | ribosomal protein L28 | RPL28 | NR | Ribosomes are involved in protein synthesis and thus contribute to protein homeostasis | Protein homeostasis |

(continued)

| No. | Probes etID | Title | Gene Symbol | R/ NR | supplemental annotation | Biological Category |
|---|---|---|---|---|---|---|
| 108 | 221726 _at | ribosomal protein L22 | RPL22 | NR | Ribosomes are involved in protein synthesis and thus contribute to protein homeostasis | Protein homeostasis |
| 109 | 200041 _s_at | HLA-B associated transcript 1 | BAT1 | R | Members of this family are involved in a number of cellular functions including initiation of translation, RNA splicing, and ribosome assembly and thus could have a role in protein homeostasis. | Protein homeostasis |
| 110 | 211937 _at | eukaryotic translation initiation factor 4B | EIF4B | NR | Regulates initiation of protein translation and thus is involved in protein homeostasis | Protein homeostasis |
| 111 | 200082 _s_at | ribosomal protein S7 | RPS7 | NR | Ribosomes are involved in protein synthesis and thus contribute to protein homeostasis | Protein homeostasis |
| 112 | 214167 _s_at | ribosomal protein, large, P0 | RPLPO | NR | Ribosomes are involved in protein synthesis and thus contribute to protein homeostasis | Protein homeostasis |
| 113 | 200024 _at | ribosomal protein S5 | RPS5 | NR | Ribosomes are involved in protein synthesis and thus contribute to protein homeostasis | Protein homeostasis |
| 114 | 217719 _at | eukaryotic translation initiation factor 3, subunit 6 interacting protein | EIF3S6I P | NR | Regulates initiation of protein translation and thus is involved in protein homeostasis | Protein homeostasis |
| 115 | 225797 _at | mitochondrial ribosomal protein L54 | MRPL54 | NR | involved in mitochondrial protein synthesis | Protein homeostasis |
| 116 | 200937 _s_at | ribosomal protein L5 | RPL5 | NR | Ribosomes are involved in protein synthesis and thus contribute to protein homeostasis | Protein homeostasis |
| 117 | 208985 _s_at | eukaryotic translation initiation factor 3, subunit 1 alpha, 35kDa | EIF3S1 | NR | Regulates initiation of protein translation and thus is involved in protein homeostasis | Protein homeostasis |
| 118 | 200834 _s_at | ribosomal protein S21 | RPS21 | NR | Ribosomes are involved in protein synthesis and thus contribute to protein homeostasis | Protein homeostasis |
| 119 | 216153 _x_at | reversion-inducing-cysteine-rich protein with kazal motifs | RECK | R | The protein encoded by this gene is a cysteine-rich, extracellular protein with protease inhibitor-like domains whose expression is suppressed strongly in many tumors and cells transformed by various kinds of oncogenes. In normal cells, this membrane-anchored glycoprotein may serve as a negative regulator for matrix metalloproteinase-9, a key enzyme involved in tumor invasion and metastasis. | Tumor Supressor Pathway |

(continued)

| No. | Probes etID | Title | Gene Symbol | R/ NR | supplemental annotation | Biological Category |
|---|---|---|---|---|---|---|
| 120 | 217687 _at | adenylate cyclase 2 (brain) | ADCY2 | R | Adenylate cyclase signalling regulates cell growth and differentiation; it is frequently defective in human tumors. Activation of human Adenylyl Cyclase protein(s) and inhibition of human Pde4 protein protein(s) increase apoptosis of acute lymphoblastic leukemia cells | Tumor Supressor Pathway |
| 121 | 222632 _s_at | leucine zipper transcription factor-like 1 | LZTFL1 | NR | The LZTFL1 gene has been mapped to a putative tumor suppressor region (C3CER1) on chromosome 3p21.3 | Tumor Supressor Pathway |
| 122 | 236623 _at | ATPase, Na+/K+ transporting, alpha 1 polypeptide | ATP1A1 | R | Expression regulated by p53, a tumor supressor gene | Tumor Supressor Pathway |
| 123 | 221899 _at | hypothetical protein from BCRA2 region | CG005 | R | Located in the region of BRCA2, a breast cancer susceptibility gene | Tumor Supressor Pathway |
| 124 | 221691 _x_at | nucleophosmin (nucleolar phosphoprotein B23, numatrin) | NPM1 | NR | Nucleophosmin regulates the stability and transcriptional activity of p53 | Tumor Supressor Pathway |
| 125 | 209030 _s_at | immunoglobulin superfamily, member 4 (TSLC1) | IGSF4 | NR | TSCL1 has been identified as a potential tumor supressor gene in lung cancer | Tumor Supressor Pathway |
| 126 | 222762 _x_at | LIM domains containing 1 (LIMD1) | LIMD1 | NR | Interstitial deletions of the short arm of chromosome 3 containing LIMD1 are found in a large number of tumors. IT may have a role as a tumor supressor. | Tumor Supressor Pathway |
| 127 | 240983 _s_at | cysteinyl-tRNA synthetase | CARS | NR | This gene is one of several located near the imprinted gene domain of 11p15.5, an important tumor-suppressor gene region. Alterations in this region have been associated with the Beckwith-Wiedemann syndrome, Wilms tumor, rhabdomyosarcoma, adrenocortical carcinoma, and lung, ovarian, and breast cancer. | Tumor Supressor Pathway |
| 128 | 200713 _s_at | microtubule-associated protein, RP/EB family, member 1 | MAPRE 1 | NR | MAPRE1 binds to the APC protein which is often mutated in familial and sporadic forms of colorectal cancer. This protein localizes to microtubules, especially the growing ends, in interphase cells. During mitosis, the protein is associated with the centrosomes and spindle microtubules. | Tumor Supressor Pathway |

(continued)

| No. | Probes etID | Title | Gene Symbol | R/ NR | supplemental annotation | Biological Category |
|---|---|---|---|---|---|---|
| 129 | 200814 _at | proteasome (prosome, macropain) activator subunit 1 (PA28 alpha) | PSME1 | NR | subunit of the 11S regulator of the 20S proteasome | Ubiquitin/ proteasome pathway |
| 130 | 201532 _at | proteasome (prosome, macropain) subunit, alpha type, 3 | PSMA3 | NR | core subunit of the proteasome | Ubiquitin/ proteasome pathway |
| 131 | 218011 _at | ubiquitin-like 5 | UBL5 | NR | Ubiquitin-like proteins (UBLs) are thought to be reversible modulators of protein function rather than protein degraders like ubiquitin | Ubiquitin/ proteasome pathway |
| 132 | 224747 _at | hypothetical protein LOC92912 | LOC929 12 | NR | Contains a ubiquitin conjugating enzyme domain | Ubiquitin/ proteasome pathway |
| 133 | 201758 _at | tumor susceptibility gene 101 | TSG101 | NR | The protein encoded by this gene belongs to a group of apparently inactive homologs of ubiquitin-conjugating enzymes. The gene product contains a coiled-coil domain that interacts with stathmin, a cytosolic phosphoprotein implicated in tumorigenesis. The protein may play a role in cell growth and differentiation and act as a negative growth regulator. | Ubiquitin/ proteasome pathway |
| 134 | 200019 _s_at | Finkel-Biskis-Reilly murine sarcoma virus (FBR-MuSV) ubiquitously expressed (fox derived); ribosomal protein S30 | FAU | NR | A fusion protein consisting of the ubiquitin-like protein fubi at the N terminus and ribosomal protein S30 at the C terminus. It has been proposed that the fusion protein is post-translationally processed to generate free fubi and free ribosomal protein S30. Fubi is a member of the ubiquitin family, and ribosomal protein S30 belongs to the S30E family of ribosomal proteins. | Ubiquitin/ proteasome pathway |
| 135 | 202346 _at | huntingtin interacting protein 2 | HIP2 | NR | UBIQUITIN-CONJUGATING ENZYME E2-25K has been implicated in the degradation of huntingtin and suppression of apoptosis. | Ubiquitin/ proteasome pathway |
| 136 | 201177 _s_at | SUMO-1 activating enzyme subunit 2 | UBA2 | NR | ubiquitin-like activating enzyme involved in protein homeostasis | Ubiquitin/ proteasome pathway |
| 154 | 218438 _s_at | endothelial-derived gene 1 | EG1 | NR | expressed in tumor-stimulated endothelial cells; may have role in tumor angiogenesis | |

(continued)

| No. | Probes etID | Title | Gene Symbol | R/ NR | supplemental annotation | Biological Category |
|---|---|---|---|---|---|---|
| 157 | 216288 _at | cysteinyl leukotriene receptor 1 | CYSLTR 1 | R | upregulated in colon cancer; affecting survival | |
| 166 | 210497 _x_at | synovial sarcoma, X breakpoint 2 | SSX2 | NR | A cancer antigen involved in a translocation in synovial sarcoma. May be ionvolved in transcriptional repression. | |
| 167 | 223358 _s_at | phosphodiesterase 7A | PDE7A | NR | Increased PDE7 in T cells correlated with decreased cAMP, increased interleukin-2 expression, and increased proliferation. | |
| 213 | 226882 _x_at | WD repeat domain 4 | WDR4 | NR | Members of this family are involved in a variety of cellular processes, including cell cycle progression, signal transduction, apoptosis, and gene regulation. | |
| 242 | 225647 _s_at | cathepsin C | CTSC | NR | a lysosomal cysteine proteinase that appears to be a central coordinator for activation of many serine proteinases in immune/inflammatory cells | |
| 251 | 208642 _s_at | X-ray repair complementing defective repair in Chinese hamster cells 5 (double-strand-break rejoining; Ku autoantigen, 80kDa) | XRCC5 | NR | Invoved in DNA repair, a pathway important to cancer. Defects in this pathway can lead to cancer and overactivity of this pathway can lead to chemotherapeutic resistance in cancer cells | |
| 286 | 37793_ r_at | RAD51-like 3 (S. cerevisiae) | RAD51L 3 | R | Possibly invoved in DNA damage repair based on sequence homology | |
| 333 | 218467 _at | hepatocellular carcinoma susceptibility protein | HCCA3 | NR | A novel full-length cDNA was cloned and differentiated, which was highly expressed in liver cancer tissues. | |
| 346 | 209031 _at | immunoglobulin superfamily, member 4 | IGSF4 | NR | | |
| 442 | 208013 _s_at | acrosomal vesicle protein 1 | ACRV1 | R | a testis differentiation antigen | |

Proteasome inhibitor resistant cell lines

In order to better understand the specific mechanism(s) by which proteasome inhibitors exert their apoptotic effects, as well as to elucidate mechanisms by which those effects may be subverted, bortezomib resistant tumor cell lines were generated. Tumor cell lines were treated with a very low dose of bortezomib (approximately 1/20 the LD50 - a dose that would kill 50% of the cells) for 24 hours. The drug was then removed and surviving cells were allowed to recover for 24 to 72 hours. This process was then repeated for multiple rounds with the bortezomib dose doubled each time. After cells had been dosed with 3-5 times the LD50, several individual cell lines were sub-cloned from single cell colonies. Subsequent analyses demonstrated that these lines exhibit 5-10 fold resistance to bortezomib and that this characteristic is stable over months in culture and unaffected by inhibitors of multi-drug resistance pumps. This strategy was applied to both ovarian tumor cell lines (OVCAR-3) and myeloma tumor cell lines (RPMI8226) and multiple sub-clones were characterized. The resistant cell lines were then subject to gene expression profiling using the Affymetrix U133 microarray. A comparison of genes differentially expressed in sensitive parental (S) versus resistant sub-clones (R) highlighted several genes that were also identified in analysis of sensitive and resistant myeloma biopsies. See table 8. The number identified in Table 8 corresponds to the marker number identification in Table 1. Such results not only highlight a potential relationship between expression of these genes and bortezomib sensitivity, but also support the validity of methods used to define response genes in clinical samples.

**TABLE 8 Gene Identification in Proteasome Inhibition Sensitive / Resistant Cell Lines**

| No. | Probeset ID | Title | R/S | Ratio Resistant / Parental |
|---|---|---|---|---|
| 156 | 202075_s_at | gb:NM_006227.1 /DEF=Homo sapiens phospholipid transfer protein (PLTP), mRNA. /FEA=mRNA /GEN=PLTP /PROD=phospholipid transfer protein /DB_XREF=gi:5453913 /UG=Hs.283007 phospholipid transfer protein /FL=gb:L26232.1 gb:NM_006227.1 | S | 0.36 |
| 166 | 210497_x_at | gb:BC002818.1 /DEF=Homo sapiens, Similar to synovial sarcoma, X breakpoint 2, clone MGC:3884, mRNA, complete cds. /FEA=mRNA /PROD=Similar to synovial sarcoma, X breakpoint 2 /DB_XREF=gi:12803942 /UG=Hs.289105 synovial sarcoma, X breakpoint 2 /FL=gb:BC002818.1 | R | 2.82 |
| 332 | 210715_s_at | gb:AF027205.1 /DEF=Homo sapiens Kunitz-type protease inhibitor (kop) mRNA, complete cds. /FEA=mRNA /GEN=kop /PROD=Kunitz-type protease inhibitor /DB_XREP=gi:2598967 /UG=Hs.31439 serine protease inhibitor, Kunitz type, 2 /FL=gb:AF027205.1 | S | 0.37 |
| 211 | 219373_at | gb:NM_018973.1 /DEF=Homo sapiens dolichyl-phosphate mannosyltransferase polypeptide 3 (DPM3), mRNA. /FEA=mRNA /GEN=DPM3 /PROD=dolichyl-phosphate mannosyltransferasepolypeptide 3 /DB_XREF=gi:9506552 /UG=Hs.110477 dolichyl-phosphate mannosyltransferase polypeptide 3 /FL=gb:AF312923.1 gb:AF312922.1 gb:AB028128.1 gb:NM_018973.1 | S | 0.37 |
| 343 | 200030_s_at | gb:NM_002635.1 /DEF=Homo sapiens solute carrier family 25 (mitochondrial carrier; phosphate carrier), member 3 (SLC25A3), nuclear gene encoding mitochondrial protein, transcript variant 1b, mRNA. /FEA=mRNA /GEN=SLC25A3 /PROD=phosphate carrier precursor isoform 1 b /DB_XREF=gi:4505774 /UG=Hs.78713 solute carrier family 25 (mitochondrial carrier; phosphate carrier), member 3 /FL=gb:BC000998.1 gb:BC001328.1 gb:BC003504.1 gb:BC004345.1 gb:NM_002635.1 | R | 2 |
| 447 | 222975_s_at | Consensus includes gb:AI423180 /FEA=EST /DB_XREF=gi:4269111 /DB_XREF=est:tf32e08.x1 /CLONE=IMAGE:2097926 /UG=Hs.69855 NRAS-related gene /FL=gb:AB020692.1 | R | 1.16 |

(continued)

| No. | Probeset ID | Title | R/S | Ratio Resistant / Parental |
|---|---|---|---|---|
| 280 | 224673_ at | Consensus includes gb:AI613244 /FEA=EST /DB_XREF=gi:4622411 /DB_XREF=est:ty35a06.x1 /CLONE=IMAGE:2281042 /UG=Hs.306121 leukocyte receptor cluster (LRC) encoded novel gene 8 | S | 0.44 |
| 129 | 200814_ at | gb:NM_006263.1 /DEF=Homo sapiens proteasome (prosome, macropain) activator subunit 1 (PA28 alpha) (PSME1), mRNA. /FEA=mRNA /GEN=PSME1 /PROD=proteasome (prosome, macropain) activatorsubunit 1 (PA28 alpha) /DB_XREF=gi:5453989 /UG=Hs.75348 proteasome (prosome, macropain) activator subunit 1 (PA28 alpha) /FL=gb:BC000352.1 gb:L07633.1 gb:NM_006263.1 | R | 2.11 |
| 390 | 204610_s _at | gb:NM_006848.1 /DEF=Homo sapiens hepatitis delta antigen-interacting protein A (DIPA), mRNA. /FEA=mRNA /GEN=DIPA /PROD=hepatitis delta antigen-interacting protein A /DB_XREF=gi:5803004 /UG=Hs.66713 hepatitis delta antigen-interacting protein A /FL=gb:U63825.1 gb:NM_006848.1 | R | 2.09 |
| 429 | 222646_s _at | Consensus includes gb:AW268365 /FEA=EST /DB_XREF=gi:6655395 /DB_XREF=est:xv50d03.x1 /CLONE=IMAGE:2816549 /UG=Hs.25740 ERO1 (S. cerevisiae)-like /FL=gb:AF081886.1 gb:NM_014584.1 | R | 2.74 |

Sensitivity Assays

A sample of cancerous cells is obtained from a patient. An expression level is measured in the sample for a marker corresponding to at least two of the predictive markers set forth in Table 1, Table 2 and/or Table 3. Preferably a marker set is utilized comprising markers idenitifed in Table 1, Table 2 and/or Table 3 and put together in a marker set using the methods described herein. For example, marker sets can comprise the marker sets identified in Table 4, Table 5 and/or Table 6 or any marker set prepared by similar methods. Such analysis is used to obtain an expression profile of the tumor in the patient. Evaluation of the expression profile is then used to determine whether the patient is a responsive patient and would benefit from proteasome inhibition therapy (e.g., treatment with a proteasome inhibitor (e.g., bortezomib) alone, or in combination with additional agents). Evaluation can include use of one marker set prepared using any of the methods provided or other similar scoring methods known in the art (e.g., weighted voting, CTF). Still further, evaluation can comprise use of more than one prepared marker set. A proteasome inhibition therapy will be identified as appropriate to treat the cancer when the outcome of the evaluation demonstrates decreased non-responsiveness or increased responsiveness in the presence of the agent.

Examining the expression of one or more of the identified markers or marker sets in a tumor sample taken from a patient during the course of proteasome inhibition treatment, it is also possible to determine whether the therapeutic agent is continuing to work or whether the cancer has become non-responsive (refractory) to the treatment protocol. For example, a patient receiving a treatment of bortezomib would have tumor cells removed and monitored for the expression of the a marker or marker set. If the expression profile of one or more marker sets identified in Table 1, Table 2 and/or Table 3 demonstrates increased responsiveness in the presence of the agent, the treatment with proteasome inhibitor would continue. However, if the expression profile of one or more marker sets identified in Table 1, Table 2 or Table 3 demonstrates increased non-responsiveness in the presence of the agent, then the cancer may have become resistant to proteasome inhibition therapy and another treatment protocol should be initiated to treat the patient.

Importantly, these determinations can be made on a patient by patient basis or on an agent by agent (or combinations of agents). Thus, one can determine whether or not a particular proteasome inhibition therapy is likely to benefit a particular patient or group/class of patients, or whether a particular treatment should be continued.

## Claims

**1.** An *in vitro* method for determining a proteasome inhibition therapy regimen for treating a tumor in a patient comprising:

a) determining the level of expression of markers in a marker set comprising at least two Predictive markers

selected from the group consisting of the markers identified in Table 1, Table 2 and Table 3 in a patient sample comprising tumor cells; and
b) determining a proteasome inhibition-based regimen for treating the tumor based on the expression of the predictive markers, wherein a significant expression level is indicative that the patient is either a responsive patient or a non-responsive patient.

**2.** The method of claim 1 wherein

a) the level of expression of the markers in the predictive marker set is determined by detection of mRNA or protein; and/or
b) the predictive markers selected from the markers identified in Table 1, Table 2 and Table 3 comprise markers identified in any of Table 4, Table 5, or Table 6.

**3.** The method of claim 1 or 2, wherein determining the significant level of expression is determined by comparison with a control marker or by comparison to a predetermined standard.

**4.** The method of claim 1 or 2, wherein the tumor is selected from liquid or solid tumors, optionally wherein the liquid tumor is selected from the group consisting of myelomas, multiple myeloma, Non-Hodgkins Lymphoma, B-cell lymphomas, Waldenstrom's syndrome, chronic lymphocytic leukemia, and other leukemias.

**5.** The method of claim 1 to 4, wherein the proteasome inhibition-based regimen for treating the tumor comprises treatment with a proteasome inhibitor is selected from the group consisting of a peptidyl aldehyde, a peptidyl boronic acid, a peptidyl boronic ester, a vinyl sulfone, an epoxyketone, and a lactacystin analog.

**6.** The method of any preceding claim, wherein the proteasome inhibition-based regimen for treating the tumor comprises treatment with bortezomib.

**7.** The method of any preceding claim, wherein the patient sample comprising tumor cells is obtained from the subject any time selected from prior to tumor therapy, concurrently with tumor therapy or after tumor therapy.

**8.** The method of claim 1, wherein the predictive markers are selected from the Markers identified in Table 1 or Table 2 associated with a biological function selected from the group consisting of cellular adhesion, apoptotic signalling, cancer antigen, cell cycle, drug metabolism, drug resistance, growth control, hematopoesis, mitogenic signaling, myeloma signaling, myeloma translocation, NFkB pathway, oncogenes, oncogenic signaling, protein homeostasis, tumor suppressor pathway, and the ubiquitin/proteasome pathway.

**9.** A marker set for use in the method of claim 1 wherein the marker set comprises at least two predictive markers selected from the group consisting of the markers identified in Table 1, Table 2 and Table 3 and is constructed using the weighted voting method or the combination of threshold features model.

**10.** A kit for use in determining a proteasome inhibition therapy for treating a tumor in a patient comprising reagents for assessing the expression of at least one predictive marker, and instructions for use, wherein the reagents comprise

a) a plurality of nucleic acid probes, wherein the probes specifically bind the markers in a predictive marker set comprising at least two markers identified in any of Table 1, Table 2, or Table 3; and/or
b) a plurality of detecting reagents selected from the group consisting of an antibody, an antibody derivative, an antibody fragment, and peptide probe, wherein the detecting reagents specifically bind to the proteins encoded by predictive markers in a predictive marker set comprising at least two markers identified in any of Table 1, Table 2, or Table 3.

**11.** The method of claim 1, wherein the predictive marker set comprises markers selected from Table 7.

## Patentansprüche

**1.** *In vitro* Verfahren zur Bestimmung einer Proteasom-Inhibierungs-Therapie zur Behandlung eines Tumors in einem Patienten, welches umfasst:

a) Bestimmen des Expressions-Niveaus von Markern in einem Marker-Satz, der mindestens zwei Voraussage-Marker umfasst, ausgewählt aus der Gruppe bestehend aus den in Tabelle 1, Tabelle 2 und Tabelle 3 aufgeführten Markern, in einer Patienten-Probe, die Tumorzellen umfasst; und
b) Bestimmen einer auf Proteasom-Inhibierung basierenden Therapie zur Behandlung des Tumors basierend auf der Expression der Voraussage-Marker, wobei ein wesentliches Expressions-Niveau anzeigt, dass der Patient entweder auf die Therapie anspricht oder nicht.

**2.** Verfahren nach Anspruch 1, wobei

a) das Expressions-Niveau der Marker in dem Voraussage-Markersatz bestimmt wird durch Nachweis von mRNA oder Protein; und/oder
b) die Voraussage-Marker, die unter den in Tabelle 1, Tabelle 2 und Tabelle 3 aufgeführten Markern ausgewählt sind, Marker umfassen, die in einer der Tabelle 4, Tabelle 5 oder Tabelle 6 aufgeführt sind.

**3.** Verfahren nach Anspruch 1 oder 2, wobei die Bestimmung des wesentlichen Expression-Niveaus durch Vergleich mit einem Kontroll-Marker oder Vergleich mit einem bestimmten Standard durchgeführt wird.

**4.** Verfahren nach Anspruch 1 oder 2, wobei der Tumor ausgewählt ist unter liquiden oder festen Tumoren, wahlweise wobei der liquide Tumor ausgewählt ist aus der Gruppe bestehend aus Myelomen, multiplen Myelomen, Nicht-Hodgkins-Lymphomen, B-Zell-Lymphomen, Waldenstrom's Syndrom, chronischer lymphozytischer Leukämie und anderen Leukämien.

**5.** Verfahren nach Anspruch 1 bis 4, wobei die auf Proteasom-Inhibierung basierende Therapie zur Behandlung des Tumors umfasst, Behandlung mit einem Proteasom-Inhibitor, ausgewählt aus der Gruppe bestehend aus einem Peptidylaldehyd, einer Peptidylborsäure, einem Peptidylborsäureester, einem Vinylsulfon, einem Epoxyketon und einem Lactacystin-Analog.

**6.** Verfahren nach einem der vorstehenden Ansprüche, wobei die auf Protcasom-Inhibierung basierende Therapie zur Behandlung des Tumors Behandlung mit Bortezomib umfasst.

**7.** Verfahren nach einem der vorstehenden Ansprüche, wobei die Patienten-Probe mit Tumorzellen von dem Individuum zu jedem Zeitpunkt vor der Tumortherapie, während der Tumortherapie oder nach der Tumortherapie erhalten wird.

**8.** Verfahren nach Anspruch 1, wobei die Voraussage-Marker ausgewählt sind unter Markern, die in Tabelle 1 oder Tabelle 2 als mit einer biologischen Funktion assoziiert aufgeführt sind, die ausgewählt ist aus der Gruppe bestehend aus Zelladhäsion, apoptotischer Signalübertragung, Krebsantigen, Zellzyklus, Arzneimittel-Metabolismus, Arzneimittel-Resistenz, Wachstumssteuerung, Hematopoiese, mitogener Signalübertragung, myelomer Signalübertragung, myelomer Translokation, NfkB-Weg, Onkogenese, onkogener Signalübertragung, Protein-Homeostase, Tumorsupressor-Weg und dem Ubiquitin/Proteasom-Weg.

**9.** Markersatz zur Verwendung in dem Verfahren nach Anspruch 1, wobei der Markersatz mindestens zwei Voraussage-Marker umfasst, ausgewählt aus der Gruppe bestehend aus den in Tabelle 1, Tabelle 2 und Tabelle 3 aufgeführten Markern und unter Verwendung der gewichteten Votierungs-Methode oder der Kombination des Grenzwertmerkmalsmodells konstruiert wird.

**10.** Kit zur Verwendung bei der Bestimmung einer Proteasom-Inhibierungs-Therapie zur Behandlung eines Tumors in einem Patienten, welcher Reagenzien zur Bestimmung der Expression mindestens eines Voraussage-Markers umfasst, und Anweisungen zur Verwendung, worin die Reagenzien umfassen

a) mehrere Nukleinsäuresonden, worin die Sonden die Marker in einem Voraussage-Marker-Satz spezifisch binden, welcher mindestens zwei Marker umfasst, die in Tabelle 1, Tabelle 2 oder Tabelle 3 aufgeführt sind; und/oder
b) mehrere Erfassungsreagienzen ausgewählt aus der Gruppe bestehend aus einem Antikörper, einem Antikörper-Derivat, einem Antikörper-Fragment, und einer Peptid-Probe, worin die Erfassungsreagenzien spezifisch an von den Voraussage-Markern kodierten Proteine in einem Voraussage-Marker-Satz binden, der mindestens zwei der in Tabelle 1, Tabelle 2 oder Tabelle 3 aufgeführten Marker umfasst.

**11.** Verfahren nach Anspruch 1, wobei der Voraussage-Markersatz Marker umfasst, die aus Tabelle 7 ausgewählt sind.

## Revendications

1. Procédé *in vitro* de détermination d'un régime thérapeutique par inhibition du protéasome pour traiter une tumeur chez un patient comprenant :

   a) la détermination du taux d'expression de marqueurs dans un ensemble de marqueurs comprenant au moins deux marqueurs prédictifs choisis dans le groupe constitué des marqueurs identifiés dans le tableau 1, le tableau 2 et le tableau 3 dans un échantillon de patient comprenant des cellules tumorales ; et
   b) la détermination d'un régime basé sur l'inhibition du protéasome pour le traitement de la tumeur en se basant sur l'expression des marqueurs prédictifs, où un taux d'expression significatif indique que le patient est soit un patient sensible soit un patient non sensible.

2. Procédé selon la revendication 1, dans lequel

   a) le taux d'expression des marqueurs dans l'ensemble des marqueurs prédictifs est déterminé par détection de l'ARNm ou de la protéine ; et/ou
   b) les marqueurs prédictifs choisis parmi les marqueurs identifiés dans le tableau 1, le tableau 2 et le tableau 3 comprennent des marqueurs identifiés dans l'un quelconque du tableau 4, du tableau 5, ou du tableau 6.

3. Procédé selon la revendication 1 ou 2, dans lequel la détermination du taux d'expression significatif est déterminée par comparaison à un marqueur témoin ou par comparaison à un étalon prédéterminé.

4. Procédé selon la revendication 1 ou 2, dans lequel la tumeur est choisie parmi des tumeurs liquides ou solides, éventuellement où la tumeur liquide est choisie dans le groupe constitué de myélomes, myélome multiple, lymphome non hodgkinien, lymphomes à lymphocytes B, syndrome de Waldenström, leucémie lymphocytaire chronique, et autres leucémies.

5. Procédé selon les revendications 1 à 4, dans lequel le régime basé sur l'inhibition du protéasome pour le traitement de la tumeur comprend le traitement avec un inhibiteur du protéasome choisi dans le groupe constitué d'un aldéhyde de peptidyle, un acide boronique de peptidyle, un ester boronique de peptidyle, une vinylsulfone, une époxycétone, et un analogue de lactacystine.

6. Procédé selon l'une quelconque des revendications précédentes, dans lequel le régime basé sur l'inhibition du protéasome pour le traitement de la tumeur comprend le traitement avec du bortézomib.

7. Procédé selon l'une quelconque des revendications précédentes, dans lequel l'échantillon du patient comprenant des cellules tumorales est obtenu du sujet à n'importe quel moment choisi parmi avant le traitement de la tumeur, simultanément au traitement de la tumeur ou après le traitement de la tumeur.

8. Procédé selon la revendication 1, dans lequel les marqueurs prédictifs sont choisis parmi les marqueurs identifiés dans le tableau 1 ou le tableau 2 associés à une fonction biologique choisie dans le groupe constitué de l'adhérence cellulaire, la signalisation apoptosique, l'antigène cancéreux, le cycle cellulaire, le métabolisme du médicament, la résistance au médicament, le contrôle de la croissance, l'hématopoïèse, la signalisation mitogène, la signalisation du myélome, la translocation du myélome, la voie du NFκB, les oncogènes, la signalisation oncogène, l'homéostasie des protéines, la voie du suppresseur tumoral, et la voie de l'ubiquitine/ protéasome.

9. Ensemble de marqueurs pour une utilisation dans le procédé selon la revendication 1, où l'ensemble de marqueurs comprend au moins deux marqueurs prédictifs choisis dans le groupe constitué des marqueurs identifiés dans le tableau 1, le tableau 2 et le tableau 3 et est construit en utilisant le procédé du vote pondéré ou la combinaison du modèle des caractères seuil.

10. Kit pour une utilisation dans la détermination d'un traitement par inhibition du protéasome pour le traitement d'une tumeur chez un patient comprenant des réactifs pour l'estimation de l'expression d'au moins un marqueur prédictif, et des instructions d'utilisation, où les réactifs comprennent

    a) une pluralité de sondes d'acide nucléique, où les sondes lient spécifiquement les marqueurs dans un ensemble de marqueurs prédictifs comprenant au moins deux marqueurs identifiés dans l'un quelconque du tableau 1, du tableau 2, ou du tableau 3 ; et/ou

b) une pluralité de réactifs de détection choisis dans le groupe constitué d'un anticorps, un dérivé d'anticorps, un fragment d'anticorps, et une sonde peptidique, où les réactifs de détection se lient spécifiquement aux protéines codées par des marqueurs prédictifs dans un ensemble de marqueurs prédictifs comprenant au moins deux marqueurs identifiés dans l'un quelconque du tableau 1, du tableau 2, ou du tableau 3.

**11.** Procédé selon la revendication 1, dans lequel l'ensemble de marqueurs prédictifs comprend des marqueurs choisis à partir du tableau 7.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 43151402 P
- WO 9525533 A
- US 5780454 A
- US 6066730 A
- US 6083903 A
- US 5631169 A, Lakowicz
- US 4868103 A, Stavrianopoulos
- US 4843155 A, Chomczynski
- US 4683202 A, Mullis
- US 5854033 A
- US 6548668 B
- WO 9113904 A, Siman
- US 5693617 A, Stein
- WO 9524914 A
- US 5756764 A, Fentany
- US 6147223 A
- US 6645999 B, Schreiber
- WO 8809810 A
- WO 8910134 A
- US 5876930 A
- US 4816567 A, Cabilly
- US 4816397 A, Boss
- US 5585089 A, Queen
- WO 8702671 A
- EP 184187 A
- EP 171496 A
- EP 173494 A
- WO 8601533 A
- EP 125023 A
- US 5225539 A
- US 5625126 A
- US 5633425 A
- US 5569825 A
- US 5661016 A
- US 5545806 A
- US 4676980 A
- US 5223409 A


### Non-patent literature cited in the description


- *Science,* 1996, vol. 274, 1652-1659
- *Immunity,* 1995, vol. 2, 493-506
- *Seminars in Cancer Biology,* 1993, vol. 4, 219-229
- *Science,* 1996, vol. 274, 782
- **BLADE et al.** *Br J Haematol.,* September 1998, vol. 102 (5), 1115-23
- **SJOLANDER, S. ; URBANICZKY, C.** *Anal. Chem.,* 1991, vol. 63, 2338-2345
- **SZABO et al.** *Curr. Opin. Struct. Biol.,* 1995, vol. 5, 699-705
- *Biochem,* vol. 18 (8), 284-7
- **HEEGAARD, N.H.** *J. Mol. Recognit.,* 1998, vol. 11 (1-6), 141-8
- **HAGE, D.S. ; TWEED, S.A.** *J Chromatogr B Biomed Sci Appl,* 10 October 1997, vol. 699 (1-2), 499-525
- Current Protocols in Molecular Biology. John Wiley & Sons, 1987
- **BARANY.** *Proc. Natl. Acad. Sci. USA,* 1991, vol. 88, 189-193
- **GUATELLI et al.** *Proc. Natl. Acad. Sci. USA,* 1990, vol. 87, 1874-1878
- **KWOH et al.** *Proc. Natl. Acad. Sci. USA,* 1989, vol. 86, 1173-1177
- **LIZARDI et al.** *Bio/Technology,* 1988, vol. 6, 1197
- **IQBAL et al.** *J. Med. Chem.,* 1995, vol. 38, 2276-2277
- **BOUGET et al.** *Bioorg Med Chem,* 2003, vol. 17, 4881-4889
- **FENTEANY et al.** *Proc. Natl. Acad. Sci. USA,* 1994, vol. 91, 3358
- **BOGYO et al.** *Proc. Natl. Acad. Sci.,* 1997, vol. 94, 6629
- **SPALTENSTEIN et al.** *Tetrahedron Lett.,* 1996, vol. 37, 1343
- **MENG L.** *Proc. Natl. Acad Sci,* 1999, vol. 96, 10403
- **MENG LH.** *Cancer Res,* 1999, vol. 59, 2798
- **KOGUCHI Y.** *Antibiot (Tokyo),* 2000, vol. 53, 105
- **KROLL M.** *Chem Biol,* 1999, vol. 6, 689
- **NAM S.** *J. Biol Chem,* 2001, vol. 276, 13322
- **GILMAN A.G. et al.** The Pharmacological Basis of Therapeutics. 1990, 1202-1263
- **SAMBROOK et al.** Molecular Cloning: A Laboratory Manual. Cold Spring Harbor Laboratory Press, 1989
- **HYRUP et al.** *Bioorganic & Medicinal Chemistry,* 1996, vol. 4 (1), 5-23

- **PERRY-O'KEEFE et al.** *Proc. Natl. Acad. Sci. USA,* 1996, vol. 93, 14670-675
- **FINN et al.** *Nucleic Acids Res.,* 1996, vol. 24 (17), 3357-63
- **MAG et al.** *Nucleic Acids Res.,* 1989, vol. 17, 5973-88
- **PETERSER et al.** *Bioorganic Med. Chem. Lett.,* 1975, vol. 5, 1119-11124
- **LETSINGER et al.** *Proc. Natl. Acad. Sci. USA,* 1989, vol. 86, 6553-6556
- **LEMAITRE et al.** *Proc. Natl. Acad. Sci. USA,* 1987, vol. 84, 648-652
- **KROL et al.** *Bio/Techniques,* 1988, vol. 6, 958-976
- **ZON.** *Pharm. Res.,* 1988, vol. 5, 539-549
- **KARLIN ; ALTSCHUL.** *Proc. Natl. Acad. Sci. USA,* 1990, vol. 87, 2264-2268
- **KARLIN ; ALTSCHUL.** *Proc. Natl. Acad. Sci. USA,* 1993, vol. 90, 5873-5877
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **MYERS ; MILLER.** *CABIOS,* 1988, vol. 4, 11-17
- **PEARSON ; LIPMAN.** *Proc. Natl. Acad. Sci. USA,* 1988, vol. 85, 2444-2448
- **KOHLER ; MILSTEIN.** *Nature,* 1975, vol. 256, 495-497
- **KOZBOR et al.** *Immunol. Today,* 1983, vol. 4, 72
- **COLE et al.** Monoclonal Antibodies and Cancer Therapy. Alan R. Liss, Inc, 1985, 77-96
- Current Protocols in Immunology. John Wiley & Sons, 1994
- **BETTER et al.** *Science,* 1988, vol. 240, 1041-1043
- **LIU et al.** *Proc. Natl. Acad. Sci. USA,* 1987, vol. 84, 3439-3443
- **LIU et al.** *J. Immunol.,* 1987, vol. 139, 3521-3526
- **SUN et al.** *Proc. Natl. Acad. Sci. USA,* 1987, vol. 84, 214-218
- **NISHIMURA et al.** *Cancer Res.,* 1987, vol. 47, 999-1005
- **WOOD et al.** *Nature,* 1985, vol. 314, 446-449
- **SHAW et al.** *J. Natl. Cancer Inst.,* 1988, vol. 80, 1553-1559
- **MORRISON.** *Science,* 1985, vol. 229, 1202-1207
- **OI et al.** *Bio/Techniques,* 1986, vol. 4, 214
- **JONES et al.** *Nature,* 1986, vol. 321, 552-525
- **VERHOEYAN et al.** *Science,* 1988, vol. 239, 1534
- **BEIDLER et al.** *J. Immunol.,* 1988, vol. 141, 4053-4060
- **LONBERG ; HUSZAR.** *Int. Rev. Immunol.,* 1995, vol. 13, 65-93
- **JESPERS et al.** *Bio/technology,* 1994, vol. 12, 899-903
- Monoclonal Antibodies For Immunotargeting Of Drugs In Cancer Therapy. **ARNON et al.** Monoclonal Antibodies And Cancer Therapy. Alan R. Liss, Inc, 1985, 243-56
- Antibodies For Drug Delivery. **HELLSTROM et al.** Controlled Drug Delivery. Marcel Dekker, Inc, 1987, 623-53
- Antibody Carriers Of Cytotoxic Agents In Cancer Therapy: A Review. **THORPE et al.** Monoclonal Antibodies '84: Biological And Clinical Applications. 1985, 475-506
- Analysis, Results, And Future Prospective Of The Therapeutic Use Of Radiolabeled Antibody In Cancer Therapy. Monoclonal Antibodies For Cancer Detection And Therapy. Academic Press, 1985, 303-16
- **THORPE et al.** The Preparation And Cytotoxic Properties Of Antibody-Toxin Conjugates. *Immunol. Rev.,* 1982, vol. 62, 119-58
- **ZUCKERMANN et al.** *J. Med. Chem.,* 1994, vol. 37, 2678-85
- **LAM.** *Anticancer Drug Des.,* 1997, vol. 12, 145
- **DEWITT et al.** *Proc. Natl. Acad. Sci. U.S.A.,* 1993, vol. 90, 6909
- **ERB et al.** *Proc. Natl. Acad. Sci. USA,* 1994, vol. 91, 11422
- **ZUCKERMANN et al.** *J. Med. Chem.,* 1994, vol. 37, 2678
- **CHO et al.** *Science,* 1993, vol. 261, 1303
- **CARRELL et al.** *Angew. Chem. Int. Ed. Engl.,* 1994, vol. 33, 2059
- **CARELL et al.** *Angew. Chem. Int. Ed. Engl.,* 1994, vol. 33, 2061
- **GALLOP et al.** *J. Med. Chem.,* 1994, vol. 37, 1233
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc Natl Acad Sci USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 87, 6378-6382
- **FELICI.** *J. Mol. Biol.,* 1991, vol. 222, 301-310
- **RIVAS, G. ; MINTON, A.P.** *Trends Biochem Sci,* August 1993, vol. 18 (8), 284-7
- **HEEGAARD.** *J Mol. Recognit.,* 1996, vol. 11, 141-148
- **HAGE ; TWEED.** *J. Chromatogr. B. Biomed. Sci. Appl.,* 1997, vol. 699, 499-525
- Current Protocols in Molecular Biology. J. Wiley & Sons, 1999

- **KRAUT et al.** *J. Clin. Oncol.,* 1998, vol. 16 (2), 589-592
- **BLADE et al.** *Br. J. Haematol.,* 1998, vol. 102 (5), 1115-1123
- **RICHARDSON PG et al.** *New Eng. J. Med.,* 2003, vol. 348, 2609-17
- **GOLUB et al.** Molecular Classification of Cancer: Class discovery and class prediction by marker expression monitoring. *Science,* 1999, vol. 286, 531-537
- **CONOVER, W. J.** Practical Nonparametric Statistics. John Wiley & Sons, 1980
- **E.T. LEE.** Statistical Methods for Survival Data Analysis. John Wiley& Sons, 1992
- **DASH ; LIU.** Feature Selection for Classification. *Intelligent Data Analysis,* 1997, vol. 1, 131-156
- **KOHAVI ; JOHN.** Wrappers for Feature Subset Selection. *Artificial Intelligence,* 1997, vol. 97 (1-2), 273-324